# EUROPEAN PATENT SPECIFICATION

(11) **EP 1 776 138 B1**
(45) Date of publication and mention of the grant of the patent: **21.10.2009**
(21) Application number: 05759912.8
(22) Date of filing: 21.07.2005
(51) Int. Cl.: A61K 38/53, A61P 7/02, A61P 35/00, A61P 17/06, A61P 19/02, A61P 17/02

(54) **tRNA SYNTHETASE FRAGMENTS**
tRNA-SYNTHETASE-FRAGMENTE
FRAGMENTS D'ARNt SYNTHÉTASE

(30) Priority: 02.08.2004 US 598019 P; 07.10.2004 US 962218; 07.10.2004 US 961529; 07.10.2004 US 962171; 07.10.2004 US 962217; 07.10.2004 US 962058; 07.10.2004 US 961528; 07.10.2004 US 962375; 07.10.2004 US 962062; 07.10.2004 US 961526; 07.10.2004 US 961486; 02.11.2004 US 980866; 02.11.2004 US 624656 P; 20.12.2004 US 19969
(43) Date of publication of application: 25.04.2007
(73) Proprietor: Angiosyn, Inc., New York NY 10017 (US)
(72) Inventor: GLIDDEN, Paul, F., San Diego, CA 92128 (US)
(74) Representative: Rutt, Jason Edward
(86) International application number: PCT/IB2005/002180
(87) International publication number: WO 2006/016217

(56) References cited:
- WO-A-01/74841
- WO-A-03/009813
- GB-A- 2 327 945
- US-A1- 2002 182 666
- US-A1- 2003 017 564
- OTANI A ET AL: "Truncated fragments of aminoacyl-tRNA synthetases are potent angiostatic agents for retinal angiogenesis" IOVS, vol. 42, no. 4, 15 March 2001 (2001-03-15), page S93, XP009054159 & ANNUAL MEETING OF THE ASSOCIATION FOR RESEARCH IN VISION AND OPHTHALMOLOGY; FORT LAUDERDALE, FLORIDA, USA; APRIL 29-MAY 04, 2001
- YU YADONG ET AL: "Crystal structure of human tryptophanyl-tRNA synthetase catalytic fragment: insights into substrate recognition, tRNA binding, and angiogenesis activity." THE JOURNAL OF BIOLOGICAL CHEMISTRY. 27 FEB 2004, vol. 279, no. 9, 27 February 2004 (2004-02-27), pages 8378-8388, XP002286863 ISSN: 0021-9258
- KISE YOSHIAKI ET AL: "A short peptide insertion crucial for angiostatic activity of human tryptophanyl-tRNA synthetase." NATURE STRUCTURAL & MOLECULAR BIOLOGY. FEB 2004, vol. 11, no. 2, February 2004 (2004-02), pages 149-156, XP009032966 ISSN: 1545-9993
- WARD W H ET AL: "Protein engineering of homodimeric tyrosyl-tRNA synthetase to produce active heterodimers." THE JOURNAL OF BIOLOGICAL CHEMISTRY. 25 JUL 1986, vol. 261, no. 21, 25 July 1986 (1986-07-25), pages 9576-9578, XP002346228 ISSN: 0021-9258
- DATABASE WPI Section Ch, Week 198937 Derwent Publications Ltd., London, GB; Class A96, AN 1989-268323 XP002346234 & JP 01 196295 A (TOSOH CORP) 8 August 1989 (1989-08-08)
- DATABASE WPI Section Ch, Week 198945 Derwent Publications Ltd., London, GB; Class A14, AN 1989-327392 XP002346235 & JP 01 242105 A (TORAY IND INC) 27 September 1989 (1989-09-27)
- DATABASE WPI Section Ch, Week 199018 Derwent Publications Ltd., London, GB; Class A96, AN 1990-137268 XP002346236 & KR 8 902 068 B (CHEIL SUGAR & CO) 16 June 1989 (1989-06-16)
- DATABASE WPI Section Ch, Week 199235 Derwent Publications Ltd., London, GB; Class A18, AN 1992-288535 XP002346237 & JP 04 197436 A (TERUMO CORP) 17 July 1992 (1992-07-17)
- DATABASE WPI Section Ch, Week 198937 Derwent Publications Ltd., London, GB; Class A14, AN 1989-268322 XP002346238 & JP 01 196294 A (TOSOH CORP) 8 August 1989 (1989-08-08)

## Description

### CROSS REFERENCES

This application is a continuation-in-part of U.S. Application No. 11/019,969 filed on December 20, 2004, which is a continuation-in-part of U.S. Application No. 10/962,218 filed on October 7, 2004; this application is also a continuation-in-part of U.S. Application No. 10/980,866 filed on November 2, 2004, which is a continuation-in-part of U.S. Application No. 10/961,529 filed on October 7, 2004; this application also claims priority to U.S. Provisional Application No. 60/598,019 filed on August 2, 2004, and U.S. Applications Nos. 10/962,171, 10/962,217, 10/962,058, 10/961,528, 10/962,375, 10/962,062, 10/962,218, 10/961,529, 10/961,526, and 10/961,486, all of which were filed on October 7, 2004.

### BACKGROUND

Normal tissue growth, which occurs during embryonic development, wound healing, and menstrual cycle is characterized by dependence on new vessel formation for the supply of oxygen and nutrients as well as removal of waste products. Angiogenesis is the name given to the development of new capillaries from pre-existing blood vessels. The extent of angiogenesis is determined by the balance between pro-angiogenic factors and anti-angiogenic factors. Pro-angiogenic factors include, but are not limited to, vascular endothelial growth factor (VEGF), fibroblast growth factor (FGF), interleukin-8 (IL-8), angiogenin, angiotropin, epidermal growth factor (EGF), platelet derived endothelial cell growth factor, transforming growth factor α (TGF-α), transforming growth factor β (TGF-β), and nitric oxide. Anti-angiogenic factors include, but are not limited to, thrombospondin, angiostatin, and endostatin.

While in most normal tissues the balance favors the anti-angiogenic factors and angiogenesis is inhibited, numerous conditions may become manifested upon a switch to an angiogenesis-stimulating phenotype. Such angiogenic conditions include, but are not limited to, age-related macular degeneration (AMD), cancer (both solid and hematologic), developmental abnormalities (organogenesis), diabetic blindness, endometriosis, ocular neovascularization, psoriasis, rheumatoid arthritis (RA), skin disclolorations (e.g., hemangioma, nevus flammeus, or nevus simplex) and wound healing.

It is desirable to identify compositions and methods that modulate or inhibit angiogenesis.

### SUMMARY OF THE INVENTION

### COMPOSITIONS OF AND PURIFICATION METHODS FOR LOW-ENDOTOXIN THERAPEUTIC AGENTS

The present invention relates to methods for purifying therapeutic agents i.e. tRNA Synthetase Fragments so that they are substantially free of endotoxins. Also presented herein are preparations suitable for therapeutic administration comprising a pharmaceutical agent, wherein the preparations are substantially free of endotoxins. Such preparations may be used in a variety of therapeutic applications, including, but not limited to applications in the therapy of cancers, applications in the therapy of neovascular disorders, applications in inhibiting angiogenesis, and applications in the therapy of ophthalmic conditions.

In one aspect, the present disclosure relates to pharmaceutical preparations suitable for administration to a human comprising a pharmaceutical agent and a pharmaceutically acceptable carrier wherein the amount of endotoxins in the pharmaceutical preparation is less than about 10 endotoxin units per milligram of pharmaceutical agent. In one embodiment, the pharmaceutical agent is a polypeptide.

In another aspect, the present disclosure relates to pharmaceutical preparations suitable for administration to a human comprising a polypeptide and a pharmaceutically acceptable carrier wherein the amount of endotoxins in the pharmaceutical preparation is less than about 10 endotoxin units per milligram of polypeptide

In another aspect, present disclosure relates to pharmaceutical preparations suitable for use in oncological therapy or ophthalmic administration in a human comprising a polypeptide and a pharmaceutically acceptable carrier wherein the amount of endotoxins in the pharmaceutical preparation is less than about 10 endotoxin units per milligram of polypeptide.

In any one of the aforementioned aspects, the polypeptide is synthesized recombinantly. In another of these aspects, the polypeptide is produced in and recovered from a transformed prokaryotic cell or its progeny. In another aspect, the polypeptide is produced in and recovered from a transformed eukaryotic cell or its progeny. In another aspect, the polypeptide is produced in and recovered from the cytoplasm of the transformed eukaryotic cell or its progeny. In another aspect, the polypeptide can modulate angiogenesis. In another aspect, the polypeptide can be used to treat macular degeneration, diabetic retinopathy, or other diseases or conditions associated with unwanted ocular neovascularization. In another aspect, the polypeptide has an isoelectric point of less than about 8.0. In another aspect, the polypeptide has an isolectric point between about 5.5 and about 8.0. In another aspect, the polypeptide has an isoelectric point between about 6.0 and about 7.5. In another aspect, the polypeptide comprises a hydrophobic cleft, and in a further refinement of this aspect, the polypeptide also has an isoelectric point of less than about 8.0.

The polypeptide is preferably part of a tryptophan tRNA synthetase. In any one of the aformentioned aspects, the preparation comprises a T2-TrpRS or a homolog thereof:

In an embodiment, the present invention relates to methods for purifying any of the aforementioned polypeptides or Pharmaceutical agents, comprising performing an endotoxin-reduction filtration step after performing a clarification step and prior to performing a buffer exchange step. Further, the endotoxin-reduction filtration step may be performed prior to performing a cation exchange chromatographic step. Alternatively, the endotoxin-reduction filtration step may be performed prior to performing a concentration step.

In another embodiment, the present invention relates to methods for purifying any of the aforementioned polypeptides or pharmaceutical agents, comprising performing an endotoxin-reduction filtration step after performing a clarification step and prior to performing a concentration step. Further, the endotoxin-reduction filtration step may be performed prior to performing a cation exchange chromatographic step. Alternatively, the endotoxin-reduction filtration step may be performed prior to performing a buffer exchange step.

In another embodiment, the present invention relates to methods for purifying any of the aforementioned polypeptides or pharmaceutical agents, comprising performing an endotoxin-reduction filtration step after performing a clarification step and prior to performing a cation-exchange chromatographic step. Alternatively, the endotoxin-reduction filtration, step may be performed prior to performing a concentration step. Alternatively, the endotoxin-reduction filtration step may be performed prior to performing a buffer exchange step.

In another embodiment, the present invention relates to methods for purifying any of the aforementioned polypeptides or pharmaceutical agents in which an endotoxin-reduction filtration step is performed prior to performing a concentration step and prior to performing a cation-exchange chromatographic step and prior to a buffer exchange step.

The order of the concentration, buffer exchange, and cation-exchange chromatography steps in any of the aforementioned purification methods may vary, but in one embodiment, at least one concentration step is performed prior to the buffer exchange step. Alternatively, a cation-exchange chromatographic step is performed after the buffer exchange step. Alternatively, at least one concentration step is performed prior to the cation-exchange chromatographic step. Alternatively, the cation-exchange chromatographic step is performed after a buffer exchange step and at least one concentration step. Alternatively, at least one concentration step is performed prior to the buffer exchange step and the cation-exchange chromatographic step. And alternatively, an additional concentration step is performed after any buffer exchange step.

In a further embodiment of any of the aforementioned purification methods, the endotoxin-reduction filtration step is performed after an anion-exchange chromatographic step. The anion-exchange chromatographic step comprises use of an anion-exchange resin. The anion-exchange resin is selected from the group consisting of Q Sepharose, DEAE Sepharose, and ANX Sepharose. In still a further embodiment, the anion-exchange resin is Q Sepharose. In any of these uses of anion-exchange resins, a variety of grades and sizes may be used, including, but not limited to Source grade, fast flow grade and high performance grade.

In an embodiment of any of the aforementioned purification methods that involve a cation-exchange chromatography step, the cation-exchange chromatographic step comprises use of a cation-exchange resin. The cation-exchange resin is selected from the group consisting of CM Sepharose, SP Sepharose, and DEAE Sepharose. In still a further embodiment, the cation exchange resin is CM Sepharose. In any of these uses of cation-exchange resins, a variety of grades and sizes may be used, including, but not limited to Source grade, fast flow grade and high performance grade.

In another embodiment, the present invention relates to methods for purifying a polypeptide suitable for administration to a patient comprising an anion-exchange chromatographic step, a step comprising a means for reducing endotoxins, and a buffer exchange step, wherein the step comprising a means for reducing endotoxins is performed prior to the buffer exchange step. In a further embodiment, the polypeptide suitable for administration to a patient is suitable for ophthalmic administration. In still a further embodiment, the polypeptide suitable for ophthalmic administration is a modulator of angiogenesis. In yet a further embodiment, the polypeptide suitable for ophthalmic administration can be used to treat macular degeneration, diabetic retinopathy or diseases or conditions associated with unwanted ocular neovascularization. In a further refinement of any of the embodiments noted in this paragraph, the polypeptide is substantially free of endotoxins.

The present invention provides to polypetide preparations for use in ophthalmic administration comprising the purified polypeptide prepared by a method comprising an anion-exchange chromatographic step, a step comprising a means for reducing endotoxins, and a buffer exchange step, wherein the step comprising a means for reducing endotoxins is performed prior to the buffer exchange step. The polypeptide preparation may further comprise a pharmaceutically acceptable carrier. The polypeptide is part of a tRNA synthetase. The polypeptide may be part of a tryptophanyl-tRNA synthetase. In yet a further possibility, the polypeptide is a T2-TrpRS or a homolog thereof. In the polypeptide preparations mentioned in this paragraph, the concentration of endotoxins in the polypeptide preparation may be less than about 10 endotoxin units per milligram of polypeptide.

The present invention provides polypeptide compositions comprising a polypeptide, wherein the polypeptide is part of a tRNA synthetase, wherein the polypeptide composition is substantially free of endotoxins. The polypeptide may be part of a tryptophanyl-tRNA synthetase. In an alternative aspect are polypeptide compositions comprising a polypeptide, wherein the polypeptide is all or part of a T2-TrpRS or a homolog thereof, wherein the polypeptide composition is substantially free of endotoxins. In a further of the aspects mentioned in this paragraph, the polypeptide composition further comprises a pharmaceutically acceptable carrier.

In another embodiment, the present invention relates to methods for preparing the polypeptide compositions mentioned in the prior paragraph, comprising performing a concentration step on collected polished polypeptide fractions, wherein the collected polished polypeptide fractions are substantially free of endotoxins. In a further embodiment are methods of preparing the collected polished polypeptide fractions of the previous embodiment comprising performing a cation-exchange chromatographic step on an unpolished polypeptide sample thereby producing the collected polished polypeptide fractions of the previous embodiment, wherein the unpolished polypeptide sample is substantially free of endotoxins. In further embodiments are methods of producing the unpolished polypeptide sample of the previous embodiment comprising performing a buffer exchange step on a polypeptide sample in a post-anion exchange buffer thereby producing the unpolished polypeptide sample of the previous embodiment, wherein the polypeptide sample in the post-anion exchange buffer is substantially free of endotoxins. In further embodiments are methods of producing the polypeptide sample in the post-anion exchange buffer of the previous embodiment comprising performing a concentration step on collected polypeptide fractions from an anion-exchange column prior to the buffer exchange step thereby producing the polypeptide sample in the post-anion exchange buffer of the previous embodiment, wherein the collected polypeptide fractions from an anion-exchange column are substantially free of endotoxins. In further embodiments are methods of producing the collected polypeptide fractions from an anion-exchange column of the previous embodiment comprising performing an endotoxin-reduction filtration step prior to the concentration step of the previous embodiment. In a further embodiment are methods comprising performing an anion-exchange chromatographic step prior to the endotoxin-reduction filtration step.

Herein disclosed are methods of treating a patient having an ophthalmic disease or condition comprising administering a therapeutically effective amount of a polypeptide, wherein the level of endotoxins in the therapeutically effective amount of the polypeptide is less than about 10 endotoxin units per milligram of polypeptide. In one treatment, the ophthalmic disease or condition is associated with unwanted ocular neovascularization. The polypeptide may be isolated from a transformed prokaryotic cell or progeny thereof. The isolation may comprise an endotoxin-reduction filtration step prior to a polishing step. In still a further embodiment, the isolation further comprises a clarification step prior to the endotoxin-reduction filtration step. In yet a further embodiment, the isolation further comprises a concentration step after the polishing step. In still a further embodiment, the isolation further comprises a buffer-exchange step after the endotoxin-reduction filtration step.

### MULTI-UNIT COMPLEXES AND USES THEREOF

The present invention also provides a composition comprising a multi-unit complex of a tRNA synthetase fragment. Preferably, the multi-unit complex of the tRNA synthetase fragment is isolated and/or soluble. Examples of multi-unit complexes include dimers (including homodimers), trimers etc. A multi-unit complex of the present invention can include a first monomer and a second monomer, wherein the first and the second monomers are covalently linked or non-covalently associated.

A tRNA synthetase fragment of the present invention can be, for example, a tryptophanyl tRNA synthetase fragment, a human tRNA synthetase fragment, or any angiostatic fragment of a tRNA synthetase fragment. In some embodiments, the tRNA synthetase fragment is selected from the group consisting of SEQ ID NOS: 12-17, 24-29, 36-41, 48-53, and any homologs and analogs thereof.

### DIVERSE MULTI-UNIT COMPLEXES INCLUDING A tRNA SYNTHETASE FRAGMENT

The present invention also provides a composition comprising a first tRNA synthetase fragment and a second tRNA synthetase fragment, wherein the first tryptophanyl tRNA synthetase fragment has a methionine at its N-terminus and the second tryptophanyl tRNA synthetase does not have a methionine at its N-terminus.

More than 50% of the composition may comprise the first tRNA synthetase fragment. More than 50% of the composition may comprise the second tRNA synthetase fragment.

The first and/or second tRNA synthetase fragments may be tryptophanyl tRNA synthetase fragments, human tryptophanyl tRNA synthetase fragments, or any angiostatic fragments of a tRNA synthetase. Examples of angiostatic fragments of a tRNA synthetase include but are not limited to the polypeptide of SEQ ID NOS: 15-17, 27-29, 39-41, 51-53, and any homologs or analogs thereof.

In some embodiments, a composition herein has a pl of about 7.4-7.8. In more preferred embodiments, a composition herein has a pl of about 7.6.

The compositions herein may also include a therapeutic agent. A therapeutic agent of the present invention may be selected from the group consisting of an antineoplastic agent, an anti-inflammatory agent, an antibacterial agent, an antiviral agent, and an anti-angiogenic agent.

Any of the multi-unit complexes of the present invention may be formulated into a pharmaceutical formulation comprising a multi-unit complex and a pharmaceutically acceptable excipient. The formulation can also include a second therapeutic agent selected from the group consisting of: an antineoplastic agent, an anti-inflammatory agent, an antibacterial agent, an angiogenic agent, an antiviral agent, and an anti-angiogenic agent. For ocular administration, a pharmaceutical formulation does not include a preservative. In preferred embodiments, a pharmaceutical formulation is a solution.

Any of the compositions (including pharmaceutical formulations) herein may be lypholized. The compositions (including pharmaceutical formulations) herein may be used to inhibit angiogenesis in a cell by contacting a cell with a composition of the present invention. The compositions herein may also be used to treat an individual suffering from an angiogenic condition by administering to the individual a pharmaceutical formulation of the present invention.

### COMPOSITIONS AND METHODS FOR MODULATING ANGIOGENESIS

The present invention also provides pharmaceutical formulations comprising a first tRNA synthetase fragment and a second tRNA synthetase fragment, wherein said first and said second tRNA synthetase fragments are non-covalently dimerized and do not include a marker-sequence, such as hexa-Histidine tag. Such pharmaceutical formulations may have a first tRNA synthetase fragment having a methionine at its N-terminus, and a second tRNA synthetase that does not include a methionine at its N-terminus.

The first and second tRNA synthetase fragments of such pharmaceutical formulations may be tryptophanyl tRNA synthetase fragments. Optionally, the first tRNA synthetase fragment is selected from the group consisting of SEQ ID NOS: 15-17, 27-29, 39-41, 51-53, homologs, and analogs thereof. Optionally, the second tRNA synthetase fragment is selected from the group consisting of SEQ ID NOS: 12-14, 24-26, 36-38, 48-50, homologs, and analogs thereof. Optionally, the first tRNA synthetase fragment is SEQ ID NO: 15, or a homolog or analog thereof and/or the second tRNA synthetase fragment is SEQ ID NO: 12, or a homolog or analog thereof. Optionally, the first tRNA synthetase fragment is SEQ ID NO: 27, or a homolog or analog thereof and/or the second tRNA synthetase fragment is SEQ ID NO: 24, or a homolog or analog thereof.

In any of the pharmaceutical formulations herein, the first tRNA synthetase fragment can be less than about 5% by weight of total amount of the first and second tRNA synthetase fragments. In some of the pharmaceutical formulations herein, the second tRNA synthetase fragment is at least about 5% by weight of total amount of the first and second tRNA synthetase fragments. In some cases a pharmaceutical formulation has a first tRNA synthetase fragment that is about 50% by weight of total amount of the first and second tRNA synthetase fragments, and a second tRNA synthetase fragment that is about 50% by weight of total amount of the first and second tRNA synthetase fragments.

In any of the pharmaceutical formulations herein the endotoxin concentration can be less than 1 endotoxin units per milligram of tRNA synthetase fragments. Moreover, the pharmaceutical formulations herein are preferably substantially free or completely free of detergent and/or preservatives.

The present invention also provides a kit that includes a container containing any of the pharmaceutical formulation herein and a set of instruction for modulating angiogenesis. Such kits can also include one or more pre-filled syringes wherein each syringe includes a single dose of such pharmaceutical formulation.

The invention also provides methods for modulating angiogenesis in a cell or an organism. Such methods include contacting a cell or organism with a pharmaceutical formulation of the invention. Preferably such angiogenesis is ocular angiogenesis or ocular neovascularization.

The present invention also provides a method for treating a patient suffering from a condition comprising administering to said patient a pharmaceutical formulation disclosed herein. Preferably such ccondition involves ocular angiogenesis or ocular neovascularization. Treatment or prevention may involve administering the pharmaceutical formulations herein locally (e.g., to the eye).

### POLYNUCLEOTIDES ENCODING tRNA SYNTHETASE FRAGMENTS AND USES THEREOF

Also disclosed is a polynucleotide sequence encoding a first tRNA synthetase fragment and a second tRNA synthetase fragment. Optionally, at least one of such tRNA synthetase fragments is a tryptophanyl tRNA synthetase fragment. Optionally, both of such tRNA synthetase fragments are tryptophanyl tRNA synthetase fragments. The tryptophanyl tRNA synthetase fragments may be mammalian or human and have angiostatic activity. Optionally, a first tRNA synthetase fragment and/or a second tRNA synthetase fragment are selected from the group consisting of SEQ ID NOS: 12-17, 24-29, 36-41, 48-53, and any homologs and analogs thereof. A polynucleotide sequence may encode a first and a second tRNA synthetase fragments in tandem. Such polynucleotide sequences may also encode a linker. A polynucleotide sequence encoding a linker may be situated between the polynucleotide sequences encoding the first and second tRNA synthetase fragments. A linker is long enough to allow the expressed first and second tRNA synthetase fragments to freely rotate and dimerize with one another. The linker and the first and second tRNA synthetase fragments are preferably in the same open reading frame.

Optionally, the polynucleotide sequence encoding at least two tRNA synthetase fragments also encodes a leader sequence. A leader can be an antibody or antibody fragment that localizes the polypeptide to a particular region. The polynucleotide sequence may also encode a prosequence. A prosequence may be cleaved once the encoded tRNA synthetase polypeptides reach a desired location (e.g., the vitreous of an eye).

Preferably, the tRNA synthetase fragment is selected from the group consisting of SEQ ID NOS: 12-17, 24-29, 36-41, 48-53, and any homologs or analogs thereof. Such polypeptides may be encoded, for example, by SEQ ID NOS: 18-23, 30-35, 42-47, 54-59, and any homologs and analogs thereof.

Also disclosed is an expression vector comprising a polynucleotide sequence disclosed herein, as well as a host cell comprising such expression vector.

The present invention also provides a targeted liposome comprising an expression vector as disclosed.

The expression vectors herein may be useful for preparing a multi-unit complex. Thus, a method for creating a multi-unit complex is disclosed wherein the method includes the steps of: providing an expression vector disclosed herein; transfecting a host cell with said expression vector; and maintaining said host cell under condition suitable for expression.

### ANTIBODIES AND EPITOPES SPECIFIC TO tRNA SYNTHETASE FRAGMENTS

Also disclosed are antibodies that specifically bind to a tRNA synthetase or a fragment, homolog or analog thereof. For example, an antibody may bind to an epitope of a tRNA synthetase, or a fragment, homolog, or analog thereof. The tRNA synthetase (or fragment thereof) can be a tryptophanyl-tRNA synthetase, a human tRNA synthetase, or any angiostatic fragment of a tRNA synthetase. Preferably, the antibody specifically binds to a polypeptide selected from the group consisting of SEQ ID NOS: 12-17, 24-29, 36-41, 48-53 and any homologs and analogs thereof. An antibody may be a polyclonal antibody, a monoclonal antibody, a chimeric antibody, an anti-idiotypic antibody, an antibody fragments.

The antibody may bind to an epitope-bearing polypeptide, wherein the epitope-bearing polypeptide comprises of about 5 to about 30 amino acids of a tRNA synthetase (or a fragment, homolog, or analog thereof). Such epitope-bearing polypeptides, or epitopes, are preferably N-terminus epitopes or includes the N-terminus of the tRNA synthetase (or a fragment, homolog, or analog thereof).

An epitope-bearing polypeptide can include at least about 5 amino acid sequence of a tRNA synthetase fragment. The tRNA synthetase fragment can be a tryptophanyl tRNA synthetase fragment, a human tryptophanyl tRNA synthetase fragment, or any angiogenic fragment of a tRNA synthetase.

Examples of epitope-bearing polypeptides include polypeptide comprising, or alternatively consisting of: amino acid residues of from about 1 to about 5, about 1 to about 15, or about 1 to about 25 of SEQ ID NOS: 12-17, 24-29, 36-41, 48-53, and any homologs and analogs thereof; amino acid residues of from about 10 to about 15, about 10 to about 25, or about 10 to about 35 of SEQ ID NOS: 12-17, 24-29, 36-41, 48-53 and any homologs and analogs thereof; amino acid residues of from about 20 to about 25, about 20 to about 35, or about 20 to about 45 of SEQ ID NOS: 12-17, 24-29, 36-41, 48-53 and any homologs and analogs thereof.

Also disclosed is a polynucleotide sequence encoding one or more of the epitope-bearing polypeptides herein.

### VARIANTS OF tRNA SYNTHETASE FRAGMENTS AND USES THEREOF

Also disclosed is a composition comprising an isolated tRNA synthetase fragment, wherein the tRNA synthetase fragment comprises, consists essentially of, or consists of an amino acid sequence SEQ ID NO: 12, 15, 24, 27, 36, 39, 48 or 51. Preferably, such tRNA synthetase fragment is less than 45 kD, more preferably less than 44 kD, less than 43.9 kD, 43.8 kD, 43.7 kD, 43.6 kD, or more preferably less than 43.5 kD. Preferably such tRNA synthetase fragment is anti-angiogenic.

Optionally, a composition comprising an isolated tRNA synthetase fragment, wherein the tRNA synthetase fragment comprises, consists essentially of, or consists of SEQ ID NO: 13, 16, 25, 28, 37, 40, 49 or 52. Preferably, such tRNA synthetase fragment is less than 48 kD, more preferably less than 47 kD, or more preferably less than 46 kD. Preferably such tRNA synthetase fragment is anti-angiogenic.

Optionally, the present invention provides a composition comprising an isolated tRNA synthetase fragment, wherein the tRNA synthetase fragment comprises, consists essentially of, or consists of SEQ ID NO: 14, 17, 26, 29, 38, 41, 50 or 53. Preferably, such tRNA synthetase fragment is less than 53 kD, more preferably less than 52 kD, more preferably less than 51 kD, more preferably less than 50 kD, or more preferably less than 49 kD. Preferably such tRNA synthetase fragment is anti-angiogenic.

Optionally a tRNA synthetase fragment is isolated. Optionally, a tRNA synthetase fragment is purified. Such purification step may reduce the amout of an endotoxin in a pharmaceutical composition. The amount of endotoxin in a composition is preferably less than 30, 20, 10, or more preferably 9, 8, 7, 6, 5, 4, 3, 2, or 1 endounits.

### METHODS FOR TREATING ANGIOGENESIS

The present invention also provides methods for treating an individual suffering from an angiogenic condition. The methods include the step of administering to such an individual a pharmaceutical formulation comprising a multi-unit complex of a tRNA synthetase fragment or a homolog or analog thereof.

Examples of angiogenic conditions that may be treated by the present invention include, but are not limited to, age-related macular degeneration, cancer, developmental abnormalities, diabetic blindness, endometriosis, ocular neovascularization, psoriasis, rheumatoid arthritis (RA), skin discolorations, such as hymengioma, and wound healing.

The tRNA synthetase fragment used in the method may be a tryptophanyl-tRNA synthetase fragment, or a human tryptophanyl-tRNA synthetase fragment, or any angiostatic fragment thereof. Examples of fragments contemplated include, but are not limited to, those of SEQ ID NOS: 12-17, 24-29, 36-41, 48-53, and any homologs and analogs thereof.

Optionally, the multi-unit complex is a dimer or a homodimer. When a multi-unit complex is a dimer, the dimer may include a first monomer and a second monomer, wherein the first monomer and the second monomer are different, homologous, substantially homologous, or identical. The first and a second monomer and the multi-unit complex contemplated herein may be covalently linked or non-covalently linked.

Optionally, an individual suffering and/or susceptible to angiogenesis may further be administered or co-administered a therapeutic agent selected from the group consisting of: an antineoplastic agent, an anti-inflammatory agent, an antibacterial agent, an antiviral agent, and an anti-angiogenic agent.

The pharmaceutical formulations used may be administered systemically or locally. For systemic administration, the pharmaceutical formulations herein may be administered at a dose of 0.1-100 mg/kg. For topical administration, the pharmaceutical formulations herein may be administered at a dose of 50-1000 µg/cm². In particularly, for intraocular administration, the pharmaceutical formulations herein may be administered at a dose of 50-1000 µg/eye. When administered to the eye, the pharmaceutical formulations preferably do not include a preservative are packaged in single unit dosages.

### METHODS FOR SCREENING FOR ANTI-ANGIOGENIC AGENTS

The present invention also provides methods for screening for an angiostatic agent wherein the methods include the steps of contacting a receptor of a tRNA synthetase fragment with a member of a library of candidate agents and selecting a candidate agent from the library that selectively binds to the receptor. Candidate agents of a library (two or more agents) may be, for example, polypeptides, peptidomimetic, peptide nucleic acids, nucleic acids, carbohydrates, and small or large, organic or inorganic molecules.

Optionally, the above methods of screening further include the step of evaluating the ability of a candidate agent to inhibit angiogenesis. Evaluation can include the step of administering the candidate agent to a retina of a mammal and visualizing neovascularization of said retina.

Examples of tRNA synthetase fragments used in the method of screening include tryptophanyl tRNA synthetase fragments, human tryptophanyl tRNA synthetase fragment, and other angiostatic fragments of a tRNA synthetase.

The present invention provides methods for obtaining an optimized ligand for a receptor of a tRNA synthetase fragment. Such methods include the steps of obtaining an X-ray structure of said receptor with said fragment; using a computer program to analyze the point of contact between the receptor and the tRNA synthetase fragment; and modifying the tRNA synthetase fragment to increase its affinity to the receptor. Examples of computer programs that may be used in these embodiments include, but are not limited to, GRID, MCSS, AUTODOCK, DOCK, AMBER, QUANTA, and INSIGHT II.

The tRNA synthetase fragment used in the methods for obtaining an optimized ligand may be a tryptophanyl tRNA synthetase fragment, a human tryptophanyl tRNA synthetase fragment, or any angiostatic fragment of a tRNA synthetase. Such fragments may be selected from the group consisting of SEQ ID NOS: 12-17, 24-29, 36-41, 48-53, and any homologs or analogs thereof.

### METHODS OF MODULATING ANGIOGENESIS

The present invention also provides methods of modulating angiogenesis. Such methods comprise the step of contacting to a cell or tissue susceptible or experiencing angiogenesis with a multi-unit complex comprising a tRNA synthetase fragment, or a homolog or analog thereof.

A tRNA synthetase fragment can be, for example, a tryptophanyl tRNA synthetase fragment, a human tRNA synthetase fragment, or any angiostatic fragment of a tRNA synthetase. Examples of tRNA synthetase fragments include those selected from the group consisting of SEQ ID NOS: 12-17, 24-29, 36-41, 48-53, and any homologs and analogs thereof.

A multi-unit complex may be soluble and/or isolated. A multi-unit complex can include two or more monomers. In some examples, a multi-unit complex is a dimer or a homodimer. Two or more monomer units of a multi-unit complex can be covalently linked or non-covalently associated.

Optionally, a multi-unit complex can have a first monomer and a second monomer, wherein said first monomer comprises a tRNA synthetase fragment having a methionine at its N-terminus, and wherein said second monomer comprises a tRNA synthetase fragment not having a methionine at its N-terminus. Such compositions can have a pl value of about 7.4-7.8.

Examples of a first monomer include those selected from the group consisting of SEQ ID NOS: 15-17, 27-29, 39-41, 51-53, and any homologs or analogs thereof.

Examples of a second monomer include those selected from the group consisting of SEQ ID NOS: 12-14, 24-26, 36-38, 48-50, and any homologs or analogs thereof.

Optionally, a multi-unit complex comprises of a first monomer and a second monomers, wherein said first monomer comprises a tRNA synthetase fragment modified to include at least one non-naturally occurring cysteine in its dimerization domain and said second monomer comprises a tRNA synthetase fragment modified to include at least one non-naturally occurring cysteine in its dimerization domain.

Such tRNA synthetase fragments can be, for example, tryptophanyl tRNA synthetase fragments, human tRNA synthetase fragments, or any angiostatic fragment of a tRNA synthetase.

A cell or tissue may further be contacted with a second therapeutic agent selected from the group consisting of: an antineoplastic agent, an anti-inflammatory agent, an antibacterial agent, an antiviral agent, and an anti-angiogenic agent.

### KITS FOR MODULATING ANGIOGENESIS

The present invention provides kits for modulating angiogenesis. Optionally, a kit comprises a container comprising a multi-unit complex wherein at least one unit of said multi-unit complex comprises a tRNA synthetase fragment or a homolog or analog thereof; and written instructions for use thereof in treating an individual. A multi-unit complex can be, for example, a dimer having two units. Monomers of a multi-unit complex can be different from each other, homologous, substantially homologous, or identical. Optionally a multi-unit complex is a dimer having two homologous monomers.

Optionally a tRNA-synthetase fragment can be a tryptophanyl tRNA synthetase fragment, a human tryptophanyl tRNA-synthetase, or any angiostatic fragment of a tRNA synthetase fragment. For example, a tRNA synthetase fragment can be selected from the group consisting of SEQ ID NOS: 12-17, 24-29, 36-41, 48-53, and any homologs or analogs thereof.

Any two monomers within a multi-unit complex may be covalently linked or non-covalently linked. The composition in the first container may be packaged for systemic administration in a single unit dosage. When packaged in single unit dosages, a dose may range between 50-1000 µg/dose. The kit herein may also include a second therapeutic agent. Such second therapeutic agent may be contained in a second container. Examples of a second therapeutic agent include, but are not limited to an antineoplastic agent, an anti-inflammatory agent, an antibacterial agent, an antiviral agent, and an anti-angiogenic agent.

A kit can include a container, comprising an antibody that specifically binds to an epitope of a tRNA synthetase fragment and written instructions for use thereof. In such examples, the tRNA synthetase fragment is a tryptophanyl tRNA synthetase fragment or a human tryptophanyl tRNA synthetase fragment, or any angiostatic fragment of a tRNA synthetase. Optionally an angiostatic tRNA synthetase fragment is one selected from the group consisting of SEQ ID NOS: 12-17, 24-29, 36-41, 48-53, and any homologs and analogs thereof.

Optionally, a kit comprises a container comprising a composition of a first tRNA synthetase fragment and a second tRNA synthetase fragment wherein the first tRNA synthetase fragment has a methionine at its N-terminus and wherein the second tRNA synthetase fragment does not have a methionine at its N-terminus; and written instructions for use thereof.

The first tRNA synthetase fragment can be, for example, a tryptophanyl tRNA synthetase fragment, a human tRNA synthetase fragment, or an angiostatic fragment of a tRNA synthetase. The second tRNA synthetase fragment can be, for example, a tryptophanyl tRNA synthetase fragment, a human tRNA synthetase fragment, or an angiostatic fragment of a tRNA synthetase.

Examples of angiostatic tRNA synthetase fragments having a methionine at their N-terminus include, but are not limited to those selected from the group consisting of SEQ ID NOS 15-17, 27-29, 36-38, 48-50 and any homologs and analogs thereof.

Examples of angiostatic tRNA synthetase fragments not having a methionine at their N-terminus include, but are not limited to those selected from the group consisting of SEQ ID NOS 12-14, 24-26, 36-38, 48-50, and any homologs and analogs thereof.

A composition in the first container may have a pl of about 7.4 -7.8.

Such kits may further include a second therapeutic agent, such as an antineoplastic agent, an anti-inflammatory agent, an antibacterial agent, an antiviral agent, or an anti-angiogenic agent. The second therapeutic agent may be contained in a separate container.

### BUSINESS METHODS FOR MODULATING ANGIOGENESIS

The present invention also provides business methods for modulating angiogenesis. The business methods herein include the steps of search for an agent that modulates or binds to a receptor of a tRNA synthetase fragment; and commercialize said agent.

A tRNA synthetase fragment of the present invention can be, for example, a tryptophanyl tRNA synthetase fragment or a tyrosyl tRNA synthetase. Preferably such fragment is mammalian, or more preferably human. Optionally, a tRNA synthetase fragment has angiostatic activity. Examples of such angiostatic tRNA synthetase fragments include but are not limited to a fragment selected from the group consisting of SEQ ID NOS: 12-17, 24-29, 36-41, 48-53, and any homologs and variants thereof.

A searching step may involve screening a library of candidate agents to identify an agent that modulates angiogenesis. Such agent can be, for example, a small molecule, a peptide, or a peptidomimetic. A searching step may involve the use of a computer program to generate a peptidomimetic of said tRNA synthetase fragment.

The present invention also provides business methods comprising the steps of (i) modifying a tRNA synthetase fragment to enhance its dimerization capabilities; and (ii) commercializing the enhanced tRNA synthetase fragment or dimerized form thereof.

Such tRNA synthetase fragments are preferably angiostatic fragments, tryptophanyl tRNA synthetase fragments, and/or human tRNA synthetase fragments. Examples of such fragments include a fragment selected from the group consisting of SEQ ID NOS: 12-17, 24-29, 36-41, 48-53 and any homologs and analogs thereof.

The modifying step herein can involve generating an expression vector encoding a tRNA synthetase fragment modified in its dimerization domain to include one or more non-naturally occurring cysteines.

Optionally the modifying step involves generating an expression vector encoding two tRNA synthetase fragments. An expression vector may also encode a linker. Such linker may be situated between the first and the second tRNA synthetase fragments.

Optionally, the modifying step involves the use of a computer program to optimize the tRNA synthetase fragment. Examples of useful computer programs includes, but are not limited to GRID, MCSS, AUTODOCK, DOCK, AMBER, QUANTA, and INSIGHT II.

Optionally the present invention provides business methods that include the steps of (i) preparing a recombinant tRNA synthetase fragment; and (ii) commercializing said fragment for modulating angiogenesis. Examples of recombinant tRNA synthetase fragments that may be prepared include, but are not limited to, tryptophanyl tRNA synthetase fragments and tyrosyl tRNA synthetase fragments. Preferably such fragments are human. Also, preferably, such fragments can modulate angiogenesis.

Examples of angiostatic fragments include, but are not limited to, SEQ ID NOS: 12-17, 24-29, 36-41, 48-53, and any homologs and analogs thereof.

Any of the angiogenesis-modulating tRNA synthetase fragments herein may be in monomer units or part of a multi-unit complex.

The business methods herein prepare such angiogenesis-modulating tRNA synthetase fragments by first preparing an expression vector encoding such fragments, then tranfecting a host cell with said expression vector, and finally maintaining said host cell under a condition that permits the expression of said tRNA synthetase fragment.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

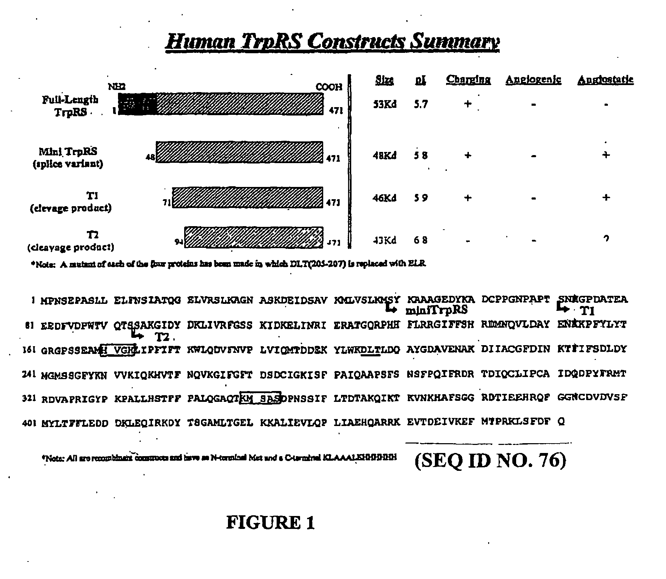

Figure 1 illustrates the amino acid residue sequence of tryptophanyl-tRNA synthetase polypeptide (SEQ ID NO: 63); mini-tryptophanyl-tRNA synthetase polypeptide (SEQ ID NO: 29), which corresponds to amino acid residues 48-471 of SEQ ID NO: 63; T1-tryptophanyl-tRNA-synthetase polypeptide (SEQ ID NO: 25), which corresponds to amino acid residues 71-471 of SEQ ID NO: 63; and T2-tryptophanyl-tRNA synthetase polypeptide (SEQ ID NO: 24), which corresponds to amino acid residues 94-471 of SEQ ID NO: 63. All sequences are recombinant constructs and have an N-terminal Met and a Terminal KLAAALEHHHHHH motif (SEQ ID NO. 76).
Figure 2 is a photomicrograph that illustrates retinal vascular development in a mouse model.
Figure 3 is a graphical representation of data reported in Example 3, below.
Figure 4 is a graphical representation of data reported in Example 4, below.
Figure 5 is a photomicrograph that illustrates the binding localization of his-tagged T2 (SEQ ID NO: 7) in the retina in a mouse model.
Figure 6 illustrates experimental pl of a polypeptide recombinantly produced by an expression vector encoding SEQ ID NO: 27.
Figure 7 illustrates a flowchart illustration of one possible method for purifying the compositions herein.
Figure 8 illustrates another embodiment of the purification methods of the invention.
Figure 9 illustrates an 4-20% Tris-Glycine SDS-PAGE analysis (reducing reconditions) demonstrating purity of a polypeptide produced by a bacteria host cell transfected with a vector of SEQ ID NO: 70, encoding SEQ ID NO: 27 and further purified using one of the methods disclosed herein.
Figure 10 illustrates an SDS-PAGE gel of samples produced by recombinantly expressing in *E. coli* a vector of SEQ ID NO: 70, which encodes SEQ ID NO: 27, wherein some product is heated.
Figure 11 illustrates a native PAGE gel of a product produced by recombinantly expressing in *E. coli* a vector of SEQ ID NO: 70, which encodes SEQ ID NO: 27.
Figure 12 illustrates a calibration curve wherein the x-axis is the retention time of calibrants per minute and the y-axis is the log MW.
Figure 13 illustrates a product produced by recombinantly expressing in *E*. *coli* a vector of SEQ ID NO: 70, which encodes SEQ ID NO: 27, as detected at UV absorbance of 215 nm.
Figure 14 illustrates a product produced by recombinantly expressing in *E. coli* a vector of SEQ ID NO: 70, which encodes SEQ ID NO: 27, as detected at UV absorbance of 254 nm.
Figure 15 illustrates a product produced by recombinantly expressing in *E. coli* a vector of SEQ ID NO: 70, which encodes SEQ ID NO: 27, as detected at UV absorbance of 280 nm.
Figure 16 illustrates results from a PPi exchange assay.
Figure 17 illustrates counts per minute results from a PPi exchange assay.
Figure 18 illustrates various inhibition levels in post-natal mouse.
Figure 19 illustrates a comparison of percentage inhibition of angiogenesis by product produced by *E*. *Coli* expression of SEQ ID NO: 71, SEQ ID NO: 70 purified to about 95% purity and SEQ ID NO: 70 purified to about 100% purity at various dosages.
Figure 20 illustrates results from a reverse phase HPLC column of a product produced by *E. coli* expression of a polynucleotide encoding SEQ ID NO: 27, purified to reduce endotoxin levels.
Figure 21 illustrates MALDI-TOF spectrum of a product produced recombinant *E*. *Coli* expression of vector SEQ ID NO: 70, which is then purified to about 95% purity ±4%.
Figure 22 illustrates a MALDI-TOF spectrum of a product produced recombinant *E*. *Coli* expression of vector SEQ ID NO: 70, which is then purified to about 100% ±1% purity.
Figure 23 illustrates mass spectrum of a product produced by recombinant expression of SEQ ID NO: 70 in *E*. *coli,* followed by purification to greater than 99% purity and removal of substantially all endotoxins, which is then digested by GluC.
Figure 24 illustrates mass spectrum of a product produced by recombinant expression of SEQ ID NO: 70 in *E*. *coli,* followed by purification to greater than 99% purity and removal of substantially all endotoxins, which is then digested with trypsin.
Figure 25 illustrates mass spectrum of a product produced by recombinant expression of SEQ ID NO: 70 in *E. coli,* followed by purification to greater than 99% purity and removal of substantially all endotoxins, which is then digested with GluC showing the N-terminal peptide without a methionine at 494 m/z (Mr=2468).
Figure 26 illustrates mass spectrum of a product produced by recombinant expression of SEQ ID NO: 70 in *E. coli*, followed by purification to greater than 99% purity and removal of substantially all endotoxins, which is then digested with GluC showing N-terminal peptide without a methionine at 618 m/z (Mr=2468).
Figure 27 illustrates the mass spectrum of a a product produced by recombinant expression of SEQ ID NO: 70 in *E. coli*, followed by purification to greater than 99% purity and removal of substantially all endotoxins, which is then digested with GluC, showing the N-terminal peptide without a methionine.
Figure 28 illustrates a fragmentation of the doubly charged mass at m/z = 759 of a product produced by recombinant expression of SEQ ID NO: 70 in *E. coli,* followed by purification to greater than 99% purity and removal of substantially all endotoxins.
Figure 29 illustrates a MALDI-TOF mass spectrum of a product produced by recombinant expression of SEQ ID NO: 70 in *E*. *coli,* followed by purification to greater than 99% purity and removal of substantially all endotoxins, which is then digested by GluC.
Figure 30 illustrates a MALDI-TOF mass spectrum of a product produced by recombinant expression of SEQ ID NO: 70 in *E*. *coli,* followed by purification to greater than 99% purity and removal of substantially all endotoxins, which is then digested by trypsin.
Figure 31 illustrates an electrospray ionization spectrum of a product produced by recombinant expression of SEQ ID NO: 70 in *E. coli*, followed by purification to greater than 99% purity and removal of substantially all endotoxins, which is then desalted with a C₄ZipTip (Millipore).
Figure 32 illustrates the convoluted electrospray spectrum Figure 32.
Figure 33 illustrates a MALDI-TOF mass spectrum of a product produced by recombinant expression of SEQ ID NO: 70 in *E. coli,* followed by purification to greater than 99% purity and removal of substantially all endotoxins, which is then desalted with a C₄ preparatory column (ZipTip, Millipore).

### DETAILED DESCRIPTION OF THE INVENTION

### Definitions

The term "amino acid" or "amino acid residue" refers to an amino acid which is preferably in the L-isomeric form. When an amino acid residue is part of a polypeptide chain, the D-isomeric form of the amino acid can be substituted for the L-amino acid residue, as long as the desired functional property is retained. NH₂ refers to the free amino group present at the amino terminus of a polypeptide. COOH refers to the free carboxy group present at the carboxyl terminus of a polypeptide.

In keeping with standard polypeptide nomenclature described in J. Biol. Chem., 243:3552-59 (1969) and adopted at 37 C.F.R. §§ 1.821-1.822, all amino acid residue sequences represented herein by formulae have a left to right orientation in the conventional direction of amino-terminus to carboxyl-terminus. In addition, the phrase "amino acid residue" is broadly defined to include modified and unusual amino acids, such as those referred to in 37 C.F.R. §§ 1.821-1.822. A dash at the beginning or end of an amino acid residue sequence indicates a peptide bond to a further sequence of one or more amino acid residues or to an amino-terminal group such as IVH₂ or to a carboxyl-terminal group such as COOH.

In a peptide or protein, suitable conservative substitutions of amino acids are known to those of skill in this art and can be made generally without altering the biological activity of the resulting molecule. Those of skill in this art recognize that, in general, single amino acid substitutions in non-essential regions of a polypeptide do not substantially alter biological activity (see, e.g., Watson et al. Molecular Biology of the Gene, 4th Edition, 1987, The Benjamin/Cummings Pub. Co. p.224).

Such substitutions are preferably made with those set forth as follows:

| Original residue | Conservative substitution(s) |
|---|---|
| Ala | Gly; Ser |
| Arg | Lys |
| Asn | Gln; His |
| Cys | Ser |
| Gln | Asn |
| Glu | Asp |
| Gly | Ala; Pro |
| His | Asn; Gln |
| lie | Leu; Val |
| Leu | Ile; Val |
| Lys | Arg; Gln; Glu |
| Met | Leu; Tyr, Ile |
| Phe | Met; Leu; Tyr |
| Ser | Thr |
| Thr | Ser |
| Trp | Tyr |
| Tyr | Trp; Phe |
| Val | Ile; Leu |

The term "analog(s)" as used herein refers to a composition that retains the same structure or function (e.g., binding to a receptor) as a polypeptide or nucleic acid herein. Examples of analogs include peptidomimetics, peptide nucleic acids, small and large organic or inorganic compounds, as well as derivatives and variants of a polypeptide or nucleic acid herein. The term "derivative" or "variant" as used herein refers to a peptide or nucleic acid that differs from the naturally occurring polypeptide or nucleic acid by one or more amino acid or nucleic acid deletions, additions, substitutions or side-chain modifications. Amino acid substitutions include alterations in which an amino acid is replaced with a different naturally-occurring or a non-conventional amino acid residue. Such substitutions may be classified as "conservative", in which case an amino acid residue contained in a polypeptide is replaced with another naturally-occurring amino acid of similar character either in relation to polarity, side chain functionality or size.

Substitutions encompassed by the present invention may also be "non-conservative", in which an amino acid residue which is present in a peptide is substituted with an amino acid having different properties, such as naturally-occurring amino acid from a different group (e.g., substituting a charged or hydrophobic amino acid with alanine), or alternatively, in which a naturally-occurring amino acid is substituted with a non-conventional amino acid. Preferably, amino acid substitutions are conservative.

Amino acid substitutions are typically of single residues, but may be of multiple residues, either clustered or dispersed. Additions encompass the addition of one or more naturally occurring or non-conventional amino acid residues. Deletion encompasses the deletion of one or more amino acid residues.

As stated above peptide derivatives include peptides in which one or more of the amino acids has undergone side-chain modifications. Examples of side chain modifications contemplated by the present invention include modifications of amino groups such as by reductive alkylation by reaction with an aldehyde followed by reduction with NaBH₄ ; amidination with methylacetimidate; acylation with acetic anhydride; carbamoylation of amino groups with cyanate; trinitrobenzylation of amino groups with 2,4,6-trinitrobenzene sulphonic acid (TNBS); acylation of amino groups with succinic anhydride and tetrahydrophthalic anhydride; and pyridoxylation of lysine with pyridoxal-5-phosphate followed by reduction with NaBH₄.

The guanidine group of arginine residues may be modified by the formation of heterocyclic condensation products with reagents such as 2,3-butanedione, phenylglyoxal and glyoxal. The carboxyl group may be modified by carbodiimide activation via O-acylisourea formation followed by subsequent derivitisation, for example, to a corresponding amide. Sulphydryl groups may be modified by methods such as carboxymethylation with iodoacetic acid or iodoacetamide; performic acid oxidation to cysteic acid; formation of a mixed disulphides with other thiol compounds; reaction with maleimide, maleic anhydride or other substituted maleimide; formation of mercurial derivatives using 4-chloromercuribenzoate, 4-chloromercuriphenylsulphonic acid, phenylmercury chloride, 2-chloromercuri-4-nitrophenol and other mercurials; carbamoylation with cyanate at alkaline pH. Any modification of cysteine residues must not affect the ability of the peptide to form the necessary disulphide bonds. It is also possible to replace the sulphydryl groups of cysteine with selenium equivalents such that the peptide forms a diselenium bond in place of one or more of the disulphide bonds.

Tryptophan residues may be modified by, for example, oxidation with N-bromosuccinimide or alkylation of the indole ring with 2-hydroxy-5-nitrobenzyl bromide or sulphenyl halides. Tyrosine residues on the other hand, may be altered by nitration with tetranitromethane to form a 3-nitrotyrosine derivative. Modification of the imidazole ring of a histidine residue may be accomplished by alkylation with iodoacetic acid derivatives or N-carbethoxylation with diethylpyrocarbonate. Proline residue may be modified by, for example, hydroxylation in the 4-position. Other derivatives contemplated by the present invention include a range of glycosylation variants from a completely unglycosylated molecule to a modified glycosylated molecule. Altered glycosylation patterns may result from expression of recombinant molecules in different host cells.

Additional derivatives include alterations that are caused by expression of the polypeptide in bacteria or other host system as well as through chemical modifications. Preferably, the derivatives retain the desired activity. For example, a derivative of T2 may be a truncated version of T2 that retains T2's ability to bind one of its naturally occurring receptors or to inhibit angiogenesis.

The term "antagonist" is used herein to refer to a molecule inhibiting a biological activity. Examples of antagonist molecules include but are not limited to antibodies, antisense nucleic acids, siRNA nucleic acids, and other binding agents.

The term "antibody" or "antibodies" as used herein includes polyclonal antibodies, monoclonal antibodies (mAbs), chimeric antibodies, anti-idiotypic (anti-Id) antibodies to antibodies that can be labeled in soluble or bound form, as well as fragments, regions or derivatives thereof (e.g., separate heavy chains, light chains, Fab, Fab', F(ab')2, Fabc, and Fv).

The term "effective amount" as used herein means that amount of composition necessary to achieve the indicated effect.

The terms "gene therapy" and "genetic therapy" refer to the transfer of heterologous nucleic acids to the certain cells, target cells, of a mammal, particularly a human, with a disorder or conditions for which such therapy is sought. The nucleic acid is introduced into the selected target cells in a manner such that the heterologous DNA is expressed and a therapeutic product encoded thereby is produced. Alternatively, the heterologous nucleic acids can in some manner mediate expression of a nucleic acid that encodes the therapeutic product; it can encode a product, such as a peptide or RNA that in some manner mediates, directly or indirectly, expression of a therapeutic product. Genetic therapy can also be used to nucleic acid encoding a gene product replace a defective gene or supplement a gene product produced by the mammal or the cell in which it is introduced. The introduced nucleic acid can encode a therapeutic compound, such as a growth factor inhibitor thereof, or a tumor necrosis factor or inhibitor thereof, such as a receptor thereof, that is not normally produced in the mammalian host or that is not produced in therapeutically effective amounts or at a therapeutically useful time. The heterologous DNA encoding the therapeutic product can be modified prior to introduction into the cells of the afflicted host in order to enhance or otherwise alter the product or expression thereof.

The term "homodimer" as used herein refers to two monomers that are complexed together either covalently or non-covalently wherein the two compounds are identical.

The term "homolog" or "homologous" as used herein refers to homology with respect to structure and/or function. With respect to sequence homology, sequences are homologs if they are at least 50%, preferably at least 60%, more preferably at least 70%, more preferably at least 80%, more preferably at least 90%, more preferably at least 95% identical, more preferably at least 97% identical, or more preferably at least 99% identical. The term "substantially homologous" refers to sequences that are at least 90%, more preferably at least 95% identical, more preferably at least 97% identical, or more preferably at least 99% identical. Homologous sequences can be the same functional gene in different species.

The term "host" as used herein refers to an organism that expresses a nucleic acid of this invention in at least one of its cells. The term "host cell" as used herein refers to a cell which expresses the nucleotide sequences according to this invention.

The term "inhibit" as used herein refers to prevention or any detectable reduction or elimination of a condition.

The term "isolated" as used herein refers to a compound or molecule (e.g., a polypeptide or a nucleic acid) that is relatively free of other compounds or molecules that it normally is associated with *in vivo.* In general, an isolated polypeptide constitutes at least about 75%, more preferably about 80%, more preferably about 85%, more preferably about 90%, more preferably about 95%, or more preferably about 99% by weight of a sample containing it.

The term "mini-TrpRS" as used herein refers to a polypeptide having amino acid sequence selected from the group consisting of SEQ ID NOS: 2, 3, 14, 17, 26, 29, 38, 41, 50, 53, and any homologs and analog thereof.

The term "multi-unit complex" as used herein refers to a complex of one or more monomer units that are complexed together covalently or non-covalently. Examples of multi-unit complexes include dimers, trimers, etc.

The term "nucleic acid" or "nucleic acid molecule" as used herein refers to an oligonucleotide sequence, polynucleotide sequence, including variants, homologs, fragments, or analogs thereof. A nucleic acid may include DNA, RNA, or a combination thereof. A nucleic acid may be naturally occurring or synthetic, double-stranded or single-stranded, sense or antisense strand.

As used herein the term "operably linked" wherein referring to a first nucleic acid sequence which is operably linked with a second nucleic acid sequence refers to a situation when the first nucleic acid sequence is placed in a functional relationship with the second nucleic acid sequence. For instance, a promoter is operably linked to a coding sequence if the promoter effects the transcription or expression of the coding sequence. Generally, operably linked nucleic acid sequences are contiguous and, where necessary to join two protein coding regions, the open reading frames are aligned.

The term "peptidomimetic" as used herein refers to both peptide and non-peptide agents that mimic aspects of a polypeptide. Non-hydrolyzable peptide analogs of critical residues can be generated using benzodiazepine (see Freidinger et al. in Peptides: Chemistry and Biology, G. R. Marshall ed., ESCOM Publisher: Leiden, Netherlands, 1988), azepine (see Huffman et al. in Peptides: Chemistry and Biology, G. R. Marshall ed., ESCOM Publisher: Leiden, Netherlands, 1988), substituted γ lactam rings (Garvey et al. in Peptides: Chemistry and Biology, G. R. Marshall ed., ESCOM Publisher: Leiden, Netherlands, 1988), keto-methylene pseudopeptides (Ewenson et al. (1986) J Med Chem 29:295; and Ewenson et al. in Peptides: Structure and Function (Proceedings of the 9th American Peptide Symposium) Pierce Chemical Co. Rockland, III, 1985), β-turn dipeptide cores (Nagai et al. (1985) Tetrahedron Lett 26:647; and Sato et al. (1986) J Chem Soc Perkin Trans 1:1231), and β-aminoalcohols (Gordon et al. (1985) Biochem Biophys Res Commun 126:419; and Dann et al. (1986) Biochem Biophys Res Commun 134:71).

The term "polypeptide", "peptide", "oligopeptides" or "protein" refers to any composition that includes two or more amino acids joined together by a peptide bond. It will be appreciated that polypeptides often contain amino acids other than the 20 amino acids commonly referred to as the 20 naturally occurring amino acids, and that many amino acids, including the terminal amino acids, may be modified in a given polypeptide, either by natural processes such as glycosylation and other post-translational modifications, or by chemical modification techniques which are well known in the art.

Among the known modifications which may be present in polypeptides include, but are not limited to, acetylation, acylation, ADP-ribosylation, amidation, covalent attachment of flavin, covalent attachment of a heme moiety, covalent attachment of a polynucleotide or polynucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, y-carboxylation, glycation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination.

The term "receptor" refers to a biologically active molecule that specifically binds to (or with) other molecules. The term "receptor protein" can be used to more specifically indicate the proteinaceous nature of a specific receptor. For example, the term "T2 receptor" refers to a biologically active molecule that specifically binds to (or with) T2.

The term "T1" or "T1-TrpRS" refers to a polypeptide having an amino acid sequence comprising of SEQ ID NO: 13, 25, 37, 49, homologs or analogs thereof, and any polynucleotide sequence encoding the same.

The term "T2" or "T2-TrpRS" refers to a polypeptide having an amino acid sequence comprising of SEQ ID NO: 12, 24, 36, 48, homologs or analogs thereof, and any polynucleotide sequence encoding the same.

The term "treating" as used herein refers to eliminating, reducing, or alleviating symptoms in a subject, or preventing symptoms from occurring, worsening, or progressing.

The term "TrpRS" or "tryptophanyl tRNA synthetase" as used herein refers to the full length tryptophanyl-tRNA synthetase as illustrated in Figure 1, wherein amino acid residues 213 is either Gly or Ser and amino acid residue 214 is either Asp or Tyr (independently of the other). Thus, the terms "GD variant" "SD variant" "GY variant" and "SY variant" as used herein refer to TrpRS or fragment thereof with the corresponding amino acid residues in the above location within the polypeptide.

The term "tRS" as used herein means a tRNA synthetase polypeptide and/or nucleic acids encoding such polypeptide, whether naturally occurring or non-naturally occurring.

The term "truncated tRNA synthetase polypeptides" means polypeptides that are shorter than the corresponding full length tRNA synthetase.

### Compositions

Aminoacyl-tRNA synthetases (tRS) are ancient proteins that are essential for decoding genetic information during the process of translation. There are two classes of tRS. The first class, class I, contains a common loop with the signature sequence KMSKS (and HIGH, as part of a Rossman dinucletide binding fold of parallel β sheets ("Rossman fold domain")). Sever et al., Biochem. 35, 32-40 (1996). The second class, Class II, have an entirely different topology of dinucleotide binding bases on anti-parallel β sheets.

Tryptophanyl-tRNA synthetase (TrpRS) is a Class I tRS. It is believed that expression of TrpRS is stimulated by interferon ("IFN") (e.g, IFN-γ) and/or tumor necrosis factor ("TNF") (e.g., TNF-α). IFN-γis responsible for antiviral and anti-proliferative state of animal cells. See Kisselev, L., Biochimie 75, 1027-1039 (1993). Stimulation of TrpRS by IFN occurs at the transcriptional level by a consensus regulatory sequence designated IFN-stimulated response element ("ISRE"). An examination of ISRE sequences from a number of IFN-response genes indicates a common motif of GGAAAN(N/-)GAAA. Thus the present invention provides the use of the compositions herein to treat IFN and/or TNF mediated conditions, and in particular IFN-γ and/or TNF-α mediated conditions.

Mammalian TrpRS molecules have an amino-terminal appended domain. In normal human cells, there are two forms of TrpRS that can be detected: a major form consisting of the full-length molecule (amino acid residues 1-471 of SEQ ID NO: 1) and a minor truncated form ("mini-TrpRS"; a polypeptide comprising amino acid sequence SEQ ID NOS: 3, 14, 19, or 20). In any of the Trp-RS embodiments herein amino acids 213 can be either a Gly or Ser and amino acid 214 can be either an Asp or Tyr. Such variants may be referred to herein as the GD variant, GY variant, SD variant and SY variant.

The minor form is generated by the deletion of the amino-terminal domain through alternative splicing of the pre-mRNA (Tolstrup et al., J. Biol. Chem. 270:397-403 (1995)). The amino-terminus of mini-TrpRS has been determined to be the methionine residue at position 48 of the full-length TrpRS molecule. Alternatively, truncated TrpRS can be generated by proteolysis. Lemaire et al., Eur. J. Biochem. 51:237-52 (1975). For example, bovine TrpRS is highly expressed in the pancreas and is secreted into the pancreatic juice (Kisselev, Biochimie 75:1027-39 (1993)), thus resulting in the production of a truncated TrpRS molecule. These observations suggest that truncated TrpRS could have a function other than the aminoacylation of tRNA.

Studies indicate that the full-length TrpRS does not inhibit angiogenesis, whereas mini-TrpRS inhibits VEGF-induced cell proliferation and migration (Wakasugi et al., Proc. Natl. Acad. Sci. 99: 173-177 (2002)). In particular, a chick CAM assay shows that mini-TrpRS blocks angiogenic activity of VEGF. Thus, removal of the first 47 amino acid residues exposes the anti-angiogenic activity of TrpRS. TrpRS and mini-TrpRS are further described in International Application Nos. PCT/US01/08966 and PCT/US01/8975, both filed March 21, 2001.

Additional fragments of TrpRS that have angiostatic activity are referred to herein as T1 and T2. Treatment of TrpRS with PMN elastase results in two additional products: a 47 kDa fragment (super mini-TrpRS or T1; *e.g*., SEQ ID NO: 13, 16, 25, 28, 37, 40, 49, and 52) and an approximately 43 kDa fragment (T2-TrpRS or T2; *e.g*., SEQ ID N: 12, 15, 24, 27, 36, 39, 48, and 51). Terminal amino acid analysis has revealed Ser-71 and Ser-94, respectively, as the NH₂-terminal residues for these fragments. Both T1 and T2 have been shown to be potent antagonists of *in vivo* angiogenesis as illustrated in the examples below. T1 and T2 are further described in U.S. Provisional Application No. 60/270,951 filed on February 23, 2001, for "Tryptophanyl-tRNA Synthetase Derived Polypeptides Useful for the Regulation of Angiogenesis" as well as U.S. Patent Application No. 10/080,839, filed February 22, 2002, and International Application No. PCT/US02/05185, filed February 22, 2002. Methods for preparing T2 are further disclosed in U.S. Provisional Application No. 60/598,019, filed August 8, 2004, entitled "Composition of and Purification Methods for Low-Endotoxin Therapeutic Agents".

### 1. Polypeptides.

The present invention provides compositions comprising a tRNA synthetase fragment having angiogenic or angiostatic (anti-angiogenic) activity.

Preferably such compositions and/or tRNA synthetase fragments are substantially pure. In other embodiments, the compositions and/or tRNA synthetase fragments herein are at least 20%, 30%, 40%, 50%, 55% 60%, 65%, 70%, 75%, 80%, 85%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, 99.9% ot 99.95% pure. Percent purity refers to the weight of the composition and/or tRNA synthetase fragment per total total weight of the composition and/or tRNA synthetase fragment (w/w), respectively. When referring to a composition comprising a tRNA synthetase fragment, the composition is deemed to be, e.g., 80% pure, if 80% of total product is observed under a single chromatographic peak at UV absorbance bewteen 180-220 nm. Similarly, when referring to a tRNA synthetase fragment, the tRNA synthetase fragment is deemed to be, e.g., 90% pure, if 90% of total product is observed under a single chromatographic peak at UV absorbance between 180-220 nm.

In some cases, tRNA synthetase fragments (and compositions comprising such fragments) are angiogenic. In some cases, tRNA synthetase fragments (and compositions comprising such fragments) are angiostatic. When referring to angiostatic activity, a tRNA synthetase fragment is said to have angiostatic activity as measured by the methods disclosed in Example 18. Preferably, a tRNA synthetase fragment (or composition comprising the tRNA synthetase fragment) has angiostatic activity of more than 10 15, 20, 25, 30, 35, 40, 45, 50, 55, 60, 65, 70, or 75 angiostatic activity units. Optionally, a tRNA synthetase fragment (and compositions comprising thereof) has angiostatic activity greater than 50 angiostatic activity units.

Examples of tRNA synthetase fragments include tryptophanyl tRNA synthetase fragments and tyrosyl tRNA synthetase fragments. Such fragments are preferably mammalian, or more preferably human. Such fragments preferably do not include a His-tag (e.g., a series of histidine amino acid residues, commonly added to the C-terminus). Examples of tRNA synthetase fragments that do not include His-tags include SEQ ID NOS: 12-17, 24-29, 36-41, 48-53, and homologs and variants thereof. Removal of His-tag is preferred for pharmaceutical formulations administered to an organism because of the His-tag affinity for certain compounds and effect on solubility of a polypeptide and the potential for the His-tag to be antigenic and potentially elicit an unwanted immunologic effect. However, removal of a His-tag is not trivial and may sometimes affect other aspects of a polypeptide.

Examples of tryptophanyl tRNA synthetase fragments that are disclosed by the present invention include mini-TrpRS, T1, T2 and any angiogenic or angiostatic fragments thereof. Preferably, such polypeptides have an amino acid sequence comprising, consisting essentially of, or consisting of SEQ ID NOS: 12-17, 24-29, 36-41, 48-53, or any homologs, analogs, or fragments thereof. Such fragments may be naturally occurring or non-naturally occurring. Such fragments are preferably isolated and/or purified.

Optionally a composition of the present invention comprises a tRNA synthetase fragment, wherein the tRNA synthetase fragment comprises, consists essentially of, or alternatively consists of an amino acid sequence selected from the group of SEQ ID NO: 12, 15, 24, 27, 36, 39, 48, 51, and any homologs and analogs thereof. Preferably, such tRNA synthetase fragment does not include a His-tag. Preferably, such tRNA synthetase fragment is less than 45 kD, more preferably less than 44 kD, 43.9 kD, 43.8 kD, 43.7 kD, 43.6 kD, or more preferably less than 43.5 kD. Preferably such fragments are anti-angiogenic. Such tRNA synthetase fragment may be isolated and/or purified by the methods herein or other methods known in the art.

In some cases a composition of the present invention comprises a tRNA synthetase fragment, wherein the tRNA synthetase fragment comprises, consists essentially of, or alternatively consists of an amino acid sequence selected from the group of SEQ ID NO: 13, 16, 25, 28, 37, 40, 49, 52, and any homologs and analogs thereof. Preferably, such tRNA synthetase fragment does not include a His-tag. Preferably, such tRNA synthetase fragment is less than 48 kD, more preferably less than 47 kD, or more preferably less than 46 kD. Preferably such tRNA synthetase fragment is anti-angiogenic. Such tRNA synthetase fragment may be isolated and/or purified by the methods herein or other methods known in the art.

In some cases, a composition of the present invention comprises a tRNA synthetase fragment, wherein the tRNA synthetase fragment comprises, consists essentially of, or alternatively consists of an amino acid sequence selected from the group of SEQ ID NO: 14, 17, 26, 29, 38, 41, 50, 53, and any homologs and analogs thereof. Preferably, such tRNA synthetase fragment does not include a His-tag. Preferably, such tRNA synthetase fragment is less than 53 kD, more preferably less than 52 kD, more preferably less than 51 kD, more preferably less than 50 kD, or more preferably less than 49 kD. Preferably, such fragments are greater than 43 kD. Preferably such tRNA synthetase fragment is anti-angiogenic. Such tRNA synthetase fragment may be isolated and/or purified by the methods herein or other methods known in the art.

A tRNA synthetase fragment is preferably isolated. Moreover, a tRNA synthetase fragment is preferably purified. Methods for purifying a tRNA synthetase fragment are described in U.S. Provisional Application No. 60/598,019.

In some cases a composition comprising a tRNA synthetase fragment or a tRNA synthetase fragment has an experimental isoelectric point (pl) of less than 10.0, more preferably less than 9.0, or more preferably less than 8.0. In some cases, a tRNA synthetase fragment has an isoelectric point of 5.0 to 9.0, more preferably 6.0 to 8.0, or more preferably 7.4 to 7.8. In some cases a tRNA synthetase fragment of the invention has an experimental pl greater than 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, or 7.5. Preferably, a tRNA synthetase fragment of the invention has experimental pl of about 7.6. In some embodiments, a tRNA synthetase fragment herein has a hydrophobic cleft.

The tRNA synthetase fragments herein may be monomer(s) in a multi-unit complex. A multi-unit complex can include, for example, at least 2, 3, 4, 5, or 6 monomers. Both the monomer and multi-unit complexes may be soluble and may be isolated or purified to homogeneity. A multi-unit complex comprises at least two monomer units that are associated with each other covalently, non- covalently, or both covalently and non-covalently. A multi-unit complex, made of non-covalently bound monomers, can be broken down to individual monomeric units under certain conditions such as high salt concentrations, detergent, and/or heat. Therefore, in order to maintain multi-unit complex formations one should avoid applying denaturants to the product, such as substantial heat, detergent and/or high salt concentrations.

Monomer units in a multi-unit complex may be different, homologous, substantially homologous, or identical to one another. A multi-unit complex includes at least one, two, three, four, five or six monomer units that comprise of, consist essentially of, or consist of a tRNA synthetase fragment herein.

For example, a composition can comprise a dimer, wherein each monomer unit of the dimer is selected from the group consisting of SEQ ID NOS: 12-17, 24-29, 36-41, 48-53, and homologs and analogs thereof. Preferably, a composition comprises a dimer wherein at least one of the two monomers comprises, consists essentially of, or consists of SEQ ID NO: 24. In some embodiments, both monomer units of a dimer comprise, consist essentially of, or consist of SEQ ID NO: 24.

For example, the present invention discloses a dimer having two monomers that are T2 fragments. The present invention discloses a dimer having two monomers comprising, consisting essentially of, or consisting of SEQ ID NO: 12, 15, 24, 27, 36, 39, 48, 51, or any homologs or analogs thereof. The present invention contemplates a dimer having two monomers comprising, consisting essentially of, or consisting of SEQ ID NO: 12, 24, 36, 48 or homologs or analogs thereof. More preferably, a dimer of the present invention comprises, consists essentially of, or consisting of SEQ ID NO: 24, or any homolog or analog thereof. Preferably each monomer unit does not include a His-tag. Optionally such dimer compositions are isolated and/or purified. In some cases such dimer compositions are soluble. In some cases such dimers are homodimers.

Two or more monomers in a multi-unit complex may be covalently linked. Covalently linked monomers can be linked directly (by bonds) or indirectly (e.g., via a linker). For directly linking the monomers herein, it may be beneficial to modify the polypeptides herein to enhance dimerization. For example, one or more amino acid residues of a tRNA synthetase fragment may be modified by the addition or substitution by one or more cysteines. A tRNA synthetase fragment modified is preferably a tryptophanyl tRNA synthetase fragment. Such fragments are preferably mammalian, or more preferably human. Such fragments have angiostatic activity and preferably comprise of, consist essentially of, or consist of a polypeptide having an amino acid sequence selected from the group consisting of SEQ ID NOS: 12-17, 24-29, 36-41, 48-53, and any homologs and analogs thereof. Preferably such amino acid sequence does not include a His-tag. Methods for creating cysteine substitutions, such as by site directed mutagenesis, are known to those skilled in the art.

Preferably, such modification occurs in the dimerization domain of the tRNA synthetase fragment. A dimerization domain refers to that domain which forms covalent and/or non-covalent bonds with a second monomer. For example, the dimerization domain of full length Trp-RS (SEQ ID NO: 1) is between amino acid residues about 230 to about 300, or more preferably between amino acid residues about 237 to about 292. In another example, the dimerization domain for a polypeptide of SEQ ID NO: 13, a T1, is between amino acid residues about 160 to about 230, or more preferably between amino acid residues about 167 to about 222. In another example, the dimerization domain for a polypeptide of SEQ ID NO: 12, 24, 36, or 42, a T2, is between amino acid residues about 137 to about 157, or more preferably between amino acid residues about 144 to about 149. For other angiogenic fragments of a tRNA synthetase, the dimerization region may be any region that is homologous to the above regions or SEQ ID NO: 60.

The addition or substitution of cysteines can create disulfide bridges, linking two or more monomers covalently. Preferably, two or more of the modified polypeptide herein are covalently linked to form a multi-unit (monomer) complex. A multi-unit complex comprises at least two, three, four, five, or six monomers. The various monomers in a multi-unit complex may be different, homologous, substantially homologous, or identical to one another. Optionally two or more of the various monomers in a multi-unit complex are substantially homologous to one another or identical to one another.

Two or more monomers may also be covalently bonded via a linker. A linker of the present invention is preferably long enough to allow the two or more monomer to align in the head-to-tail orientation (N-terminus to C-terminus). In some cases linker is at least about 3, more preferably about 30, more preferably about 150, more preferably about 300, or more preferably about 450 atoms in length. Linker sequences, which are generally between 2 and 25 amino acids in length, are well known in the art and include, but are not limited to, the glycine(4)-serine spacer (GGGGS x3) described by Chaudhary et al. (1989). These and other linkers can be used in the present invention.

In some cases a linker can be used to localize a multi-unit complex. For example, a linker can comprise, consist essentially of, or consist of an antibody fragment or binding agent. In some cases a linker comprises, consists essentially of, or consists of an antibody or antibody fragment or a binding agent that specifically binds to a photoreceptor or another receptor located in the eye.

Examples of non-covalent bonds (associations) include electrostatic bonds, ionic bonds, hydrogen bonds, Van der Waals bonds, and hydrophobic effect.

A polypeptide can be any of the above wherein (i) one or more of the amino acid residues are substituted with a conserved or non-conserved amino acid residue (preferably a conserved amino acid residue) and such substituted amino acid residue is or is not encoded by the genetic code; (ii) one or more of the amino acid residues includes a substituent group; (iii) the polypeptide is fused with another compound, (e.g., a compound to increase the half-life of the polypeptide or target it to a specific receptor, cell, tissue, or organelle), (iv) additional amino acids are fused to the polypeptide, such as a leader or secretory sequence or a sequence which is employed for purification of the polypeptide or a proprotein sequence; or (v) one or more of the amino acid residues are substituted with a non-conserved amino acid residue (preferably cysteine) and such substituted amino acid residue form a disulfide bridge with a second polypeptide (e.g., to form a dimer or homodimer). Such derivatives are deemed to be within the scope of those skilled in the art from the teachings herein.

For example, any of the polypeptides herein can be modified to improve stability and increase potency by means known in the art. For example, L-amino acids can be replaced by D-amino acids, the amino terminus can be acetylated, or the carboxyl terminus modified, e.g., ethylamine-capped (Dawson, D. W., et al., Mol. Pharmacol., 55: 332-338 (1999)) or glycosylated.

In another example, the polypeptides herein can be fused to another protein or portion thereof. For example, mini-TrpRS, T1 or T2 polypeptide or portion thereof, can be operably linked to another polypeptide moiety to enhance solubility. In some cases a polypeptide having an amino acid sequence comprising, consisting essentially of, or consisting of SEQ ID NO: 12-17, 24-29, 36-41, 48-53, and any homologs and analogs thereof is operable linked to another polypeptide moiety to enhance solubility. Preferably such polypeptide does not include a His-tag. Examples of a protein which can be fused with mini-TrpRS, T1 or T2 or portions thereof to enhance solubility include a plasma protein or fragment thereof. In other cases mini-TrpRS, T1 or T2 polypeptide or portion thereof, can be operably linked to another polypeptide moiety to target the molecule to a specific tissue or cell type. For example, mini-TrpRS, T1 or T2 polypeptides or portions thereof, can be operable linked to an antibody that specifically binds the photoreceptor cells in the eye, a particular tumor cell, or a particular organelle. In some cases mini-TrpRS, T1 or T2 polypeptide may be operably linked to a polypeptide moiety that helps reduce immune response, for example, a constant F(c) region of an immunoglobulin.

Optionally the polypeptides herein include a leader sequence. A leader sequence can be used to allow the polypeptide to enter into a specific cell or cell compartment. Thus, the present invention discloses a polypeptide comprising, consisting essentially of, or consisting of SEQ ID NOS: 12-17, 24-29, 36-41, 48-53, and any analogs and homologs thereof having a leader sequence. In some cases such polypeptide does not include a His-tag.

In another example, the polypeptides herein can be modified for enhanced dimerization. Modifications that enhance dimerization of a polypeptide include alternations (e.g., substitutions or additions) to the naturally occurring sequence which enhances covalent and/or non-covalent interactions of the polypeptide with another monomer. Preferably modifications are made within a dimerization domain.

For tryptophanyl-tRNA synthetase and fragments thereof, the dimerization domain is approximately between amino acid residues 230 and 300, or more preferably approximately between amino acid residues 237 and 292 of the full length Trp-tRS (SEQ ID NO: 1). Such polypeptides (preferably mini-TrpRS, T1, and T2) have enhanced dimerization capabilities. Thus, the present invention discloses a mini-TrpRS monomer with a cysteine addition or substitution approximately between amino acid residues 183 and 253, or more preferably approximately between amino acid residues 190 and 245. The present invention discloses a T1 monomer with a cysteine addition or substitution approximately between amino acid residues 160 and 230, or more preferably between amino acid residues 167 and 222. The present invention discloses a T2 monomer with a cysteine addition or substitution approximately between amino acid residue 137 and 208, or more preferably between amino acid residue 144 and 200.

It is further disclosed by the present invention that any of the cysteine modified polypeptides may dimerize to form tRNA synthetase dimers. Preferably such dimerization occurs naturally and/or spontaneously as a result of expressing and/or purifying any of the above polypeptide(s) using a vector that encodes a single tRNA synthetase fragment, and allowing such expressed fragments to naturally dimerize.

Thus, a composition comprises homodimers of preferably identical monomer units. For example, a composition comprises a dimer of two monomers having SEQ ID NO: 12, a dimer of two monomers having SEQ ID NO: 13, a dimer of two monomers having SEQ ID NO: 14, a dimer of two monomers having SEQ ID NO: 15, a dimer of two monomers having SEQ ID NO: 16, a dimer of two monomers having SEQ ID NO: 17, a dimer of two monomers having SEQ ID NO: 24, a dimer of two monomers having SEQ ID NO: 25, a dimer of two monomers having SEQ ID NO: 26, a dimer of two monomers having SEQ ID NO: 27, a dimer of two monomers having SEQ ID NO: 28, a dimer of two monomers having SEQ ID NO: 29, a dimer of two monomers having SEQ ID NO: 36, a dimer of two monomers having SEQ ID NO: 37, a dimer of two monomers having SEQ ID NO: 38, a dimer of two monomers having SEQ ID NO: 39, a dimer of two monomers having SEQ ID NO: 40, a dimer of two monomers having SEQ ID NO: 41, a dimer of two monomers having SEQ ID NO: 48, a dimer of two monomers having SEQ ID NO: 49, a dimer of two monomers having SEQ ID NO: 50, a dimer of two monomers having SEQ ID NO: 51, a dimer of two monomers having SEQ ID NO: 52, or a dimer of two monomers having SEQ ID NO: 53.

A composition herein comprises a combination of any of the above identical homodimers. For example, a composition can comprise a dimer of two monomers having SEQ ID NO: 12 and a dimer of two monomers having SEQ ID NO: 24. All other combinations of the dimers above are also contemplated.

The present invention provides a composition comprising a first tRNA synthetase fragment and a second tRNA synthetase fragment, wherein the first tRNA synthetase fragment has a methionine at its N-terminus ("Met-tRS fragment") and wherein the second tRNA synthetase does not have a methionine at its N-terminus ("non-Met-tRS fragment").

Preferably, the tRNA synthetase fragments herein are tryptophanyl-tRNA synthetase fragments. As such in some embodiments, a first tRNA synthetase fragment having a methionine at its N-terminus is a "Met-TrpRS fragment", and the second tRNA synthetase fragment not having a methionine at its N-terminus is a "non-Met-TrpRS fragment".

Examples of Met-TrpRS fragments, or tryptophanyl tRNA synthetase fragments having a methionine at their N-terminus include polypeptides comprising, consisting essentially of, or consisting of an amino acid sequence SEQ ID NOS: 15-17, 27-29, 39-41, 51-53, or any homologs, analogs, or fragments thereof. Preferably such fragments do not include a His-tag.

Examples of Trp-RS fragments, or tryptophanyl tRNA synthetase fragments that do not have methionine at their N-terminus, include polypeptides comprising, consisting essentially of, or consisting SEQ ID NOS: 12-14, 24-26, 36-38, 48-50, or any homologs, analogs, or fragments thereof. All other angiostatic fragments of Trp-tRNA synthetase are contemplated herein. Preferably, such fragments do not include a His-tag.

In some cases the first tRNA synthetase fragment is a polypeptide having an amino acid sequence comprising, consisting essentially of, or consisting of SEQ ID NO: 15, or any homolog, analog, or fragment thereof. Preferably such fragment does not include a His-tag. The second tRNA synthetase fragment may be a polypeptide having an amino acid sequence comprising, consisting essentially of, or consisting of SEQ ID NO: 12., or any homolog, analog, or fragment thereof. Preferably such fragment does not include a His-tag.

In some cases the first tRNA synthetase fragment is a polypeptide having an amino acid sequence comprising, consisting essentially of, or consisting of SEQ ID NO: 16, or any homolog, analog, or fragment thereof. Preferably such fragment does not include a His-tag. The second tRNA synthetase fragment may be a polypeptide having an amino acid sequence comprising, consisting essentially of, or consisting of SEQ ID NO: 13., or any homolog, analog, or fragment thereof. Preferably such fragment does not include a His-tag.

In some cases the first tRNA synthetase fragment is a polypeptide having an amino acid sequence comprising, consisting essentially of, or consisting of SEQ ID NO: 17, or any homolog, analog, or fragment thereof. Preferably such fragment does not include a His-tag. The second tRNA synthetase fragment may be a polypeptide having an amino acid sequence comprising, consisting essentially of, or consisting of SEQ ID NO: 14., or any homolog, analog, or fragment thereof. Preferably such fragment does not include a His-tag.

In some cases the first tRNA synthetase fragment is a polypeptide having an amino acid sequence comprising, consisting essentially of, or consisting of SEQ ID NO: 27, or any homolog, analog, or fragment thereof. Preferably such fragment does not include a His-tag. The second tRNA synthetase fragment may be a polypeptide having an amino acid sequence comprising, consisting essentially of, or consisting of SEQ ID NO: 24., or any homolog, analog, or fragment thereof. Preferably such fragment does not include a His-tag.

In some cases the first tRNA synthetase fragment is a polypeptide having an amino acid sequence comprising, consisting essentially of, or consisting of SEQ ID NO: 28, or any homolog, analog, or fragment thereof. Preferably such fragment does not include a His-tag. The second tRNA synthetase fragment may be a polypeptide having an amino acid sequence comprising, consisting essentially of, or consisting of SEQ ID NO: 25., or any homolog, analog, or fragment thereof. Preferably such fragment does not include a His-tag.

In some cases the first tRNA synthetase fragment is a polypeptide having an amino acid sequence comprising, consisting essentially of, or consisting of SEQ ID NO: 29, or any homolog, analog, or fragment thereof. Preferably such fragment does not include a His-tag. The second tRNA synthetase fragment may be a polypeptide having an amino acid sequence comprising, consisting essentially of, or consisting of SEQ ID NO: 26., or any homolog, analog, or fragment thereof. Preferably such fragment does not include a His-tag.

In some cases the first tRNA synthetase fragment is a polypeptide having an amino acid sequence comprising, consisting essentially of, or consisting of SEQ ID NO: 39, or any homolog, analog, or fragment thereof. Preferably such fragment does not include a His-tag. The second tRNA synthetase fragment may be a polypeptide having an amino acid sequence comprising, consisting essentially of, or consisting of SEQ ID NO: 36., or any homolog, analog, or fragment thereof. Preferably such fragment does not include a His-tag.

In some cases the first tRNA synthetase fragment is a polypeptide having an amino acid sequence comprising, consisting essentially of, or consisting of SEQ ID NO: 40, or any homolog, analog, or fragment thereof. Preferably such fragment does not include a His-tag. The second tRNA synthetase fragment may be a polypeptide having an amino acid sequence comprising, consisting essentially of, or consisting of SEQ ID NO: 37., or any homolog, analog, or fragment thereof. Preferably such fragment does not include a His-tag.

In some cases the first tRNA synthetase fragment is a polypeptide having an amino acid sequence comprising, consisting essentially of, or consisting of SEQ ID NO: 41, or any homolog, analog, or fragment thereof. Preferably such fragment does not include a His-tag. The second tRNA synthetase fragment may be a polypeptide having an amino acid sequence comprising, consisting essentially of, or consisting of SEQ ID NO: 38., or any homolog, analog, or fragment thereof. Preferably such fragment does not include a His-tag.

In some cases the first tRNA synthetase fragment is a polypeptide having an amino acid sequence comprising, consisting essentially of, or consisting of SEQ ID NO: 51, or any homolog, analog, or fragment thereof. Preferably such fragment does not include a His-tag. The second tRNA synthetase fragment may be a polypeptide having an amino acid sequence comprising, consisting essentially of, or consisting of SEQ ID NO: 48, or any homolog, analog, or fragment thereof. Preferably such fragment does not include a His-tag.

In some cases the first tRNA synthetase fragment is a polypeptide having an amino acid sequence comprising, consisting essentially of, or consisting of SEQ ID NO: 52, or any homolog, analog, or fragment thereof. Preferably such fragment does not include a His-tag. The second tRNA synthetase fragment may be a polypeptide having an amino acid sequence comprising, consisting essentially of, or consisting of SEQ ID NO: 49, or any homolog, analog, or fragment thereof. Preferably such fragment does not include a His-tag.

In some cases the first tRNA synthetase fragment is a polypeptide having an amino acid sequence comprising, consisting essentially of, or consisting of SEQ ID NO: 53, or any homolog, analog, or fragment thereof. Preferably such fragment does not include a His-tag. The second tRNA synthetase fragment may be a polypeptide having an amino acid sequence comprising, consisting essentially of, or consisting of SEQ ID NO: 50., or any homolog, analog, or fragment thereof. Preferably such fragment does not include a His-tag.

In some cases herein which contain a first tRNA synthetase fragment having a methionine at its N-terminus and a second tRNA synthetase fragment not having a methionine at its N-terminus, the first tRNA synthetase fragment can comprise about 5%, 10%, 15%, 20%, 25%, 30%, 35%, 40%, 45%, 50%, 55%, 60%, 65%, 70%, 75%, 80%, 85%, 90%, or 95% by weight of the total amount tRNA synthetase fragments. In other cases the first tRNA synthetase fragment comprises less than about 5%, 10%, 15%, 20%, 25%, 30%, 35%, 40%, 45%, 50%, 55%, 60%, 65%, 70%, 75%, 80%, 85%, 90%, or 95% by weight of the total amount tRNA synthetase fragments.

In some cases herein which contain a first tRNA synthetase fragment having a methionine at its N-terminus and a second tRNA synthetase fragment not having a methionine at its N-terminus, the second tRNA synthetase fragment comprises about 5%, 10%, 15%, 20%, 25%, 30%, 35%, 40%, 45%, 50%, 55%, 60%, 65%, 70%, 75%, 80%, 85%, 90%, or 95% by weight of the total amount tRNA synthetase fragments. In other cases the second tRNA synthetase fragment not having a methionine at its N-terminus comprises at least about 5%, 10%, 15%, 20%, 25%, 30%, 35%, 40%, 45%, 50%, 55%, 60%, 65%, 70%, 75%, 80%, 85%, 90%, or 95% by weight of the total amount tRNA synthetase fragments.

The term "about" as used to describe a percentage by weight of a composition means the percentage by weight +/- 4, 3, 2, or 1 %.

A composition can comprise about 50% by weight of a first tRNA synthetase fragment and about 50% by weight of a second tRNA synthetase fragment. For example, a composition comprises about 50% by weight of a Met-tRS fragment and about 50% by weight of a non-Met-tRS fragment. In some cases a composition comprises about 50% by weight of a Met-TrpRS fragment and about 50% by weight of a non-Met-TrpRS fragment. In other cases more than 50% of a composition comprises either a Met-Trp-RS fragment or a non-Met-Trp-RS fragment. Preferably the fragments above do not include a His-tag.

Any of the above compositions can further comprise a therapeutic agent, such as an antineoplastic agent, an anti-inflammatory agent, an antibacterial agent, an angiogenic agent, an antiviral agent, and an anti-angiogenic agent. Examples of such agents are disclosed herein. Preferably, the therapeutic agent is an anti-angiogenic agent and is either a VEGF antagonist or an integrin antagonist.

### 2. Antibodies

In another aspect, the invention provides a peptide comprising, consisting essentially of, or consisting of an epitope-bearing portion of the polypeptides described herein. The term "epitope" as used herein, refers to a portion of a polypeptide having antigenic or immunogenic activity in an animal, preferably a mammal, and most preferably in a human. Antigenic epitope-bearing peptides of the polypeptides of the invention are useful to raise antibodies, including monoclonal antibodies that bind specifically to a polypeptide of the invention. The term "antigenic epitope," as used herein, is defined as a portion of a protein to which an antibody can specifically bind its antigen as determined by any method well known in the art, for example, by the immunoassays

Antigenic epitope-bearing polypeptides preferably contain a sequence of at least about five or about seven, more preferably at least about nine or about eleven amino acids, and more preferably between at least about 5 to about 30 or more preferably between about 10 to about 20 amino acids contained within a tRNA synthetase fragment, or more preferably a tryptophanyl tRNA synthetase fragment. Such fragments are preferably mammalian, or more preferably human. The tRNA fragments herein have angiostatic activity. Examples of human tryptophanyl tRNA synthetase fragments with angiostatic activity include, but are not limited to SEQ ID NOS: 12-17, 24-29, 36-41, 48-53, and homologs and analogs thereof. In this context "about" includes the particularly recited value and values larger or smaller by several (5, 4, 3, 2, or 1) amino acids.

In some cases such epitope-bearing polypeptides are "N-terminus epitopes." The phrase "N-terminus epitopes" as used herein refer to a peptide having an amino acid sequence that is closer to the N-terminus than the C-terminus of a polypeptide of the invention (e.g., SEQ ID NOS: 12-17, 24-29, 36-41, 48-53, and homologs and analogs thereof). In some cases such epitope-bearing polypeptides comprise or consist of the N-terminus of a polypeptide (e.g., SEQ ID NOS: 12-17, 24-29, 36-41, 48-53, and homologs and analogs thereof).

Examples of such epitope-bearing polypeptides include polypeptide comprising, or alternatively consisting of: amino acid residues of about 1 to about 5, about 1 to about 15, or about 1 to about 25 of SEQ ID NOS: 12-17, 24-29, 36-41, 48-53, and any homologs or analogs thereof; amino acid residues of about 10 to about 15, about 10 to about 25, or about 10 to about 35 of SEQ ID NOS: 12-17, 24-29, 36-41, 48-53, and homologs or analogs thereof; amino acid residues of about 20 to about 25, about 20 to about 35, or about 20 to about 45 of SEQ ID NOS: 12-17, 24-29, 36-41, 48-53 and any homologs and analogs thereof.

The above polypeptides can be used for research purposes (e.g., to distinguish between one fragment and another), for diagnostic purposes (e.g., to identify and quantify angiogenic/angiostatic fragments); and/or for therapeutic purposes (e.g., to inhibit angiostatic activity of an angiostatic tRNA synthetase fragment).

For example, antibodies of the present invention can distinguish between any two of the following: TrpRS, mini-TrpRS, T1, and T2. In some cases antibodies of the present invention can distinguish between a tRNA synthetase fragment having and not having a methionine in its N-terminus. (For example, an antibody can distinguish between SEQ ID NOS: 12 and 15; or between SEQ ID NOS: 13 and 16; or between SEQ ID NOS: 14 and 17; or homologs or analogs thereof.) In some cases antibodies of the present invention can distinguish between two variants of a tRNA synthetase fragment. (For example, an antibody of the present invention may distinguish between two polypeptide selected from the following group: SEQ ID NOS: 12, 24, 36, and 48.)

Other antibodies that bind the dimerization domain or receptor binding domain may also be useful as therapeutics to treat or prevent a condition associated with diminished vascular growth (an anti-angiogenic condition).

Moreover, calibration of the amount of tRNA fragments that are angiogenic and/or non-angiogenic may permit the diagnosis of angiogenesis-mediated condition.

Polynucleotides encoding these antigenic epitope-bearing peptides are also disclosed by the present invention.

Epitope-bearing polypeptides may be used to induce antibodies according to methods well known in the art including, but not limited to, *in vivo* immunization, *in vitro* immunization, and phage display methods.

If *in vivo* immunization is used, animals may be immunized with free peptide; however, anti-peptide antibody titer may be boosted by coupling the peptide to a macromolecular carrier, such as keyhole limpet hemacyanin (KLH) or tetanus toxoid. For instance, peptides containing cysteine residues may be coupled to a carrier using a linker such as maleimidobenzoyl-N-hydroxysuccinimide ester (MBS), while other peptides may be coupled to carriers using a more general linking agent such as glutaraldehyde.

For making a polyclonal antibody, animals such as, for example, rabbits, rats, and mice are immunized with either free or carrier-coupled peptides, for instance, by intraperitoneal and/or intradermal injection of emulsions containing about 100 micrograms of an epitope-bearing peptide and possibly a carrier protein and Freund's adjuvant or any other adjuvant known for stimulating an immune response. Several booster injections may be needed, for instance, at intervals of about two weeks, to provide a useful titer of anti-peptide antibody that can be detected, for example, by ELISA assay using free peptide adsorbed to a solid surface. The titer of anti-peptide antibodies in serum from an immunized animal may be increased by selection of anti-peptide antibodies, for instance, by adsorption to the peptide on a solid support and elution of the selected antibodies according to methods well known in the art.

More preferably, the present invention discloses monoclonal antibodies that are able to specifically bind to one or more of the polypeptides herein. Monoclonal antibodies can be readily prepared through use of well-known techniques such as those exemplified in U.S. Pat. No. 4,196,265. Typically, a technique involves first immunizing a suitable animal with a selected antigen (e.g., a polypeptide or polynucleotide of the present invention) in a manner sufficient to provide an immune response. Rodents such as mice and rats are preferred animals. Spleen cells from the immunized animal are then fused with cells of an immortal myeloma cell. Where the immunized animal is a mouse, a preferred myeloma cell is a murine NS-1 myeloma cell.

The fused spleen/myeloma cells are cultured in a selective medium to select fused spleen/myeloma cells from the parental cells. Fused cells are separated from the mixture of non-fused parental cells, for example, by the addition of agents that block the *de novo* synthesis of nucleotides in the tissue culture media. This culturing provides a population of hybridomas from which specific hybridomas are selected. Typically, selection of hybridomas is performed by culturing the cells by single-clone dilution in microtiter plates, followed by testing the individual clonal supernatants for reactivity with antigen-polypeptides. The selected clones can then be propagated indefinitely to provide the monoclonal antibody. Preferably, a monoclonal antibody is also humanized.

As one of skill in the art will appreciate, and as discussed above, the polypeptides comprising an immunogenic or antigenic epitope can be fused to other polypeptide sequences. For example, the polypeptides may be fused with the constant domain of immunoglobulins (IgA, IgE, IgG, IgM), or portions thereof (CH1, CH2, CH3, or any combination thereof and portions thereof) resulting in chimeric polypeptides. Such fusion proteins may facilitate purification and may increase half-life *in vivo.*

The present invention also discloses fragment, regions or derivatives of the above antibodies. Such fragments include separate heavy chains, light chains, Fab, Fab', F(ab')2, Fabc, and Fv.

### 3. Nucleic Acids

The present invention also provides polynucleotide sequences encoding any of the polypeptides herein. In some cases a polynucleotide sequence encodes two or more of the polypeptides herein. Preferably, the polynucleotide sequences are isolated.

For example, the present invention provides polynucleotide sequences that encode one or more, or two or more tRNA synthetase fragments. The tRNA synthetase fragments can be fragments of any one or more of the tRNA synthetases known in the art, but more preferably either of a tryptophanyl tRNA synthetase or a tyrosyl tRNA synthetase. A tRNA synthetase of the present invention is preferably mammalian, or more preferably human. Furthermore, fragments of such tRNA synthetases preferably have angiostatic activity.

For example, in some cases a polynucleotide sequence encodes one or more angiostatic fragments of a tRNA synthetase. Examples of angiostatic fragments of a tryptophanyl tRNA synthetase include mini-TrpRS, T1, and T2 and any angiostatic fragments, homologs or analogs thereof. Thus, in some cases a polynucleotide encodes a tryptophanyl tRNA synthetase fragment comprising, consisting essentially of, or consisting of a polypeptide selected from the group consisting of SEQ ID NOS: 12-17, 24-29, 36-41, 48-53 and any homologs and analogs thereof. Preferably, a polynucleotide encodies a tryptophanyl fragment comprising, consisting essentially of, or consisting of SEQ ID NO: 24 or 27.

Examples of polynucleotide sequences encoding such fragments are the polynucleotide sequence of SEQ ID NOS: 18-23, 30-35, 42-47, 54-59, and homologs and analogs thereof. Additional examples of isolated polynucleotides include the polynucleotides of SEQ ID NOS: 70-75.

As the DNA code is degenerative, such that more than one codon can encode a single amino acid residue, the above polynucleotide sequences are exemplary and not intended to be limiting in any way. Any of the above polynucleotides are preferably isolated.

In some cases a polynucleotide sequence of the present invention encodes two or more of the polypeptides herein. For example, a polynucleotide of the present invention can encode a first tRNA synthetase fragment and a second tRNA synthetase fragment. The first tRNA synthetase fragment can be a polypeptide having an amino acid comprising, consisting essentially of, or consisting of SEQ ID NOS: 12-17, 24-29, 36-41, 48-53, or homologs or analogs thereof. The second tRNA synthetase fragment can be a polypeptide having an amino acid comprising, consisting essentially of, or consisting of SEQ ID NOS: 12-17, 24-29, 36-41, 48-53, or homologs or analogs thereof. The first and the second tRNA synthetase fragments can be different, homologous, substantially homologous, or identical.

In some cases the nucleotide sequences encoding two or more copies of a polypeptide sequence can be fused in tandem. When two nucleotide sequences encoding polypeptides are fused in tandem each polypeptide can have its own orientation such that when the two nucleotide sequences are expressed the encoded polypeptides can result in a C-N, N-N, C-C, or C-N terminal connection. In preferred cases expression of the nucleotide sequences herein result in the N terminus of the second polypeptide being covalently linked to the C-terminus of the first polypeptide.

In some cases a polynucleotide sequence encoding two or more tRNA synthetase fragments may also encode a linker. A nucleotide sequence encoding a linker can be inserted between two nucleotide sequences tRNA synthetase fragments. A nucleotide sequence encoding a linker can be long enough to allow a first tRNA synthetase fragment and a second tRNA synthetase fragments to productively arrange and dimerize with one another. In some cases a nucleotide sequence encoding a linker is at least 9, at least 30, at least around 60, at least around 90, at least around 120, at least around 150, at least around 180, at least around 210, at least around 240, at least around 270, or at least around 300 nucleotides in length.

In some cases a polynucleotide sequence encoding a first tRNA synthetase fragment can be inserted within a polynucleotide sequence encoding a second tRNA synthetase fragment. This will result in translation of a first segment of the first tRNA synthetase fragment, the complete translation of the second tRNA synthetase fragment, and then translation of the remaining segment of the first tRNA synthetase fragment.

In some cases a polynucleotide sequence herein encodes a modified tRNA synthetase fragment. An example of a modified tRNA synthetase fragment is one wherein the fragment has been modified (e.g., by addition or substitution of amino acids) to insert one or more non-naturally occurring cysteines into the fragment. Preferably, the tRNA synthetase fragment is a tryptophanyl tRNA synthetase fragment, or more preferably a fragment selected from the group consisting of SEQ ID NOS: 12-17, 24-29, 36-41, 48-53, and any homologs or analogs thereof.

Preferably, non-naturally occurring cysteine(s) are inserted (e.g., by addition or substitution) into the dimerization domain of the fragment. The insertion of such a cysteine can be made at the nucleic acid level using recombinant technology. Nucleic acid sequences that can be modified by the following invention to include cysteines include, but are not limited to, SEQ ID NOS: 18-23, 30-35, 42-47, 54-59, and any homologs, and analogs thereof.

In some cases a polynucleotide of the invention encodes two or more modified tRNA synthetase fragments. For example, a polynucleotide can encode 2 or more tryptophanyl tRNA synthetase fragments wherein each fragment is modified to include at least one non-naturally occurring cysteine in its dimerization domain. Examples of tryptophanyl tRNA synthetase fragments that can be modified as follows include, but are not limited to SEQ ID NOS: 12-17, 24-29, 36-41, 48-53, and any homologs or analogs thereof.

Any of the polynucleotides herein are preferably fused in the same reading frame to a polynucleotide sequence which aids in expression and secretion of a polypeptide from a host cell. This results in an expression vector. An expression vector can be used to express the polynucleotides in a host cell.

In some cases a leader sequence which functions as a secretory sequence for controlling transport of a polypeptide from the cell can be fused after the open reading frame sequence. A polypeptide having a leader sequence is a preprotein and can have the leader sequence cleaved by the host cell to form the mature form of the polypeptide. The polynucleotides can also encode for a proprotein which is the mature protein plus additional 5' amino acid residues. A mature protein having a prosequence is a proprotein and is an inactive form of the protein. Once the prosequence is cleaved an active mature protein remains. Thus, for example, the polynucleotide of the present invention can encode for a mature protein, or for a protein having a prosequence or for a protein having both a prosequence and presequence (leader sequence). Preferably, when a polynucleotide sequence of the present invention encodes a prosequence, such prosequence is cleaved in the vitreous of the eye or at a target cancer cell or tumor.

In some cases the pre or pro sequences encode for antibodies or antibody fragments that bind to a target cell (e.g., photoreceptors). Again, the pre or pro sequence can include a protease cleavage site that will allow for the sequence to be automatically cleaved upon reaching its desired site, thus activating the compositions herein.

The polynucleotides can also have the coding sequence fused in frame to a marker sequence which allows for purification of the polypeptide. The marker sequence can be a hexa-histidine tag supplied by a pQE-9 vector to provide for purification of the mature polypeptide fused to the marker in the case of a bacterial host, or, for example, the marker sequence can be a hemagglutinin (HA) tag when a mammalian host, e.g. COS-7 cells, is used. The HA tag corresponds to an epitope derived from the influenza hemagglutinin protein (Wilson, I., et al., Cell, 37:767 (1984)).

The present invention further discloses polynucleotides that hybridize to any of the sequences described herein, preferably under stringent conditions. A stringent condition refers to a condition that allows nucleic acid duplexes to be distinguished based on their degree of mismatch. Such polynucleotides (e.g., antisense and RNAi) can be used to inhibit the expression of an angiostatic tRNA fragment or angiogenic tRNA fragment depending upon the desired outcome. Such polynucleotides can also serve as probes and primers for research and diagnostic purposes.

Antisense nucleic acids are nucleotide sequences which are complementary to the coding strand of a double-stranded cDNA molecule or to an mRNA sequence of a target nucleotide sequence, preferably encoding a positive angiogenesis factor, e.g., VEGF. Antisense nucleic acids can be used as an agent to inhibit angiogenesis in the methods described herein. It inhibits translation by forming hydrogen bonds with a sense nucleic acid. Antisense nucleic acid can be complementary to an entire angiogenic coding region (e.g., VEGF) or only to a portion thereof.

An antisense oligonucleotide herein can be, for example, about 5, 10, 15, 20, 25, 30, 35, 40, 45 or 50 nucleotides in length. An antisense nucleic acid can be constructed using chemical synthesis and enzymatic ligation reactions using procedures known in the art. For example, an antisense nucleic acid (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. Examples of modified nucleotides which can be used to generate the antisense nucleic acid include 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, β-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, β-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (i.e., RNA transcribed from the inserted nucleic acid will be of an antisense orientation to a target nucleic acid of interest).

In some cases double stranded nucleic acids can be used to silence genes associated with angiogenesis (e.g., tryptophanyl tRNA synthetase and/or tyrosyl tRNA synthetase) by RNA interference. RNA interference ("RNAi") is a mechanism of post-transcriptional gene silencing in which double-stranded RNA (dsRNA) corresponding to a gene (or coding region) of interest is introduced into a cell or an organism, resulting in degradation of the corresponding mRNA. The RNAi effect persists for multiple cell divisions before gene expression is regained. RNAi is therefore an extremely powerful method for making targeted knockouts or "knockdowns" at the RNA level. RNAi has proven successful in human cells, including human embryonic kidney and HeLa cells (see, e.g., Elbashir et al. Nature May 24, 2001;411 (6836):494-8).

In one case transfection of small (less than 50, more preferably 40, more preferably 30 or more preferably 20 nucleotides (nt) dsRNA specifically inhibits gene expression (reviewed in Caplen (2002) Trends in Biotechnology 20:49-51). Briefly, RNAi is thought to work as follows. dsRNA corresponding to a portion of a gene to be silenced is introduced into a cell. The dsRNA is digested into small dsRNA nucleotide siRNAs, or short interfering RNAs. The siRNA duplexes bind to a nuclease complex to form what is known as the RNA-induced silencing complex, or RISC. The RISC targets the homologous transcript by base pairing interactions between one of the siRNA strands and the endogenous mRNA. It then cleaves the mRNA at about 12 nucleotides from the 3' terminus of the siRNA (reviewed in Sharp et al (2001) Genes Dev 15: 485-490; and Hammond et al. (2001) Nature Rev Gen 2: 110-119).

RNAi technology in gene silencing utilizes standard molecular biology methods. dsRNA corresponding to the sequence from a target gene to be inactivated can be produced by standard methods, e.g., by simultaneous transcription of both strands of a template DNA (corresponding to the target sequence) with T7 RNA polymerase. Kits for production of dsRNA for use in RNAi are available commercially, e.g., from New England Biolabs, Inc. Methods of transfection of dsRNA or plasmids engineered to make dsRNA are routine in the art.

Gene silencing effects similar to those of RNAi have been reported in mammalian cells with transfection of a mRNA-cDNA hybrid construct (Lin et al., Biochem Biophys Res Commun Mar. 2, 2001 ;281 (3):639-44), providing yet another strategy for gene silencing. In some embodiments, the present invention relates to methods of modulating angiogenesis by contacting a cell or tissue with an RNAi or antisense complementary to a tRNA synthetase (e.g., TyrRS or TrpRS) or a fragment thereof. For example an antisense or RNAi can be complementary to a polynucleotide sequence selected from the group consisting of SEQ ID NOS: 18-23, 30-35, 42-47, 54-60, and any homologs and analogs thereof.

The polynudeotides are preferably provided in an isolated form, and preferably are purified to homogeneity.

### 4. Vectors

The present invention also provides vectors (preferably expression vectors) which include polynucleotides, host cells which are genetically engineered with vectors and the production of polypeptides by recombinant techniques.

The vectors can be constructed using standard recombinant techniques widely available to one skilled in the art. Such techniques can be found in common molecular biology references such as Sambrook, et al., Molecular Cloning: A Laboratory Manual, Cold Spring Harbor Laboratory Press (1989), D. Goeddel, ed., Gene Expression Technology, Methods in Enzymology series, Vol. 185, Academic Press, San Diego, Calif. (1991), and Innis, et al. PCR Protocols: A Guide to Methods and Applications Academic Press, San Diego, Calif. (1990).

The present invention contemplates recombinant construction of a vector which comprises one or more, or more preferably two or more, of the polynucleotide sequences described above. The constructs comprise a vector, such as a plasmid or viral vector, into which one or more, or more preferably two or more, polynucleotide sequence of the invention are inserted, in a forward or reverse orientation. Preferably, two polynucleotide sequences are inserted into a vector in tandem. The polynucleotide sequences can be adjacent to one another or separated by a linker.

### 5. Host Cells

Host cells are cells that express the nucleotide sequences described herein. Representative examples of appropriate hosts include bacterial cells, such as *E. coli, Salmonella typhimurium, Streptomyces;* fungal cells, such as yeast; insect cells, such as Drosophila and Sf9; animal cells such as CHO, COS or Bowes melanoma; plant cells, etc. The selection of an appropriate host is deemed to be within the scope of those skilled in the art from the teachings herein.

There are available to one skilled in the art multiple viral and non-viral methods suitable for introduction such nucleotide sequences into a target host cell.

Viral transduction methods can comprise the use of a recombinant DNA or an RNA virus comprising a nucleic acid sequence that drives or inhibits expression of a protein having sialyltransferase activity to infect a target cell. A suitable DNA virus for use in the present invention includes but is not limited to an adenovirus (Ad), adeno-associated virus (AAV), herpes virus, vaccinia virus or a polio virus. A suitable RNA virus for use in the present invention includes but is not limited to a retrovirus or Sindbis virus. It is to be understood by those skilled in the art that several such DNA and RNA viruses exist that can be suitable for use in the present invention.

"Non-viral" delivery techniques that have been used or proposed for gene therapy include DNA-ligand complexes, adenovirus-ligand-DNA complexes, direct injection of DNA, CaPO₄ precipitation, gene gun techniques, electroporation, liposomes and lipofection. Any of these methods are widely available to one skilled in the art and would be suitable for use in the present invention. Other suitable methods are available to one skilled in the art, and it is to be understood that the present invention can be accomplished using any of the available methods of transfection. Several such methodologies have been utilized by those skilled in the art with varying success. Lipofection can be accomplished by encapsulating an isolated DNA molecule within a liposomal particle and contacting the liposomal particle with the cell membrane of the target cell. Liposomes are self-assembling, colloidal particles in which a lipid bilayer, composed of amphiphilic molecules such as phosphatidyl serine or phosphatidyl choline, encapsulates a portion of the surrounding media such that the lipid bilayer surrounds a hydrophilic interior. Unilammellar or multilammellar liposomes can be constructed such that the interior contains a desired chemical, drug, or, an isolated DNA molecule.

### a. Expression

Expression vectors can be used to express the polynucleotides herein in host cells. Expression vectors contain the appropriate polynucleotide sequences, such as those described herein, as well as an appropriate promoter or control sequence, can be employed to transform an appropriate host to permit the host to express the protein. Preferably an expression vector expresses a polypeptide selected from the group consisting of SEQ ID NOS: 12-17, 24-29, 36-41, 48-53, and any homologs and analogs thereof. A composition may therefore be produced by transfecting a host cell with an expression vector or polynucleotide sequence that encodes a polypeptide comprising, consisting essentially or, or consisting of an amino acid sequence SEQ ID NOS: 12-17, 24-29, 36-41, 48-53, or any homologs or analogs thereof. The host cell is then maintained under a condition which allows the polypeptide or composition to be produced.

In order to obtain transcription of the polynucleotide sequences herein within a host cell, a transcriptional regulatory region capable of driving gene expression in the target cell is utilized. The transcriptional regulatory region can comprise a promoter, enhancer, silencer or repressor element and is functionally associated with a nucleic acid. Preferably, the transcriptional regulatory region drives high level gene expression in the target cell. Transcriptional regulatory regions suitable for use in the present invention include but are not limited to the human cytomegalovirus (CMV) immediate-early enhancer/promoter, the SV40 early enhancer/promoter, the JC polyomavirus promoter, the albumin promoter, PGK and the α-actin promoter coupled to the CMV enhancer, the *E*. *coli lac* or trp promoters, the phage lambda P_{L} promoter and other promoters known to control expression of genes in prokaryotic or eukaryotic cells or their viruses. The expression vector can also contain a ribosome binding site for translation initiation and a transcription terminator.

In addition, the expression vectors may also contain a gene to provide a phenotypic trait for selection of transformed host cells such as dihydrofolate reductase or neomycin resistance for eukaryotic cell culture, or such as tetracycline, kanamycin, or ampicillin resistance in *E. coli.*

In a preferred aspect, the construct further comprises regulatory sequences, including, for example, a promoter, operably linked to the sequence. Large numbers of suitable vectors and promoters are known to those of skill in the art, and are commercially available. The following vectors are provided by way of example: (a) Bacterial: pQE70, pQE-9 (Qiagen), pBs, phagescript, PsiX174, pBluescript SK, pBsKS, pNH8a, pNH16a, pNH18a, pNH46a (Stratagene), pTrc99A, pKK223-3, pKK233-3, pDR540, and PRIT5 (Pharmacia); (b) Eukaryotic: pWLneo, pSV2cat, pOG44, pXT1, pSG (Stratagene) pSVK3, pBPV, PMSG, pSVL (Pharmacia) and pET20B. Optionally the vector is pET24B which is a kanamycin screening vector. However, any other plasmid or vector can be used as long as they are replicable and viable in the host.

Promoter regions can be selected from any desired gene using CAT (chloramphenicol transferase) vectors or other vectors with selectable markers. Two appropriate vectors are pKK232-8 and pCM7. Particular named bacterial promoters include lacI, lacZ, T3, T7, gpt, lambda P_{R}, PL and trp. Eukaryotic promoters include CMV immediate early, HSV thymidine kinase, early and late SV40, LTRs from retrovirus, and mouse metallothionein-1. Selection of the appropriate vector and promoter is well within the level of ordinary skill in the art.

In a further case the present invention provides host cells containing the above-described construct. The host cell can be a higher eukaryotic cell, such as a mammalian cell, or a lower eukaryotic cell, such as a yeast cell, or the host cell can be a prokaryotic cell, such as a bacterial cell. Introduction of the construct into the host cell can be effected by calcium phosphate transfection, DEAE-Dextran mediated transfection, electroporation, viral transfection (e.g., using adenovirus or a retrovirus), as well as other means known in the art. See Davis, L., et al., Basic Methods in Molecular Biology, 1986; see also WO 0/009813.

The constructs in host cells can be used in a conventional manner to produce the polypeptide products encoded by the recombinant sequence. For example, the present invention provides methods for preparing a multi-unit complex that has angiostatic activity. Such method includes the steps of providing an expression vector encoding one or more tRNA synthetase fragments, transfecting a host cell with such expression vector, and maintaining the host cell under conditions suitable for expression. Optionally an expression vector used to transfect a host cell encodes one, two or more tRNA synthetase fragments. More preferably, such tRNA synthetase fragments are tryptophanyl tRNA synthetase fragments. In some cases such fragments are derived from mammalian tRNA synthetase, or more preferably, human tRNA synthetase. In some cases the expression vector encodes a tRNA synthetase fragment selected from the group consisting of SEQ ID NOS: 12-17, 24-29, 36-41, 48-53, and any fragments, homologs, and analogs thereof. In some cases such expression vector encodes a second tRNA synthetase fragment, wherein the second tRNA synthetase fragment is also selected from the group consisting of SEQ ID NOS: 12-17, 24-29, 36-41, 48-53, and any fragments, homologs, and analogs thereof. The two tRNA synthetase fragments can be different, homologous, substantially homologous, or identical.

The present invention also provides that a host cell (e.g., a bacteria) may or may not cleave the Methionine at the N-terminus of any of the polypeptides herein, depending upon the natural processes within the host cell. As such, it is further contemplated that a composition can comprise of a combination of Met- and non-Met-tRNA synthetase fragments. For example, a bacteria transfected with a polynucleotide sequence encoding SEQ ID NO: 15-17, 27-29, 39-41, 51-53, may result in a combination of both Met-tRNA synthetase fragments and non-met tRNA synthetase fragments, all met-tRNA synthetase fragments, or all non-met tRNA synthetase fragments.

Alternatively, the polypeptides can be synthetically produced by conventional peptide synthesizers.

Proteins can be expressed in mammalian cells, yeast, bacteria, or other cells under the control of appropriate promoters. Cell-free translation systems can also be employed to produce such proteins using RNAs derived from the DNA constructs of the present invention. Appropriate cloning and expression vectors for use with prokaryotic and eukaryotic hosts are described by Sambrook. et al., Molecular Cloning: A Laboratory Manual, Second Edition, Cold Spring Harbor, N.Y., (1989).

Transcription of a polynucleotide sequence encoding the polypeptides by higher eukaryotes is increased by inserting an enhancer sequence into the vector. Enhancers are cis-acting elements of DNA, usually about from 10 to about 300 base pairs (bp), that act on a promoter to increase its transcription. Examples include the SV40 enhancer on the late side of the replication origin (bp 100 to 270), a cytomegalovirus early promoter enhancer, a polyoma enhancer on the late side of the replication origin, and adenovirus enhancers.

Generally, recombinant expression vectors will include origins of replication and selectable markers permitting transformation of the host cell, e.g., the ampicillin resistance gene of *E*. *coli,* kanamycin for pET24B, and S. cerevisiae TRP1 gene, and a promoter derived from a highly-expressed gene to direct transcription of a downstream structural sequence. Such promoters can be derived from operons encoding glycolytic enzymes such as 3-phosphoglycerate kinase (PGK), α-factor, acid phosphatase, or heat shock proteins, among others. The heterologous structural sequence is assembled in appropriate phase with translation initiation and termination sequences, and preferably, a leader sequence capable of directing secretion of translated protein into the periplasmic space or extracellular medium. Optionally, the heterologous sequence can encode a fusion protein including an N-terminal identification peptide imparting desired characteristics, e.g., stabilization or simplified purification of expressed recombinant product.

Following transformation of a suitable host strain and growth of the host strain to an appropriate cell density, the selected promoter is derepressed by appropriate means (e.g., temperature shift or chemical induction) and cells are cultured for an additional period.

Cells are typically harvested by centrifugation, disrupted by physical or chemical means, and the resulting crude extract retained for further purification.

Microbial cells employed in expression of proteins can be disrupted by any convenient method, including freeze-thaw cycling, sonication, mechanical disruption, or use of cell lysing agents.

Various mammalian cell culture systems can also be employed to express recombinant protein. Examples of mammalian expression systems include the COS-7 lines of monkey kidney fibroblasts, described by Gluzman, Cell, 23:175 (1981), and other cell lines capable of expressing a compatible vector, for example, the C127, 3T3, CHO, HeLa and BHK cell lines. Mammalian expression vectors will comprise an origin of replication, a suitable promoter and enhancer, and also any necessary ribosome binding sites, polyadenylation site, splice donor and acceptor sites, transcriptional termination sequences, and 5' flanking nontranscribed sequences. DNA sequences derived from the SV40 viral genome, for example, SV40 origin, early promoter, enhancer, splice, and polyadenylation sites can be used to provide the required nontranscribed genetic elements.

Thus, in its most basic form, a polypeptide can be prepared by providing the appropriate expression vector, transfecting a host cell with such expression vector, and maintaining the host cell under a condition suitable for expression. Preferably, expression vectors used herein include at least one nucleotide sequence encoding a tRNA synthetase fragment, or more preferably a tryptophanyl tRNA synthetase fragment, or any homolog or analog thereof. The vector encoding such tryptophanyl tRNA synthetase fragments may be modified to encode one or more non-naturally occurring cysteines in the dimerization domain of the polypeptide. In some embodiments, an expression vector encodes two or more tRNA synthetase fragments, or more preferably two or more tryptophanyl tRNA synthetase fragments. Such vectors preferably encode a linker situated between the first and second fragments.

Polypeptides are recovered and purified from recombinant cell cultures by methods used heretofore, including ammonium sulfate or ethanol precipitation, acid extraction, anion or cation exchange chromatography, phosphocellulose chromatography, hydrophobic interaction chromatography, affinity chromatography, hydroxyapatite chromatography and lectin chromatography. It is preferred to have low concentrations (approximately 0.1-5 mM) of calcium ion present during purification (Price, et al., J. Biol. Chem., 244:917 (1969)). Protein refolding steps can be used, as necessary, in completing configuration of the mature protein. Finally, high performance liquid chromatography (HPLC) can be employed for final purification steps. Additional purifications methods are disclosed herein.

### b. Gene Therapy

The polynucleotides can also be employed as gene therapy by expression of such polypeptide *in vivo.*

Various viral vectors that can be utilized for gene therapy as taught herein include adenovirus, herpes virus, vaccinia, adeno-associated virus (AAV), or, preferably, an RNA virus such as a retrovirus. Preferably, the retroviral vector is a derivative of a murine or avian retrovirus, or is a lentiviral vector. The preferred retroviral vector is a lentiviral vector. Examples of retroviral vectors in which a single foreign gene can be inserted include, but are not limited to: Moloney murine leukemia virus (MoMuLV), Harvey murine sarcoma virus (HaMuSV), murine mammary tumor virus (MuMTV), SIV, BIV, HIV and Rous Sarcoma Virus (RSV). A number of additional retroviral vectors can incorporate multiple genes. All of these vectors can transfer or incorporate a gene for a selectable marker so that transduced cells can be identified and generated. By inserting a zinc finger derived-DNA binding polypeptide sequence of interest into the viral vector, along with another gene that encodes the ligand for a receptor on a specific target cell, for example, the vector is made target specific. Retroviral vectors can be made target specific by inserting, for example, a polynucleotide encoding a protein (dimer). Preferred targeting is accomplished by using an antibody to target the retroviral vector. Those of skill in the art will know of, or can readily ascertain without undue experimentation, specific polynucleotide sequences which can be inserted into the retroviral genome to allow target specific delivery of the retroviral vector containing the zinc finger-nucleotide binding protein polynucleotide.

Since recombinant retroviruses are defective, they require assistance in order to produce infectious vector particles. This assistance can be provided, for example, by using helper cell lines that contain plasmids encoding all of the structural genes of the retrovirus under the control of regulatory sequences within the LTR. These plasmids are missing a nucleotide sequence which enables the packaging mechanism to recognize an RNA transcript for encapsitation. Helper cell lines which have deletions of the packaging signal include but are not limited to PSI.2, PA317 and PA12, for example. These cell lines produce empty virions, since no genome is packaged. If a retroviral vector is introduced into such cells in which the packaging signal is intact, but the structural genes are replaced by other genes of interest, the vector can be packaged and vector virion produced. The vector virions produced by this method can then be used to infect a tissue cell line, such as NIH 3T3 cells, to produce large quantities of chimeric retroviral virions.

### c. Zinc Fingers

Another targeted delivery system for polynucleotides encoding zinc finger derived-DNA binding polypeptides is a colloidal dispersion system. Colloidal dispersion systems include macromolecule complexes, nanocapsules, microspheres, beads, and lipid-based systems including oil-in-water emulsions, micelles, mixed micelles, and liposomes. The preferred colloidal system of this invention is a liposome. Liposomes are artificial membrane vesicles which are useful as delivery vehicles *in vitro* and *in vivo.* It has been shown that large unilamellar vesicles (LUV), which range in size from 0.2-4.0 µm, can encapsulate a substantial percentage of an aqueous buffer containing large macromolecules. RNA, DNA and intact virions can be encapsulated within the aqueous interior and be delivered to cells in a biologically active form (Fraley, et al., Trends Biochem. Sci., 6:77, (1981)).

### d. Targeted Liposomes

Targeted liposomes may be used to delivery the polynucleotides herein. In some cases the polynucleotide sequence is an expression vector as described herein. In order for a liposome to be an efficient gene transfer vehicle, the following characteristics should be present: (1) encapsulation of the genes of interest at high efficiency while not compromising their biological activity; (2) preferential and substantial binding to a target cell in comparison to non-target cells; (3) delivery of the aqueous contents of the vesicle to the target cell cytoplasm at high efficiency; and (4) accurate and effective expression of genetic information (Mannino, et al., Biotechniques, 6:682, (1988)).

The composition of the liposome is usually a combination of phospholipids, particularly high-phase-transition-temperature phospholipids, usually in combination with steroids, especially cholesterol. Other phospholipids or other lipids can also be used. The physical characteristics of liposomes depend on pH, ionic strength, and the presence of divalent cations.

Examples of lipids useful in liposome production include phosphatidyl compounds, such as phosphatidylglycerol, phosphatidylcholine, phosphatidylserine, phosphatidylethanolamine, sphingolipids, cerebrosides, and gangliosides. Particularly useful are diacylphosphatidylglycerols, where the lipid moiety contains from 14-18 carbon atoms, particularly from 16-18 carbon atoms, and is saturated. Illustrative phospholipids include egg phosphatidylcholine, dipalmitoylphosphatidylcholine and distearoylphosphatidylcholine.

The targeting of liposomes has been classified based on anatomical and mechanistic factors. Anatomical classification is based on the level of selectivity, for example, organ-specific, cell-specific, and organelle-specific. Mechanistic targeting can be distinguished based upon whether it is passive or active. Passive targeting utilizes the natural tendency of liposomes to distribute to cells of the reticulo-endothelial system (RES) in organs which contain sinusoidal capillaries. Active targeting, on the other hand, involves alteration of the liposome by coupling the liposome to a specific ligand such as a monoclonal antibody, sugar, glycolipid, or protein, or by changing the composition or size of the liposome in order to achieve targeting to organs and cell types. For example, a targeted liposome delivery system can include antibodies that specifically bind to cancer cells, tumor cells, photoreceptor cells, myocardial tissue, etc.

The surface of the targeted delivery system can be modified in a variety of ways. In the case of a liposomal targeted delivery system, lipid groups can be incorporated into the lipid bilayer of the liposome in order to maintain the targeting ligand in stable association with the liposomal bilayer. Various linking groups can be used for joining the lipid chains to the targeting ligand.

In general, the compounds bound to the surface of the targeted delivery system will be ligands and receptors which will allow the targeted delivery system to find and "home in" on the desired cells. A ligand can be any compound of interest which will bind to another compound, such as a receptor.

In general, surface membrane proteins which bind to specific effector molecules are referred to as receptors. In the present invention, antibodies are preferred receptors. Antibodies can be used to target liposomes to specific cell-surface ligands. For example, certain antigens expressed specifically on tumor cells, referred to as tumor-associated antigens (TAAs), can be exploited for the purpose of targeting antibody-zinc finger-nucleotide binding protein-containing liposomes directly to the malignant tumor. Since the zinc finger-nucleotide binding protein gene product can be indiscriminate with respect to cell type in its action, a targeted delivery system offers a significant improvement over randomly injecting non-specific liposomes. A number of procedures can be used to covalently attach either polyclonal or monoclonal antibodies to a liposome bilayer. Antibody-targeted liposomes can include monoclonal or polyclonal antibodies or fragments thereof such as Fab, or F(ab')₂, as long as they bind efficiently to an the antigenic epitope on the target cells. Liposomes can also be targeted to cells expressing receptors for hormones or other serum factors.

### e. Cell based therapy

Cells transfected with the polynucleotides herein can be administered to a patient. In some cases the cells transfected originate from the patient. In other cases the cells transfected do not originate from the patient. In any event, the cells can be transfected by the constructs herein *in vivo*, *ex vivo,* or *in vitro*. Optionally, the cells transfected are stem cells. Methods for making hematopoietic stem cells are described in PCT/US2003/024839.

### Analogs

The present invention provides methods for screening for analogs for the compositions herein, and in particular, analogs for mini-TrpRS, T1, and T2. The term "analogs" as used herein means compounds that share structure and/or function, such as, for example, peptidomimetics, and any small or large organic or inorganic compounds. In preferred cases an analog of the present invention is a small organic or inorganic compound that mimics the function and structure of mini-TrpRS, T1, or T2, by having similar interactions with their receptor(s).

### 1. Purification

In any of the embodiments herein, and especially for ophthalmic applications, the compositions (e.g., pharmaceutical formulation and/or polypeptides) herein are preferably substantially free of endotoxins.

The levels of endotoxins in a pharmaceutical or polypeptide preparation may be determined by any known technique; such techniques are widespread and commonly used by those of skill in the art in the pharmaceutical and biotechnology fields. For example, the FDA published Good Guidance Practices in February 1997 that noted several methods for quantifying endotoxin levels in a sample, including Limulus Amebocyte Lysate tests using chromagenic, endpoint-turbidimetric and kinetic-turbidimetric techniques. All of these techniques, as well as other techniques (including, but not limited to the use of rabbit pyrogen testing colonies) may be appropriately used to determine the endotoxin levels of the samples described herein.

Thus, for example, a pharmaceutical formulation for systemic administration or topical administration can have a concentration of endotoxins that is preferably, less than about 500, 400, 300, 200, 100, 90, 80, 70, 50, 40, 30, 25, 20, or 15, or more preferably less than about 10, 9, 8, 7, 6, 5, 4, 3, 2, or 1, or more preferably less than about 0.5, 0.1, 0.05, 0.01, 0.005, or 0.001 endotoxin units per milligram of product (e.g., polypeptide).

For other forms of administration e.g., intraocular, via inhalation, via eye drops, vaginal, rectal, etc, a pharmaceutical formulation of the present invention preferably has a concentration of endotoxins that is less than 50, 40, 30, 25, 20, or 15, or more preferably less than about 10, 9, 8, 7, 6, 5, 4, 3, 2, or 1, or more preferably less than about 0.5, 0.1, 0.05, 0.01, 0.005, or 0.001 endotoxin units per milligram of a product (e.g., polypeptide).

The amount of endotoxins in a sample refers to the amount of endotoxins (such as measured in endotoxin units (or E.U.s) in a sample relative to the amount of desired polypeptide or pharmaceutical agent in that sample (generally provided per mg of polypeptide or pharmaceutical agent). The amount of endotoxins can be measured by any of a variety of techniques. However, the particular units employed herein are exemplary only, and are used throughout for reasons of consistency and readability. That is, the methods and materials presented herein are not limited by the particular "units" used to present the amount of endotoxins in a sample. Conversion between various units (by way of example only, E.U./mg of polypeptide to E.U./mL of sample) is considered well within the abilities of one of ordinary skill in the art.

In some embodiments, endotoxin reduction is the last or nearly last step in a purification process. In other embodiments, the endotoxin reduction step occurs at an early stage of the purification process (e.g., prior to steps that may lead to strong and/or irreversible binding of endotoxin to polypeptide).

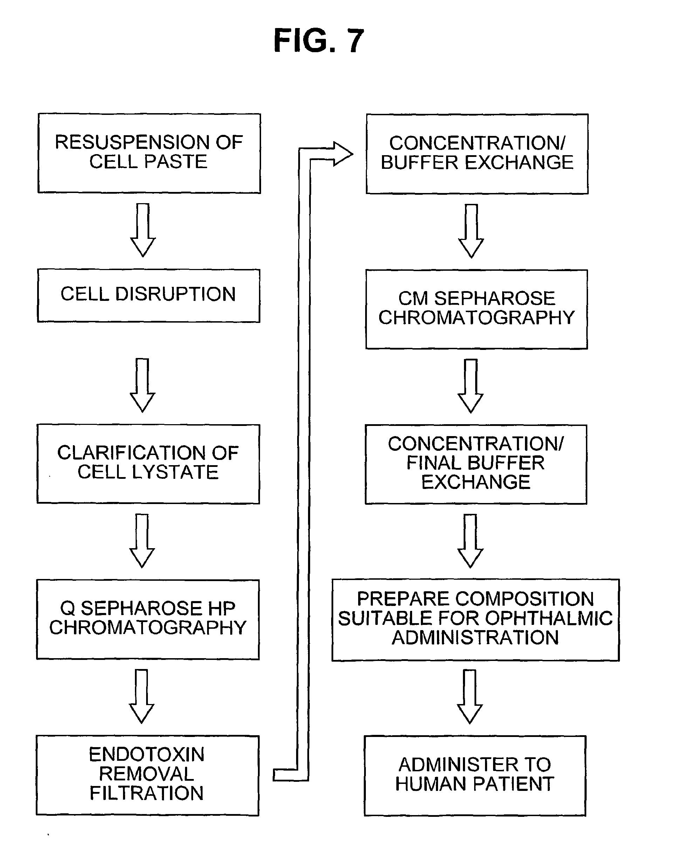

Figure 7 illustrates a flowchart illustrating a sequence of purification steps (each occurring prior to the next) for purifying a pharmaceutical agent and/or polypeptide of the present invention. When a step occurs "prior to" another step, then the first step has been at least partially completed on a particular sample containing a polypeptide before the subsequent step is initiated.

At step 100 a cell paste is formed from cells grown in a fermentor (the cell paste may be properly stored until needed). Next at step 120, the cell paste is resuspended in a buffer. At step 130, the cells are disrupted.

At step 140, cell lysate is clarified. Clarification generally involves removal of insoluble matter (*e.g*., cellular debris, organelles and membranes) in all or in part from a solution containing a polypeptide of interest (e.g., a cell lysate or homogenate). The methods and compositions described herein are not limited by the technique used to produce the cell paste, lysate, or homogenate (or any other analogous term used in the art for the material). Clarification may be achieved by numerous methods known in the art, including by way of example only, simple filtration, centrifugation, dialysis, depth filtration, ultrafiltration using membranes with cut-offs in the vicinity of 100K (in which the desired product is the filtrate and the retentate is discarded), decanting or other appropriate means known to those of skill in the art for such separations. That is, in general, after clarification, one fraction comprises mostly the insoluble portions of a cell, whereas the other fraction comprises mostly the soluble portions of a cell. In another aspect of a clarification step, a slurry becomes a clarified solution. It is of course appreciated by those in the art that endotoxin reduction does arise non-specifically during a clarification step by means of selecting against inclusion of remaining cell membranes (large fragments). However, clarification, by itself, is not designed to provide a polypeptide preparation that is substantially free of endotoxins.

In step 150, anion-chromatography is performed on the clarified cell lysate. This step can include collecting desired eluant fractions. Anion-exchange chromatography refers to the use of a positively charged surface with which a negatively-charged protein can form an ionic interaction. The protein may then be selectively eluted from the positively charged surface by manipulating the salt concentration and/or the pH of the eluting solvent. Examples of positively charged surfaces include anion-exchange resins.

Examples of anion exchange resins include, but are not limited to, diethylaminoethyl- (DEAE-), the quarternary ammonium- (Q- or QAE-), and the Amberlite-based resins. Different resin substrates, sizes (*e.g*., fast flow or FF, Source, or high performance or HP), and pore-diameters for anion-exchange resins are commercially available from standard chemical suppliers and their use is considered within the scope of the methods described herein. Preferably, an anion-exchange resin is selected from the group consisting of Q Sepharose, DEAE Sepharose, and ANX Sepharose. In still a further embodiment, the anion-exchange resin is Q-Sepharose. As is appreciated by those of skill in the art, smaller-sized resins may provide cleaner separation of products, but with a consequent trade-off in the speed with which such products are eluted from the chromatography column. Analyzing such trade-offs in selecting an anion-exchange resin is considered well within the ability of one of ordinary skill in the art. The anion-exhange chromatography is preferably performed prior to the reducing of the levels of endotoxins from the collected eluant.

Step 160 involves reducing the levels of endotoxins from the collected eluant fractions. Such step can remove all, substantially all, or some endotoxins from a sample. This step need not necessarily increase the overall purity of the protein (e.g., T1, T2, mini-trpRS). Techniques for endotoxin reduction include, by way of example, ultrafiltration (e.g., using membranes with cut-offs in the vicinity of 100K in which the desired polypeptide product is in the retentate and the filtrate is discarded); reverse-phase, affinity, size-exclusion, hydrophobic interaction and/or anion-exchange chromatography (e.g., including Q Sepharose); sucrose centrifugation gradients; absorption of endotoxin onto activated charcoal, silica, hydroxyapatite, glass, and/or polystyrene; precipitation with isopropanol, ammonium acetate, or polyethylene glycol; phase-separation techniques using surfactants, such as detergents; use of charged-filter surfaces, and proprietary detoxifying media such as Acticlean Etox^{™}, Prosep-Remtox, Mustang E, and CUNO Zeta Plus ZA. The latter are typically provided in devices through which the polypeptide sample flows. In any of the embodiments herein, filtration-based techniques are preferable over column-based techniques based upon the recovery of product in relation to the reduction in endotoxin levels.

In some embodiments, the level of endotoxins is reduced by using ultrafiltration. Ultrafiltration involves separating all or at least some or at least one desired polypeptide(s) from different-sized molecules and/or molecules having a molecular weight different from the desired polypeptide(s). Ultrafiltration may involve a technique known as tangential flow filtration (as opposed to axial flow filtration). By passing the solution over the membrane in a tangential manner and having the ability to recirculate the solution (also called the retentate), the materials can pass through the membrane in a more gentle manner. The ability to pass through the membrane is determined by two factors: the membrane pore size (also known as the molecular weight cut-off), and the transmembrane pressure (set by the user by means of the pumps and valves). Using various embodiments of this set up, the protein of interest may either pass through the membrane (into the filtrate, this is used in clarification systems) or not pass through the membrane (stays in the retentate, this is used in buffer exchanges and concentration systems). In some embodiments, ultrafiltration is used to filter a liquid medium and small solute molecules through a semipermeable membrane having pores with an average cut-off molecular weight ranging from 100 kDa to 1,000 kDa, 200 kDa to 900 kDa, 300 kDa to 800 kDa, or 400 kDa to 500 kDa. In some embodiments, ultrafiltration is used to filter a liquid medium and small solute molecules through a semipermeable membrane having pores with an average cut-off molecular weight of at least 90 kDa, 100 kDa, 200 kDa, 300 kDa , 400 kDa, 500 kDa, 600 kDa, 700 kDa, 800 kDa, 900 kDa, or 1,000 kDa. Performing an ultrafiltration step may include a dialysis process for separating globular proteins in solution from low-molecular weight solutes. Such a step can utilize a semipermeable membrane to retain protein molecules and allow small solute molecules and water to pass through. Such membranes may have a molecular weight cut-offs ranging, by way of example only, from 1 kDa to 100 kDa, 2 kDa to 90 kDa, 3 kDa to 80 kDa, 4 kDa to 70 kDa, 5 kDa to 60 kDa, or 6 kDa to 50 kDa, 7 kDa to 40 kDa, 8 kDa to 30 kDa, or 9 kDa to 20 kDa. In some embodiments, the molecular weight cut-offs may be less than 90 kDa, 85kDa, 80 kDa, 75 kDa, 70 kDa, 65 kDa, 60 kDa, 55 kDa, 50 kDa, 45 kDa, 40 kDa, 35 kDa, 30 kDa, 25 kDa, 20 kDa, 15 kDa, 10 kDa, 5 kDa, or 1 kDa. Preferably, the molecular weight cut-off for to retain tRNA synthetase molecules and allow small solute molecules and water to pass through is less than 50 kDa, less than 25 kDa, or less than 1 kDa.

In preferred embodiments, the polypeptides purified by the present invention are not modified or denatured during the endotoxin-reduction process. The endotoxin-reduction step is preferably made prior to the buffer exchange step.

In step 170 the filtered eluant fractions are concentrated. Performing a concentration step can result in an increase of concentration of a desired polypeptide or pharmaceutical agent (e.g., any of the polypeptides herein) in the solvent by at least a factor of 2, 3, 4, 5, 6, 7, 8, 9, 10, 20, 30, 40, 50, 100, 200,300, 400, or 500. Preferably such a concentration-increasing process is conducted after a first chromatography step (e.g., anion-exchange and/or cation-exchange chromatography). Generally, a concentration step involves reducing the relative amount of solvent from a sample. Methods for effecting such a concentration step include, but are not limited to, ultrafiltration, evaporation, lyophilization, and precipitation (followed by resolubilization).

A concentration step will typically be performed at least once, 2, 3, 4, 5, or 6 times during the purification of a polypeptide and/or pharmaceutical agent. For example, if the first ion-exchange chromatography step is an anion-exchange chromatography step, then a concentration step may be performed on the amalgamation of eluted fractions containing the desired polypeptide and/or pharmaceutical agent (in this case, also known as the collected polypeptide fractions from the anion-exchange column). Similarly, if the second ion-exchange chromatography column is a cation-exchange chromatography step (also known as a polishing step), then a concentration step may be performed on the amalgamation of eluted fractions containing the desired polypeptide and/or pharmaceutical agent (in this case, the collected polished polypeptide fractions, or the collected polypeptide fractions from the cation-exchange column).

The concentration step(s) can occur either prior to a buffer exchange step or simultaneous to a buffer exchange step.

In step 180, buffer(s) are exchanged in preparation for a cation-exchange chromatography step. Buffer exchange involves changing of a 'solvent' *i.e*., the liquid environment of a polypeptide is changed, in whole or in part. Solvents can include micromolecular solutes (*e.g*. salts) of the medium in which a desired polypeptide is found and/or macromolecule solutes. One suitable technique to perform a buffer exchange is ultrafiltration. Another suitable technique is dialysis of the solution containing the polypeptide against substantially larger quantities of a different buffer. Other buffer exchange techniques include, for example, gel permeation and diafiltration. The buffer exchange step can occur prior to, after, or simultaneously with a concentration step. One example of the latter approach is via the technique known as constant volume diafiltration. A buffer exchange step might be used once or multiple times in purifying a pharmaceutical agent and/or a polypeptide. By way of example only, if a particular polypeptide sample (i.e., T2 produced by recombinantly expressing vector of SEQ ID NO: 70) comprises an amalgamation of samples collected from an anion-exchange column (i.e., an anion-exchange chromatography step), then this polypeptide sample (known herein as a polypeptide sample in a post-anion exchange buffer) may undergo buffer exchange prior to loading the polypeptide sample through a cation-exchange column (i.e., a cation-exchange chromatography step). Another example wherein a buffer exchange step might be advantageously performed on a polypeptide sample is prior to storage of the finished polypeptide sample, but after the polishing step (e.g., the last ion-exchange chromatography step).

In step 190, a cation-exchange chromatography is performed. This step may include collection of desired eluant-fractions. Cation-exchange chromatography refers to the use of a negatively charged surface with which the positively-charged protein can form an ionic interaction. When a cation-exchange chromatography step is performed on a sample that has already undergone an anion-exchange chromatography step, the cation-exchange chromatography step is sometimes referred to as a "polishing step"; the sample loaded onto the cation-exchange column is the unpolished sample and the eluted fractions containing the desired polypeptide sample have been polished and may be referred to as a polished polypeptide sample. The protein may then be selectively eluted from the negatively charged surface by manipulating the salt concentration and/or the pH of the eluting solvent. Examples of negatively charged surfaces include cation-exchange resins.

Examples of cation exchange resins include, by way of example only, carboxymethyl- (CM-) and sulfopropyl- (SP-) based resins. Different resin substrates, sizes (*e.g*., fast flow or FF, Source, or high performance or HP), and pore-diameters for cation-exchange resins are commercially available from standard chemical suppliers and their use is considered within the scope of the methods described herein. As is appreciated by those of skill in the art, smaller-sized resins may provide cleaner separation of products, but with a consequent trade-off in the speed with which such products are eluted from the chromatography column. Analyzing such trade-offs in selecting a cation-exchange resin is considered well within the ability of one of ordinary skill in the art.

Finally, at step 200, the sample is again concentrated and, optionally, buffers are again exchanged. This results in a polypeptide sample that has reduced endotoxin levels. The low-endotoxin preparation may be further formulated in step 210 prior to administration to an organism (e.g., human) in step 220.

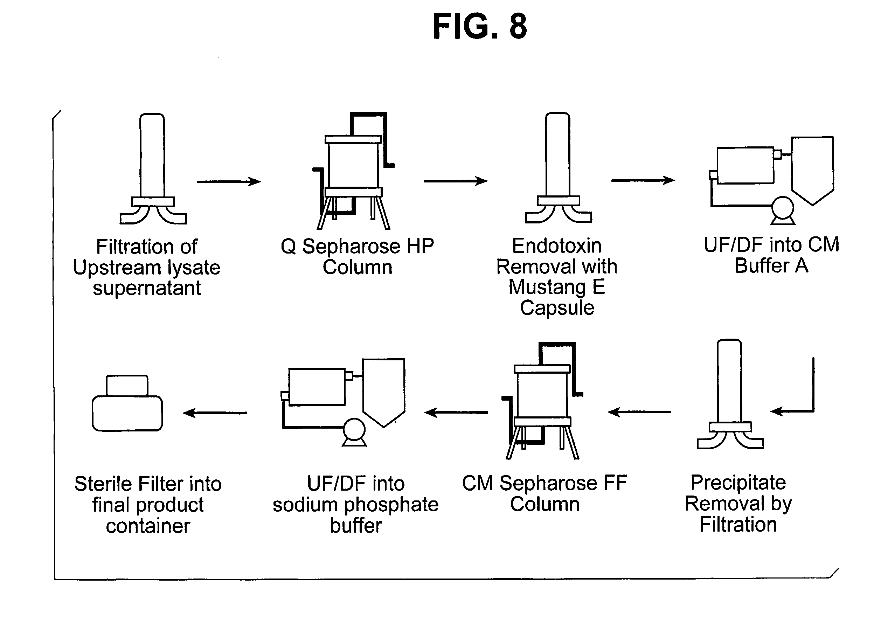

Figure 8 is another illustration of the purification methods disclosed herein.

In some aspects of the methods herein, an endotoxin-reduction filtration step is performed after performing a clarification step and prior to performing a buffer exchange step. Furthermore, the endotoxin-reduction filtration step may be performed prior to performing a cation exchange chromatographic step. Alternatively, the endotoxin-reduction filtration step may be performed prior to performing a concentration step.

In some aspects of the methods herein, an endotoxin-reduction filtration step is performed after performing a clarification step and prior to performing a concentration step. Furthermore, the endotoxin-reduction filtration step may be performed prior to performing a cation exchange chromatographic step. Alternatively, the endotoxin-reduction filtration step may be performed prior to performing a buffer exchange step.

In some aspects of the methods herein, an endotoxin-reduction filtration step is performed after performing a clarification step and prior to performing a cation-exchange chromatographic step. Alternatively, the endotoxin-reduction filtration step may be performed prior to performing a concentration step. Alternatively, the endotoxin-reduction filtration step may be performed prior to performing a buffer exchange step.

In some aspects of the methods herein, an endotoxin-reduction filtration step is performed prior to performing a concentration step and prior to performing a cation-exchange chromatographic step and prior to a buffer exchange step.

The order of the concentration, buffer exchange, and cation-exchange chromatography steps in any of the purification methods herein may vary, but in one embodiment, at least one concentration step is performed prior to the buffer exchange step. Alternatively, a cation-exchange chromatographic step is performed after the buffer exchange step. Alternatively, at least one concentration step is performed prior to the cation-exchange chromatographic step. Alternatively, the cation-exchange chromatographic step is performed after a buffer exchange step and at least one concentration step. Alternatively, at least one concentration step is performed prior to the buffer exchange step and the cation-exchange chromatographic step. And alternatively, an additional concentration step is performed after any buffer exchange step.

In a further embodiment of any of the purification methods herein, the endotoxin-reduction filtration step is performed after an anion-exchange chromatographic step. In a further embodiment, the anion-exchange chromatographic step comprises use of an anion-exchange resin. In yet a further embodiment, the anion-exchange resin is selected from the group consisting of Q Sepharose, DEAE Sepharose, and ANX Sepharose. In still a further embodiment, the anion-exchange resin is Q Sepharose. In any of these uses of anion-exchange resins, a variety of grades and sizes may be used, including, but not limited to Source grade, fast flow grade and high performance grade.

In any of the purification methods herein, ae cation-exchange chromatographic step may comprise use of a cation-exchange resin. In a further embodiment, the cation-exchange resin is selected from the group consisting of CM Sepharose, SP Sepharose, and DEAE Sepharose. In still a further embodiment, the cation exchange resin is CM Sepharose. In any of these uses of cation-exchange resins, a variety of grades and sizes may be used, including, but not limited to Source grade, fast flow grade and high performance grade.

In an alternative aspect, methods for purifying a polypeptide can comprise an anion-exchange chromatographic step, a step comprising a means for reducing endotoxins, and a buffer exchange step, wherein the step comprising a means for reducing endotoxins is performed prior to the buffer exchange step. In a further embodiment, the polypeptide suitable for administration to a patient is suitable for ophthalmic administration. In still a further embodiment, the polypeptide suitable for ophthalmic administration is a modulator of angiogenesis. In yet a further embodiment, the polypeptide suitable for ophthalmic administration can be used to treat macular degeneration, diabetic retinopathy or diseases or conditions associated with unwanted ocular neovascularization. In a further refinement of any of the embodiments noted in this paragraph, the polypeptide is substantially free of endotoxins.

In some embodiments, purification of a polypeptide can comprise an anion-exchange chromatographic step, a step comprising a means for reducing endotoxins, and a buffer exchange step, wherein the step comprising a means for reducing endotoxins is performed prior to the buffer exchange step.

In any of the embodiments herein, a purification step can comprise of a concentration step of collected polished polypeptide fractions, wherein the collected polished polypeptide fractions are substantially free of endotoxins. In a further embodiment are methods of preparing the collected polished polypeptide fractions of the previous embodiment comprising performing a cation-exchange chromatographic step on an unpolished polypeptide sample thereby producing the collected polished polypeptide fractions of the previous embodiment, wherein the unpolished polypeptide sample is substantially free of endotoxins. In further embodiments are methods of producing the unpolished polypeptide sample of the previous embodiment comprising performing a buffer exchange step on a polypeptide sample in a post-anion exchange buffer thereby producing the unpolished polypeptide sample of the previous embodiment, wherein the polypeptide sample in the post-anion exchange buffer is substantially free of endotoxins. In further embodiments are methods of producing the polypeptide sample in the post-anion exchange buffer of the previous embodiment comprising performing a concentration step on collected polypeptide fractions from an anion-exchange column prior to the buffer exchange step thereby producing the polypeptide sample in the post-anion exchange buffer of the previous embodiment, wherein the collected polypeptide fractions from an anion-exchange column are substantially free of endotoxins. In further embodiments are methods of producing the collected polypeptide fractions from an anion-exchange column of the previous embodiment comprising performing an endotoxin-reduction filtration step prior to the concentration step of the previous embodiment. In a further embodiment are methods comprising performing an anion-exchange chromatographic step prior to the endotoxin-reduction filtration step.

The purity of the polypeptide sample may be ascertained before, during and/or after any of the aforementioned steps.

As described above a variety of host-expression vector systems may be utilized to express any of the polypeptide herein (e.g., a tRNA synthetase fragment, such as T1, T2, or miniTrpRS, preferably comprising, consisting essentially of, or consisting of a polypeptide of SEQ ID NO: 12-17, 24-29, 36-41, or 48-53). The expression systems that may be used include but are not limited to microorganisms such as bacteria (*e.g., E. coli,* and *B. subtilis*) transformed with recombinant bacteriophage DNA, plasmid DNA or cosmid DNA expression vectors containing a polynucleotide sequence encoding any of the polypeptide herein at least in part; yeast (*e.g., Saccharomyces,* and Pichia) transfected with recombinant yeast expression vectors containing a polynucleotide sequence encoding any of the polypeptide herein at least in part; insect cell systems infected with recombinant virus expression vectors (*e.g*., baculovirus) containing a polynucleotide sequence encoding any of the polypeptide herein at least in part; plant cell systems infected with recombinant virus expression vectors (*e.g*., cauliflower mosaic virus, CaMV; tobacco mosaic virus, TMV) or transfected with recombinant plasmid expression vectors (*e.g*., Ti plasmid) containing a nucleotide sequence encoding any of the polypeptide herein at least in part; or mammalian cell systems (*e.g*., COS, CHO, BHK, 293, 3T3, U937) harboring recombinant expression constructs containing promoters derived from the genome of mammalian cells (*e.g*., metallothionein promoter) or from mammalian viruses (*e.g*., the adenovirus late promoter; the vaccinia virus 7.5K promoter).

In eukaryotic systems, a number of selection systems may be used, including but not limited to genes such as the herpes simplex virus thymidine kinase (Wilkie et al., 1979, Nucleic Acids Res., 7:859-77), hypoxanthine-guanine phosphoribosyltransferase (Szybalska & Szybalski, 1962, Proc. Natl. Acad. Sci. USA 48:2026), and adenine phosphoribosyltransferase (Lowy et al., 1980, Cell 22:817) that can be employed in tk-, hprt- or aprt-cells, respectively. Also, antimetabolite resistance can be used as the basis of selection. The following genes exemplify this approach: dhfr, which confers resistance to methotrexate (Subramani S, et al., Mol Cell Biol. 1:854-64 (1981); Gasser et al., Proc Natl. Acad. Sci, 1982, 79(21):6522-26 (1982); O'Hare et al., (1981), Proc. Natl. Acad. Sci. USA 78:1527), especially in dhfr cells (Urlaub & Chasin, Proc. Natl. Acad. Sci, (1980), 77(7):4216-4220); gpt, which confers resistance to mycophenolic acid (Mulligan & Berg, (1981), Proc. Natl. Acad. Sci. USA 78:2072); neo, which confers resistance to the aminoglycoside G-418 (Colberre-Garapin et al., (1981), J. Mol. Biol. 150:1); hygro, which confers resistance to hygromycin (Santerre et al., (1984), Gene 30:147); the bar gene, which confers resistance to bialaphos; and D-amino acid oxidase, which confers resistance to D-alanine or D-serine (Erikson et al., Nat Biotechnol., (2004), 22(4):455-58).

In bacterial systems, a number of expression vectors may be selected depending upon the use intended for any of the polypeptide herein or homolog or analogs thereof. Suitable bacteria include, by way of example only, gram positive and gram-negative bacteria. In one embodiment, the polypeptide is expressed in *E. coli* bacteria and subsequently isolated from the cells using the purification methods described herein.

The polypeptide can be expressed in a prokaryotic cell using expression systems known to those of skill in the art of biotechnology. Expression systems useful for the practice our methods and compositions are described in U.S. Pat. Nos. 5,795,745; 5,714,346; 5,637,495; 5,496,713; 5,334,531; 4,634,677; 4,604,359; 4,601,980.

Prokaryotic cells can be grown under a variety of conditions known to the skilled artisan. In one aspect, the cells are grown in a medium suitable for growth of such cells, for example, minimal media or complete (*i.e*., rich) media. Generally, the medium used to grow the cells should not contain concentrations of salts or other chemicals, for example, urea, that are so high as to interfere with the partitioning of the polypeptide or with the formation of phases during the extraction methods.

Any of the polypeptide herein or homologs or analogs thereof may be expressed in transgenic animals. Animals species including, but not limited to, mice, rats, rabbits, guinea pigs, pigs, micro-pigs, goats, and non-human primates, *e.g*., baboons, monkeys, and chimpanzees may be used to generate transgenic animals expressing a transgene encoding any of the polypeptide herein or a homolog or analog thereof. Additionally, any of the polypeptide herein, including by way of example only, T1-TrpRS, T2-TrpRS, and mini-TrpRS may be used in the compositions and methods described herein, may also be expressed in transgenic plants.

The purification methods herein are useful for purifying any of the polypeptide herein from a crude mixture that may be rich in contaminants, such as cell extracts or cellular debris. Cells that express the polypeptide herein can be prepared prior to the purification procedure in a variety of ways. For example, one may prepare a paste of frozen dead cells, or one may use living cells that are frozen, or living cells can be used directly in an extraction procedure.

If the polypeptide herein is purified from cells, the cells are disrupted or homogenized prior to extraction of the polypeptide. The purpose for disrupting or homogenizing the cells is to release the polypeptide herein from the cells. A variety of ways to disrupt or homogenize cells of diverse origin are well known in the art, for example, use of bead mills, osmotic shock, french presses, douncing, sonication, microfluidizing, high-pressure homogenization, and freeze fracture. If the polypeptide is secreted from the cells in which it is synthesized, the cells do not have to be lysed but the polypeptide can be extracted from the extracellular fluid or culture medium, *e.g*., a phase-forming agent may be added directly to the fermentor.

The purification methods described herein may include any techniques for separating the desired pharmaceutical agent or polypeptide from other undesired materials. These techniques include, by way of example only, tangential flow filtration (also known at TFF), depth filtration, ultrafiltration, dialysis, two-phase extractions, decantation, "salting out" techniques, an expanded bed adsorption system, and centrifugation.

In accordance with the compositions and purification methods described herein, the polypeptide can be purified from cells, a cell homogenate, disrupted cells, a crude mixture obtained following chemical synthesis of the polypeptide, or any kind of mixture that contains the polypeptide of interest and contaminants such that purification of the polypeptide is desirable.

Following each purification step, the polypeptide can be detected by a variety of methods including, but not limited to, bioassays, HPLC, amino acid determination or immunological assays, *e.g*., radioimmunoassay, ELISA, Western blot using antibody binding, SDS-PAGE. Such antibodies include but are not limited to polyclonal antibodies, monoclonal antibodies (mAbs), humanized or chimeric antibodies, single chain antibodies, Fab fragments, F(ab')₂ fragments, fragments produced by a Fab expression library, and epitope-binding fragments of any of the above.

The amount of the purified polypeptide and their level of purity can be determined by methods well known in the art. For example, and not by way of limitation, one may examine a polypeptide formulation that was prepared using our purification methods with polyacrylamide gel electrophoresis followed by staining the gel to visualize the total polypeptide in the gel. In one embodiment, the yield and purity of the polypeptide following two-phase extraction are determined using reverse phase HPLC.

The purity of a formulation of a polypeptide prepared using our purification methods may vary depending on the starting material. By way of example only, when purifying a polypeptide that is expressed in *E*. *coli,* the resulting preparation contains at least about 50% by weight of the polypeptide of interest, more preferably at least about 50%, more preferably at least about 70%, more preferably at least about 85% and preferably at least about 95%, preferably at least about 96%, preferably at least about 97%, preferably at least about 98%%, preferably at least about 99%, or more preferably at least about 99.5%.

All polypeptide purification methods known to the skilled artisan may be used for further purification. Such techniques have been extensively described in Berger and Kimmel, Guide to Molecular Cloning Techniques, Methods in Enzymology, Volume 152, Academic Press, San Diego, Calif. (1987); Molecular Cloning: A Laboratory Manual, 2d ed., Sambrook, J., Fritsch, E. F., and Maniatis, T. (1989); Current Protocols in Molecular Biology, John Wiley & Sons, all Viols., (1989), and periodic updates thereof); New Polypeptide Techniques: Methods in Molecular Biology, Walker, J. M., ed., Humana Press, Clifton, N.J., (1988); and Polypeptide Purification: Principles and Practice, 3rd. Ed., Scopes, R. K., Springer-Verlag, New York, N.Y., (1987). Additional methods for further purifying the polypeptide include, but are not limited to ammonium sulfate precipitation, ion exchange, gel filtration, reverse-phase chromatography (and the HPLC or FPLC forms thereof), and hydrophobic interaction chromatography.

### 2. Library Screening

A receptor of any of the compositions herein may be used to screen for agents that can modulate the receptor. Preferably the agent is combined with a library of two or more candidate agents. Candidate agents that bind or interact with the receptor can be selected for further evaluation (e.g., by detecting ability to prevent/treat ocular neovascularization in mice or other mammals, see Examples 3 and 4). Examples of candidate agents include polypeptides (e.g., linear, cyclic, natural amino acids, unnatural amino acids, peptidomimetic compounds, and peptide nucleic acids), nucleic acids, carbohydrates, and small or large organic or inorganic molecules. Such libraries can be generated by a person of ordinary skill in the art and tailored for specific assays.

Candidate agents may be obtained from a wide variety of sources including libraries of synthetic or natural compounds. For example, numerous means are available for random and directed synthesis of a wide variety of organic compounds and bio-molecules, including expression of randomized oligonucleotides. Alternatively, libraries of natural compounds in the form of bacterial, fungal, plant and animal extracts are available or readily produced. Additionally, natural or synthetically produced libraries and compounds are readily modified through conventional chemical, physical and biochemical means. Known pharmacological agents may be subjected to directed or random chemical modifications, such as acylation, alkylation, esterification, or amidification to produce structural analogs.

Agents that bind to the receptor can be then further evaluated for their angiostatic activity using any of the angiogenic assay models disclosed herein or otherwise known in the art. Examples of assays to determine angiogenesis include those described in Example 3 and the Matrigel angiogenesis assay described in Example 4. Agents which have a significant affect on angiogenesis are deemed analogs of the compositions herein.

### 3. Molecular Modeling

The compositions may be modified or new compositions may be designed using computer modeling tools. Once there is confirmation of binding between a ligand (T2 or any of the other homodimers herein) and its receptor(s), modifications of the ligand may allow for increased binding capabilities or rational drug design.

This typically involves solving the crystal structure of the ligand/receptor complex; analyzing the contacts made between the ligand and receptor components; comparing how the ligand would interact with the receptor using computer simulation and the appropriate software; and altering those portions of the ligand that are sterically hindered from or otherwise incompatible with binding to the ligand. The software typically utilized in molecular modeling is capable of achieving each of these steps, as well as suggesting potential replacements for various moieties of the ligand that would increase association with the native second kinase. Preferably, the software can also suggest small organic or inorganic compounds that can be used in lieu of the ligand (e.g., T2) to achieve the same affects.

Preferably, a molecular modeling system is used to analyze the interaction made by a tryptophanyl tRNA synthetase fragment and its receptor. Subsequently tryptophanyl tRNA synthetase fragment may be modified to improve the binding affinities of these two compounds.

One skilled in the art may use one of several methods to screen chemical moieties to replace portions of the ligand so that binding to the native receptor is optimized. This process may begin by side-by-side visual inspection of the ligand and receptor on the computer screen based on the X-ray structure of the two compounds. Modified ligands may then be tested for their ability to dock to the native receptor using software such as DOCK and AUTODOCK followed by energy minimization and molecular dynamics with standard molecular mechanics force fields, such as CHARMM and AMBER.

Other specialized computer programs that may also assist in the process of replacement fragments include the following:
1. GRID (P. J. Goodford, "A Computational Procedure for Determining Energetically Favorable Binding Sites on Biologically Important Macromolecules", J. Med. Chem., 28, pp. 849-857 (1985)). GRID is available from Oxford University, Oxford, UK.
2. MCSS (A. Miranker et al., "Functionality Maps of Binding Sites: A Multiple Copy Simultaneous Search Method." Proteins: Structure, Function and Genetics, 11, pp. 29-34 (1991)). MCSS is available from Molecular Simulations, Burlington, Mass.
3. AUTODOCK (D. S. Goodsell et al., "Automated Docking of Substrates to Proteins by Simulated Annealing", Proteins: Structure, Function. and Genetics, 8, pp. 195-202 (1990)). AUTODOCK is available from Scripps Research Institute, La Jolla, Calif.
4. DOCK (I. D. Kuntz et al., "A Geometric Approach to Macromolecule-Ligand Interactions", J. Mol. Biol., 161, pp. 269-288 (1982)). DOCK is available from University of California, San Francisco, Calif.

Other molecular modeling techniques may also be employed in accordance with this invention. See, e.g., N. C. Cohen et al., "Molecular Modeling Software and Methods for Medicinal Chemistry, J. Med. Chem., 33, pp. 883-894 (1990). See also, M. A. Navia et al., "The Use of Structural Information in Drug Design", Current Opinions in Structural Biology, 2, pp. 202-210 (1992).

Once a compound has been designed or selected by the above methods, the efficiency with which that entity may bind to the receptor may be tested and further optimized by computational evaluation.

An entity designed or selected as binding to the native receptor may be further computationally optimized so that in its bound state it would preferably lack repulsive electrostatic interaction with the target receptor. Such non-complementary (e.g., electrostatic) interactions include repulsive charge-charge, dipole-dipole and charge-dipole interactions. Specifically, the sum of all electrostatic interactions between the ligand and the receptor when ligand is bound to the receptor preferably make a neutral or favorable contribution to the enthalpy of binding.

Specific computer software is available in the art to evaluate compound deformation energy and electrostatic interaction. Examples of programs designed for such uses include: Gaussian 92, revision C [M. J. Frisch, Gaussian, Inc., Pittsburgh, Pa. © 1992]; AMBER, version 4.0 [P. A. Kollman, University of California at San Francisco, © 1994]; QUANTA/CHARMM [Molecular Simulations, Inc., Burlington, Mass. © 1994]; and Insight II/Discover (Biosysm Technologies Inc., San Diego, Calif. © 1994). These programs may be implemented, for instance, using a Silicon Graphics workstation, Indigo₂ or IBM RISC/6000 workstation model 550. Other hardware systems and software packages will be known to those skilled in the art.

Once the modified ligand has been optimally selected or designed, as described above, substitutions may then be made in some of its atoms or side groups in order to improve or modify its binding properties. Generally, initial substitutions are conservative, i.e., the replacement group will have approximately the same size, shape, hydrophobicity and charge as the original group. Such substituted chemical compounds may then be analyzed for efficiency of fit to the receptor by the same computer methods described in detail, above.

### Pharmaceutical Formulations

Any of the compositions and analogs and any salts, prodrugs, or metabolites thereof, can be formulated for administration to an individual by the addition of a pharmaceutically acceptable carrier. Pharmaceutically acceptable salts are non-toxic salts at the concentration at which they are administered. The preparation of such salts can facilitate the pharmacological use by altering the physical-chemical characteristics of the composition without preventing the composition from exerting its physiological effect. Examples of useful alterations in physical properties include lowering the melting point to facilitate transmucosal administration and increasing the solubility to facilitate the administration of higher concentrations of the drug.

Pharmaceutically acceptable salts include acid addition salts such as those containing sulfate, hydrochloride, phosphate, sulfonate, sulfamate, sulfate, acetate, citrate, lactate, tartrate, methanesulfonate, ethanesulfonate, benzenesulfonate, p-toluenesulfonate, cycloexylsulfonate, cyclohexylsulfamate, and quinate. Pharmaceutically acceptable salts can be obtained from acids such as hydrochloric acid, sulfuric acid, phosphoric acid, sulfonic acid, sulfamic acid, acetic acid, citric acid, lactic acid, tartaric acid, malonic acid, methanesulfonic acid, ethanesulfonic acid, benzenesulfonic acid, p-toluenesulfonic acid, cyclohexylsulfonic acid, cyclohexylsulfamic acid, and quinic acid. Such salts may be prepared by, for example, reacting the free acid or base forms of the product with one or more equivalents of the appropriate base or acid in a solvent or medium in which the salt is insoluble, or in a solvent such as water which is then removed in vacuo or by freeze-drying or by exchanging the ions of an existing salt for another ion on a suitable ion exchange resin.

Ophthalmically acceptable carriers are agents that have no persistent detrimental effect on the treated eye or the functioning thereof, or on the general health of the subject being treated. Typically, pharmaceutical formulations for intraocular administrations will be substantially free of detergent and/or preservative, or completely free of detergent and/or preservative.

Useful aqueous suspensions for ophthalmic formulations can contain one or more polymers as suspending agents. Useful polymers include water-soluble polymers such as cellulosic polymers, *e.g*., hydroxypropyl methylcellulose, and water-insoluble polymers such as cross-linked carboxyl-containing polymers. Useful ophthalmic formulations can also comprise of an ophthalmically acceptable mucoadhesive polymer, selected for example from carboxymethylcellulose, carbomer (acrylic acid polymer), poly(methylmethacrylate), polyacrylamide, polycarbophil, acrylic acid/butyl acrylate copolymer, sodium alginate and dextran.

Ophthalmically acceptable solubilizing agent to aid in the solubility of any of the compositions herein include agents that result in the formation of a micellar solution or a true solution of the agent. Certain nonionic surfactants, for example polysorbate 80, can be useful as solubilizing agents, as can glycols, polyglycols, *e.g*., polyethylene glycol 400, and glycol ethers. In general, however, such surfactants and glycols are not used in compositions for intraocular administration except in very low doses because of their potential to cause certain harmful side effects, such as retinal detachment. Accordingly, such surfactants and glycols are preferably not used, or if required, in only small quantities.

Useful ophthalmically acceptable pH adjusting agents or buffering agents include, for example, acids such as acetic, boric, citric, lactic, phosphoric and hydrochloric acids; bases such as sodium hydroxide, sodium phosphate, sodium borate, sodium citrate, sodium acetate, sodium lactate and tris-hydroxymethylaminomethane; and buffers such as citrate/dextrose, sodium bicarbonate and ammonium chloride. Such acids, bases and buffers are included in an amount required to maintain pH of the composition in an ophthalmically acceptable range.

Useful ophthalmically acceptable salts include those having sodium, potassium or ammonium cations and chloride, citrate, ascorbate, borate, phosphate, bicarbonate, sulfate, thiosulfate or bisulfite anions; suitable salts include sodium chloride, potassium chloride, sodium thiosulfate, sodium bisulfite and ammonium sulfate.

Useful ophthalmically acceptable surfactants to enhance physical stability or for other purposes include polyoxyethylene fatty acid glycerides and vegetable oils, *e.g*., polyoxyethylene (60) hydrogenated castor oil; and polyoxyethylene alkylethers and alkylphenyl ethers, *e.g*., octoxynol 10, octoxynol 40.

The ophthalmic pharmaceutical formulations herein may also take the form of a solid article that can be inserted between the eye and eyelid or in the conjunctival sac, where it releases the agent. Release is to the lacrimal fluid that bathes the surface of the cornea, or directly to the cornea itself, with which the solid article is generally in intimate contact. Solid articles suitable for implantation in the eye in such fashion are generally composed primarily of polymers and can be biodegradable or non-biodegradable.

The pharmaceutically acceptable carrier can be one that does not destroy or affect a multi-unit complex of a tRNA synthetase fragment.

The pharmaceutical formulations herein can further include a therapeutic agent selected from the group consisting of: an antineoplastic agent, an anti-inflammatory agent, an antibacterial agent, an antiviral agent, an angiogenic agent, and an anti-angiogenic agent. Examples of such agents are disclosed herein.

For example, an antineoplastic agent may be selected from the group consisting of Acodazole Hydrochloride; Acronine; Adozelesin; Aldesleukin; Altretamine; Ambomycin; Ametantrone Acetate; Aminoglutethimide; Amsacrine; Anastrozole; Anthramycin; Asparaginase; Asperlin ; Azacitidine; Azetepa; Azotomycin; Batimastat; Benzodepa; Bicalutamide; Bisantrene Hydrochloride; Bisnafide Dimesylate; Bizelesin; Bleomycin Sulfate; Brequinar Sodium; Bropirimine; Busulfan; Cactinomycin; Calusterone; Caracemide; Carbetimer; Carboplatin; Carmustine; Carubicin Hydrochloride; Carzelesin; Cedefingol; Chlorambucil; Cirolemycin ; Cisplatin; Cladribine; Crisnatol Mesylate; Cyclophosphamide ; Cytarabine; Dacarbazine; Dactinomycin; Daunorubicin Hydrochloride; Decitabine; Dexormaplatin; Dezaguanine; Dezaguanine Mesylate; Diaziquone; Docetaxel; Doxorubicin; Doxorubicin Hydrochloride; Droloxifene; Droloxifene Citrate; Dromostanolone Propionate; Duazomycin; Edatrexate; Eflornithine Hydrochloride ; Elsamitrucin; Enloplatin; Enpromate; Epipropidine; Epirubicin Hydrochloride; Erbulozole; Esorubicin Hydrochloride; Estramustine; Estramustine Phosphate Sodium; Etanidazole; Ethiodized Oil I 131; Etoposide; Etoposide Phosphate; Etoprine; Fadrozole Hydrochloride; Fazarabine; Fenretinide; Floxuridine; Fludarabine Phosphate; Fluorouracil; Flurocitabine; Fosquidone; Fostriecin Sodium; Gemcitabine; Gemcitabine Hydrochloride; Gold Au 198 ; Hydroxyurea; Idarubicin Hydrochloride; Ifosfamide; Imofosine; Interferon Alfa-2a; Interferon Alfa-2b ; Interferon Alfa-n1; Interferon Alfa-n3; Interferon β-la; Interferon γ-lb; Iproplatin; Irinotecan Hydrochloride; Lanreotide Acetate; Letrozole; Leuprolide Acetate Liarozole Hydrochloride; Lometrexol Sodium; Lomustine; Losoxantrone Hydrochloride; Masoprocol; Maytansine; Mechlorethamine Hydrochloride; Megestrol Acetate; Melengestrol Acetate; Melphalan; Menogaril; Mercaptopurine; Methotrexate; Methotrexate Sodium; Metoprine; Meturedepa; Mitindomide; Mitocarcin; Mitocromin; Mitogillin; Mitomalcin; Mitomycin; Mitosper; Mitotane; Mitoxantrone Hydrochloride; Mycophenolic Acid; Nocodazole; Nogalamycin; Ormaplatin; Oxisuran; Paclitaxel; Pegaspargase; Peliomycin; Pentamustine; Peplomycin Sulfate; Perfosfamide; Pipobroman; Piposulfan; Piroxantrone Hydrochloride; Plicamycin; Plomestane; Porfimer Sodium; Porfiromycin; Prednimustine; Procarbazine Hydrochloride; Puromycin; Puromycin Hydrochloride; Pyrazofurin; Riboprine; Rogletimide; Safingol; Safingol Hydrochloride; Semustine; Simtrazene; Sparfosate Sodium; Sparsomycinl, Spirogermanium Hydrochloride; Spiromustine; Spiroplatin; Streptonigrin; Streptozocin; Strontium Chloride Sr 89; Sulofenur; Talisomycin; Taxane; Taxoid; Tecogalan Sodium; Tegafur; Teloxantrone Hydrochloride; Temoporfin; Teniposide; Teroxirone; Testolactone; Thiamiprine; Thioguanine; Thiotepa; Tiazofurin; Tirapazamine; Topotecan Hydrochloride; Toremifene Citrate; Trestolone Acetate; Triciribine Phosphate; Trimetrexate; Trimetrexate Glucuronate; Triptorelin; Tubulozole Hydrochloride; Uracil Mustard; Uredepa; Vapreotide; Verteporfin; Vinblastine Sulfate; Vincristine Sulfate; Vindesine; Vindesine Sulfate; Vinepidine Sulfate; Vinglycinate Sulfate; Vinleurosine Sulfate; Vinorelbine Tartrate; Vinrosidine Sulfate; Vinzolidine Sulfate; Vorozole; Zeniplatin; Zinostatin; Zorubicin Hydrochloride.

Anti-angiogenic agents are any agents that inhibit angiogenesis, whether disclosed herein or known in the art. In preferred cases an anti-angiogenic agent is an anti-VEGF agent, such as Macugen™ (Eyetech, New York, NY); or anti-VEGF antibody.

Pharmaceutical compositions can be formulated by standard techniques using one or more suitable carriers, excipients, and dilutents. *See, e.g.,* Remington's Pharmaceutical Sciences, (19th Ed. Williams & Wilkins, 1995).

Examples of suitable carriers, excipients and diluents include lactose, dextrose, sucrose, sorbitol, mannitol, starches, gum acacia, calcium phosphate, alginates, calcium silicate, microcrystalline cellulose, polyvinyl pyrrolidine, cellulose, tragacanth, gelatin syrup, methylcellulose, methyl and propyl hydroxybenzoates, talc, magnesium stearate, water and mineral oil. Other additives optionally include lubricating agents, wetting agents, emulsifying and suspending agents. An ophthalmic carrier is preferable in sterile, substantially isotonic aqueous solutions.

The pharmaceutical compositions may be formulated to provide immediate, sustained or delayed release of the compound. For applications providing slow release, certain carriers may be particularly preferred. Suitable slow release carriers may be formulated from dextrose, dextran, polylactic acid, and various cellulose derivatives, for example ethylhydroxycellulose in the form of microcapsules.

Various additives may be added to the formulations herein. Such additives include substances that serve for emulsification, preservation, wetting, improving consistency and so forth and which are conventionally employed in pharmaceutical preparations. Other additives include compounds that have surfactant properties, either ionic or non-ionic such as sorbitan monolaurate triethanolamine oleate, polyoxyethylenesorbitan monopalmitate, dioctyl sodium sulfosuccinate, monothioglycerol, thiosorbitol, ethylenediamine tetra-acetic acid, etc.

For non-ocular indications, an excipient may include a preservative. Suitable preservatives for use in non-ocular pharmaceutical preparations include benzalkonium chloride, benzethonium, phenylethyl alcohol, chlorobutanol, thimerosal and the like. Suitable buffers include boric acid, sodium and potassium bicarbonate, sodium and potassium borates, sodium and potassium carbonate, sodium acetate, sodium biphosphate, Tris, and the like, in amounts sufficient to maintain the pH between about pH 3 and about pH 9.5, most preferably between about pH 7 and pH 7.5. Suitable tonicity agents are dextran 40, dextran 70, dextrose, glycerin, potassium chloride, propylene glycol, sodium chloride and the like, such that the sodium chloride equivalent of the ophthalmic solution is in the range of 0.9 ± 0.2%.

Suitable antioxidant and stabilizers include sodium and potassium bisulfite, sodium and potassium metabisulfite, sodium thiosulfate, thiourea and the like. Suitable wetting and clarifying agents include polysorbate 80, polysorbate 20, poloxamer 282 and tyloxapol. Suitable viscosity increasing agents include dextran 40, gelatin, glycerin, hydroxyethyl cellulose, hydroxymethyl propyl cellulose, lanolin, methylcellulose, petrolatum, polyethylene glycol, polyvinyl alcohol, polyvinyl polyvinylpyrrolidone, carboxymethyl cellulose and the like. Stabilizers such as chelating agents that may be used include, for example, EDTA, EGTA, DTPA, DOTA, ethylene diamine, bipyridine, 1,10-phenanthrolene, crown ethers, aza crown, catechols, dimercaprol, D-penicillamine and deferoxamine. Antioxidants that may also act as stabilizers include such compounds as ascorbic acid, sodium bisulfite, ascorbyl palmitate, butylated hydroxyanisole, butylated hydroxytoluene, potassium metabisulfite and sodium metabisulfite.

Formulations can include capsules, gels, cachets, tablets, effervescent or non-effervescent powders or tablets, powders or granules; as a solution or suspension in aqueous or non-aqueous liquid; or as an oil-in-water liquid emulsion or a water-in-oil emulsion. Capsule or tablets can be easily formulated and can be made easy to swallow or chew. Tablets may contain suitable carriers, binders, lubricants, diluents, disintegrating agents, coloring agents, flavoring agents, flow-inducing agents, or melting agents. A tablet may be made by compression or molding, optionally with one or more additional ingredients. Compressed tables may be prepared by compressing the active ingredient in a free flowing form (e.g., powder, granules) optionally mixed with a binder (e.g., gelatin, hydroxypropylmethylcellulose), lubricant, inert diluent, preservative, disintegrant (e.g., sodium starch glycolate, cross-linked carboxymethyl cellulose) surface-active or dispersing agent. Suitable binders include starch, gelatin, natural sugars such as glucose or β-lactose, com sweeteners, natural and synthetic gums such as acacia, tragacanth, or sodium alginate, carboxymethylcellulose, polyethylene glycol, waxes, or the like. Tablets may optionally be coated or scored and may be formulated so as to provide slow- or controlled-release of the active ingredient. Tablets may also optionally be provided with an enteric coating to provide release in parts of the gut other than the stomach.

Formulations suitable for topical administration (e.g., wound healing) in the mouth wherein the active ingredient is dissolved or suspended in a suitable carrier include lozenges which may comprise the active ingredient in a flavored carrier, usually sucrose and acacia or tragacanth; gelatin, glycerin, or sucrose and acacia; and mouthwashes comprising the active ingredient in a suitable liquid carrier. Topical applications for administration include ointments, cream, suspensions, lotions, powder, solutions, pastes, gels, spray, aerosol or oil. Alternately, a formulation may comprise a transdermal patch or dressing such as a bandage impregnated with an active ingredient and optionally one or more carriers or diluents.

To be administered in the form of a transdermal delivery system, the dosage administration will, of course, be continuous rather than intermittent throughout the dosage regimen. The topical formulations may desirably include a compound that enhances absorption or penetration of the active ingredient through the skin or other affected areas. Examples of such dermal penetration enhancers include dimethylsulfoxide and related analogs.

Formulations suitable for parenteral administration include aqueous and non-aqueous formulations isotonic with the blood of the intended recipient; and aqueous and non-aqueous sterile suspensions which may include suspending systems designed to target the compound to blood components or one or more organs. The formulations may be presented in unit-dose or multi-dose sealed containers, for example, ampoules or vials. For intraocular formulations, unit dosages are preferred because no preservatives are in the formulation. For other parenteral formulations, preservative may be used, which would allow for multi dose containers

Extemporaneous injections solutions and suspensions may be prepared from sterile powders, granules and tablets of the kind previously described. Parenteral and intravenous forms may also include minerals and other materials to make them compatible with the type of injection or delivery system chosen.

Particular parenteral administrations contemplated by the present invention include intraocular and intravitreous administrations to the eye. Pharmaceutical formulations for intraocular and intravitreous administrations include phosphate buffered saline (PBS) and balanced isotonic salt solution (BSS) with or without excipients such as mannitol or sorbitol as protein stabilizers.

In general, water, suitable oil, saline, aqueous dextrose (glucose), or related sugar solutions and glycols such as propylene glycol or polyethylene glycols are suitable carriers for parenteral solutions. Solutions for parenteral administration preferably contain the active ingredient, suitable stabilizing agents and, if necessary, buffer substances. Antioxidizing agents, such as sodium bisulfite, sodium sulfite, or ascorbic acid, either alone or combined, are suitable stabilizing agents. Also used are citric acid salts thereof, or sodium EDTA. In addition, parenteral solutions may contain preservatives, such as benzalkonium chloride, methyl- or propyl-paraben, or chlorobutanol. Suitable pharmaceutical carriers are described in Remington, cited supra.

A composition or pharmaceutical formulation herein may be lyophilized.

The pharmaceutical formulations preferably have less than about 30, 20 or 10, more preferably less than 9, 8, 7, 6, 5, 4, 3, 2, or 1, or more preferably less 0.1, 0.01, or 0.001 endotoxin unit(s) per milligram of therapeutic agents

### Indications

It is disclosed that any of the compositions (including pharmaceutical formulations) herein may be used to modulate angiogenesis in a cell or tissue. Such methods involve contacting the cell or tissue with an appropriate anti-angiogenic (e.g., angiostatic) or angiogenic agent. For example, in some cases a cell or tissue experiencing or susceptible to angiogenesis (e.g., an angiogenic condition) may be contacted with a multi-unit complex of a tRNA synthetase fragment, or a homolog or analog thereof to inhibit an angiogenic condition. In other cases a cell or tissue experiencing or susceptible to insufficient angiogenesis (e.g., an angiostatic condition) may be contacted with an inhibitor of a tRNA synthetase fragment, e.g., an RNAi, antisense nucleic acid, antibody, or other binding agent or agent that interferes with angiostatic activity of a tryptophanyl-tRNA synthetase fragment.

The cells/tissue that may be modulated are preferably mammalian cells, or more preferably human cells. Such cells can be of a healthy state or of a diseased state. In some embodiments, a cancerous cell, tumor cell, or a cell experiencing neovascularization is contacted with a composition of the present invention. In some cases a cell experiencing angiogenesis due to an increase in VEGF, interferon γ, and/or TNF-α is contacted with a composition of the present invention. In one example, a photoreceptor cell is contacted with a multi-unit complex of the present invention.

Angiogenesis can be modulated in a cell or tissue by contacting the cell with a multi-unit complex, such as a dimer, trimer, etc. of the present invention. In preferred cases such multi-unit complex is isolated. Furthermore, a multi-unit complex may be soluble.

When modulating angiogenesis, the rate of angiogenesis may be inhibited by contacting a cell or tissue with an effective amount of a multi-unit complex. An example of the multi-unit complex includes a first monomer and a second monomer. The first and second monomers may be different, homologous, substantially homologous, or identical to each other. Any of the monomers can comprise a tRNA synthetase fragment. A tRNA synthetase fragment can be, for example, a tryptophanyl tRNA synthetase fragment, a human tryptophanyl tRNA synthetase fragment, and/or any angiostatic fragment of a tRNA synthetase. Examples of angiostatic tryptophanyl tRNA synthetase fragments include those selected from the group consisting of SEQ ID NOS: 12-17, 24-29, 36-41, 48-53, and any homologs and analogs thereof.

Units of a multi-unit complex may be covalently linked or non-covalently linked. Covalently linked monomers can be linked by any method disclosed herein, e.g., a linker, a disulfide bond. In some cases, two or more monomers are linked by one or more non-naturally occurring cysteines. Such cysteines are preferably located in a dimerization domain of a monomer. In some cases monomers are linked by a linker. A linker should be long enough to allow two or more monomers the freedom to productively arrange and dimerize with one another.

When modulating angiogenesis, the rate of angiogenesis may be enhanced by contacting a cell or tissue with an effective amount of an inhibitor of a tRNA synthetase fragment that has angiostatic activity. Examples of such inhibitors include, but are not limited to an antibody, an antisense nucleic acid, a RNAi nucleic acid, a peptidomimetic, a peptide nucleic acid, a peptide, and a small or large organic or inorganic molecule. Such inhibitors may function, for example, by competitively binding to a receptor of said tRNA synthetase fragment; binding to the binding site of said tRNA synthetase fragment; binding to said tRNA synthetase fragment and changing its conformation; inhibiting the expression of said tRNA synthetase, and/or inhibiting the cleavage of a full length tRNA synthetase which forms said tRNA synthetase fragment.

The compositions herein can be used to modulate neovascular stabilization and/or maturation. As such the compositions herein can be used to enhance would healing and regulating vascular endothelial cell function.

It is further contemplated that any of the compositions herein may be administered to a patient susceptible to or suffering from a condition associated with increased angiogenesis (vascular formation) ("an angiogenic condition") or a diminished capacity for vascular formation ("an anti-angiogenic condition") (collectively, "angiogenesis-mediated conditions").

Examples of angiogenic conditions that may be treated/prevented by the compositions/methods include, but are not limited to, age-related macular degeneration (AMD), neoplastic condition (both solid tumour and haematological disorders), developmental abnormalities (organogenesis), diabetic blindness, endometriosis, ocular neovascularization, psoriasis, rheumatoid arthritis (RA), treat retinopathy of prematurity (ROP) and skin disclolorations (e.g., hemangioma, nevus flammeus, or nevus simplex).

Examples of anti-angiogenic conditions that may be treated/prevented by the compositions/methods include, but are not limited to, cardiovascular disease (e.g., atherosclerosis (see Moulton, K., PNAS, Vol. 100, No. 8: 4736-4741 (2003)), restenosis (see Brasen JH., Arterioscler. Thromb. Vasc. Biol. Nov;21 (11):1720-6 (2001)), peripheral vascular disease, peripheral arterial disease, tissue damage after reperfusion of ischemic tissue or cardiac failure (see The U. of Tenn., The Vessel, 4(1) (2003)), chronic inflammation, and wound healing.

For example, the present invention discloses methods for treating or preventing conditions associated with ocular neovascularization using any of the compositions/methods herein. Conditions associated with ocular neovascularization include, but are not limited to, diabetic retinopathy, age related macular degeneration ("ARMD"), rubeotic glaucoma, interstitial keratitis, retinopathy of prematurity, ischemic retinopathy (e.g., sickle cell), pathological myopic, ocular histoplasmosis, pterygia, punitiate inner choroidopathy, and the like.

Examples of neoplastic conditions that may be treatable or preventable by the compositions/methods herein include, but are not limited to, breast cancer; skin cancer; bone cancer; prostate cancer; liver cancer; lung cancer; brain cancer; cancer of the larynx; gallbladder; pancreas; rectum; parathyroid; thyroid; adrenal; neural tissue; head and neck; colon; stomach; bronchi; kidneys; basal cell carcinoma; squamous cell carcinoma of both ulcerating and papillary type; metastatic skin carcinoma; osteo sarcoma; Ewing's sarcoma; veticulum cell sarcoma; myeloma; giant cell tumor; small-cell lung tumor; gallstones; islet cell tumor; primary brain tumor; acute and chronic lymphocytic and granulocytic tumors; hairy-cell leukemia; adenoma; hyperplasia; medullary carcinoma; pheochromocytoma; mucosal neuronms; intestinal ganglioneuromas; hyperplastic corneal nerve tumor; marfanoid habitus tumor; Wilm's tumor; seminoma; ovarian tumor; leiomyomater tumor; cervical dysplasia and in situ carcinoma; neuroblastoma; retinoblastoma; soft tissue sarcoma; malignant carcinoid; topical skin lesion; mycosis fungoide; rhabdomyosarcoma; Kaposi's sarcoma; osteogenic and other sarcoma; malignant hypercalcemia; renal cell tumor; polycythemia vera; adenocarcinoma; glioblastoma multiforme; leukemias (including acute myelogenous leukemia); lymphomas; malignant melanomas; epidermoid carcinomas; chronic myeloid lymphoma; gastrointestinal stromal tumors; and melanoma.

Methods include a method for treating an individual suffering from an angiogenic condition by administering to the individual a pharmaceutical formulation comprising a multi-unit complex. A multi-unit complex of the present invention is a complex of 2 or more monomers, 3 or more monomers, 4 or more monomers, 5 or more monomers, or 6 or more monomers.

In some cases a monomer of a multi-unit complex is a tRNA synthetase fragment, or a homolog or an analog thereof. Preferably, the tRNA synthetase fragment is a fragment of tryptophanyl tRNA synthetase (SEQ ID NO: 61-64), or any homologs or derivatives thereof. The tRNA synthetase fragment is preferably a fragment from a mammalian tRNA synthetase, or more preferably human tRNA synthetase. In some cases a monomer of the multi-unit complex is selected from the group consisting of SEQ ID NOS: 12-17, 24-29, 36-41, and 48-53. A first monomer and a second monomer of the multi-unit complex can be different, homologous, substantially homologous, or identical. In preferred embodiments, a multi-unit complex is a dimer (with homologous or substantially homologous monomers), or more preferably a homodimer (with identical monomers).

The two or more monomers in a multi-unit complex may be covalently linked, non-covalently associated, or both.

It is further contemplated herein that the compositions herein can specifically interact with at least one angiogenic receptor. An angiogenic receptor is any cell surface receptor that can mediate angiogenesis (including abnormal developmental growth, tumorgenesis, lymphogenesis, and vasculogenesis). Angiogenic receptors are preferably located on an endothelium cell, or more preferably vascular endothelium cell. In some cases the compositions herein are used to modulate an angiogenic receptor or to treat an angiogenic-receptor mediated condition.

Known angiogenic receptors include, but are not limited to, growth factor receptors of VEGF, IGF, EGF, PDGF and FGF. Other preferred angiogenic receptors include cell adhesion molecules as described below. Angiogenic receptors also include CXC-receptors or chemokine receptors. Examples of CXC receptors include, but are not limited to, the group consisting of, IL8RA, IL8RB, IL8RBP, CXCR3, CXCR4, BLR1, and CXCR6. Examples of chemokine receptors include, but are not limited to, the group consisting of CCR1-CCR9, GPR2, CCRL1-CCRL2, and FPRL1.

The methods of treatment disclosed herein further include administering to an individual suffering from an angiogenic condition one or more therapeutic agents selected from the group consisting of antineoplastic agents, antiviral agents, anti-inflammatory agents, antibacterial agents, anti-angiogenic agents, or anti-angiogenic agents.

Such combination treatments can be achieved by either administering to an individual a co-formulating of the compositions herein with the additional therapeutic agent(s) or by administering the compositions herein and the therapeutic agent(s) as two separate pharmaceutical formulations. In embodiments wherein more than one composition/therapeutic agent is administered to an individual, lower dosages of the compositions and/or therapeutic agent(s) may be utilized as a result of the synergistic effect of both active ingredients.

Examples of antineoplastic agents are provided herein and are known in the art.

Antibacterial agents that may be administered to an individual include, but are not limited to, penicillins, aminoglycosides, macrolides, monobactams, rifamycins, tetracyclines, chloramphenicol, clindamycin, lincomycin, imipenem, fusidic acid, novobiocin, fosfomycin, fusidate sodium, neomycin, polymyxin, capreomycin, colistimethate, colistin, gramicidin, minocycline, doxycycline, vanomycin, bacitracin, kanamycin, gentamycin, erythromycin and cephalosporins.

Anti-inflammatory agents that may be administered to an individual include, but are not limited to, NSAIDS (*e.g*., aspirin (salicylamide), sodium salicylamide, indoprofen, indomethacin, sodium indomethacin trihydrate, Bayer™, Buffering™, Celebrex™, diclofenac, Ecotrin™ , diflunisal, fenoprofen, naproxen, sulindac, Vioxx™), corticosteroids or corticotropin (ACTH), colchicine, and anecortave acetate.

Antiviral agents that may be administered to an individual include, but are not limited to, α-methyl-P-adamantane methylamine, 1,-D-ribofuranosyl-1,2,4-triazole-3 carboxamide, 9-[2-hydroxy-ethoxy]methylguanine, adamantanamine, 5-iodo-2'-deoxyuridine, trifluorothymidine, interferon, adenine arabinoside, CD4, 3'-azido-3'-deoxythymidine (AZT), 9-(2-hydroxyethoxymethyl)-guanine (acyclovir), phosphonoformic acid, 1-adamantanamine, peptide T, and 2',3'dideoxycytidine.

Angiogenic agents that may be administered to an individual include, but are not limited to, Angiogenin, Angiopoietin-1, Del-1, Fibroblast growth factors: acidic (aFGF) and basic (bFGF), Follistatin, Granulocyte colony-stimulating factor (G-CSF), Hepatocyte growth factor (HGF) /scatter factor (SF), Interleukin-8 (IL-8), Leptin, Midkine, Placental growth factor, Platelet-derived endothelial cell growth factor (PD-ECGF), Platelet-derived growth factor-BB (PDGF-BB), Pleiotrophin (PTN), Progranulin, Proliferin, Transforming growth factor-α (TGF-α), Transforming growth factor-β (TGF-β), Tumor necrosis factor-α (TNF-α), and Vascular endothelial growth factor (VEGF)/vascular permeability factor (VPF).

Anti-angiogenic agents that may be administered to an individual include antagonists of angiogenic material. The term "antagonists of angiogenic material" is used herein to refer to any molecule that inhibiting the biological activity of an angiogenic material. Examples of antagonists of angiogenic material include, but are not limited to, antibodies that specifically bind the angiogenic material, iRNA that inhibit translation of the angiogenic material, and other agents that bind/interfere with the biological activity of the angiogenic material.
Examples of angiogenic materials include but are not limited to: (1) growth factors and their receptors; (2) remodeling and morphogenic receptors and their ligands; (3) adhesion receptors and their ligands; (4) matrix-degrading enzymes, such as Matrix-Metalo Proteinases (MMPs); (5) signaling molecules, such as Raf and MAPK, PKA, Rhos-family GTPases, PKB; and (6) transcription factors and regulators (e.g., hypoxia inducible factor (HIF)-1, Id 1/3, and Nuclear Factor-B) and homobox gene products (e.g., Hox D3, and B3).

In some cases the angiogenic material is a growth factor and/or its receptor. Examples of growth factors receptors include VEGF receptors (e.g., soluble VEGFR1, VEGFR1 (Flt-1), VEGFR2 (Flk-1), and VEGFR3 (Flt-4)) and their ligands (e.g., VEGF A, B, C, and D). Thus, in some embodiments, an anti-angiogenic agent is an antagonist to a VEGF receptor, such as VEGFR1, VEGFR2, VEGFR3, or an antagonist to a VEGF ligand, such as VEGFA, VEGFB, VEGFC, or VEGFD. In some embodiments, an anti-angiogenic agent is antagonist to a VEGF ligand (e.g., VEGFA-VEGFD). More preferably, an anti-angiogenic agent is antagonist to VEGFA. Examples of anti-VEGF, anti-angiogenic agents include Avastin (Genentech, Inc.), Macugen (EyeTech Pharmaceuticals, Inc.) or Visudyne (Novartis, Crop.) and anti-VEGF monoclonal antibody M293. Additional examples of anti-VEGF anti-angiogenic agents are disclosed in U.S. Pat. Nos. 5,730,977, 6,383,484, 6,403,088, 6,479,654, 6,559,126, and 6,676,941.

Additional examples of growth factors and their receptors include, but are not limited to, angiogenin, angiopoietin-1, Del-1, fibroblast growth factors ("FGF") and FGFR (including acidic aFGF and basic bFGF), follistatin, granulocyte colony-stimulating factor (G-CSF), hepatocyte growth factor (HGF), Interleukin-8 (IL-8), leptin, midkine, placental growth factor, platelet-derived endothelial growth factor (PD-ECGF), platelet-derived growth factor-BB (PDFG-BB), pleiotrophin (PTN), progranulin, proliferin, transforming growth factor (TGF)-α, TGF-β, and tumor necrosis factor (TNF)-α.

In some cases an anti-angiogenic agent is an antagonist of a remodeling and morphogenic receptor and/or ligand. Examples of remodeling and morphogenic receptors and ligands include, but are not limited to, the Tie receptors (e.g., Tie1 and Tie2) and their ligands (e.g., ANG-1, ANG-2, and ANG-3/4), as well as the Ephrin receptors (e.g., EphB1, EphB2, EphB3, EphB4, EphB6, EphA4) and their ligands (e.g., ephrin B1, B2, and B3).

In some cases an anti-angiogenic agent is an antagonist of an adhesion receptor and/or its ligand. Examples of adhesion receptors and their ligands include, but are not limited to, the integrins, cadherins, semophorins, and fibronectin. There are eighteen α and eight β mammalian subunits which assemble to form 24 different heterodimers of integrin receptors. In some cases an antagonist of an adhesion receptor is an antagonist of a vascular integrin receptor selected from the group consisting of α1β1, α2β1, α3β1, α4β1, α5β1, α6β1, α8β1, α9β1, α Vβ1, αVβ3, αVβ5, α6β4, and αVβ8. In more preferred cases an antagonist of an adhesion receptor is an antagonist of a vascular integrin receptor selected from the group consisting of α1β1, α2β1, α5β1, and αVβ3. In more preferred cases an antagonist of an adhesion receptor is an antagonist of αVβ3.

Peptide and antibody antagonists of this integrin inhibit angiogenesis by selectively inducing apoptosis of the proliferating vascular endothelial cells. Integrin antibodies are commercially available from, e.g., Chemicon Internation, Biocompare, Soretec, etc.

Two cytokine-dependent pathways of angiogenesis exist and can be defined by their dependency on distinct vascular cell integrins, αVβ3 and αVβ5. Specifically, basic FGF- and VEGF-induced angiogenesis depend on integrin αVβ3 and αVβ5, respectively, since antibody antagonists of each integrin selectively block one of these angiogenic pathways in the rabbit corneal and chick chorioallantoic membrane (CAM) models. Peptide antagonists that block all αV integrins inhibit FGF-and VEGF-stimulated angiogenesis. While normal human ocular blood vessels do not display either integrin, αVβ3 and αVβ5 integrins are selectively displayed on blood vessels in tissues from patients with active neovascular eye disease. While only αVβ3 was consistently observed in tissue from patients with ARMD, αVβ3 and αVβ5 both were present in tissues from patients with PDR. Systemically administered peptide antagonists of integrins blocked new blood vessel formation in a mouse model of retinal vasculogenesis.

There are many different types of cadherins. The most extensively studied group of cadherins is known as the classical, or type I, cadherins. Cadherins that contain calcium binding motifs within extracellular domain cadherin repeats, but do not contain an HAV CAR sequence, are considered to be nonclassical cadherins. To date, nine groups of nonclassical cadherins have been identified (types II-X). These cadherins are membrane glycoproteins. Type II, or atypical, cadherins include OB-cadherin, also known as cadherin-11 (Getsios et al., Developmental Dynamics 211:238-247, (1998)); cadherin-5, also known as VE-cadherin (Navarro et al., J. Cell Biology 140:1475-1484 (1998)); cadherin-6, also known as K-cadherin (Shimoyama et al., Cancer Research 55:2206-2211 (1995)); cadherin-7 (Nakagawa et al., Development 121:1321-1332 (1995); cadherin-8 (Suzuki et al., Cell Regulation 2:261-270 (1991)), cadherin-12, also known as Br-cadherin (Tanihara et al., Cell Adhesion and Communication 2:15-26, (1994)); cadherin-14 (Shibata et al., J. Biological Chemistry 272:5236-5240 (1997)), cadherin-15, also known as M-cadherin (Shimoyama et al., J. Biological Chemistry 273:10011-10018 (1998)), and PB-cadherin (Sugimoto et al., J. Biological Chemistry 271:11548-11556 (1996)). For a general review of atypical cadherins, see Redies and Takeichi, Developmental Biology 180:413-423 (1996) and Suzuki et al., Cell Regulation 2:261-270 (1991).

Additional examples of angiogenic receptors include neuropillins (e.g., neuropillin -1 and neuropillin-2), endoglin, PDFGβR, CXCR-4, Tissue Factor ("TF"), thrombin receptor, Gα₁₃, and EP3. It has been suggested that T-2 also binds to neuropillin-1 and 2, see, *e.g.,* International Appl. No. PCT/US02/23868, having publication No. WO 03/009813. Thus, the present invention discloses methods for identifying other binding partners that can specifically interact with and/or bind tRS, or more preferably T2. Such methods include the use of a yeast two hybrid system, a phage display library system, screening peptide libraries, computer imaging programs, and the like.

Anti-angiogenic agents can include nucleic acids, polypeptides, peptidomimetics, PNAs, antibodies, fragments of antibodies, small or large organic or inorganic nucleic acids that bind to angiogenesis associated molecules.

Other known anti-angiogenic agents that are found in the body include, but are not limited to, angioarrestin, angiostatin (plasminogen fragment), antiangiogenic antithrombin III, cartilage-derived inhibitor (CDI), CD59 complement fragment, endostatin (collagen XVIII fragment), fibronectin fragment, Gro-β, heparinases, heparin hexasaccharide fragment, human chorionic gonadotropin (hCG), interferon α/β/γ, interferon inducible protein (IP-10), interleukin-12, kringle 5 (plasminogen fragment), metalloproteinase inhibitors (TIMPs), 2-methoxyestradiol, placental ribonuclease inhibitor, plasminogen activator inhibitor, platelet factor-4 (PF4), prolactin 16 kDa fragment, proligerin-related protein (PRP), retinoids, tetrahydrocortisol-S, thrombosponrin-1 (TSP-1), transforming growth factor-β, vasculostatin, vasostatin (calreticulin fragment).

### Administration

Administration of a composition to a target cell *in vivo* can be accomplished using any of a variety of techniques well known to those skilled in the art.

For example, compositions can be administered systemically or locally by any means known in the art (e.g., orally, intraocularly, intravascularly (i.v.), intradermally, intramuscularly, transdermally, transmucosally, enterically, parentally, by inhalation spray, rectally, or topically) in dosage unit formulations and containing conventional pharmaceutically acceptable carriers, adjuvants, and vehicles.

For purposes of this invention the term "ophthalmic administration" encompasses, but is not limited to, intraocular injection, subretinal injection, intravitreal injection, periocular administration, subconjuctival injections, retrobulbar injections, intracameral injections (including into the anterior or vitreous chamber), sub-Tenon's injections or implants, ophthalmic solutions, ophthalmic suspensions, ophthalmic ointments, ocular implants and ocular inserts, intraocular solutions, use of iontophoresis, incorporation in surgical irrigating solutions, and packs (by way of example only, a saturated cotton pledget inserted in the fornix).

As used herein the term parenteral includes subcutaneous, intravenous, intramuscular, intrasternal, infusion techniques or intraperitoneal injections. Suppositories for rectal administration of the drug can be prepared by mixing the drug with a suitable non-irritating excipient such as cocoa butter and polyethylene glycols that are solid at ordinary temperatures but liquid at the rectal temperature and will therefore melt in the rectum and release the drug.

The dosage regimen for treating a disorder or a disease with the vectors and/or compositions is based on a variety of factors, including the type of disease, the age, weight, sex, medical condition of the patient, the severity of the condition, the route of administration, and the particular compound employed. Thus, the dosage regimen can vary widely, but can be determined routinely using standard methods.

For systemic administration, the polypeptides (preferably dimers or homodimers) and/or small molecules are preferably administered at a dose of at least 0.05, 0.1, 0.2, 0.3, 0.4, 0.5, 0.6, 0.7, 0.8, 0.9, 1.0, 2.0, 3.0, 4.0, 5.0, 6.0, 7.0, 8.0, 9.0, 10, 20, 30, 40, 50, 75, 100, or 150 mg/kg body weight. In other cases the polypeptides (preferably dimers or homodimers) and/or small molecules herein are administered systemically at a dose of 0.1-100 mg/kg, more preferably 0.5-50 mg/kg, more preferably 1-30 mg/kg body weight, or more preferably 5-20 mg/kg.

For localized administration, the polypeptides (preferably dimers or homodimers) and/or small molecules are preferably administered at a dose of at least 50 µg, 100 µg, 150 µg, 200 µg, 250 µg, 300 µg, 350 µg, 400 µg, 450 µg, 500 µg, 550 µg, 600 µg, 650 µg, or 700 µg. In other embodiments, the polypeptides (preferably dimers or homodimers) and/or small molecules herein are administered locally at a dose of 50-1000 µg, more preferably 100-800 µg, more preferably 200-500 µg, or more preferably 300-400 µg per site. In other cases the polypeptides (preferably dimers or homodimers) and/or small molecules herein are administered locally at a dose of at less than 1000 µg, 900 µg, 800 µg, 700 µg, 600 µg, 500 µg, 400 µg, 300 µg, 200 µg, 100 µg, 50 µg, 25 µg, 10 µg, or 5 µg per site.

For example, for dermal administration the polypeptides (e.g., dimers) and/or peptidomimetics and/or small molecules are administered at a dose of 50-1000 µg/cm², more preferably 100-800 µg/cm², or more preferably 200-500 µg/cm². In another example, for ocular administration, the polypeptides (e.g., dimers) and/or peptidomimetics and/or small molecules of the present invention are administered at a dose of 50-1000 µg/eye, more preferably 100-800 µg/eye, or more preferably 200-500 µg/eye.

The pharmaceutical compositions preferably include the active ingredient (e.g., T2) in an effective amount, i.e., in an amount effective to achieve therapeutic or prophylactic benefit. The actual amount effective for a particular application will depend on the condition being treated and the route of administration. Determination of an effective amount is well within the capabilities of those skilled in the art, especially in light of the disclosure herein.

Preferably, the effective amount of the active ingredient, e.g., T2, is from about 0.0001 mg to about 500 mg active agent per kilogram body weight of a patient, more preferably from about 0.001 to about 250 mg active agent per kilogram body weight of the patient, still more preferably from about 0.01 mg to about 100 mg active agent per kilogram body weight of the patient, yet still more preferably from about 0.5 mg to about 50 mg active agent per kilogram body weight of the patient, and most preferably from about 1 mg to about 15 mg active agent per kilogram body weight of the patient.

In terms of weight percentage, the formulations will preferably comprise the active agent, e.g., T2-TrpRS, in an amount of from about 0.0001 to about 10 wt. %, more preferably from about 0.001 to about 1 wt. %, more preferably from about 0.05 to about 1 wt. %, or more preferably about 0.1 wt. to about 0.5 wt. %. In some ophthalmic formulations, the composition herein is formulated between 0.01-1000 mg/mL, 0.1-100 mg/mL, 1-10 mg/mL, 2-10 mg/mL, 2-9 mg/mL, 3-9 mg/mL, 4-8 mg/mL, 5-8 mg/mL, 5-7 mg/mL, or 6-7 mg/mL. For systemic formulations, the compositions herein can be formulated between 0.001 - 100 mg/mL, 0.01-10 mg/mL, 0.1-10 mg/mL, 2-10 mg/mL, 2-9 mg/mL, 3-9 mg/mL, 4-8 mg/mL, 5-8 mg/mL, 5-7 mg/mL, or 6-7 mg/mL.

### Screening/Diagnosis

A cell or tissue may be screened for an angiogenesis mediated condition (e.g., an anti-angiogenic condition or an angiogenic condition). This can be accomplished by any technology known in the art. For example, tagged probes, tagged probes described in WO 2004/011900 may be used to identify and/or quantify angiostatic and/or angiogenic tRNA synthetase fragments in a sample. Generally, such tagged probes include a binding moiety that is specific to a tRNA synthetase fragment (e.g., miniTrp-RS, T1, or T2), a detectable reporter (such as a fluorescent group), and optionally a mobility modifier. The mobility modifier and detectable reporter are linked to the binding moiety by a cleavable linker. The binding moiety can be, for example, an antibody specific to a tRNA synthetase fragment disclosed herein (e.g., a polypeptide selected from SEQ ID NOS: 12-17, 24-29, 36-41, 48-53, and any homologs and analogs thereof.

After binding the target agent, the cleavable tags can be cleaved and separated according to their mobility. More than one tagged probe may be used simultaneously to determine the angiogenic state of a cell/tissue/organism.

In some cases a patient may be diagnosed or screened for one or more conditions associated with angiogenesis (an angiogenesis mediated condition) prior to or subsequent a treatment. For example, an individual may be screened for a condition selected from the group consisting of adiposity, cardiovascular diseases, restenosis, cancer, chronic inflammation, tissue damage after reperfusion, neurodegeneration, rheumatoid arthritis, Crohn's disease, Alzheimer's disease, Parkinson's disease, diabetes, endometriosis, psoriasis, failure in wound healing, and ocular neovascularization. If a patient is diagnosed as having such a condition or being susceptible to such a condition, a therapeutically effective amount of the compositions herein may be administered to the patient. Similarly, a patient may be monitored after a therapeutic treatment is administered to see if additional treatments are required.

Methods for diagnosing or screening patients for conditions are known in the art and include detection of single nucleotide polymorphisms (SNPs) or alleles that are associated with resistance or susceptibility to such conditions. Preferably such diagnosis is made using a microarray device. Examples of SNPs that may be used to detect/diagnose an individual with an ocular neovascular condition (or susceptibility thereof) are disclosed in U.S. Patent No. 6,713,300. Additional SNPs related to angiogenesis-mediated conditions can be identified on the dbSNP database maintained by NCBI at <http://www.ncbi.nlm.nih.gov>.

### Business Methods

The invention herein also discloses business methods by providing therapeutics and/or diagnostics for treating individuals suffering from or susceptible to angiogenic conditions. In some cases a business method contemplates searching for an agent that modulates or binds to a receptor of tRNA synthetase fragment and commercializing such an agent. A tRNA synthetase fragment is preferably a tryptophanyl tRNA synthetase fragment. The tryptophanyl tRNA synthetase fragments herein are preferably mammalian, or more preferably human. Examples of human tryptophanyl tRNA synthetase fragments include polypeptide that comprise, consist essentially of, or consist of SEQ ID NOS: 12-17, 24-29, 36-41, 48-53, homologs and analogs thereof. Preferably a tRNA synthetase fragment herein is angiostatic. In some cases the step of searching for an agent that modulates or binds to a receptor of tRNA synthetase fragment involves using a computer programs to generate peptidomimetics of the tRNA synthetase fragment. In some cases the step of searching involves screening a library of candidate agents to identify an agent that modulates or binds to the receptor. There are various forms of libraries available for screening candidate agents. Such libraries include peptide libraries, and small molecule libraries, as well as others disclosed herein or known in the art.

The present invention also provides a business method that includes the steps of modifying a tRNA synthetase fragment to enhance its dimerization capabilities and commercializing the enhanced fragment or dimer form thereof. Again, the tRNA synthetase fragment can be tryptophanyl tRNA synthetase fragment, or more preferably a fragment that are polypeptides comprising, consisting essentially of, or consisting of SEQ ID NOS: 12-17, 24-29, 36-41, 48-53, homologs and analogs thereof. In some cases such business methods contemplate the use of a computer program to optimize the tRNA synthetase fragments herein. Examples of computer programs that can be used to optimize a ligand include, but are not limited to GRID, MCSS, AUTODOCK, DOCK, AMBER, QUANTA, and INSIGHT II. In other cases the business methods herein contemplate generating an expression vector that encodes a tRNA synthetase fragment modified to include one or more non-naturally occurring cysteines. Preferably, such modifications occur in the dimerization domain of the fragment. In other cases the business methods herein contemplate generating an expression vector that encodes two tRNA synthetase fragments. Such vectors can also encode a linker that is preferably situated between the two fragments.

The business methods herein also contemplate commercializing fragments of a tRNA synthetase that modulate angiogenesis. In some cases such fragments may inhibit angiogenesis (e.g., angiostatic fragments of a tRNA synthetase). In other cases such fragments may enhance angiogenesis (.e.g., inhibitors of angiostatic fragments of a tRNA synthetase). Preferably, a business method contemplates commercializing compositions that can be used to modulate angiogenesis. Such compositions can be any of the compositions described by the present invention. Preferably, such compositions comprise a first tRNA synthetase fragment having a methionine at its N-terminus and a second tRNA synthetase fragment not having a methionine at its N-terminus. The methionine can be naturally occurring or non-naturally occurring. Examples of a first tRNA synthetase fragment having a methionine at its N-terminus include, but are not limited to, SEQ ID NOS: 15-17, 27-29, 39-41, 51-53, and any homologs, analogs, or fragments thereof. Examples of a second tRNA synthetase fragment not having a methionine at its N-terminus include, but are not limited to, SEQ ID NOS: 12-14, 24-26, 36-38, 48-50, and any homologs, analogs, or fragments thereof. In some cases the compositions herein include about 50% by weight of a tRNA synthetase fragment having a methionine at its N-terminus and about 50% by weight of a tRNA synthetase fragment not having a methionine at its N-terminus. Preferably, such compositions are isolated and/or purified. Such tRNA synthetase may under appropriate conditions form dimers.

Optionnally the present invention relates to a business method which includes the steps of expressing an expression vector encoding a tRNA synthetase fragment and commercializing said fragment for modulating angiogenesis. A tRNA synthetase fragment can be, for example, a tryptophanyl tRNA synthetase fragment, a human tRNA synthetase fragment, or any angiostatic fragment of a tRNA synthetase. Examples of such fragments include but are not limited to SEQ ID NOS: 12-17, 24-29, 36-41, 48-53, and any homologs and analogs thereof.

In some cases the fragments commercialized are part of a multi-unit complex. A multi-unit complex of the present invention can include two or more monomer units covalently bound or non-covalently associated.

In some cases the expression vector also encodes a second tRNA synthetase fragment. The first tRNA synthetase fragment and the second tRNA synthetase fragment can be different, homologous, substantially homologous, or identical. Moreover, in some embodiments, the first tRNA synthetase fragment and the second tRNA synthetase fragment are modified to include at least one non-naturally occurring cysteine. Such non-naturally occurring cysteine is preferably situated in the dimerization domain of the tRNA synthetase fragments.

An expression vector encoding two or more tRNA synthetase fragments can have the two or more fragments aligned in tandem. In some cases the expression vector can also encode a linker. The polynucleotide sequence encoding the linker can be situated between the sequence encoding the first and the sequence encoding the second tRNA synthetase fragments. A linker of the present invention is preferably sufficiently long to allow said first and said second tRNA synthetase fragments to free rotate and dimerize.

The fragments and multi-unit complexes herein can be prepared by tranfecting a host cell with the expression vectors disclosed herein, and maintaining the host cell under a condition that permits the expression of the one or more tRNA synthetase fragments.

The business methods herein also contemplate commercializing diagnostics for detection of angiogenesis-mediated conditions (e.g., either an angiostatic or angiogenic condition).

For example, a diagnostic may be commercialized to detect an angiogenic condition, such as an ocular neovascularization condition or AMD, either independently or in combination with an angiostatic composition disclosed herein (e.g., an angiostatic fragment of a tRNA synthetase, more preferably an angiostatic fragment of a tryptophanyl tRNA synthetase, or more preferably mini-trpRS, T1 and/or T2). Examples of genetic variations and diagnostics that may be used to detect ocular neovascularization conditions include those disclosed in U.S. Patent No. 6,713,300.

In another example, a diagnostic may be commercialized to detect an anti-angiogenic condition, such as a cardiovascular disease, either independently or in combination with an angiogenic composition disclosed herein (e.g., an inhibitor of an angiostatic fragment of a tRNA synthetase, such as a tryptophanyl tRNA synthetase, e.g., mini-trpRS, T1 and/or T2).

In some cases a diagnostic is used to measure the amount of a composition of the present invention (e.g., mini-TrpRS, T1, or T2) in a patient or an organism. Such data can be used for pharmacokinetic or pharmacodynamic studies. Detection of the composition herein can be made using methods such as ELISA, HPLC, and/or any of the antibodies herein. The amount or level of a composition in a patient or organism can subsequently be used to determine if additional treatment should be administered.

Herein further contemplated is the step of partnering with a third party partner to commercialize the compositions and/or diagnostics herein. Examples of partners can include biotech partners, pharmaceutical partners, consumer products partners, agricultural partners, scientific partners, government partners, etc.

In some cases partners can provide funding or research capabilities to, for example, discover analogs of the compositions herein, discover receptors for the compositions herein, optimize the compositions, run clinical trials on the compositions herein, develop inhibitors for the compositions herein, etc.

### Kits

The invention also provides a kit comprising one or more containers filled with one or more of the compositions herein. The kits can include written instructions on how to use such compositions (e.g., to modulate angiogenesis or treat a patient suffering from an angiogenic condition).

In one case a kit comprises a container wherein the container comprises one or more of the compositions herein. Examples of compositions that may be in a container include: a composition comprising an isolated tRNA synthetase fragment having an amino acid sequence comprising, consisting essentially of, or consisting of SEQ ID NOS: 12-17, 24-29, 36-41, 48-53 and any homologs and analogs thereof. Preferably, such tRNA synthetase fragment does not include a His-tag. Moreover, if a tRNA synthetase fragment comprises, consists essentially of, or consists of SEQ ID NOS: 12, 15, 24, 27, 36, 39, 48, 51 or any homologs or analogs thereof, then such tRNA synthetase fragment is preferable less than 45 kD, more preferably less than 44 kD, 43.9 kD, 43.8 kD, 43.7 kD, 43.6 kD, or more preferably less than 43.5 kD. If a tRNA synthetase fragment comprises, consists essentially of, or consists of SEQ ID NOS: 13, 16, 25, 28, 37, 40, 49, 52, or any homologs and analogs thereof, then such tRNA synthetase fragment is preferably less than 48 kD, more preferably less than 47 kD, or more preferably less than 46 kD. If a tRNA synthetase fragment comprises, consists essentially of, or consists of SEQ ID NOS: SEQ ID NO: 14, 17, 26, 29, 38, 41, 50, 53, or any homologs or analogs thereof, then such tRNA synthetase fragment is preferably less than 53 kD, more preferably less than 52 kD, more preferably less than 51 kD, more preferably less than 50 kD, or more preferably less than 49 kD. Preferably a tRNA synthetase fragment in a container is purified.

In some cases a kit comprises a container comprising a multi-unit complex, wherein at least one unit of the multi-unit complex comprises a tRNA synthetase, fragment or a homolog or analog thereof. A multi-unit complex can be, for example, a dimer having two units. Monomers of a multi-unit complex can be different from each other, homologous, substantially homologous, or identical. In some cases a multi-unit complex is a dimer having two homologous monomers.

In some cases a kit includes a container comprising a first tRNA synthetase fragment and a second tRNA synthetase fragment, wherein the first tRNA synthetase fragment has a methionine at its N-terminus. Preferably, such tRNA synthetase fragments are tryptophanyl tRNA synthetase fragments. More preferably, the first tRNA synthetase fragment has an amino acid sequence comprising, consisting essentially of, or consisting of SEQ ID NOS: 15-17, 27-29, 39-41, 51-53, or any homologs, analogs, or fragments thereof. Preferably, such tRNA synthetase fragments do not include a His-tag.

The second tRNA synthetase fragment may or may not have a methionine at its N-terminus. Examples of tRNA synthetase fragments that do not have a methionine at their N-terminus include polypeptide having an amino acid sequence comprising, consisting essentially of, or consisting of SEQ ID NOS: 12-14, 24-26, 36-38, 48-50, or any homologs, analogs, or fragments thereof. Preferably, such tRNA synthetase fragments do not include a His-tag.

In some cases the first and second tRNA synthetase fragments are about 50% by weight of the composition. Other ratios of a first and a second tRNA synthetase fragments may also be utilized.

A tRNA-synthetase fragment can be a tryptophanyl tRNA synthetase fragment, a human tryptophanyl tRNA-synthetase, and/or any angiostatic fragment of a tRNA synthetase fragment. Such fragments may further form multi-unit complexes that may be covalently or non-covalently linked.

The composition in the first container may be packaged for systemic administration or local administration. Preferably, the compositions are packaged in single unit dosages. When packaged in single unit dosages, a dose may range between 50-1000 µg/dose.

The kit herein may also include a second therapeutic agent. Such second therapeutic agent may be contained in a second container. Examples of a second therapeutic agent include, but are not limited to an antineoplastic agent, an anti-inflammatory agent, an antibacterial agent, an antiviral agent, an angiogenic agent, and an anti-angiogenic agent. In preferred cases a second therapeutic agent is an anti-angiogenic agent.

In any of the kits herein, a composition comprising a tRNA synthetase fragment may have an experimental pl greater than 7.1, 7.2, 7.3, 7.4 or 7.5.

A kit can include a container comprising an antibody that specifically binds to an epitope of a tRNA synthetase fragment and written instructions for use thereof. In such examples, the tRNA synthetase fragment can be a tryptophanyl tRNA synthetase fragment, a human tRNA synthetase fragment, and/or any angiostatic fragment of a tRNA synthetase. In some embodiments, an angiostatic tRNA synthetase fragment is one selected from the group consisting of SEQ ID NOS: 12-17, 24-29, 36-41, 48-53, and any homologs and analogs thereof.

The kits herein can also include one or more syringes or other delivery devices (e.g., stents, implantable depots, etc.). The kits can also include a set of written instructions for use thereof.

### EXAMPLES

### Example 1

### Preparation of Endotoxin-free Recombinant TrpRS

Endotoxin-free recombinant human TrpRS (GD and SY variants) were prepared as follows: Plasmids encoding full-length TrpRS (amino acid residues 1-471 of SEQ ID NO: 1 and the SY variant thereof), or truncated TrpRS, hereinafter referred to as T2 (SEQ ID NO: 12 (GD variant) or SEQ ID NO: 24 (SY variant)), consisting essentially of residues 94-471 of full length TrpRS and a second truncated TrpRS fragment, hereinafter referred to as T1 (SEQ ID NO: 13 (GD variant) or SEQ ID NO: 25 (SY variant)), consisting essentially of residues 71-471 of full length TrpRS were prepared.
Each plasmid also encoded a C-terminal tag consisting six histidine residues (e.g. amino acid residues 472-484 of SEQ ID NO: 1), and an initial methionine residue. The His₆-tagged T1 (SEQ ID NOS: 13 and 25) had the amino acid sequence of SEQ ID NO: 5 (or SY variant thereof), whereas the His₆-tagged T2 has the amino acid sequence of SEQ ID NO: 7 (or SY variant thereof).

The above plasmids containing SY and GD variants of T2 were introduced into *E*. *coli* strain BL 21 (DE 3) (Novagen, Madison, Wis.). Human mature EMAPII, also encoding a C-terminal tag of six histidine residues, was similarly prepared for use. Overexpression of recombinant TrpRS was induced by treating the cells with isopropyl β-D-thiogalactopyranoside for 4 hours. Cells were then lysed and the proteins from the supernatant purified on HIS-BIND® nickel affinity columns (Novagen™ ) according to the manufacturer's suggested protocol. Following purification, TrpRS proteins were incubated with phosphate-buffered saline (PBS) containing 1 µM ZnSO₄ and then free Zn²+ was removed (Kisselev et al., Eur. J. Biochem. 120:511-17 (1981)).

Endotoxin was removed from protein samples by phase separation using Triton X-114 (Liu et al., Clin. Biochem. 30:455-63 (1997)). Protein samples were determined to contain less than 0.01 units of endotoxin per mL using an E-TOXATE® gel-clot assay (Sigma, St. Louis, Mo.). Protein concentration was determined by the Bradford assay (Bio-Rad, Hercules, Calif.) using bovine serum albumin (BSA) as a standard.

### Example 2

### Cleavage of Human TrpRS by PMN Elastase

Cleavage of human full-length TrpRS by PMN elastase was examined. TrpRS was treated with PMN elastase in PBS (pH 7.4) at a protease:protein ratio of 1:3000 for 0, 15, 30, or 60 minutes. Following cleavage, samples were analyzed on 12.5% SDS-polyacrylamide gels. PMN elastase cleavage of a full-length TrpRS of about 53 kDa generated a major fragment of about 46 kDa (SEQ ID NO: 5, T1, having the C-terminal histidine tag, or an SY variant thereof) and a minor fragment of about 43.5 kDa (SEQ ID NO: 7, T2 having the C-terminal histidine tag or the SY variant thereof). In particular, cleavage of full-length TrpRS (SY variant) by PMN elastase generated a major fragment of about 46 kDa (SEQ ID NO: 25) and a minor fragment of about 43.5 kDa (SEQ ID NO: 24).

Western blot analysis with antibodies directed against the carboxyl-terminal His₆-tag of the recombinant TrpRS proteins revealed that both fragments, which were apparent at approximately 46 kDa and 43.5 kDa for either the GD or SY variants, possessed the His₆-tag at their carboxyl-terminus. Thus, only the amino-terminus of two TrpRS fragments has been truncated. The amino-terminal sequences of the TrpRS fragments were determined by Edman degradation using an ABI Model 494 sequencer. Sequencing of these fragments showed that the N-terminus sequences were S-N-H-G-P for T1 and S-A-K-G-I for T2, indicating that the amino-terminal residues of the major and minor TrpRS fragments were located at positions 71 and 94, respectively, of full-length TrpRS. These human TrpRS constructs for the GD variant are summarized in Figure 1.

The angiostatic activity of the major and minor TrpRS fragments was analyzed in angiogenesis assays. Recombinant forms of the major and minor TrpRS fragments SEQ ID NO: 5 and 7 (and SY variants thereof), each having a C-terminal histidine tag (amino acid residues 472-484 of SEQ ID NO: 1) were used in these assays. Both GD and SY variants of T2-TrpRS fragments were capable of inhibiting angiogenesis.

### Example 3

### Truncated Fragments of Trp-RS Show Potent Angiostatic Effect for Retinal Angiogenesis

Angiostatic activity of truncated forms derived from full length tryptophanyl-tRNA synthetase was examined, in a post-natal mouse retinal angiogenesis model. Friedlander et al. (Abstracts 709-B84 and 714-B89, IOVS 41(4): 138-139 (Mar. 15, 2000)) reported that postnatal retinal angiogenesis proceeds in stages in the mouse. The present invention provides a method of assaying angiogenesis inhibition by exploiting this staged retinal vascularization.

Endotoxin-free recombinant mini-TrpRS and T2 (e.g., SEQ ID NOS: 12 and 24) were prepared as recombinant proteins. These proteins were injected intravitreally into neonatal Balb/C mice on postnatal (P) day 7 or 8 and the retinas harvested on P12 or P13. Collagen IV antibody and fluorescein-conjugated secondary antibody were used to visualize the vessels in retinal whole mount preparations. Anti-angiogenic activity was evaluated by confocal microscopic examination based upon the effect of injected proteins on formation of the deep, outer, vascular plexus. Intravitreal injection and retina isolation was performed with a dissecting microscope (SMZ 645, Nikon, Japan). An eyelid fissure was created in postnatal day 7 (P7) mice with a fine blade to expose the globe for injection of T2 (5 pmol) or TrpRS (5 pmol). The samples (0.5 µL) were injected with a syringe fitted with a 32-gauge needle (Hamilton Company, Reno, Nev.). The injection was made between the equator and the corneal limbus; during injection the location of the needle tip was monitored by direct visualization to determine that it was in the vitreous cavity. Eyes with needle-induced lens or retinal damage were excluded from the study. After the injection, the eyelids were repositioned to close the fissure.

On postnatal day 12 (P12), animals were euthanized and eyes enucleated. After 10 minutes in 4% paraformaldehyde (PFA) the cornea, lens, sclera, and vitreous were excised through a limbal incision. The isolated retina was prepared for staining by soaking in methanol for 10 minutes on ice, followed by blocking in 50% fetal bovine serum (Gibco, Grand Island, N.Y.) with 20% normal goat serum (The Jackson Laboratory, Bar Harbor, Me.) in PBS for 1 hour on ice. The blood vessels were specifically visualized by staining the retina with a rabbit anti-mouse collagen IV antibody (Chemicon, Temecula, Calif.) diluted 1:200 in blocking buffer for 18 hours at 4°C. An ALEXA FLUOR@ 594-conjugated goat anti-rabbit IgG antibody (Molecular Probes, Eugene, Oregon - 1:200 dilution in blocking buffer) was incubated with the retina for 2 hours at 4°C. The retinas were mounted with slow-fade mounting media M (Molecular Probes, Eugene, Oregon).

Angiostatic activity was evaluated based upon the degree of angiogenesis in the deep, outer retinal vascular layer (secondary layer) that forms between P8 and P12. The appearance of the inner blood vessel network (primary layer) was evaluated for normal development and signs of toxicity. None of the protein constructs used in this example produced any adverse effects on the primary layer.

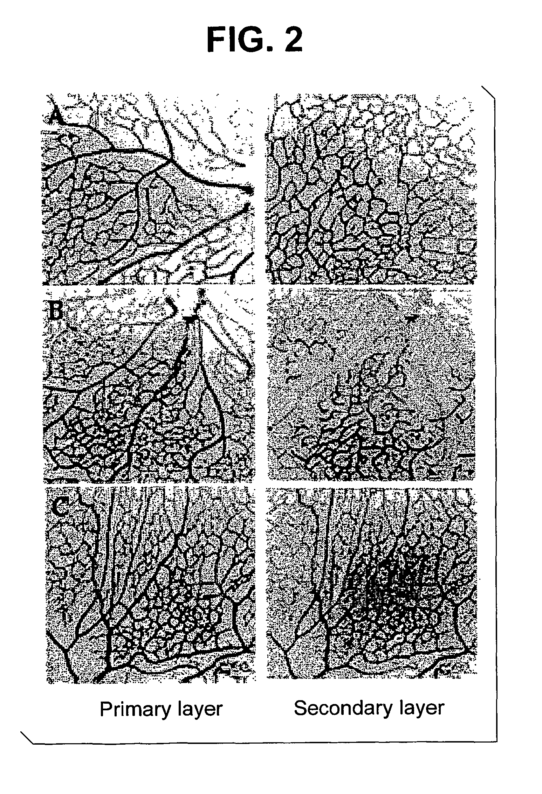

Figure 2 provides a photomicrographic depiction of the ability of T2 to inhibit vascularization of the secondary deep network of the mouse retina. In Figure 2, row A shows the vascular network of a retina exposed to TrpRS, Row B shows the vascular network of a retina exposed to Mini-TrpRS, and row C shows the vascular network of a retina exposed to polypeptide T2 of the present invention. The first (left) column shows the primary superficial network, and the second column shows the secondary deep network. As is evident from Figure 2, none of the polypeptides affected the primary superficial network, whereas only T2 significantly inhibited vascularization of the secondary deep network.

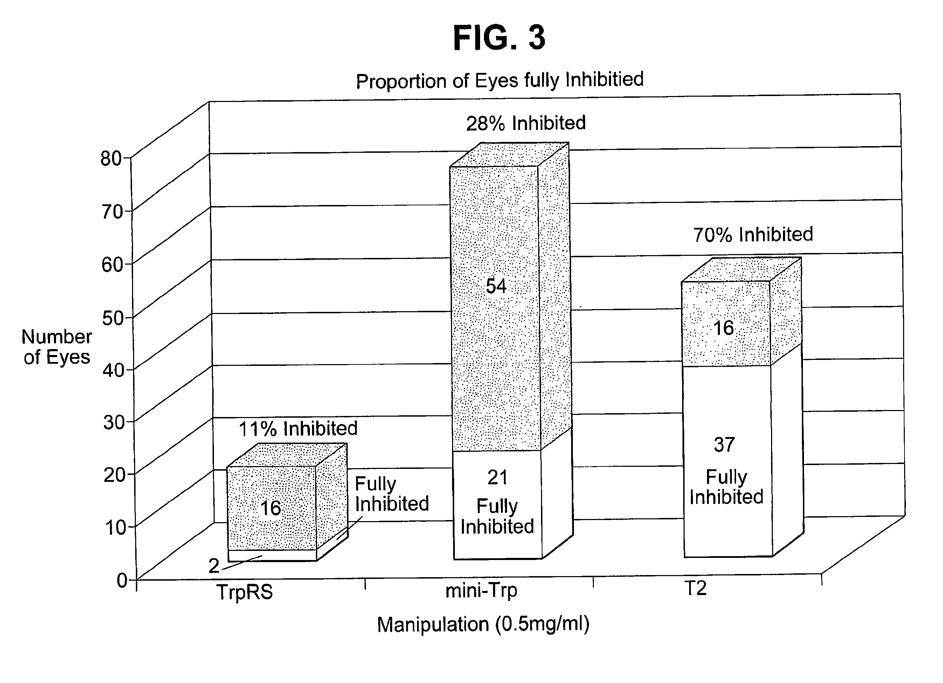

Most PBS-treated eyes exhibited normal retinal vascular development, but complete inhibition of the outer vascular layer was observed in about 8.2% (n=73) of the treated eyes. Complete inhibition of the outer network was observed in 28% of mini-TrpRS (0.5 mg/mL)-treated eyes (n=75). The smaller, truncated form (T2) was a far more potent inhibitor of angiogenesis in a dose dependent fashion; 14.3% were completely inhibited after treatment with 0.1 mg/mL of T2 (n=14), 40% after treatment with 0.25 mg/mL (n=20) and 69.8% inhibited completely after 0.5 mg/mL (n=53). The data for the 0.5 mg/mL treatments are presented graphically in Figure 3. Truncated forms of human TrpRS, especially T2 (e.g., SEQ ID NOS: 12, 24, 36, and 48), have a potent angiostatic effect on retinal vascular development.

### Example 4

### Matrigel Angiogenesis Assay

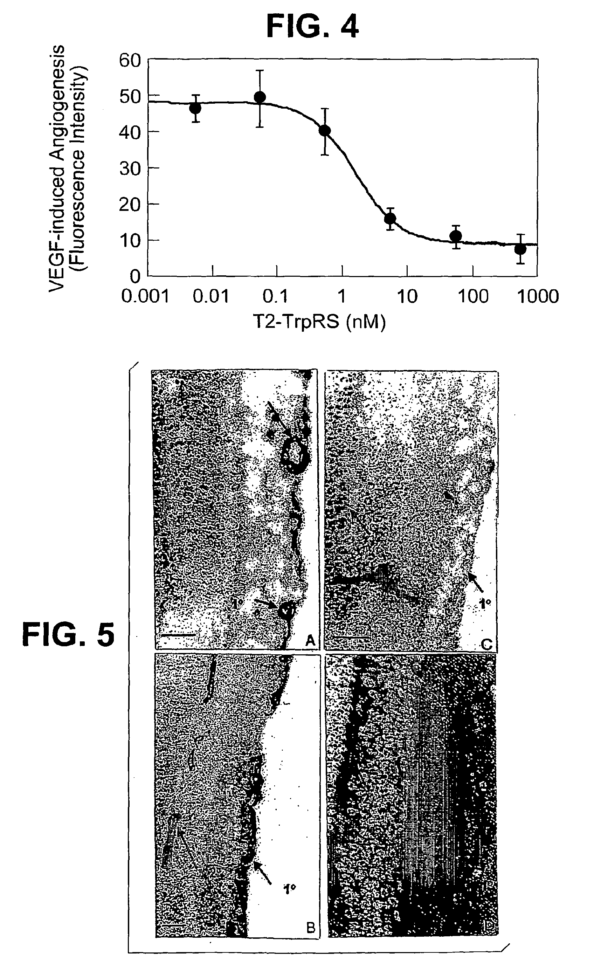

A mouse matrigel angiogenesis assay was used to examine the angiostatic activity of T2 (SEQ ID NO: 7 or SY variant thereof) according to the methods described by Brooks et al. Methods Mol. Biol., 129: 257-269 (1999) and Eliceiri et al. Mol. Cell, 4: 915-924 (1999). It was performed as described with the following modifications. Athymic WEHI mice were subcutaneously implanted with 400 µL growth-factor depleted matrigel (Becton Dickinson, Franklin Lakes, N.J.) containing 20 nM VEGF. The angiostatic activity of T2 was initially tested by including 2.5 µM T2 in the matrigel plug. The potency was determined by including various concentrations of T2 in the plug. On day 5, the mice were intravenously injected with the fluorescein-labeled endothelial binding lectin *Griffonia (Bandeiraea) Simplicifolia* I, isolectin B4 (Vector Laboratories, Burlingame, Calif.) and the matrigel plugs were resected. The fluorescein content of each plug was quantified by spectrophotometric analysis after grinding the plug in RIPA buffer (10 mM sodium phosphate, pH 7.4, 150 mM sodium chloride, 1% Nonidet P-40, 0.5% sodium deoxycholate, 0.1% sodium dodecyl sulfate). The data in Example four is illustrated in Figure 4.

### Example 5

### Localization of T2 Binding within the Retina

To assess the uptake and localization of T2 injected into the retina, ALEXA® 488-labeled (Molecular Probes, Inc., Eugene, Oregon) T2-TrpRS was injected into the vitreous of the eye on postnatal day 7 (P7). Globes were harvested on P8 and P12 and fixed in 4% PFA for 15 min. The retinas were further dissected free of adherent non-retinal tissue and placed in 4% PFA overnight at 4 °C and then embedded in medium (TISSUE-TEK® O.C.T., Sakura Fine Technical Co., Japan) on dry ice. Cryostat sections (10 micron) were rehydrated with PBS and blocked with 5% BSA, 2% normal goat serum in PBS. Blood vessels were visualized with anti-mouse collagen IV antibody as described above. VECTASHIELD® containing DAPI nuclear stain (Vector Laboratories, Burlingame, Calif.) was used to mount the tissues with a cover slip.

Alternatively, unstained retina sections were incubated with 200 nM ALEXA® 488-labeled full-length TrpRS or ALEXA® 488-labeled T2 in blocking buffer overnight at 4° C Sections were washed six times for 5 minutes each in PBS, followed by incubation with 1 µg/mL DAPI for 5 minutes for visualization of the nuclei. Pre-blocking with unlabeled T2 was performed by incubating 1 µM unlabeled T2 for 8 hours at 4° C prior to incubation with ALEXA® 488-labeled T2. Retinas were examined with a multiphoton BioRad MRC1024 confocal microscope. Three dimensional vascular images were produced from a set of Z-series images using the Confocal Assistant software (BioRad, Hercules, Calif.).

### Angiostatic Potency of T2 in the Mouse Matrigel Plug Assay

T2 fragments (SEQ ID NO: 7 and its SY variant) were examined to determine whether they had angiostatic activity, even though they had lost aminoacylation activity. The mouse matrigel assay was used to examine the angiostatic activity of T2 *in vivo.* VEGF₁₆₅-induces the development of blood vessels into the mouse matrigel plug. When T2 was added to the matrigel along with VEGF₁₆₅, angiogenesis was blocked in a dose-dependent manner with a IC₅₀ of 1.7 nM as shown in Figure 4.

ALEXA@ 488-labeled T2 Localizes to Retinal Blood Vessels. In order to visualize the intraocular localization of T2, we examined the distribution of ALEXA® 488-labeled T2 following intravitreous injection on postnatal day 7. Retinas were isolated the following day, sectioned and examined using confocal microscopy. The distribution of the injected protein was restricted to blood vessels. This localization was confirmed by co-staining labeled T2 treated eyes with a rabbit, anti-mouse collagen IV antibody (data not shown) and secondarily with an ALEXA FLUOR@ 594-labeled goat anti-rabbit IgG antibody. Five days after injection of ALEXA FLUOR@ 488-labeled T2 (on P12), the green fluorescence of the labeled T2 was still visible (Figure 5A). In these retinas, no secondary vascular layer was observed at P12, indicating that the ALEXA FLUOR@ 488-labeled T2 retained angiostatic activity comparable to unlabeled T2. Retinas injected on P7 with ALEXA FLUOR@ 488-labeled full-length TrpRS developed a secondary vascular layer by P12 but no vascular staining was observed (Figure 5B). In Figure 5, ALEXA FLUOR@ 488-labeled proteins are green, ALEXA FLUOR@ 594-labeled collagen-containing vessels are red, and nuclei are blue.

To further evaluate the binding properties of labeled T2, cross-sectioned slices of normal neonatal retinas were stained with ALEXA FLUOR@ 488-labeled T2. Under these conditions, ALEXA FLUOR@ 488-labeled T2 only bound to blood vessels (Figure 5C). The binding was specific as it was blocked by pre-incubation with unlabeled T2 (data not shown). No retinal vessel staining was observed when ALEXA FLUOR® 488-labeled full-length TrpRS was applied to the retinas (Figure 5D), consistent with the absence of angiostatic activity of the full-length enzyme.

As shown in Figure 5, ALEXA FLUOR® 488-labeled T2 is angiostatic and localizes to retinal blood vessels. ALEXA FLUOR® 488-labeled T2 (Figure 5A) or full-length TrpRS (Figure 5B) were injected (0.5 µL, intravitreous) on postnatal day 7 (P7). The retinas were harvested on P8 and stained with an anti-collagen IV antibody and DAPI nuclear stain, Labeled T2 (upper arrow pointing to vessel in Figure 5A) localized to blood vessels in the primary superficial network (1 °). Note that the secondary deep network is completely absent (2°). While both the primary (1°) and secondary (2°) vascular layers are present in eyes injected with ALEXA FLUOR® 488-labeled full-length TrpRS (arrows in Figure 5B), no labeling is observed.

In a separate study, frozen sections of P15 retinas were stained with ALEXA FLUOR® 488-labeled T2 (Figure 5C) or ALEXA FLUOR® 488-labeled full-length TrpRS (Figure 5D) and imaged in the confocal scanning laser microscope. Labeled T2 selectively localized to blood vessels and appears as a bright green vessel penetrating the primary and secondary retinal vascular layers just below the label "2°" in Figure 5C. No staining was observed with fluorescently-labeled full-length TrpRS (Figure 5D).

Full-length TrpRS contains a unique NH₂-terminal domain and lacks angiostatic activity. Removing part or this entire domain reveals a protein with angiostatic activity. The NH₂-terminal domain, which can be deleted by alternative splicing or by proteolysis, may regulate the angiostatic activity of TrpRS, possibly by revealing a binding site necessary for angiostasis that is inaccessible in full-length TrpRS.

VEGF-induced angiogenesis in the mouse matrigel model was completely inhibited by T2 as was physiological angiogenesis in the neonatal retina. Interestingly, the most potent anti-angiogenic effect of TrpRS fragments *in vitro* and in CAM and matrigel models is observed in VEGF-stimulated angiogenesis. The neonatal mouse retinal angiogenesis results are consistent with a link between VEGF-stimulated angiogenesis and the angiostatic effects of TrpRS fragments; retinal angiogenesis in this system may be driven by VEGF. In addition, the inhibition observed in the retinal model was specific for newly developing vessels; pre-existing (at the time of injection) primary vascular layer vessels were unaltered by the treatment. While the mechanism for the angiostatic activity of T2 is not known, the specific localization of T2 to the retinal endothelial vasculature and the selective effect of T2 on newly developing blood vessels suggest that T2 may function through an endothelial cell receptor expressed on proliferating or migrating cells. Further understanding of the mechanism of T2 angiostatic activity requires more detailed identification of the mechanism of action.

A variety of cell types that produce, upon interferon-γ stimulation, the angiostatic mini-TrpRS also produce angiostatic factors such as IP-10 and MIG. Thus, these results raise the possibility of a role for TrpRS in normal, physiologically relevant pathways of angiogenesis. Another ubiquitous cellular protein, pro-EMAPII (p43), has two apparently unrelated roles similar to those reported here for TrpRS. Pro-EMAPII assists protein translation by associating with the multisynthetase complex of mammalian aminoacyl tRNA synthetases. It is processed and secreted as EMAPII, and a role for EMAPII as an angiostatic mediator during lung development has been suggested.

Thus, T2 can be utilized in physiologically relevant angiogenic remodeling observed under normal or pathological conditions. In normal angiogenesis, T2 can aid in establishing physiologically important avascular zones present in some organs such as the foveal avascular zone of the central retina. Pathological angiogenesis can occur if the cleavage of full-length TrpRS was inhibited, leading to an overgrowth of vessels.

In ocular diseases, neovascularization can lead to catastrophic loss of vision. These patients can potentially receive great benefit from therapeutic inhibition of angiogenesis. Vascular endothelial growth factor has been associated with neovascularization and macular edema in the retina although it is believed that other angiogenic stimuli also have roles in retinal angiogenesis. We have observed an association between VEGF-stimulated angiogenesis and potent angiostatic activity of TrpRS fragments, making these molecules useful in the treatment of hypoxic, and other, proliferative retinopathies. There has been no report in the literature of an anti-angiogenic agent that completely inhibits angiogenesis 70% of the time, as does the T2 of the present invention (Figure 5). Another advantage of TrpRS fragments is that they represent naturally occurring and, therefore, potentially non-immunogenic, anti-angiogenics. Thus, these molecules can be delivered via targeted cell- or viral vector-based therapy. Because many patients with neovascular eye diseases have associated systemic ischemic disease, local anti-angiogenic treatment with genetically engineered cells or viral vectors placed directly into the eye is desirable.

In addition to treatment of angiogenic retinopathies, the TrpRS fragments of the present invention, particularly T2-TrpRS and angiogenesis inhibiting fragments thereof, could potentially also inhibit solid tumor growth by preventing vascularization of the tumor. The TrpRS fragments of the present invention block VEGF-induced proliferation and chemotaxis of endothelial cells *in vitro,* and are thus useful in the treatment of any pathology involving unwanted endothelial cell proliferation and vascularization.

### Example 6

Table 6 below summarizes various vector constructs of tRNA synthetase fragments.

**TABLE 6**

| **Name** | **Antiobiotic Marker** | **Characteristics Origin** |
|---|---|---|
| pAS-001 (SEQ ID NO: 70) | Kan | pET24b+ with a NdeI/HindIII insert of Human T2-TrpRS (SY variant) without 6-His Tag |
| pAS-002 (SEQ ID NO: 71) | Amp | pET20b+ with a NdeI/HindIII insert of Human T2-TrpRS (SY variant), with 6-His Tag |
| pAS-004 (SEQ ID NO: 72) | Amp | pET20b+ with a NdeI/HindIII insert of Human T2-TrpRS, 6-His Tag w/Thrombin Cleavage Site |
| pAS-006 (SEQ ID NO: 73) | Kan | pET24b+ with a NdeI/XhoI insert of Human mini-TyrRS, 6-His Tag |
| pAS-007 (SEQ ID NO: 74) | Kan | pET24b+ with a NdeI/HindIII insert of Human mini-TrpRS (SY variant), 6-His Tag |
| pAS-009 (SEQ ID NO: 75) | Kan | pET24b+ with a NdeI/XhoI insert of Human mini-TyrRS, No His Tag |

The vectors identified in Table 6 were prepared by the following methods:
***Plasmid pAS-001.*** The T2-TrpRS fragment was amplified by PCR using a full-length clone of TrpRS (Invitrogen, clone 3542671) as a template. The oligonucleotides for PCR were based on the T2-TrpRS sequence and contained a 5'- Ndel site and a 3'-HindIII site (in bold italics) (5'GGA GAT ATA ***CAT ATG*** AGT GCA AAA GGC ATA GAC TAC 3' (SEQ ID NO. 77) and 5'TGC GGC GGC ***AAG*** CTT TCA CTG AAA GTC GAA GGA CAG CTT CC 3' (SEQ ID NO. 78)). Following amplification, the purified PCR fragment was cleaved with Ndel and HindIII, and then cloned into these same restriction-digested sites of plasmid pET24b+ (Novagen). The resulting plasmid contained a T2-TrpRS sequence, immediately followed by a stop codon. Therefore the His tag sequence was not fused to the T2-TrpRS gene sequence.
***Plasmids pAS-002**.* The T2-TrpRS fragment was amplified by PCR using the full-length TrpRS clone (Invitrogen, clone 3542671) as a template. The oligonucleotides for PCR contained a 5'-Ndel site and a 3'-HindII site (in bold italics) (5'TGG ACA GTA CAG ***CAT ATG*** AGT GCA AAA GGC ATA GAC TAC 3' (SEQ ID NO. 79) and 5'TGC GGC CGC ***AAG CTT*** CTG AAA GTC GAA GGA CAG CTT CCG 3' (SEQ ID NO. 80)). Following amplification, the purified PCR fragment was cleaved with Ndel and HindIII, and then cloned into these same restriction-digested sites of plasmid pET20b+ (Novagen). The resulting plasmid contained an in-frame gene fusion between the carboxy-terminal His tag sequence present in the pET20b+ vector and the T2-TrpRS.
***Plasmid pAS-004**.* PCR based oligonucleotide-mediated introduction of a thrombin cleavage site was used to modify the vector sequence of pAS-002. The oligonucleotides for PCR were based on the T2-TrpRS sequence and contained a thrombin cleavage site (bold italics) (5'-GCT GTC CTT CGA CTT TCA GTC *TTC **TGG TCT GGT GCC ACG CGG TTC*** TAA GCT TGC GGC GGC ACT CGA GCA CCA CC 3' (SEQ ID NO. 81) and 5'GGT GGT GCT CGA GTG CGG CCG CAA GCT T***AG AAC CGC GTG GCA CCA GAC CAG AAG A***CT GAA AGT CGA AGG ACA GC 3' (SEQ ID NO. 82)). During the PCR reaction, the primers anneal to the same sequence on opposite strands of the plasmid and then were extended with Pfu turbo DNA polymerase (Stratagene), generating plasmids with the thrombin insertion immediately upstream from the 6-His tag. The thrombin cleavage site allows removal of the 6-His tag after protein purification.
***Plasmid pAS-006**.* The mini TyrRS fragment was amplified by PCR using the full-length TyrRS clone (Invitrogen, 4386850) as a template. The oligonucleotides for PCR contained a 5'-Ndel site and a 3'-Xhol site (in bold italics) (5'CCT GCT CAA ***CAT ATG*** GGG GAC GCT CCC GGC CCT GAA GAG 3' (SEQ ID No. 83) and 5'CCA GCC ***GCT CGA*** GGA TGA CCT CCT CTG GTT CTG AAT TC 3' (SEQ ID NO. 84)). Following amplification, the purified PCR fragment was cleaved with Ndel and Xhol, and then cloned into these same restriction-digested sites of plasmid pET24b+ (Novagen). The resulting plasmid contained an in-frame gene fusion between the carboxy-terminal His tag sequence present in the pET24b+ vector and the mini TyrRS.
***Plasmid pAS-007.*** The mini-TrpRS fragment was amplified by PCR using the full-length TrpRS clone (Invitrogen, 3542671) as a template. The oligonucleotides for PCR contained a 5'-Ndel site and a 3'-HindIII site (in bold italics) (GTG TCA TTA ***CAT ATG*** AGC TAC AAA GCT GCC GCG GGG 3' (SEQ ID NO. 85) and 5'CGA TGG G***AA GCT T***CT GAA AGT CGA AGG ACA GCT TCC G 3' (SEQ ID NO. 86)). Following amplification, the purified PCR fragment was leaved with NdeI and HindIII, and then cloned into these same restriction-digested sites of plasmid pET24b+ (Novagen). The resulting plasmid contained an in-frame gene fusion between the carboxy-terminal His tag sequence present in the pET24b+ vector and the mini TyrRS.
***Plasmid pAS-009.*** The mini TyrRS fragment was amplified by PCR using a full-length clone of TyrRS (Invitrogen, clone 4386850) as a template. The oligonucleotides for PCR were based on the mini TyrRS sequence and contained a 5'- Ndel site and a 3'-XhoI site (in bold italics) (5'CCT GCT CAA ***CAT ATG*** GGG GAC GCT CCC AGC CCT GAA GAG 3' (SEQ ID NO. 87) and 5'CCA GCC G***CT CGA*** GTC AGA TGA CCT CCT CTG GTT CTG AAT TC 3' (SEQ ID NO. 88)). Following amplification, the purified PCR fragment was cleaved with Ndel and Xhol, and then cloned into these same restriction-digested sites of plasmid pET24b+ (Novagen). The resulting plasmid contained a t2- TrpRS, sequence, immediately followed by a stop codon. Therefore the His tag sequence was not fused to the T2-TrpRS gene sequence.

In the case of pAS-002 and pAS-007, the gene for either mini TyrRS or T2-TrpRS was fused to a 6-His tag to aid in the purification from the host system for research grade materials. However, the 6-His tag was not used in the final system chosen for the expression and purification of material for pre-clinical development.

***Transformations**.* Plasmids were added to chemically competent *E.coli* BL21 (DE3) cells (Novagen) and allowed to incubate on ice for 30 minutes. After the incubation, the cells/DNA mixture was heat shocked for 45 seconds at 42° C. The cells were allowed to recover at 37° C on a rotator for 30 minutes and then plated on LB plates with the appropriate antibiotic.

***Protein Purification**.* Expression of research grade (His tagged proteins) the protein in BL21 (DE3) was induced at A₆₀₀ = 0.6 by addition of 1 mM isopropyl β-D-thiogalactopyranoside (Novagen) for 4 hours. Cells were harvested by centrifugation, lysed on ice by sonication in column buffer (20 mM Tris-HCl (pH 7.9), 500 mM NaCl, 30 mM imidazole and 5 mM β-mercaptoethanol), and the lysate was cleared by centrifugation at 35,000g for 30 minutes. The supernatant was loaded onto a Ni-NTA affinity column (Qiagen) pre-equilibrated with column buffer. The column was washed with column buffer containing 0.1% Triton-X 114 (Sigma) to dissociate lipopolysaccharide (LPS) from the protein, followed by additional column buffer to remove residual detergent. The protein was eluted with a gradient of 30 - 250 mM imidazole in column buffer and stored in PBS (pH 7.5)/50% Glycerol and 2 mM DTT. Purified proteins were assayed for endotoxin by the Limulus Amebocyte Lysate (LAL) assay (BioWhittaker). All purified proteins were more than 95% pure as judged by polyacrylamide gel electrophoresis (4-12% Bis-Tris NuPAGE Gels, Invitrogen). Protein concentration was determined by Bradford assay using the Bio-Rad Protein Assay reagent (Bio-Rad).

### Example 7

*E. coli* cells were transfected with a vector of SEQ ID NO: 70 identified in Example 6 above. The T2 protein product produced was purified to about 95% purity by the following methods:

### Cell Disruption and Clarification of Lysate

In the following cell disruption and lysate clarification procedure, all steps were performed at 4 °C and the pH of all buffers adjusted at 4°C.

The total mass of cell paste collected from the fermentation tank was divided into seven batches, each batch containing approximately 90 g of cell paste. The cell paste for each batch was mixed for approximately 3 minutes with 950 mL of cold Lysis Buffer (25 mM Tris pH 8.0, 10% Glycerol, 1 mM EDTA) using a homogenizer.

The suspension for each batch was then passed twice through an Avestin EmulsiFlex C-50 high pressure homogenizer at 10,000 to 20,000 PSI and collected on ice, taking care that the temperature of the lysate did not exceed 10 °C. The homogenizer was then flushed with lysis buffer to remove the residual lysate.

The lysate (~1150 mL) for each batch was then centrifuged at 38,250 g for 55 minutes. The supernatant (~1100 mL) was retained and the pellets were discarded. For each batch, the supernatant was loaded on the Q sepharose HP column as quickly as possible (see Q Sepharose chromatography). All of the above steps for the cell disruption and clarification for any additional batches of cells was performed, followed by immediate loading onto the Q Sepharose column following clarification.

### Q Sepharose Chromatography

The supernatant from the centrifugation process was loaded onto a 2.2 L (13 cm diameter, 16.6 cm height) Q Sepharose High Performance column. The column load of the protein should not exceed 5 mL of lysate per mL of resin. The column was pre-equilibrated with 2.5 L of Buffer B (25 mM Tris pH 8.0, 10% glycerol, 1 M NaCl) followed by 11 L of Buffer A (25 mM Tris pH 8.0, 10% glycerol). The load flow rate (for the soluble material) was 20-50 mL/min (-10-25 cm/hr) and the column flow through collected.

The column was washed with 30 column volumes (66 L) of Buffer A at 60 mL/min (~30 cm/hr). The column was then eluted with a 20 column volume (44 L) linear gradient, from Buffer A to 20% Buffer B at 100 mL/min (~50 cm/hr) and 500 mL fractions were collected during the elution peak ('Q fractions'). The Q fractions were analyzed by SDS-PAGE (for both the amount of T2-TrpRS in the fraction and the relative purity of the material) and the fractions containing the greatest amounts of purified T2-TrpRS were pooled. Reverse-phase HPLC represents one possible alternative to the use of SDS-PAGE for fraction analysis.

### Endotoxin reduction Filtration

The total pool of Q fractions was filtered at 4 °C through 2 Pall endotoxin reduction filtration cartridges with a Mustang E membrane at 10 mL/min, collecting the flow through. The sample was split between the two filter cartridges and only exposed to the filter membrane once. Approximately 93% of the total protein was recovered following the endotoxin reduction filtration.

### Concentration and Buffer Exchange

The endotoxin reduction filtered pool (8500 mL) was concentrated to <1 L using a Cross-Flow (Ultrafiltration) filter (molecular weight cut off of 10,000) at pressures of 5-7 psi. The filtrate was collected and checked by Bradford assay for leaking polypeptide. The concentrated pool (<1L) was diluted five-fold with CM Buffer A (25 mM HEPES pH 8.0, 10% glycerol) to increase the volume of the sample to 5L. The conductivity of the final dilution pool was 1.02 mS, whereas the conductivity of the CM Buffer A was 0.74 mS.

### CM Sepharose Chromatography

The sample from the buffer exchange process was loaded onto a 1300 mL (13 cm diameter, 9.8 cm height) CM Sepharose Fast Flow column, pre-equilibrated with 6.5 L of Buffer A (25 mM HEPES pH 8.0, 10% glycerol); the load flow rate was 90 mL/min (~40 cm/hr). The column was washed with 15 column volumes (19.5 L) of Buffer A at 70 mL/min (-30cm/hr). The column was then eluted with a 20 column volume (26 L) linear gradient, from Buffer A to 50% Buffer B (25 mM HEPES pH 8.0, 10% glycerol, 1M NaCl) at 100 mL/min (~50 cm/hr) and 500 mL fractions were collected during the elution peak. The CM fractions were analyzed by SDS-PAGE (for both the amount of T2-TrpRS in the fraction and the relative purity of the material), and fractions containing the greatest amounts of purified T2-TrpRS were pooled. Reverse-phase HPLC represents one possible alternative to the use of SDS-PAGE for fraction analysis.

### Final Sample Concentration and Buffer Exchange

The pooled CM fractions (5500 mL) were concentrated to - 150 mL using a Cross-Flow (Ultrafiltration) filter (molecular weight cut off of 10,000 daltons) at pressures of 2-7 psi. The filtrate fractions were collected and checked by Bradford assay for leaking polypeptide. The concentrated pool (145 mL) was dialyzed against 15 L of final storage buffer (5 mM sodium phosphate pH 7.4, 150 mM NaCl, 50% glycerol) using dialysis tubing having a 6000-8000 daltons molecular weight cut off at 4 °C (~16 hours).

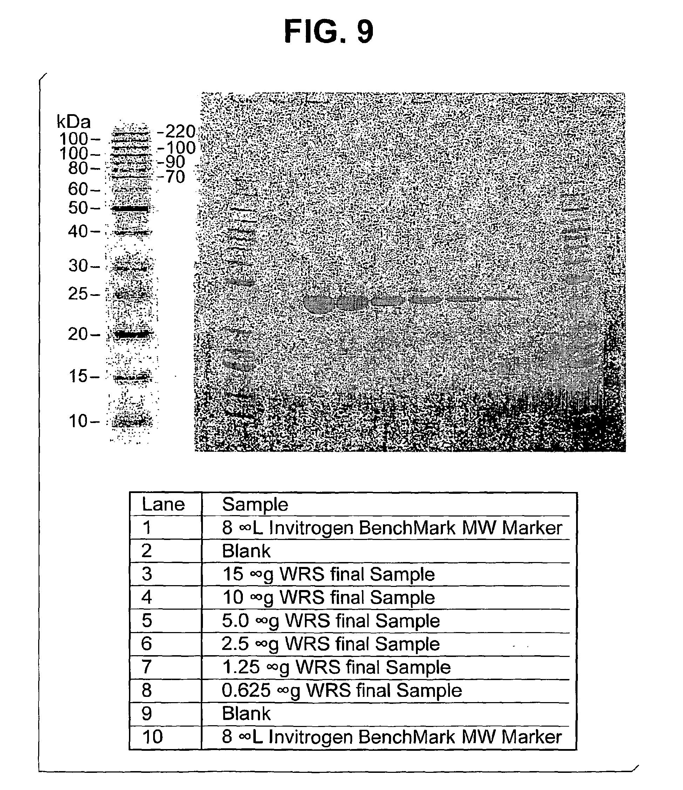

The dialyzed pool (~50 mL) was removed from dialysis and assays were completed on the sample. The final volume of the concentrated sample was 52 mL and the final concentration of the sample was 26.3 mg/mL based on the standard Bradford assay. Final denaturing SDS-PAGE analysis of the sample was completed for a purity determination and is illustrated in Figure 9. Lanes 1 and 10 illustrate the Invitrogen BenchMark MW Protein Markers. The two heavy molecular weight markers and the three lighter molecular weight markers in between them are identified on the left side of the gel. Their molecular weights vary from 20 kDa to 50 kDa. Lanes 2 and 9 are blank. Lanes 3-8 illustrate various amounts of final T2-TrpRS product. As can be visualized, the T2-TrpRS product produced by *E*. *coli* transfection of SEQ ID NO: 70 had molecular weight of about 43 kDa. The endotoxin level of this sample, measured using a PyroGene™ endotoxin assay from Invitrogen Corporation, was determined to be 6.25 E.U./mg of protein.

Table 2 below illustrates analysis of T2-TrpRS product from various stages of the purification protocol described above.

**TABLE 2. Analysis of T2-TrpRS**

| **Fraction** | **Volume (mL)** | **Protein (mg/mL)** | **Total Protein (mg)** | **Recovery %** | **Purity %** |
|---|---|---|---|---|---|
| Q HP pool | 8500 | 0.4 | 3400 | - | >85% |
| Q HP pool post endotoxin filter | 8800 | 0.36 | 3168 | 93% | >85% |
| CM load | 4800 | 0.663 | 3182 | 93.6% | >85% |
| CM pool | 5500 | 0.345 | 1897.5 | 55.8% | >95% |
| CM pool concentrated | 145 | 13.36 | 1937.2 | 57% | >95% |
| Final sample | 52 | 26.3 | 1367.6 | 40.2% | >95% |

### Example 8

Low endotoxin T2-TrpRS was produced by expression in an *E*. *coli* host (BL21-DE3) using a T7 driven plasmid having SEQ ID NO: 70. The cells were grown under cGMP conditions to produce both the Master Cell Bank (MCB) and the Working Cell Bank (WCB).

Growth medium (yeast extract 46.4 g/L, glycerol 4 g/L, and glucose 4 g/L) was prepared and filter sterilized. Kanamycin was added to the solution at a final concentration 50 µg/mL of medium. Growth medium aliquots of 250 mL were transferred into seven sterile 1 L flasks and used for inoculation.

A single stage inoculum was used for the process. A WCB vial was thawed prior to inoculation. Four shake flasks were selected for further process procedures. Fifty (50) mL of media was removed from the 3 shaker flasks not used for further processing for bioburden testing. A 0.2 mL aliquot of the WCB was added to each of four, 1 L shaker flasks containing 0.25 L of growth medium. A sterile pipet tip was used between each flask. The flasks were incubated at 37 °C, 200 rpm in an environmentally controlled shaker for 8-10 hours. One flask of the four was used to monitor growth, and the other three were used to inoculate the fermentor. During the shaker flask incubation, the fermentor was filled with fermentation medium, heat-sterilized and allowed to cool. The composition of the fermentation medium was as follows: yeast extract 37.1 g/L, KH₂PO₄ 6.67 g/L, K₂HPO₄ 9.67 g/L, Na₂HPO₄ 18.6 g/L, NH₄Cl 1.47 g/L, and NaCl 0.736 g/L.

Additional materials such as the feeding solution (MgSO₄ anhydrous 7.3 g/L, glycerol 160 g/L, CaCl₂ 0.22 g/L, glucose 32.0 g/L) and trace elements solution (FeCl₃·H₂O 27.0 g/L, ZnCl₂ 1.3 g/L, CuCl₂·2H₂O 1.0 g/L, CoCl₂ 2.0 g/L, (NH₄)₆Mo₇O₂₄·4H₂O 2.0 g/L, boric acid 0.5 g/L, concentrated HCl 100 mUL) were added to the reaction mixture at the correct proportions (0.147 L/L and 0.0022 mUL, respectively). Pluronic L-61 Antifoam solution (25%, v/v) was added to the fermentor at a ratio of 0.02 mL/L fermentation solution. Additional kanamycin was added to the fermentor to maintain the selection for transfected cells. The pH of the solution was brought to 7.0, using either ammonium hydroxide or phosphoric acid, and the temperature was maintained at 37 °C.

After the culture of the sample flask reaches an OD₆₀₀ of 3, the contents of the three other shake flasks were pooled and used to inoculate the fermentation medium in the fermentor. The contents of the inoculum pool, minus the volume of samples, were added to the fermentor. The OD₆₀₀ was measured immediately after inoculation and at 1 hour intervals. The agitation was increased, and the oxygen was supplemented as necessary to maintain the dissolved oxygen (DO) above 30% using automatic controls. When the fermentor reached an OD₆₀₀ of 10, the pre-induction samples were taken and processed.

Induction was performed by addition of IPTG to a final concentration of 0.1 mM. The growth was monitored every hour until the glycerol was exhausted. The consumption of the glycerol resulted in a spike in the DO at 6 - 8 hours post induction. At that point, samples were taken and processed. The remaining slurry was prepared for cell harvest.

The harvest procedure began with decreasing the temperature setting to 10 °C. The pH and DO controls were stopped and the stirrer was slowed to 100 rpm. When the temperature of the slurry reached 25 °C, the contents of the fermentor were distributed to centrifuge bottles. The slurry was centrifuged at 4000 (nominal 3300 x g) rpm for 15 minutes at 2-8 °C. The cell pellets were collected, weighed and resuspended in Cell Lysis Buffer (tris base 3.02 g/L, EDTA 0.29 g/L and glycerol 100 g/L, pH 8.0) such that for every 1 g of cell paste, 10 mL of buffer was used, and stored at 2-8 °C until lysis. The suspended cells were then homogenized with 3 passes through an Avestin Emulsiflux 50 at >9000 psi at 2-8 °C. The homogenized slurry was dispensed into centrifuge bottles and centrifuged at 4000 rpm for 45 minutes at 2-8 °C. The supernatant was collected and stored at 2-8 °C pending further processing.

The general downstream processing methods are diagramed in Figure 8. The purification method followed the general theme of the following steps: supernatant clarification; Q Sepharose high performance column chromatography; Mustang E filtration; concentration/ buffer exchange; CM Sepharose fast flow column chromatography; concentration/ buffer exchange; and sterile filtration and filling.

### Supernatant Clarification

Lysis buffer was flushed through a 0.45/0.2 micron sterile capsule filter (Sartobran P, 2 sq. ft membrane) while maintaining a pressure of < 20 psig. All air is purged from the system. The supernatant was passed through the system at a rate of 130 - 150 mL/min. The pump speed was adjusted to achieve < 25 psi backpressure. The resulting solution was called the "Clarified Supernatant".

### Q Sepharose High Performance (HP) Column Chromatography

The first column chromatography system was designed to increase the purity of the protein by selecting for its binding and elution characteristics. During this step, the protein purity increased to approximately 90% and the endotoxins were reduced to approximately 5% of the starting content (EU/mg protein).

Q sepharose HP resin was loaded into an Amersham BPG 200/500 column and sanitized with 0.5 N sodium hydroxide. The approximate volume of the resin bed was 5 L. The column was connected to an Amersham 6 mm Bioprocess Chromatography system. All solutions were primed, and the system was flushed with a minimum of five (5) column volumes of the loading buffer (25 mM tris + 10% (w/w) glycerol, pH 8.0). The Clarified Supernatant was loaded onto the column at a flow rate of 9.4 L/hour. The column was washed with wash buffer (25 mM tris + 10% (w/w) glycerol + 30 mM NaCl, pH 8.0) at a rate of 9.4 Uhour until 12 column volumes of solution passed through the column and the absorbance (A₂₈₀ₙₘ) droped to <0.05 AU. The product was eluted from the column by passing the elution buffer (25 mM tris + 10% (w/w) glycerol + 80 mM NaCl, pH 8.0) through the column at a rate of 15 L/hour. The product was eluted in a volume of nine (9) column volumes, which was colleted as six (6) 1000 mL fractions (F1 - F6), followed by twenty (20) 2000 mL fractions (F7- F26). The peak was collected until the absorbance (A₂₈₀ₙₘ) returned to 0.04 AU above baseline. Samples were taken from each fraction and analyzed for T2-TrpRS content. The fractions were stored at 2-8 °C until all analyses were completed. When the fractions containing ≥20% purity of T2-TrpRS were identified, they were combined into one container and renamed the "Q Sepharose HP Pool". The column was cleaned by passing regeneration buffer (25 mM tris + 10% (w/w) glycerol + 1 M NaCl, pH 8.0) through the column for a minimum of five (5) column volumes at a rate of 9.1 L/hour. Thereafter, the column was sanitized by passing 0.5 N sodium hydroxide through the column at a rate of 17 Uhour for five (5) column volumes. The column was stored in 0.1 N sodium hydroxide.

### Mustang E Filtration

The Mustang E filtration system was a solid phase filtration system specifically designed to remove endotoxin from the solution. This step did not result in any appreciable increase in the amount of T2-TrpRS compared to the total amount of protein, i.e., the purity of T2-TrpRS relative to other polypeptides in solution.

A Pall Mustang E capsule (NP6MSTGEP1) was connected to a peristaltic pump and flushed with Water for Injection (WFI). The pressure was maintained at < 20 psig, and the air was released by opening the purge valve on the non-sterile (inlet) side of the filter. Approximately three (3) L of WFI was passed through the filter. The Q Sepharose HP Pool was passed through the filter into a depyrogenated carboy at a rate that produces an inlet pressure of < 20 psig. When less than 500 mL of the Q Sepharose HP Pool remained, two (2) L of Q sepharose wash buffer (25 mM tris + 10% (w/w) glycerol + 30 mM NaCl, pH 8.0) was added to the pool. The pump setting was reduced and the remaining material was filtered. The resulting filtrate was named the "Mustang E Filtrate".

### Concentration/Buffer Exchange

This ultrafiltration/diafiltration system was designed to reduce the volume and change the buffer system to that of the next chromatography system (CM Sepharose Fast Flow Column Chromatography).

A Pellicon 2 Ultrafiltration Diafiltration (UFDF) system was fitted with five (5) 10 kDa 0.1 m² cross flow filters. The system was flushed with a minimum of 20 L of WFI, and the clear water flux rate (CWF) was calculated at a transmembrane pressure (TMP) of 10 psig. The system was sanitized with a minimum of 10 L of 0.5 N sodium hydroxide at a TMP of 5 psig. The sodium hydroxide was flushed from the system with WFI. The system was flushed with a minimum of 10 L of CM sepharose fast flow (FF) loading buffer (25 mM HEPES + 10% (w/w) glycerol, pH 8.0). The system was loaded with a fresh solution of CM sepharose FF loading buffer, and the Mustang E Filtrate was connected to the inlet line. The Mustang E Filtrate was concentrated to a final volume of 15 L at a TMP of 10-12 psig. When the concentration was complete, the diafiltration into the CM sepharose FF loading buffer began using six times the volume of the concentrated Mustang E Filtrate. When the conductivity of the solution reached 1.3 mS/cm, the diafiltration was complete. The final solution was designated "UFDF #1 Retentate". The system was cleaned with 0.5 N sodium chloride and WFI between uses and stored in 0.1 N sodium hydroxide.

### CM Sepharose Fast Flow Column Chromatography

The second column chromatography system was designed to increase the purity of the protein by selecting for its binding and elution characteristics. During this step, the protein purity increased to ≥ 98% and the endotoxins were reduced to < 10 EU/mg protein.

CM sepharose FF resin was loaded into an Amersham BPG 200/500 column and sanitized with 0.5 N sodium hydroxide. The approximate volume of the resin bed was 3.2 L. The column was connected to an Amersham 6 mm Bioprocess Chromatography system. All solutions were primed, and the system was flushed with a minimum of five (5) column volumes of the loading buffer (25. mM HEPES + 10% (w/w) glycerol, pH 8.0). The UFDF #1 Retentate was passed through a Opticap 4 inch capsule filter (0.2 µm pore size) at < 20 psig, and the solution was relabeled "UFDF #1 Retentate Filtrate". The latter solution was immediately loaded onto the CM sepharose column at 31.4 L/hour. Thereafter, the column was washed with 15 column volumes of the loading buffer at the same flow rate until the absorbance (A₂₈₀ₙₘ) drops to < 0.01 AU and the full volume of wash buffer was used. The product was eluted from the column by passing elution buffer (25 mM HEPES + 1.0 M NaCl + 10% glycerol, pH 8.0) at a rate of 31.4 Uhour for six (6) column volumes. The elution volume was collected as fractions (F1, F2, etc) in 1 L increments until the absorbance (A₂₈₀ₙₘ) falls to 0.01 AU above baseline. Samples were taken from each fraction and analyzed for T2-TrpRS content. The fractions were stored at 2-8 °C until all analyses was completed (not to exceed 24 hours). The column was cleaned by passing regeneration buffer (25 mM HEPES + 10% (w/w) glycerol + 1 M NaCl, pH 8.0) through the column for a minimum of five (5) column volumes at a rate of 31.4 L/hour. Thereafter, the column was sanitized by passing 0.5 N sodium hydroxide through the column at a rate of 31.4 Uhour for five (5) column volumes. The column was stored in 0.1 N sodium hydroxide.

### Concentration/Buffer Exchange

This ultrafiltration/diafiltration system was designed to reduce the volume and change the buffer system to that of the final drug substance formulation (5 mM sodium phosphate + 150 mM sodium chloride, pH 7.4).

A Pellicon 2 Ultrafiltration Diafiltration (UFDF) system was fitted with one (1) 10 kDa 0.1 m² cross flow filter. The system was flushed with a minimum of 10 L of WFI, and the CWF was calculated at a TMP of 5 psig. The system was sanitized with a minimum of 5 L of 0.5 N sodium hydroxide at a TMP of 5 psig. The sodium hydroxide was flushed from the system with WFI. The system was flushed with a minimum of 2 L of final drug substance formulation buffer. The system was loaded with a fresh solution of final drug substance formulation buffer, and the CM elution fractions identified as having > 95% T2-TrpRS content purity were recombined and gently mixed and designated the "CM Sepharose Elution Pool". In the Ultrafiltration mode, the CM Sepharose Elution Pool was concentrated to a target of 15.0 g/L at a TMP of 10-12 psig. When the concentration was complete, diafiltration into the final drug substance formulation buffer began using eight times the volume of the concentrated CM Sepharose Elution Pool. When the diafiltration was complete, the system was drained, and a sample was sent to Quality Control for a stat measurement of protein concentration and purity. If the concentration was in the range of 10 -15 mg/mL, the UFDF step was completed. If the concentration fell outside of this range, the system was reinitiated and corrective measures taken to adjust the concentration into the specified range. The system was cleaned with 0.5 N sodium chloride and WFI between uses and stored in 0.1 N sodium hydroxide.

### Sterile Filtration and Filling

The final solution was passed through a Millipak 20 (0.22 µm) filter into sterile 1 L PETG bottles. Endotoxin units were measured at 0.003 E.U. per mg protein.

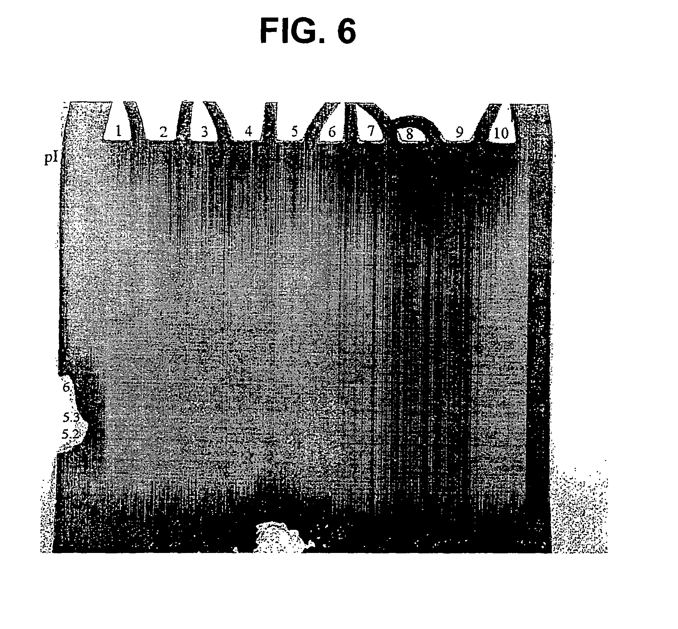

Figure 6 illustrates measurements of experimental pl (the effective charge) of a product produced recombinantly by *E. coli* after transfected with a vector of SEQ ID NO: 70 produced by the methods of Example 7 and 8. Sample 1 was produced by the methods of Example 7 and Sample 2 was produced by the methods of Example 8. The purity of Sample 1 is about 95% and wherein the purity of Sample 2 is greater than 99%. Samples were diluted 1:1 with Novex pH 3-10 sample buffer. The marker used with an IEF Marker from Invitrogen™.

The following Table 1 is a summary of each lane.

**TABLE 1**

| **Lane No.** | **Sample** | **Load** |
|---|---|---|
| 1 | Marker | 5 µL |
| 2 | Sample 1 | 1 µg |
| 3 | Sample 2 | 1 µg |
| 4 | Marker | 5 µL |
| 5 | Sample 2 | 2 µg |
| 6 | Sample 1 | 2 µg |
| 7 | Marker | 5 µL |
| 8 | Sample 2 | 4 µg |
| 9 | Sample 1 | 4 µg |
| 10 | Marker | 5 µL |

While the theoretical pl for monomer T2 having SEQ ID NO: 24 or 27 is 7.1, the experimental pl for the recombinantly produced product was measured at about 7.6, as is illustrated by Figure 6. This suggests that some of the negative charges of the primary sequence are "hidden" or inaccessible to the local environment.

### Example 10

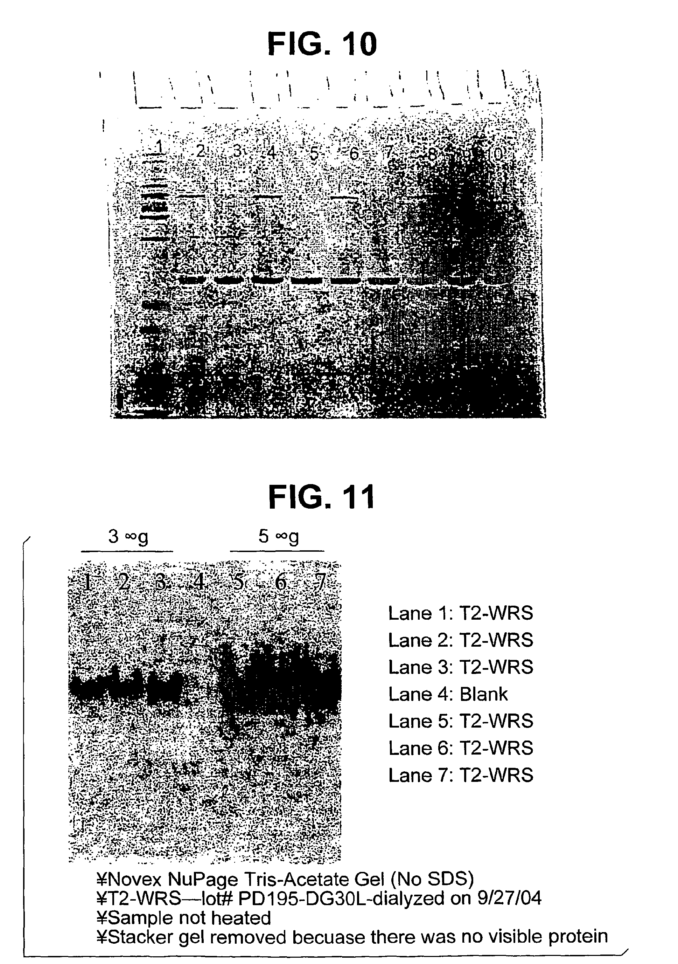

Figure 10 illustrates an SDS page gel of T2-TrpRS produced by recombinantly expressing a vector of SEQ ID NO: 70 in *E*. *coli.* The T2-TrpRS material produced by this method was approximately 99% pure and contained approximately 0.003 E.U./mg protein.

Lane 1 is a Mark 12 Ladder. Lane 2 illustrates a sample of the Load material at the processing step prior to the final purification step using a CM-sepharose column that was not heated prior to starting the gel separation. Lane 3 is the same material after heat has been applied to the sample at or near 100 °C for at least 5 minutes. Lanes 4, 6, and 8 are fractions from the CM-sepharose column without heating the sample prior to starting the gel separation. The T2-TrpRS-containing elution fractions being tested represent early, middle, and late elution from the CM-sepharose column after application of the elution buffer. There were five elution fractions in this study. Lanes 5, 7, and 9 are the fractions of Lanes 4, 6, and 8, respectively, but with heat denaturation of the protein prior to starting gel separation. Lane 10 is a Reference Standard (product approximately 95% pure) also prepared by recombinantly expressing a polynucleotide encoding SEQ ID NO: 27 in *E*. *coli.*

As is visualized by the gel, Lanes 2, 4, 6, 8, and 10, all include an upper band at roughly 86 kDa. This band disappears when the samples were heated in Lanes 3, 5, 7 and 9. All lanes include a band at roughly 43 kD, which is believed to be the monomer form of the product. This is most likely to occur because the product produced by recombinantly expressing SEQ ID NO: 27 in *E. coli* is a multi-unit complex such as a dimer that is non-covalently associated. Heating results in dissociation of the dimer and visualization of the protein's monomer components.

### Example 11

Figure 11 illustrates a native gel of T2-TrpRS produced by recombinantly expressing a vector of SEQ ID NO: 70 in *E*. *coli*, which was further purified to about 99% purity and approximately 0.003 E.U./mg protein.

The gel was a Novex NuPage Tris-Acetate Gel, which did not include SDS or detergent which could disrupt non-covalent bonds. Lanes 1-3 illustrate the product at lower concentrations than Lanes 5-7 (3µg and 5µg/lane, respectively). As can be visualized, the samples all run as a single band. This suggests that the purified product is a single form of the molecule (i.e., monomer and dimer do not exist simultaneously using this mode of detection).

### Example 12

A sizing HPLC column was used to detect the molecular weight and complexity of a T2-TrpRS product produced by recombinantly expressing vector of SEQ ID NO: 70. The T2-TrpRS product was purified to about 99% and 0.003 E.U./mg protein.

The HPLC column used was Amersham Superdex 200 10/300 GL™, which is a cross linked agarose and dextran column. The mobile phase was 0.2 M Potassium Phosphate and 0.15 Potassium Chloride (pH 6.5). The flow rate (mL/min) was 0.5. Detection was made at three different wavelengths: 215, 254 and 280 nm.

Calibration was made using blue dextran, β-amylase, alcohol dehydrogenase, albumin, carbonic anhydrase, cytochrome c, and sodium azide.

Table 3 below illustrates molecular weight (MW), log MW, retention time (RT), and elution volume for each of the calibrants. Void Volume (V₀) was measured as the elution volume of blue dextran at 8.667; Internal Volume (Vᵢ) was measured as the elution volume of sodium azide at 26.977, and Total Volume (Wₘ) was 35.654.

**TABLE 3. Molecular Weight and Retention Time**

| ***Sample*** | ***MW*** | ***LogMW*** | ***Rt*** | ***Elution Volume, m**l* |
|---|---|---|---|---|
| Blue Dextran | | | 17.353 | 8.677 |
| Sodium Azide | | | 53.954 | 26.977 |
| | | | | |
| β-amylase | 200000 | 5.30103 | 23.307 | 11.654 |
| alcohol | | | | |
| dehydrogenase | 150000 | 5.176091 | 25.546 | 12.773 |
| albumin | 66000 | 4.819544 | 28.508 | 14.254 |
| carbonic anhydrase | 29000 | 4.462398 | 32.687 | 16.344 |
| cytochrome c | 12400 | 4.093422 | 34.681 | 17.341 |

Table 4 bellow illustrates distribution coefficient for each of the calibrants.

**TABLE 4. Distribution Coefficient**

| Distribution Coefficient | KD = (Vr-Vo)/(Vm-Vo) = (Vr-Vo)Ni |
|---|---|
| blue dextran | 0.000 |
| β-amylase | 0.110 |
| alcohol | |
| dehydrogenase | 0.152 |
| albumin | 0.207 |
| carbonic anhydrase | 0.284 |
| cytochrome c | 0.321 |
| sodium azide | 0.678 |

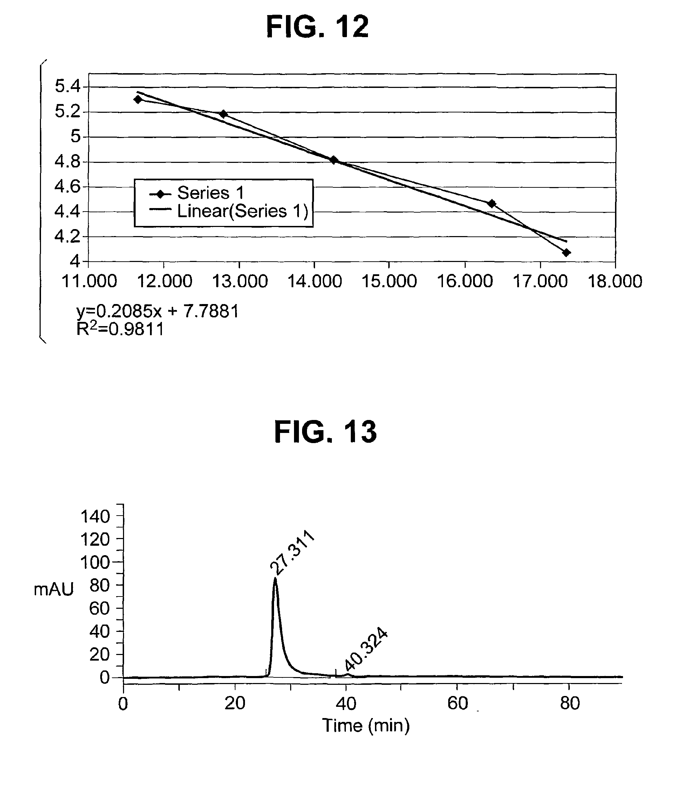

Figure 12 illustrates a calibration curve wherein the x-axis is the retention time of calibrants per minute and the y-axis is the log MW.

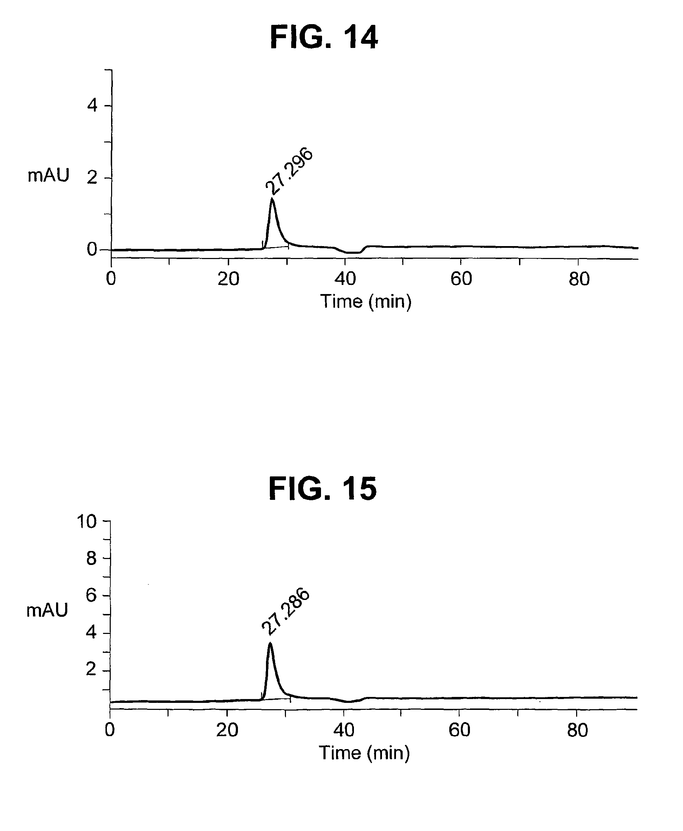

A sample of the purified protein product from expression of SEQ ID NO: 70 was loaded onto the column to identify its molecular weight. Products with larger molecular weight come off of the column sooner than products having lower molecular weight. As is illustrated in Figures 13-15, the recombinantly produced product had a retention time of about 27.3 minutes. Figure 13 illustrates the product detected at UV absorbance of 215 nm. Figure 14 illustrates the product detected at UV absorbance of 254 nm. Figure 15 illustrates the product as detected at UV absorbance of 280 nm.

Table 5 below illustrates calculations of the molecular weight of the recombinantly produced product. It was calculated that the product had a molecular weight of 87.283 kD. This confirmed that the product is composed of two monomer units, each approximately 43 kDa.

**TABLE 5. Molecular Weight of Sample**

| **Calibration Curve** | | | | | |
|---|---|---|---|---|---|
| Slope | -0.2085 | | | | |
| Intercpet | 7.7881 | | | | |
| R2 | 0.9811 | | | | |
| Flow rate | 0.5 | | | | |

| Sample | | Rt | Elution Volume, mL | logMW | MW |
|---|---|---|---|---|---|
| Reference Sample | | | | | |
| 100x dilution | | 27.311 | 13.656 | 4.940928 | 87283 |
| with mobile phase | | | | | |

### Example 13

A reverse phase HPLC was conducted to analyze the purity and establish identity of a product produced by recombinantly expressing a polynucleotide encoding a T2 fragment (e.g., SEQ ID NO: 27) herein. The HPLC system used included a Vydac Protein C4 column, 2.1 x 150 mm, 5 µm, Part # 214TP5215 and a UV detector capable of detection at 210 nm. The mobile phase A (diluent), included 0.1% TFA in water, which was prepared by mixing 1 mL TFA with 1 L water. The mobile phase B, included 0.1% TFA in acetonitrile, which was prepared by mixing 1 mL TFA with 1 L acetonitrile. The acetonitrile is less hydrophobic than water and therefore interferes with lipid interactions of proteins and the column resin surface. After the Vydac column is installed, a diluent is injected as a blank sample. Various amounts of reference material (e.g., purified T2 to about 99% purity) may further be injected to create a standard curve. Later, a sample of a partially purified T2 product (e.g., a product obtained by recombinantly expressing a polynucleotide encoding SEQ ID NO: 27) that has been left at room temperature for three days is injected at a volume of 25 µL. A gradient set of the two mobile phases (A and B) is made as follows:

**TABLE 6: HPLC Reverse Gradient Set Up**

| **Time, minute** | **%A** | **%B** |
|---|---|---|
| 0 | 85 | 15 |
| 5 | 85 | 15 |
| 25 | 30 | 70 |
| 26 | 5 | 95 |
| 31 | 5 | 95 |
| 32 | 85 | 15 |
| 37 | 85 | 15 |

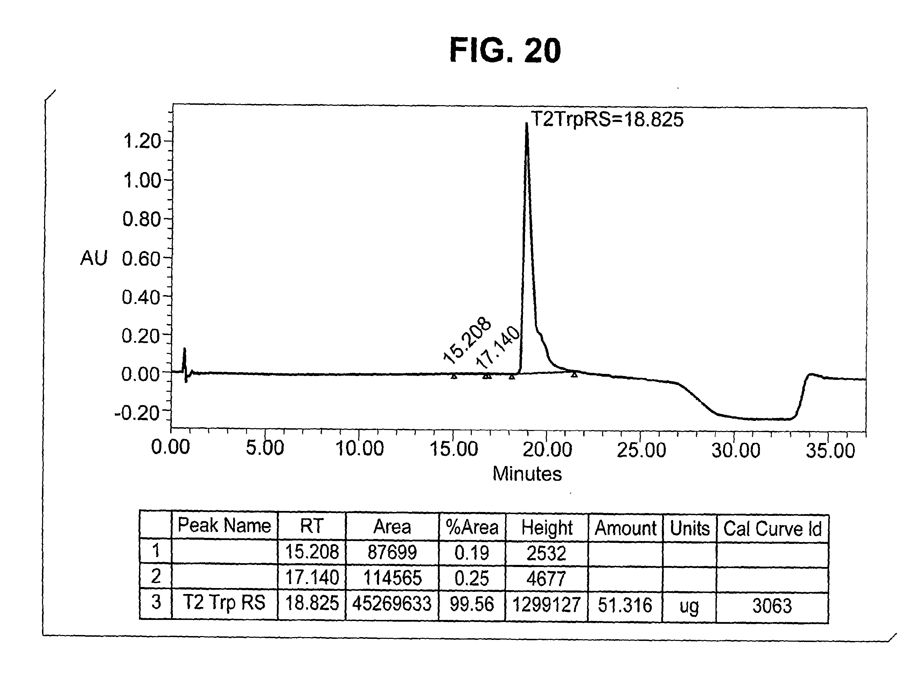

The column flow rate of the column is maintained at 0.50 mL/minute and the column temperature is maintained at 40°C. The main product peak retention time can be identified by comparing sample retention time to reference material retention time. Results from the reverse phase HPLC column are illustrated in Figure 20. The x-axis illustrates retention rate in minutes. The y-axis illustrates absorbance units.

A single peak at roughly 18.825 illustrates that the product is one species (roughly 99.56% of the area under the curve was at a retention time of 18.825 min. ±1 min.). It also demonstrates that the product, which is a dimer does not cleave or fall apart when left at room temperature for three days.

### Example 14

Edman degradation was performed on two T2-TrpRS products produced by *E*. *coli* expression of vector of SEQ ID NO: 70. The first product was purified to about 95% purity and the second product was purified to about 99.5% purity. Both products had an N-terminal sequence that began with SAK.

### Example 15

About 0.5 µL of products produced by *E. coli* transfected with a vector of SEQ ID NO: 70, and purified to about 95% ±4% purity (Product A) and to about 99.5% ±0.5% purity (Product B) were subjected to MALDI-TOF (Voyager DE-STR) mass spectrum analysis.

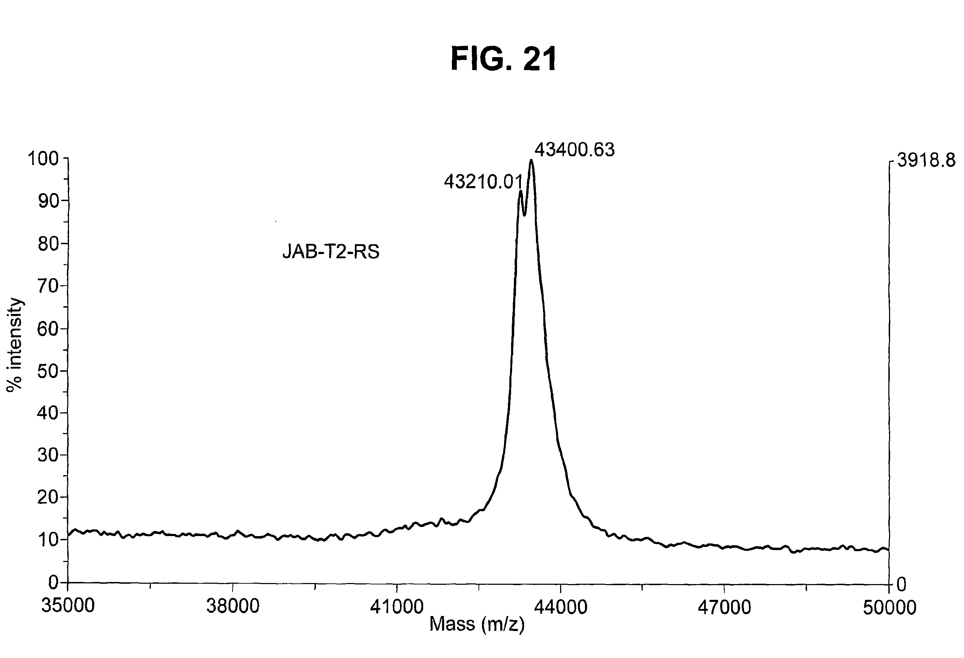

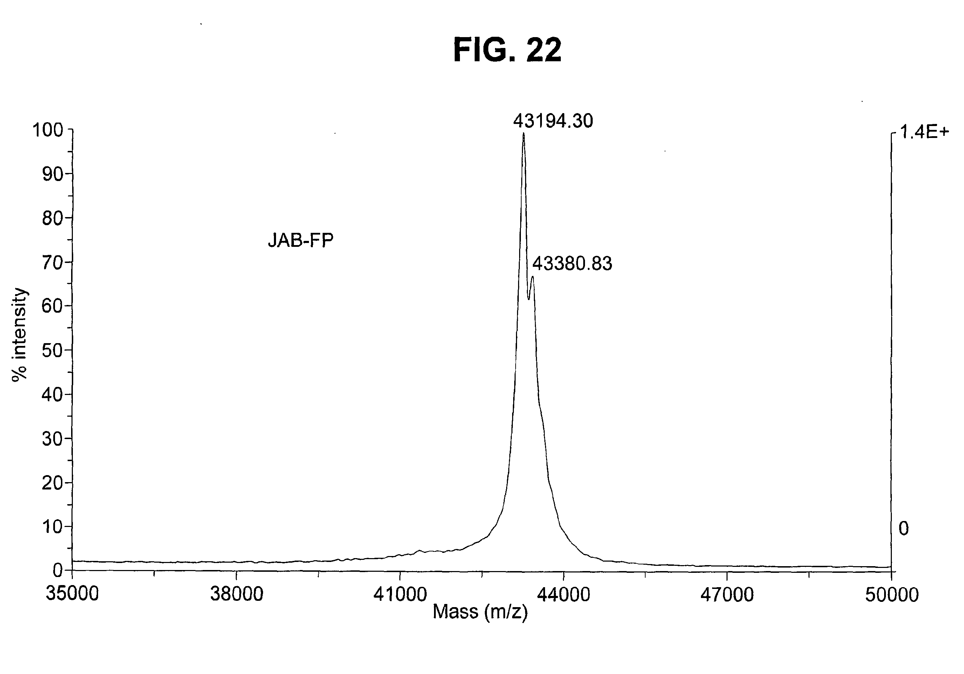

Two major masses were observed in the MALDI-TOF spectra for both the Product A (43210/43400 Da ± 30 Da) and Product B (43194/43380 Da ± 30 Da). The potentially doubly charged ions may indicate the presence of more than one protein mass per sample. Figure 21 illustrates MALDI-TOF spectrum of Product A. Figure 22 illustrates MALDI-TOF spectrum of Product B. The two peaks may be a result from having some product containing an N-formyl methionine not cleaved after protein translation; a matrix effect from the MALDI-TOF device; or other chemical or post-translational modification of the product.

### Example 16

T2-TrpRS product produced by transfection of *E*. *coli* with a vector of SEQ ID NO: 70 was analyzed using electrospray (ESI) mass spectra (QSTARpulsar, Applied Biosystems) and MALDI-TOFF mass spectra (Voyager De STR, Applied Biosystems) to further characterize the resulting T2-TrpRS product, to determine whether the ends were modified, and to determine whether the N-terminus had methionine, no methionine, or a modified methionine.

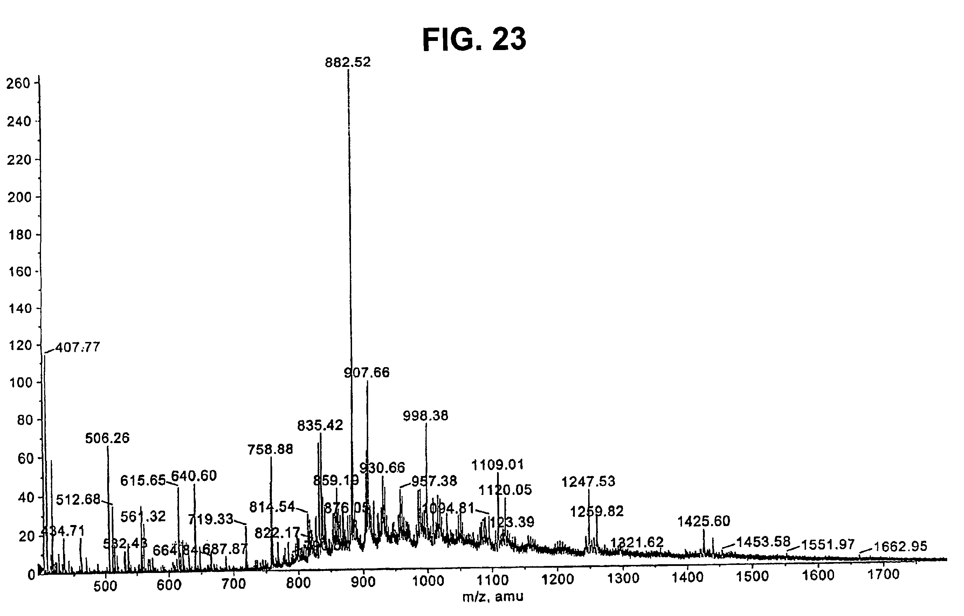

Figure 23 illustrates mass spectrum of the T2-TrpRS product produced by transfection of *E*. *coli* with a vector of SEQ ID NO: 70, and further purification of the product to about 99.5% purity and 0.003 E.U./mg protein, digested by GluC.

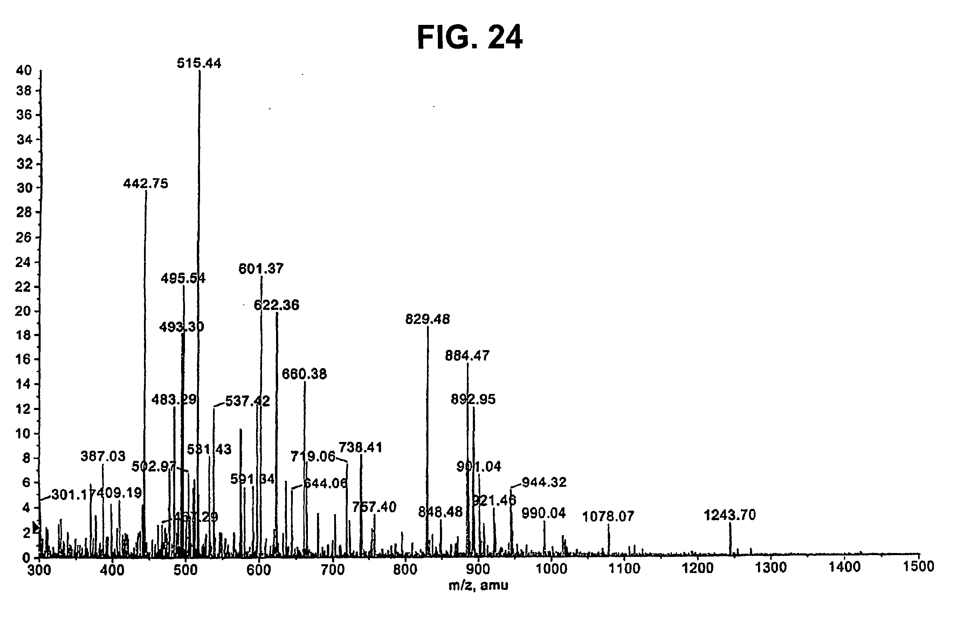

Figure 24 illustrates mass spectrum of the T2-TrpRS product produced by transfection of *E*. *coli* with a vector of SEQ ID NO: 70, and further purification of the product to about 99.5% purity and 0.003 E.U./mg protein, digested with trypsin.

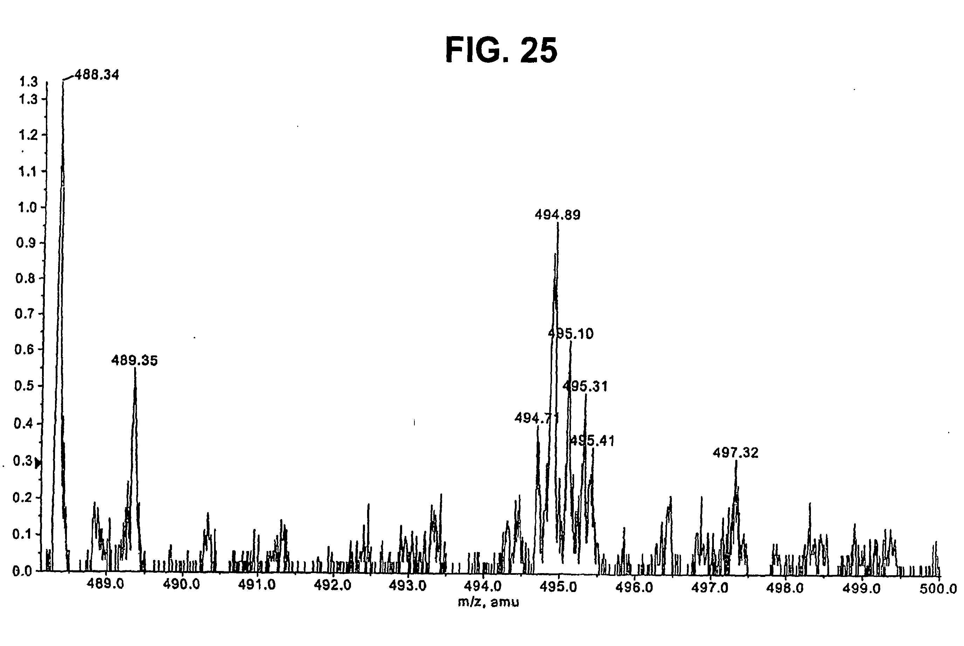

Figure 25 illustrates mass spectrum of the T2-TrpRS product produced by transfection of *E*. *coli* with a vector of SEX ID NO: 70, and further purification of the product to about 99.5% purity and 0.003 E.U./mg protein, digested with GluC showing the N-terminal peptide without a methionine at 494 m/z (Mr=2468). A mass corresponding to N-terminus with methionine or a formyl, oxidized, methylated or acetylated methoinine was not observed. It is noted that the charge state of this peptide is 5. The isotopic masses in this series differ by 1/5 or 0.2 Da. Overall, the signal intensity of the N-terminus without a methionine was below 10 counts even when the protein concentration was as high as 0.4 µg/µL.

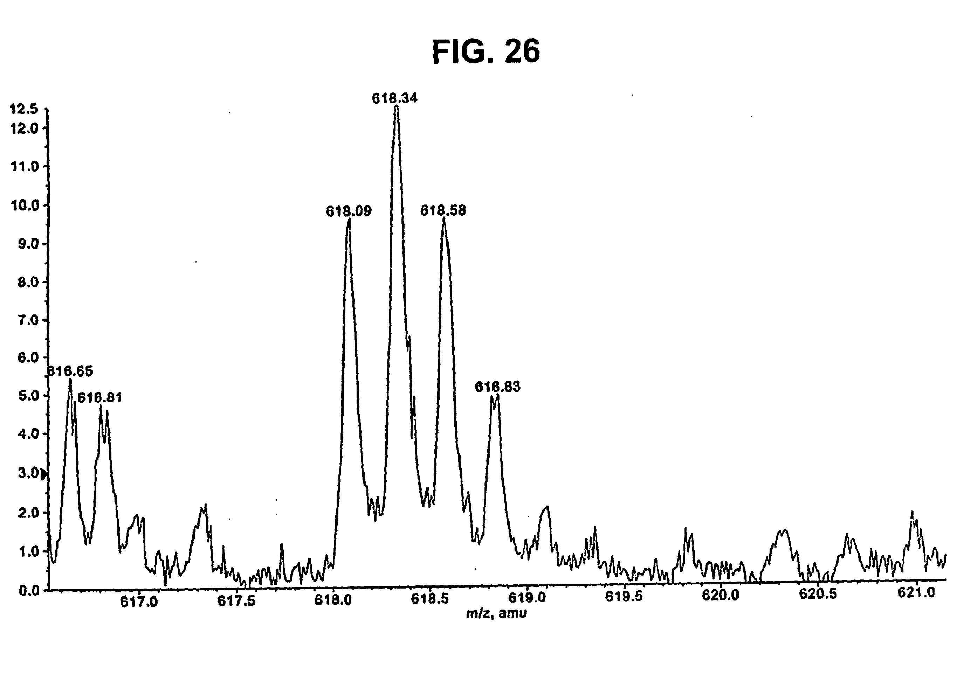

Figure 26 illustrates mass spectrum of T2-TrpRS product produced by transfection of *E. coli* with a vector of SEQ ID NO: 70, and further purification of the product to about 99.5% purity and 0.003 E.U./mg protein, digested with GluC showing N-terminal peptide without a methionine at 618 m/z (Mr=2468). It was noted that the charge state of this T2-TrpRS product was 4. The isotopic masses in this series differed by ¼ or 0.25 Da. Overall, spectra for T2-TrpRS product produced showed that the product was partially digested.

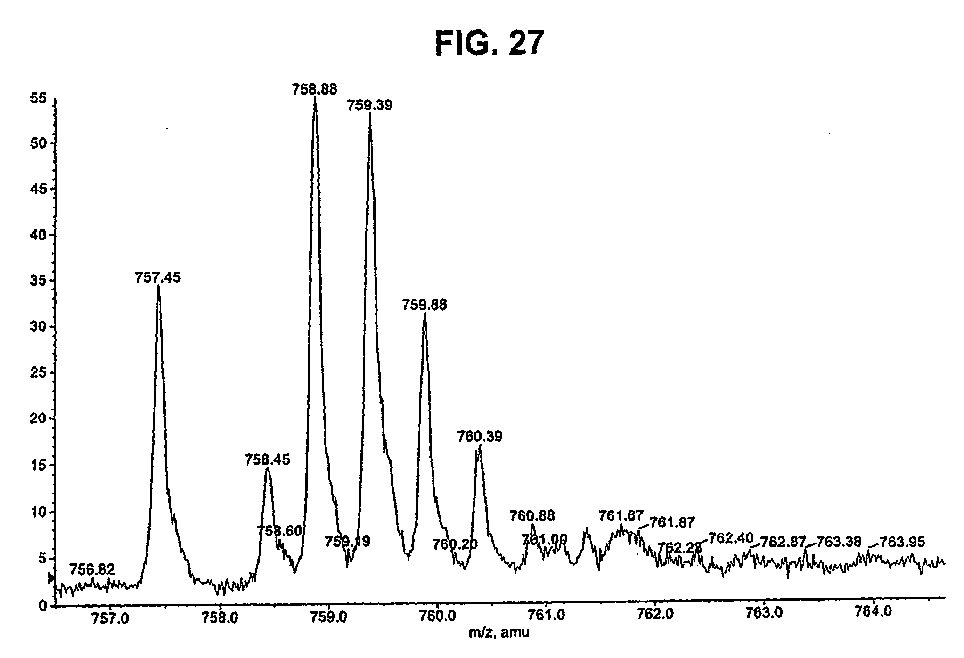

Figure 27 illustrates the mass spectrum of a GluC digested T2-TrpRS product produced by transfection of *E*. *coli* with a vector of SEQ ID NO: 70, and further purification of the product to about 99.5% purity and 0.003 E.U./mg protein, showed a C-terminal peptide without an N-terminal methionine. This peptide was at m/z = 759. As this peptide is doubly charged the mass of it was also doubled or Mr = 1516.

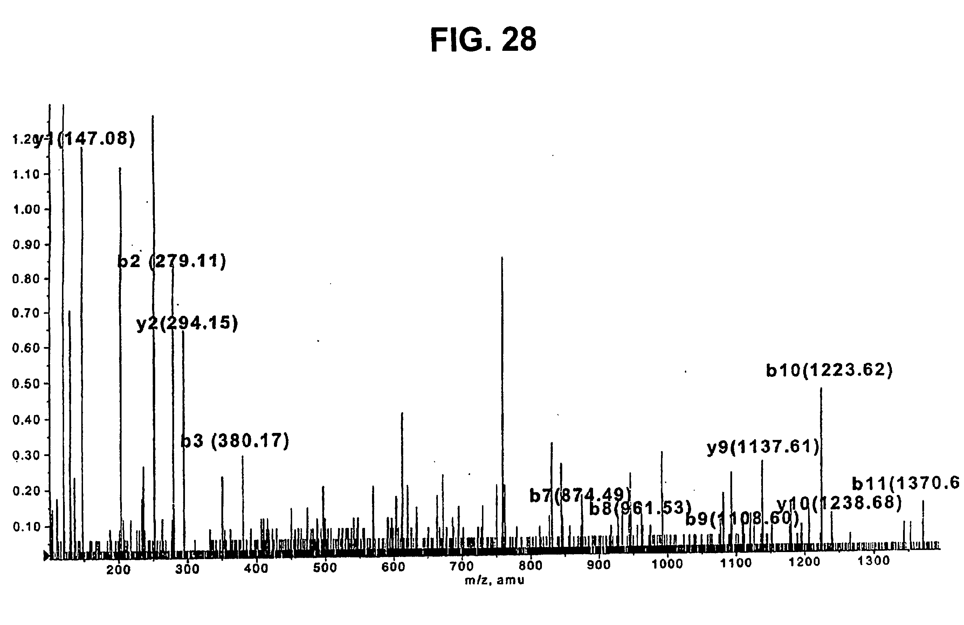

Figure 28 illustrates a fragmentation of the doubly charged mass at m/z = 759 from Figure 28. Only single charged fragments were labeled. Analysis of this spectrum confirms that the C-terminus of the T2-TrpRS product produced by recombinantly expressing the vector of SEQ ID NO: 70 had a sequence of SEQ ID NO: 69. Searching the non-redundant database with this fragmentation data returned a significant hit for human protein IFP53. The sequence of the peptide matched 100% the C-terminal peptide of the T2-TrpRS product T2-TrpRS product produced by transfection of *E*. *coli* with a vector of SEQ ID NO: 70. These results indicated that the T2-TrpRS product produced by transfection of *E*. *coli* with a vector of SEQ ID NO: 70 did not have ragged ends and that the C-terminus of the recombinant product was SEQ ID NO: 69, without a His-tag.

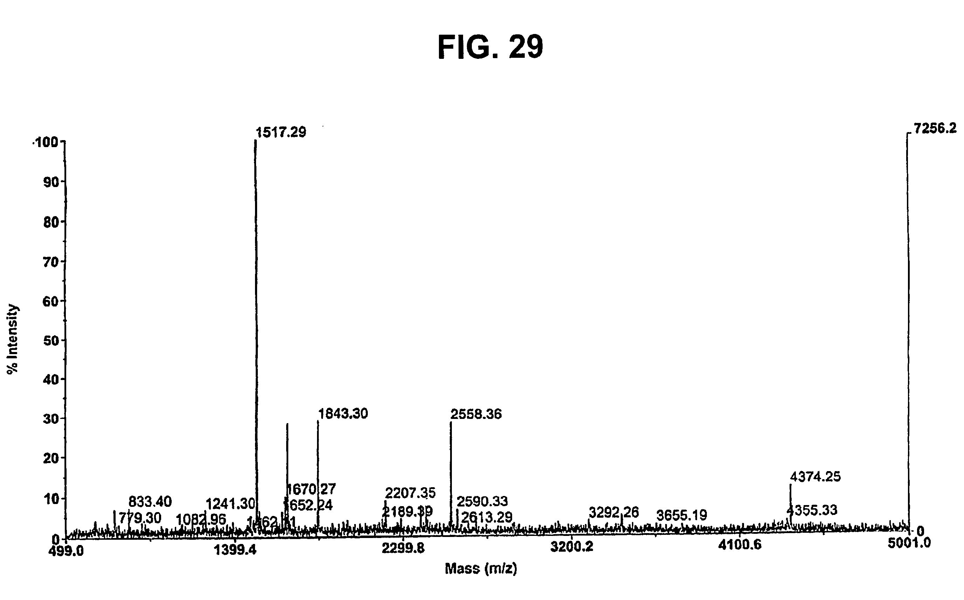

Figure 29 illustrates MALDI-TOF mass spectrum of T2-TrpRS product recombinantly produced in *E*. *coli* with a vector of SEQ ID NO: 70, wherein the product was purified to about 99.5% purity and endotoxin were removed leaving 0.003 E.U./mg protein. The product was then digested by GluC.

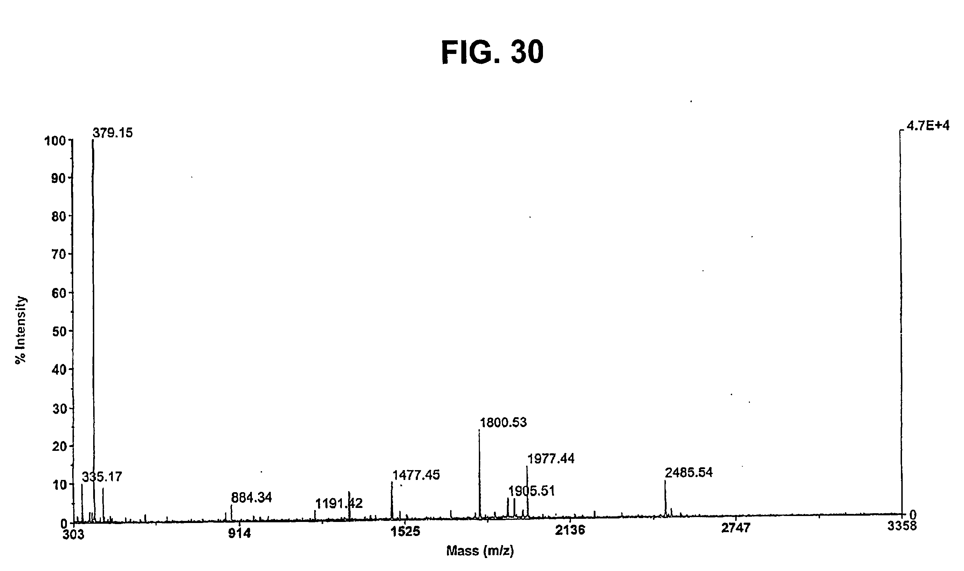

Figure 30 illustrates MALDI-TOF mass spectrum of T2-TrpRS product recombinantly produced in *E. coli* with a vector of SEQ ID NO: 70, wherein the product was purified to about 99.5% purity and endotoxin were removed leaving 0.003 E.U./mg protein. The product was then digested by trypsin.

These MALDI-TOF spectra did not show masses that would correspond to an N-terminus with or without Met.

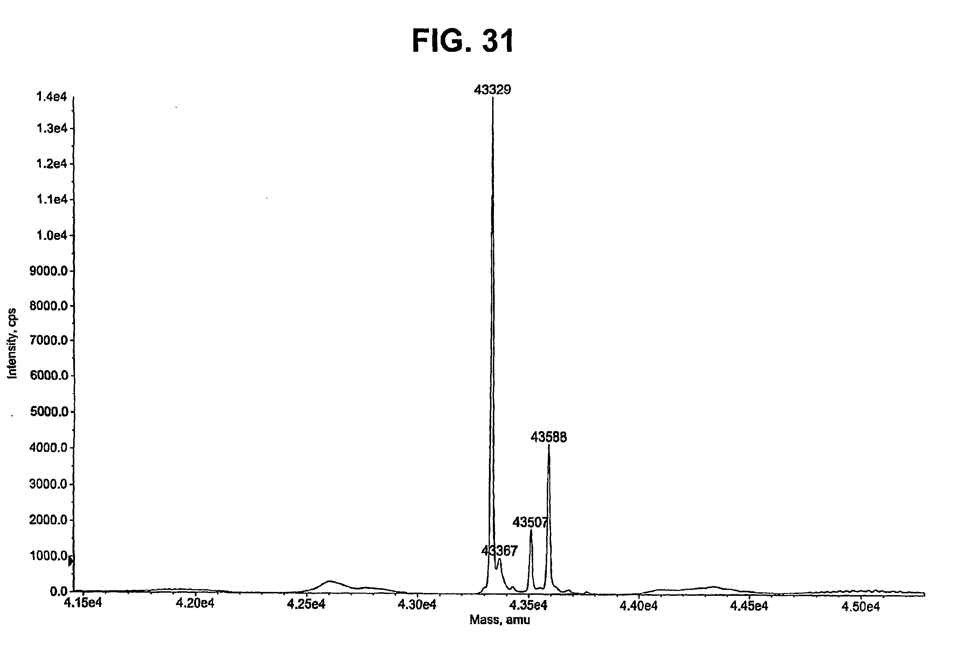

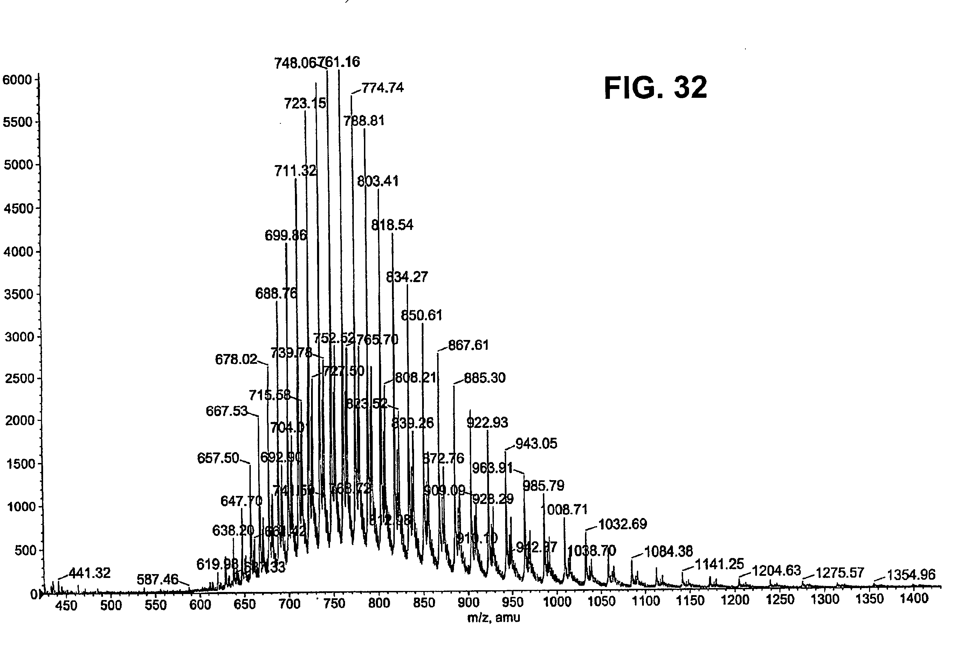

Figure 31 illustrates an electrospray ionization spectrum of a T2-TrpRS product produced by transfection of *E. coli* with a vector of SEQ ID NO: 70, purification to about 99.5% purity, and removal of endotoxins to about 0.003 E.U./mg protein. The product was desalted with a C₄ ZipTip (Millipore). This spectrum illustrates several series of possible multiply-charged ions. When convoluted, as is illustrated in Figure 32, these data show a major component with molecular mass of 43,329 Da and is consistent with the theoretical mass of 43,329 Da for the expected protein minus the N-terminus Met residue. In addition, two notable additional species are also assigned with masses of 43,507 Da and 43,588 Da. The mass difference between these components is close to that expected for phosphorylation although the difference between the major component (43329 Da) and the component with mass (43507 Da) cannot be readily assigned.

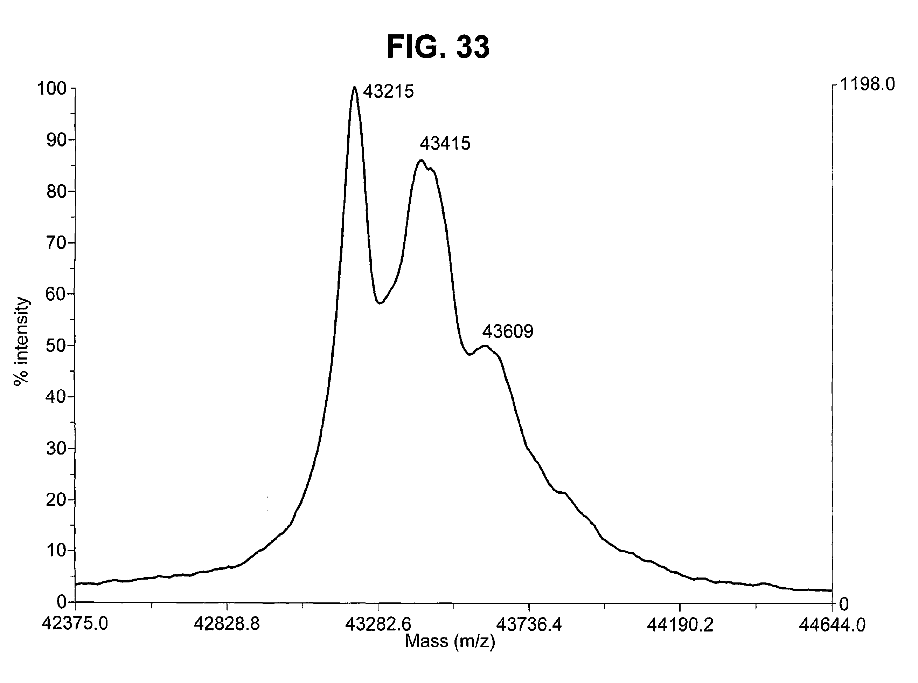

Figure 33 illustrates a MALDI-TOF mass spectrum of a T2-TrpRS product produced by transfection of *E*. *coli* with a vector of SEQ ID NO: 70, purification to about 99.5% purity, and removal of endotoxins to about 0.003 E.U./mg protein. The product was desalted with a C₄ ZipTip (Millipore). The spectrum has major singly-charged pseudomolecular ion clusters having centeres at m/z 43215 and 43415, with the associated doubly-charged ions at m/z 21621 and 21715. Expansions of the singly charged region suggests the 43415 Da cluster to be composed of more than one species and may correspond to the two higher mass species observed in the electrospray spectrum of Figure 31.

### Example 17

### Quantitative Measurements of Enzymatic Aminoacylation Activity

Figure 13 illustrates a PPi exchange assay. TrpRS covalently links tryptophan to its cognate tRNA in a two-step mechanism which is energetically driven by consumption of ATP: The PPi exchange assay measures the enzyme's catalysis of inorganic pyrophosphate (PPi) incorporation into Tryptophanyl-AMP.

The products of this reaction are free tryptophan and free ATP. (This is the reverse reaction of the one used to activate amino acids for attachment to tRNA.) It is used as a measure of enzyme activity in the first half reaction catalyzed by amino acyl tRNA synthetases. As such, it is commonly used to evaluate enzymes for activity. The other (or second half of the reaction) is the subsequent attachment of the amino acid to tRNA. The complete two-step enzyme reaction that measures the overall incorporation of Trp onto tRNA is called an "aminoacylation assay" and can be summarized as follows:
First reaction: Trp + ATP reversibly yields Trp-AMP + PPi
Second reaction: Trp-AMP + tRNA yields Trp-tRNA + AMP
Overall: Trp + ATP + tRNA yields Trp-tRNA + AMP + PPi

In the first step (termed amino acid activation), TrpRS activates the amino acid through a condensation reaction with ATP to generate Trp-AMP with the release of pyrophosphate (PPi). In the second step, the activated amino acid is attached to the 3' end of the cognate tRNA to yield the aminoacylated tRNA (Trp-tRNA) and the release of AMP.

Therefore, the catalytic activity of TrpRS can be characterized in a tryptophan-dependent ATP-PPi exchange (Eq. 1) and aminoacylation assays (sum of Eqs. 1 and 2).

The PPi exchange reactions assess the reverse of amino acid activation by measuring the incorporation of [³²P]-PPi into ATP (Eq. 1). In contrast, aminoacylation assays (sum of Eqs. 1 and 2) measures the amount of [³H]-Tryptophan ligated to its cognate tRNA.

*PPi exchange reaction-* PPi exchange reactions were performed at 100 mM Tris HCl, pH 7.8, 10 mM potassium fluoride, 2 mM magnesium chloride, 1 mM ATP, 2 mM sodium PPi, [³²P]-sodium PPi, 1 mM tryptophan, and 5 mM β-mercaptoethanol. Reactions were initiated by the addition of 0.2 µM enzyme and carried out at room temperature. At each time point, samples were quenched in 4% charcoal, 11 % perchloric acid, and 200 mM sodium PPi. The charcoal was collected and washed twice with 1% perchloric acid and 200 mM sodium PPi prior to scintillation counting.

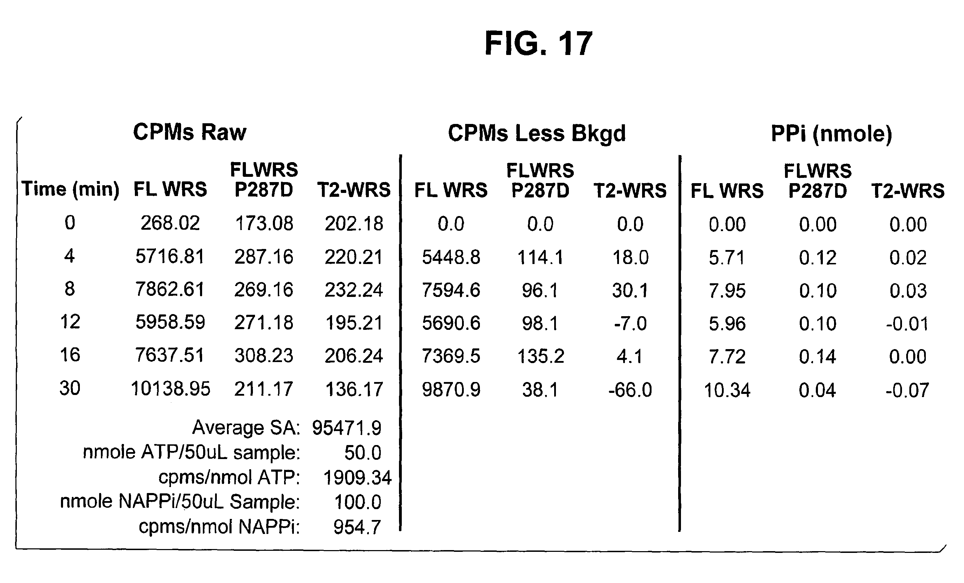

Counts per minute ("CPM's") measuring the incorporation of [³²P]-PPi into ATP were detected for full length TrpRS and T2 produced. Figure 17 (left) illustrates CPMs for full-length TrpRS ("FL WRS"; SEQ ID NO: 63 or 64); a variant of the full-length wherein Pro 287 is converted to an Asp ("FLWRS/P287D"), and of T2-TrpRS derived by recombinantly expressing the vector of SEQ ID NO: 70 in *E*. *coli* according to the methods herein) ("T2-WRS"). Figure 17 (center) illustrates CPMs less background are data wherein the CPM units at time zero have been subtracted out. Figure 17 (right) illustrates final CPM of [³²P]-PPi.

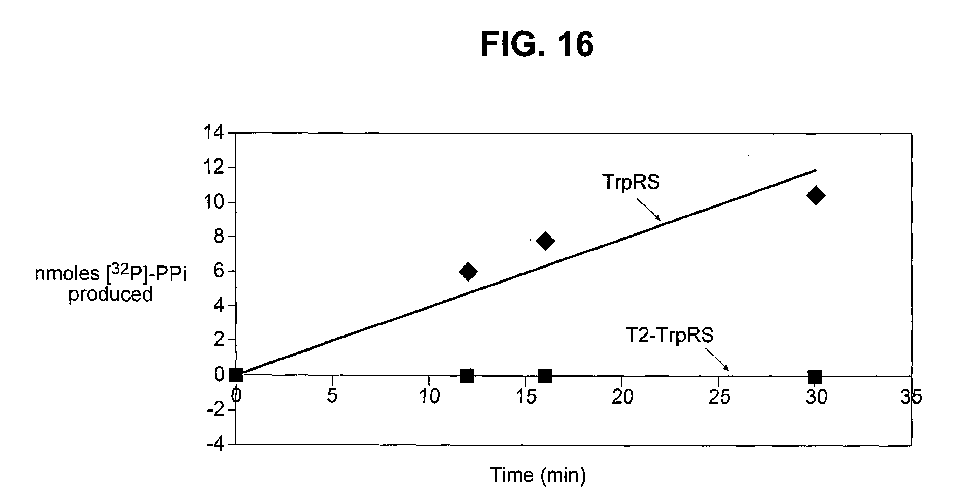

As illustrated by Figure 16, full-length TrpRS incorporated substantially more [³²P]-PPi into ATP than the T2-TrpRS. This result suggests that T2 is largely "inactive" as compared to the full-length TrpRS in its tRNA synthetase activity.

### Example 18

### Quantitative Measurements of Angiostatic Activity

Immediately after birth (P0), retinal vasculature is virtually absent in the mouse. By about three weeks post-natally (P21) the retina has attained an adult pattern of retinal vessels through a stereotypical, biphasic developmental pattern of angiogenesis. Initially, spoke-like peripapillary vessels grow radially from the central retinal artery and vein, becoming progressively interconnected by a capillary plexus that forms between them. The second phase of retinal vessel formation begins around postnatal day 8 (P8) when collateral branches sprout from capillaries of the superficial plexus and penetrate into the retina. Vascular branches then anastamose laterally to form a planar "deep vascular plexus" at the outer edge of the inner nuclear layer, which is in place by P12. An intermediate vascular plexus also forms at the inner edge of the inner nuclear layer between P14 and P20. The development of these vascular networks in the neonatal mouse is strikingly similar to the events occurring in the third trimester human fetus.

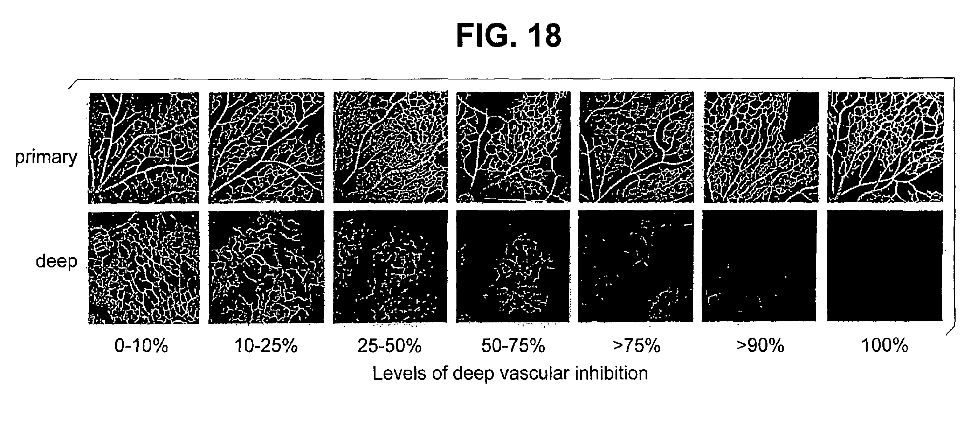

The reproducibility of this process and its easy accessibility in post-natal animals provide an opportunity to test the efficacy of anti-angiogenic compounds in a physiologically relevant model of angiogenesis. The angiostatic activity of T2-TrpRS or other angiostatic molecules was tested by intravitreal injections at P8, just prior to formation of the deep vascular sprouts, and was evaluated based upon the degree of vascular formation in the deep retinal vascular plexus by P12. The appearance of the superficial vascular plexus (primary layer) was evaluated for signs of toxicity and any adverse effects of the drug on the pre-established vasculature. For each retina, the levels of inhibition were graded based on the relative levels of inhibition throughout the entire retina. Figure 18 illustrates various percentages of inhibition by compounds injected at P8 prior to development of the deep vascular plexus, and the effects of neovascularization assed 4 days later.

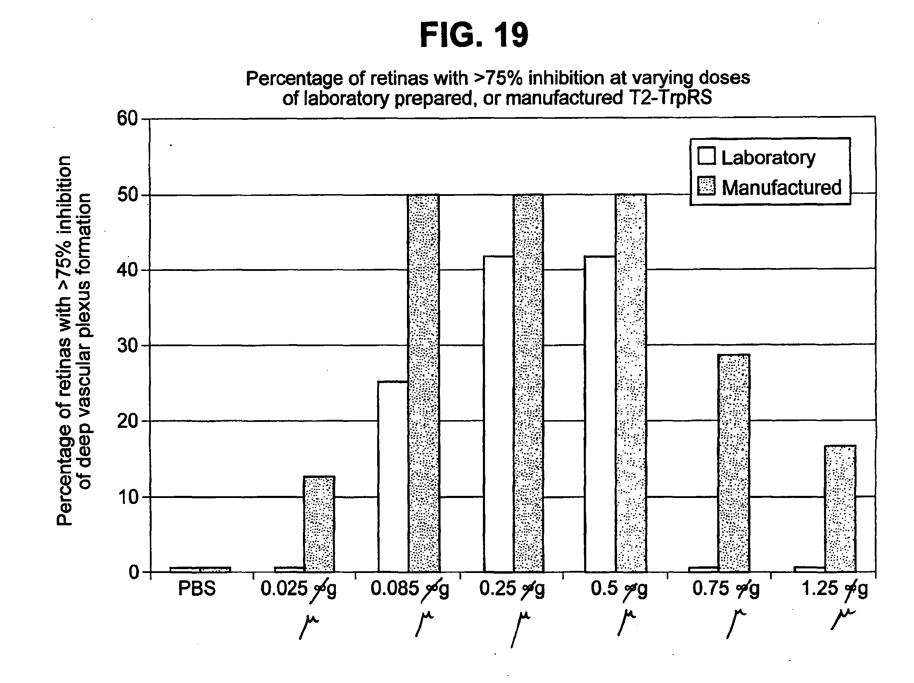

Figure 19 illustrates a comparison of percentage inhibition of angiogenesis by three different T2 manufacture lots at various dosages. On the far left of each dosage comparison is inhibition by "T2-TrpRS SY", a product produced by expressing a polynucleotide encoding SEQ ID NO: 27 with the addition of a C-terminal His₆-tag in *E*. *coli* followed by purification using laboratory techniques (nickel affinity column and Triton X-114). In the center of each dose comparison is "T2-TrpRS 40448," a product produced by expressing a polynucleotide encoding SEQ ID NO: 27 (without a C-terminal His₆-tag) in *E. coli* followed by purification using a linear gradient column chromatography system and an endotoxin filter such that the sample is about 95% pure. On the far right of each dose comparison level is inhibition by "T2-TrpRS PD195", a product produced by expressing a vector of SEQ ID NO: 70 (without a C-terminal His₆-tag) in *E*. *coli*, followed by purification using a scaled-up manufacturing process, Including batch elution column chromatography and an increased area of an endotoxin filter, such that the sample is about 99% pure and further reduced endotoxin levels.

A slight bell-shaped efficacy curve is apparent, with maximum efficacies occurring from injections of 0.25 or 0.50 µg/eye, (5.22 or 10.44 picomoles respectively). Significant Improvements in efficacy have been made with each new manufacturing protocol to date (1^{st} = T₂-TrpRS SY, 2^{nd} = T₂-TrpRS 40448, 3^{rd} = T2-TrpRS PD195-DG30L (P8195)). In addition, with each new manufactured batch, the efficacy curve became significantly broader (Figure 19). These Improvements are likely to be the result of improved purification methods which have yielded nearly 100% levels of purity by the T2-TrpRS PD195 batch.

The y-axis of Figure 19 Illustrates percentage of retinas with >75% inhibition. This percentage inhibition can also be referred to herein in activity units. For example, if 50% of retinas experienced >75% inhibition, the protein activity is deemed at 50 activity units, if 70% of retinas experiences >75% inhibition, the protein activity is deemed at 70 activity units.

### SUMMARY OF SEQUENCES

SEQ ID NO: 1. - Met-TrpRS-His tag (amino acid plus nucleic acid vector) (the GD variant)
SEQ ID NO: 2. - Met-mini-Trp-His tag (amino acid plus nucleic acid) (the GD variant)
SEQ ID NO: 3. - Met-mini-TrpRS-His tag (amino acid) (the GD variant)
SEQ ID NO: 4. - Met- T1-His tag (amino acid plus nucleic acid vector) (the GD variant)
SEQ ID NO: 5. - Met- T1-His tag (amino acid) (the GD variant)
SEQ ID NO: 6. - Met-T2-His tag (amino acid plus nucleic acid vector) (the GD variant)
SEQ ID NO: 7. - Met- T2-His tag (amino acid) (the GD variant)
SEQ ID NO: 8. - SNHGP (beginning sequence of T1) (the GD variant)
SEQ ID NO: 9. - SAKGI (beginning sequence of T2) (the GD variant)
SEQ ID NO: 10. - HVGH (internal sequence)
SEQ ID NO: 11. - KMSAS (Internal sequence)
SEQ ID NO: 12. - T2 (the GD variant)
SEQ ID NO: 13. - T1 (the GD variant)
SEQ ID NO: 14. - mini-TrpRS (the GD variant)
SEQ ID NO: 15. - Met-T2 (the GD variant)
SEQ ID NO: 16. - Met-T1 (the GD variant)
SEQ ID NO: 17. - Met-mini-TrpRS (the GD variant)
SEQ ID NO: 18. - nucleic acid encoding T2 (the GD variant)
SEQ ID NO: 19. - nucleic acid encoding Met-T2 (the GD variant)
SEQ ID NO: 20. - nucleic acid encoding T1 (the GD variant)
SEQ ID NO: 21. - nucleic acid encoding Met-T1 (the GD variant)
SEQ ID NO: 22. - nucleic acid encoding mini-TrpRS (the GD variant)
SEQ ID NO: 23. - nucleic acid encoding Met-mini-TrpRS (the GD variant)
SEQ ID NO: 24. - T2 (the SY variant)
SEQ ID NO: 25. - T1 (the SY variant)
SEQ ID NO: 26. - mini-TrpRS (the SY variant)
SEQ ID NO: 27. - Met-T2 (the SY variant)
SEQ ID NO: 28. - Met-T1 (the SY variant)
SEQ ID NO: 29. - Met-mini-TrpRS (the SY variant)
SEQ ID NO: 30. - nucleic acid encoding T2 (the SY variant)
SEQ ID NO: 31. - nucleic acid encoding Met-T2 (the SY variant)
SEQ ID NO: 32. - nucleic acid encoding T1 (the SY variant)
SEQ ID NO: 33. - nucleic acid encoding Met-T1 (the SY variant)
SEQ ID NO: 34. - nucleic acid encoding mini-TrpRS (the SY variant)
SEQ ID NO: 35. - nucleic acid encoding Met-mini-TrpRS (the SY variant)
SEQ ID NO: 36. - T2 (the GY variant)
SEQ ID NO: 37. - T1 (the GY variant)
SEQ ID NO: 38. - mini-TrpRS (the GY variant)
SEQ ID NO: 39. - Met-T2 (the GY variant)
SEQ ID NO: 40. - Met-T1 (the GY variant)
SEQ ID NO: 41. - Met-mini-TrpRS (the GY variant)
SEQ ID NO: 42. - nucleic acid encoding T2 (the GY variant)
SEQ ID NO: 43. - nucleic acid encoding Met-T2 (the GY variant)
SEQ ID NO: 44. - nucleic acid encoding T1 (the GY variant)
SEQ ID NO: 45. - nucleic acid encoding Met-T1 (the GY variant)
SEQ ID NO: 46. - nucleic acid encoding mini-TrpRS (the GY variant)
SEQ ID NO: 47. - nucleic acid encoding Met-mini-TrpRS (the GY variant)
SEQ ID NO: 48. - T2 (the SD variant)
SEQ ID NO: 49. - T1 (the SD variant)
SEQ ID NO: 50. - mini-TrpRS (the SD variant)
SEQ ID NO: 51. - Met-T2 (the SD variant)
SEQ ID NO: 52. - Met-T1 (the SD variant)
SEQ ID NO: 53. - Met-mini-TrpRS (the SD variant)
SEQ ID NO: 54. - nucleic acid encoding T2 (the SD variant)
SEQ ID NO: 55. - nucleic acid encoding Met-T2 (the SD variant)
SEQ ID NO: 56. - nucleic acid encoding T1 (the SD variant)
SEQ ID NO: 57. - nucleic acid encoding Met-T1 (the SD variant)
SEQ ID NO: 58. - nucleic acid encoding mini-TrpRS (the SD variant)
SEQ ID NO: 59. - nucleic acid encoding Met-mini-TrpRS (the SD variant)
SEQ ID NO: 60. Dimerization domain (from T2 144-199)
SEQ ID NO: 61. - Full Length GD variant, with N-terminal Met, and no His-tag
SEQ ID NO: 62. - Full Length GD variant, without N-terminal Met, and no His-tag
SEQ ID NO: 63. - Full Length SY variant, with N-terminal Met, and no His-tag
SEQ ID NO: 64. - Full Length SY variant, without N-terminal Met, and no His-tag
SEQ ID NO: 65. - Full Length GY variant, with N-terminal Met, and no His-tag
SEQ ID NO: 66. - Full Length GY variant, without N-terminal Met, and no His-tag
SEQ ID NO: 67. - Full Length SD variant, with N-terminal Met, and no His-tag
SEQ ID NO: 68. - Full Length SD variant, without N-terminal Met, and no His-tag
SEQ ID NO: 69. - C-terminus: FMTPRKLSFDFQ.
SEQ ID NO: 70. - Plasmid 01 - pET24b+ with a NdeI/HindIII insert of Human T2-TrpRS (SY variant) without 6-His Tag
SEQ ID NO: 71. - Plasmid 02: pET20b+ with a NdeI/HindIII insert of Human T2-TrpRS (SY variant), with 6-His Tag
SEQ ID NO: 72. - Plasmid 04 pET20b+ with a NdeI/HindIII insert of T2-TrpRS (SY variant), 6-His Tag with Thrombin Cleavage Site
SEQ ID NO: 73. - Plasmid 06 pET24b+ with a NdeI/XhoI insert of Human mini-TyrRS, 6-His Tag.
SEQ ID NO: 74. - Plasmid 07 pET24b+ with a NdeI/HindIII insert of Human mini-TrpRS, (SY variant) 6-His Tag
SEQ ID NO: 75. - Plasmid 09: pET24b+ with a NdeI/XhoI insert of Human mini-TyrRS, No His Tag

## Claims

1. A method for purifying a tRNA synthetase fragment, comprising performing an endotoxin- reduction filtration step after performing a clarification step and prior to performing at least one of the steps selected from: a buffer exchange step; a concentration step; and a cation-exchange chromatographic step, wherein said cation-exchange chromatographic step comprises the use of a cation-exchange resin, wherein said resin is selected from: CM Sepharose, SP Sepharose, and DEAE Sepharose.

2. The method of claim 1 wherein said method does not include the use of a denaturant.

3. A method for purifying a tRNA synthetase fragment, comprising performing an endotoxin- reduction filtration step after performing an anion-exchange chromatographic step wherein said anion-exchange chromatographic step comprises the use of an anion-exchange resin, wherein said resin is selected from: Q Sepharose, DEAE Sepharose, and ANX Sepharose.

4. The method of any one of claims 1 to 3 wherein said tRNA synthetase fragment is selected from the group consisting of SEQ ID NO: 12, 13, 14, 15, 16, 17, 24, 25, 26, 27, 28, 29, 36, 37, 38, 39, 40, 41, 48, 49, 50, 51, 52 and 53.

## Patentansprüche

1. Verfahren zur Reinigung eines tRNA-Synthetase-Fragments, umfassend Durchführen eines Endotoxin-Reduktions-Filtrationsschritts nach Durchführung eines Klärungsschritts und vor Durchführung wenigstens eines der Schritte, ausgewählt aus: einem Pufferaustauschschritt; einem Konzentrierungsschritt und einem Kationenaustauschchromatographieschritt, wobei der Kationenaustauschchromatographieschritt die Verwendung eines Kationenaustauschharzes umfasst, wobei das Harz ausgewählt ist aus: CM-Sepharose, SP-Sepharose und DEAE-Sepharose.

2. Verfahren gemäß Anspruch 1, wobei das Verfahren keine Verwendung eines Denaturierungsmittels einschließt.

3. Verfahren zur Reinigung eines tRNA-Synthetase-Fragments, umfassend Durchführen eines Endotoxin-Reduktions-Filtrationsschritt nach Durchführen eines Anionenaustauschchromatographieschritts, wobei der Anionenaustauschchromatographieschritt die Verwendung eines Anionenaustauschharzes umfasst, wobei das Harz ausgewählt ist aus: Q-Sepharose, DEAE-Sepharose und ANX-Sepharose.

4. Verfahren gemäß einem der Ansprüche 1 bis 3, wobei das tRNA-Synthetase-Fragment ausgewählt ist aus der Gruppe, bestehend aus SEQ ID NO: 12, 13, 14, 15, 16, 17, 24, 25, 26, 27, 28, 29, 36, 37, 38, 39, 40, 41, 48, .49, 50, 51, 52 und 53.

## Revendications

1. Procédé de purification d'un fragment d'ARNt synthétase, comprenant la mise en oeuvre d'une étape de réduction des endotoxines par filtration après mise en oeuvre d'une étape de clarification et avant mise en oeuvre d'au moins l'une des étapes suivantes : une étape d'échange de tampon ; une étape de concentration ; et une étape chromatographique d'échange de cations, ladite étape chromatographique d'échange de cations comprenant l'utilisation d'une résine échangeuse de cations, ladite résine étant sélectionnée parmi : la CM Sepharose, la SP Sepharose et la DEAE Sepharose.

2. Procédé selon la revendication 1, ledit procédé n'incluant pas l'utilisation d'un dénaturant.

3. Procédé de purification d'un fragment d'ARNt synthétase, comprenant la mise en oeuvre d'une étape de réduction des endotoxines par filtration après mise en oeuvre d'une étape chromatographique d'échange d'anions, ladite étape chromatographique d'échange d'anions comprenant l'utilisation d'une résine échangeuse d'anions, ladite résine étant sélectionnée parmi : la Q Sepharose, la DEAE Sepharose et l'ANX Sepharose.

4. Procédé selon l'une quelconque des revendications 1 à 3, dans lequel ledit fragment d'ARNt synthétase est sélectionné dans le groupe consistant en SEQ ID N0: 12, 13, 14, 15, 16, 17, 24, 25, 26, 27, 28, 29, 36, 37, 38, 39, 40, 41, 48, 49, 50, 51, 52 et 53.


### SEQUENCE LISTING

<110> GLIDDEN, PAUL F.
<120> COMPOSITIONS AND METHODS FOR MODULATING ANGIOGENESIS
<130> PC22120A
<140>
   <141>
<160> 75
<170> PatentIn ver. 3.2
<210> 1
   <211> 484
   <212> PRT
   <213> Artificial Sequence
<220>
   <223> Recombinant human trpRS
<400> 1
   Met Pro Asn Ser Glu Pro Ala Ser Leu Leu Glu Leu Phe Asn Ser Ile
   1               5                   10                  15
   Ala Thr Gln Gly Glu Leu Val Arg Ser Leu Lys Ala Gly Asn Ala Ser
               20                  25                  30
   Lys Asp Glu Ile Asp Ser Ala Val Lys Met Leu Val Ser Leu Lys Met
           35                  40                  45
   Ser Tyr Lys Ala Ala Ala Gly Glu Asp Tyr Lys Ala Asp Cys Pro Pro
       50                  55                  60
   Gly Asn Pro Ala Pro Thr Ser Asn His Gly Pro Asp Ala Thr Glu Ala
   65                  70                  75                  80
   Glu Glu Asp Phe Val Asp Pro Trp Thr Val Gln Thr Ser Ser Ala Lys
                   85                  90                  95
   Gly Ile Asp Tyr Asp Lys Leu Ile Val Arg Phe Gly Ser Ser Lys Ile
               100                 105                 110
   Asp Lys Glu Leu Ile Asn Arg Ile Glu Arg Ala Thr Gly Gln Arg Pro
           115                 120                 125
   His His Phe Leu Arg Arg Gly Ile Phe Phe Ser His Arg Asp Met Asn
       130                 135                 140
   Gln Val Leu Asp Ala Tyr Glu Asn Lys Lys Pro Phe Tyr Leu Tyr Thr
   145                 150                 155                 160
   Gly Arg Gly Pro Ser Ser Glu Ala Met His Val Gly His Leu Ile Pro
                   165                 170                 175
   Phe Ile Phe Thr Lys Trp Leu Gln Asp Val Phe Asn Val Pro Leu Val
               180                 185                 190
   Ile Gln Met Thr Asp Asp Glu Lys Tyr Leu Trp Lys Asp Leu Thr Leu
           195                 200                 205
   Asp Gln Ala Tyr Gly Asp Ala Val Glu Asn Ala Lys Asp Ile Ile Ala
       210                 215                 220
   Cys Gly Phe Asp Ile Asn Lys Thr Phe Ile Phe Ser Asp Leu Asp Tyr
   225 230 235 240
   Met Gly Met Ser Ser Gly Phe Tyr Lys Asn Val Val Lys Ile Gln Lys
   245 250 255
   His Val Thr Phe Asn Gln Val Lys Gly Ile Phe Gly Phe Thr Asp Ser
   260 265 270
   Asp Cys Ile Gly Lys Ile Ser Phe Pro Ala Ile Gln Ala Ala Pro Ser
   275 280 285
   Phe Ser Asn Ser Phe Pro Gln Ile Phe Arg Asp Arg Thr Asp Ile Gln
   290 295 300
   Cys Leu Ile Pro Cys Ala Ile Asp Gln Asp Pro Tyr Phe Arg Met Thr
   305 310 315 320
   Arg Asp Val Ala Pro Arg Ile Gly Tyr Pro Lys Pro Ala Leu Leu His
   325 330 335
   Ser Thr Phe Phe Pro Ala Leu Gln Gly Ala Gln Thr Lys Met Ser Ala
   340 345 350
   Ser Asp Pro Asn Ser Ser Ile Phe Leu Thr Asp Thr Ala Lys Gln Ile
   355 360 365
   Lys Thr Lys Val Asn Lys His Ala Phe Ser Gly Gly Arg Asp Thr Ile
   370 375 380
   Glu Glu His Arg Gln Phe Gly Gly Asn Cys Asp Val Asp Val Ser Phe
   385 390 395 400
   Met Tyr Leu Thr Phe Phe Leu Glu Asp Asp Asp Lys Leu Glu Gln Ile
   405 410 415
   Arg Lys Asp Tyr Thr Ser Gly Ala Met Leu Thr Gly Glu Leu Lys Lys
   420 425 430
   Ala Leu Ile Glu Val Leu Gln Pro Leu Ile Ala Glu His Gln Ala Arg
   435 440 445
   Arg Lys Glu Val Thr Asp Glu Ile Val Lys Glu Phe Met Thr Pro Arg
   450 455 460
   Lys Leu Ser Phe Asp Phe Gln Lys Leu Ala Ala Ala Leu Glu His His
   465 470 475 480
   His His His His
<210> 2
   <211> 4877
   <212> DNA
   <213> Artificial Sequence
<220>
   <223> Recombinant human mini-TrpRS nucleotide construct
<220>
   <221> CDS
   <222> (3428)..(4738)
<400> 2
   tggcgaatgg gacgcgccct gtagcggcgc attaagcgcg gcgggtgtgg tggttacgcg 60
   cagcgtgacc gctacacttg ccagcgccct agcgcccgct cctttcgctt tcttcccttc 120
   ctttctcgcc acgttcgccg gctttccccg tcaagctcta aatcgggggc tccctttagg 180
   gttccgattt agtgctttac ggcacctcga ccccaaaaaa cttgattagg gtgatggttc 240
   acgtagtggg ccatcgccct gatagacggt ttttcgccct ttgacgttgg agtccacgtt 300
   ctttaatagt ggactcttgt tccaaactgg aacaacactc aaccctatct cggtctattc 360
   ttttgattta taagggattt tgccgatttc ggcctattgg ttaaaaaatg agctgattta 420
   acaaaaattt aacgcgaatt ttaacaaaat attaacgttt acaatttcag gtggcacttt 480
   tcggggaaat gtgcgcggaa cccctatttg tttatttttc taaatacatt caaatatgta 540
   tccgctcatg agacaataac cctgataaat gcttcaataa tattgaaaaa ggaagagtat 600
   gagtattcaa catttccgtg tcgcccttat tccctttttt gcggcatttt gccttcctgt 660
   ttttgctcac ccagaaacgc tggtgaaagt aaaagatgct gaagatcagt tgggtgcacg 720
   agtgggttac atcgaactgg atctcaacag cggtaagatc cttgagagtt ttcgccccga 780
   agaacgtttt ccaatgatga gcacttttaa agttctgcta tgtggcgcgg tattatcccg 840
   tattgacgcc gggcaagagc aactcggtcg ccgcatacac tattctcaga atgacttggt 900
   tgagtactca ccagtcacag aaaagcatct tacggatggc atgacagtaa gagaattatg 960
   cagtgctgcc ataaccatga gtgataacac tgcggccaac ttacttctga caacgatcgg 1020
   aggaccgaag gagctaaccg cttttttgca caacatgggg gatcatgtaa ctcgccttga 1080
   tcgttgggaa ccggagctga atgaagccat accaaacgac gagcgtgaca ccacgatgcc 1140
   tgcagcaatg gcaacaacgt tgcgcaaact attaactggc gaactactta ctctagcttc 1200
   ccggcaacaa ttaatagact ggatggaggc ggataaagtt gcaggaccac ttctgcgctc 1260
   ggcccttccg gctggctggt ttattgctga taaatctgga gccggtgagc gtgggtctcg 1320
   cggtatcatt gcagcactgg ggccagatgg taagccctcc cgtatcgtag ttatctacac 1380
   gacggggagt caggcaacta tggatgaacg aaatagacag atcgctgaga taggtgcctc 1440
   actgattaag cattggtaac tgtcagacca agtttactca tatatacttt agattgattt 1500
   aaaacttcat ttttaattta aaaggatcta ggtgaagatc ctttttgata atctcatgac 1560
   caaaatccct taacgtgagt tttcgttcca ctgagcgtca gaccccgtag aaaagatcaa 1620
   aggatcttct tgagatcctt tttttctgcg cgtaatctgc tgcttgcaaa caaaaaaacc 1680
   accgctacca gcggtggttt gtttgccgga tcaagagcta ccaactcttt ttccgaaggt 1740
   aactggcttc agcagagcgc agataccaaa tactgtcctt ctagtgtagc cgtagttagg 1800
   ccaccacttc aagaactctg tagcaccgcc tacatacctc gctctgctaa tcctgttacc 1860
   agtggctgct gccagtggcg ataagtcgtg tcttaccggg ttggactcaa gacgatagtt 1920
   accggataag gcgcagcggt cgggctgaac ggggggttcg tgcacacagc ccagcttgga 1980
   gcgaacgacc tacaccgaac tgagatacct acagcgtgag ctatgagaaa gcgccacgct 2040
   tcccgaaggg agaaaggcgg acaggtatcc ggtaagcggc agggtcggaa caggagagcg 2100
   cacgagggag cttccagggg gaaacgcctg gtatctttat agtcctgtcg ggtttcgcca 2160
   cctctgactt gagcgtcgat ttttgtgatg ctcgtcaggg gggcggagcc tatggaaaaa 2220
   cgccagcaac gcggcctttt tacggttcct ggccttttgc tggccttttg ctcacatgtt 2280
   ctttcctgcg ttatcccctg attctgtgga taaccgtatt accgcctttg agtgagctga 2340
   taccgctcgc cgcagccgaa cgaccgagcg cagcgagtca gtgagcgagg aagcggaaga 2400
   gcgcctgatg cggtattttc tccttacgca tctgtgcggt atttcacacc gcatatatgg 2460
   tgcactctca gtacaatctg ctctgatgcc gcatagttaa gccagtatac actccgctat 2520
   cgctacgtga ctgggtcatg gctgcgcccc gacacccgcc aacacccgct gacgcgccct 2580
   gacgggcttg tctgctcccg gcatccgctt acagacaagc tgtgaccgtc tccgggagct 2640
   gcatgtgtca gaggttttca ccgtcatcac cgaaacgcgc gaggcagctg cggtaaagct 2700
   catcagcgtg gtcgtgaagc gattcacaga tgtctgcctg ttcatccgcg tccagctcgt 2760
   tgagtttctc cagaagcgtt aatgtctggc ttctgataaa gcgggccatg ttaagggcgg 2820
   ttttttcctg tttggtcact gatgcctccg tgtaaggggg atttctgttc atgggggtaa 2880
   tgataccgat gaaacgagag aggatgctca cgatacgggt tactgatgat gaacatgccc 2940
   ggttactgga acgttgtgag ggtaaacaac tggcggtatg gatgcggcgg gaccagagaa 3000
   aaatcactca gggtcaatgc cagcgcttcg ttaatacaga tgtaggtgtt ccacagggta 3060
   gccagcagca tcctgcgatg cagatccgga acataatggt gcagggcgct gacttccgcg 3120
   tttccagact ttacgaaaca cggaaaccga agaccattca tgttgttgct caggtcgcag 3180
   acgttttgca gcagcagtcg cttcacgttc gctcgcgtat cggtgattca ttctgctaac 3240
   cagtaaggca accccgccag cctagccggg tcctcaacga caggagcacg atcatgcgca 3300
   cccgtggcca ggacccaacg ctgcccgaga tctcgatccc gcgaaattaa tacgactcac 3360
   tatagggaga ccacaacggt ttccctctag aaataatttt gtttaacttt aagaaggaga 3420
   tatacat atg agc tac aaa gct gcc gcg ggg gag gat tac aag gct gac    3469
           Met Ser Tyr Lys Ala Ala Ala Gly Glu Asp Tyr Lys Ala Asp
           1               5                   10
   tgt cct cca ggg aac cca gca cct acc agt aat cat ggc cca gat gcc    3517
   Cys Pro Pro Gly Asn Pro Ala Pro Thr Ser Asn His Gly Pro Asp Ala
   15                  20                  25                  30
   aca gaa gct gaa gag gat ttt gtg gac cca tgg aca gta cag aca agc    3565
   Thr Glu Ala Glu Glu Asp Phe Val Asp Pro Trp Thr Val Gln Thr Ser
                   35                  40                  45
   agt gca aaa ggc ata gac tac gat aag ctc att gtt cgg ttt gga agt    3613
   Ser Ala Lys Gly Ile Asp Tyr Asp Lys Leu Ile Val Arg Phe Gly Ser
               50                  55                  60
   agt aaa att gac aaa gag cta ata aac cga ata gag aga gcc acc ggc    3661
   Ser Lys Ile Asp Lys Glu Leu Ile Asn Arg Ile Glu Arg Ala Thr Gly
           65                  70                  75
   caa aga cca cac cac ttc ctg cgc aga ggc atc ttc ttc tca cac aga 3709
   Gln Arg Pro His His Phe Leu Arg Arg Gly Ile Phe Phe Ser His Arg
   80 85 90
   gat atg aat cag gtt ctt gat gcc tat gaa aat aag aag cca ttt tat 3757
   Asp Met Asn Gln Val Leu Asp Ala Tyr Glu Asn Lys Lys Pro Phe Tyr
   95 100 105 110
   ctg tac acg ggc cgg ggc ccc tct tct gaa gca atg cat gta ggt cac 3805
   Leu Tyr Thr Gly Arg Gly Pro Ser Ser Glu Ala Met His Val Gly His
   115 120 125
   ctc att cca ttt att ttc aca aag tgg ctc cag gat gta ttt aac gtg 3853
   Leu Ile Pro Phe Ile Phe Thr Lys Trp Leu Gln Asp Val Phe Asn Val
   130 135 140
   ccc ttg gtc atc cag atg acg gat gac gag aag tat ctg tgg aag gac 3901
   Pro Leu Val Ile Gln Met Thr Asp Asp Glu Lys Tyr Leu Trp Lys Asp
   145 150 155
   ctg acc ctg gac cag gcc tat ggc gat gct gtt gag aat gcc aag gac 3949
   Leu Thr Leu Asp Gln Ala Tyr Gly Asp Ala Val Glu Asn Ala Lys Asp
   160 165 170
   atc atc gcc tgt ggc ttt gac atc aac aag act ttc ata ttc tct gac 3997
   Ile Ile Ala Cys Gly Phe Asp Ile Asn Lys Thr Phe Ile Phe Ser Asp
   175 180 185 190
   ctg gac tac atg ggg atg agc tca ggt ttc tac aaa aat gtg gtg aag 4045
   Leu Asp Tyr Met Gly Met Ser Ser Gly Phe Tyr Lys Asn Val Val Lys
   195 200 205
   att caa aag cat gtt acc ttc aac caa gtg aaa ggc att ttc ggc ttc 4093
   Ile Gln Lys His Val Thr Phe Asn Gln Val Lys Gly Ile Phe Gly Phe
   210 215 220
   act gac agc gac tgc att ggg aag atc agt ttt cct gcc atc cag gct 4141
   Thr Asp Ser Asp Cys Ile Gly Lys Ile Ser Phe Pro Ala Ile Gln Ala
   225 230 235
   gct ccc tcc ttc agc aac tca ttc cca cag atc ttc cga gac agg acg 4189
   Ala Pro Ser Phe Ser Asn Ser Phe Pro Gln Ile Phe Arg Asp Arg Thr
   240 245 250
   gat atc cag tgc ctt atc cca tgt gcc att gac cag gat cct tac ttt 4237
   Asp Ile Gln Cys Leu Ile Pro Cys Ala Ile Asp Gln Asp Pro Tyr Phe
   255 260 265 270
   aga atg aca agg gac gtc gcc ccc agg atc ggc tat cct aaa cca gcc 4285
   Arg Met Thr Arg Asp Val Ala Pro Arg Ile Gly Tyr Pro Lys Pro Ala
   275 280 285
   ctg ttg cac tcc acc ttc ttc cca gcc ctg cag ggc gcc cag acc aaa 4333
   Leu Leu His Ser Thr Phe Phe Pro Ala Leu Gln Gly Ala Gln Thr Lys
   290 295 300
   atg agt gcc agc gac cca aac tcc tcc atc ttc ctc acc gac acg gcc 4381
   Met Ser Ala Ser Asp Pro Asn Ser Ser Ile Phe Leu Thr Asp Thr Ala
   305 310 315
   aag cag atc aaa acc aag gtc aat aag cat gcg ttt tct gga ggg aga 4429
   Lys Gln Ile Lys Thr Lys Val Asn Lys His Ala Phe Ser Gly Gly Arg
   320 325 330
   gac acc atc gag gag cac agg cag ttt ggg ggc aac tgt gat gtg gac 4477
   Asp Thr Ile Glu Glu His Arg Gln Phe Gly Gly Asn Cys Asp Val Asp
   335 340 345 350
   gtg tct ttc atg tac ctg acc ttc ttc ctc gag gac gac gac aag ctc      4525
   Val Ser Phe Met Tyr Leu Thr Phe Phe Leu Glu Asp Asp Asp Lys Leu
                   355                 360                 365
   gag cag atc agg aag gat tac acc agc gga gcc atg ctc acc ggt gag      4573
   Glu Gln Ile Arg Lys Asp Tyr Thr Ser Gly Ala Met Leu Thr Gly Glu
               370                 375                 380
   ctc aag aag gca ctc ata gag gtt ctg cag ccc ttg atc gca gag cac      4621
   Leu Lys Lys Ala Leu Ile Glu Val Leu Gln Pro Leu Ile Ala Glu His
           385                 390                 395
   cag gcc cgg cgc aag gag gtc acg gat gag ata gtg aaa gag ttc atg      4669
   Gln Ala Arg Arg Lys Glu Val Thr Asp Glu Ile Val Lys Glu Phe Met
       400                 405                 410
   act ccc cgg aag ctg tcc ttc gac ttt cag aag ctt gcg gcc gca ctc      4717
   Thr Pro Arg Lys Leu Ser Phe Asp Phe Gln Lys Leu Ala Ala Ala Leu
   415                 420                 425                 430
   gag cac cac cac cac cac cac tgagatccgg ctgctaacaa agcccgaaag         4768
   Glu His His His His His His
                   435
   gaagctgagt tggctgctgc caccgctgag caataactag cataacccct tggggcctct 4828
   aaacgggtct tgaggggttt tttgctgaaa ggaggaacta tatccggat                4877
<210> 3
   <211> 437
   <212> PRT
   <213> Artificial Sequence
<220>
   <223> Recombinant human mini-TrpRS protein construct
<400> 3
   Met Ser Tyr Lys Ala Ala Ala Gly Glu Asp Tyr Lys Ala Asp Cys Pro
   1               5                   10                  15
   Pro Gly Asn Pro Ala Pro Thr Ser Asn His Gly Pro Asp Ala Thr Glu
               20                  25                  30
   Ala Glu Glu Asp Phe Val Asp Pro Trp Thr Val Gln Thr Ser Ser Ala
           35                  40                  45
   Lys Gly Ile Asp Tyr Asp Lys Leu Ile Val Arg Phe Gly Ser Ser Lys
       50                  55                  60
   Ile Asp Lys Glu Leu Ile Asn Arg Ile Glu Arg Ala Thr Gly Gln Arg
   65                  70                  75                  80
   Pro His His Phe Leu Arg Arg Gly Ile Phe Phe Ser His Arg Asp Met
                   85                  90                  95
   Asn Gln Val Leu Asp Ala Tyr Glu Asn Lys Lys Pro Phe Tyr Leu Tyr
               100                 105                 110
   Thr Gly Arg Gly Pro Ser Ser Glu Ala Met His Val Gly His Leu Ile
           115                 120                 125
   Pro Phe Ile Phe Thr Lys Trp Leu Gln Asp Val Phe Asn Val Pro Leu
       130                 135                 140
   Val Ile Gln Met Thr Asp Asp Glu Lys Tyr Leu Trp Lys Asp Leu Thr
   145 150 155 160
   Leu Asp Gln Ala Tyr Gly Asp Ala Val Glu Asn Ala Lys Asp Ile Ile
   165 170 175
   Ala Cys Gly Phe Asp Ile Asn Lys Thr Phe Ile Phe Ser Asp Leu Asp
   180 185 190
   Tyr Met Gly Met Ser Ser Gly Phe Tyr Lys Asn Val Val Lys Ile Gln
   195 200 205
   Lys His Val Thr Phe Asn Gln Val Lys Gly Ile Phe Gly Phe Thr Asp
   210 215 220
   Ser Asp Cys Ile Gly Lys Ile Ser Phe Pro Ala Ile Gln Ala Ala Pro
   225 230 235 240
   Ser Phe Ser Asn Ser Phe Pro Gln Ile Phe Arg Asp Arg Thr Asp Ile
   245 250 255
   Gln Cys Leu Ile Pro Cys Ala Ile Asp Gln Asp Pro Tyr Phe Arg Met
   260 265 270
   Thr Arg Asp Val Ala Pro Arg Ile Gly Tyr Pro Lys Pro Ala Leu Leu
   275 280 285
   His Ser Thr Phe Phe Pro Ala Leu Gln Gly Ala Gln Thr Lys Met Ser
   290 295 300
   Ala Ser Asp Pro Asn Ser Ser Ile Phe Leu Thr Asp Thr Ala Lys Gln
   305 310 315 320
   Ile Lys Thr Lys Val Asn Lys His Ala Phe Ser Gly Gly Arg Asp Thr
   325 330 335
   Ile Glu Glu His Arg Gln Phe Gly Gly Asn Cys Asp Val Asp Val Ser
   340 345 350
   Phe Met Tyr Leu Thr Phe Phe Leu Glu Asp Asp Asp Lys Leu Glu Gln
   355 360 365
   Ile Arg Lys Asp Tyr Thr Ser Gly Ala Met Leu Thr Gly Glu Leu Lys
   370 375 380
   Lys Ala Leu Ile Glu Val Leu Gln Pro Leu Ile Ala Glu His Gln Ala
   385 390 395 400
   Arg Arg Lys Glu Val Thr Asp Glu Ile Val Lys Glu Phe Met Thr Pro
   405 410 415
   Arg Lys Leu Ser Phe Asp Phe Gln Lys Leu Ala Ala Ala Leu Glu His
   420 425 430
   His His His His His
   435
<210> 4
   <211> 4811
   <212> DNA
   <213> Artificial sequence
<220>
   <223> Recombinant human Met-T1-His tag nucleotide construct
<220>
   <221> CDS
   <222> (3428)..(4672)
<400> 4
   tggcgaatgg gacgcgccct gtagcggcgc attaagcgcg gcgggtgtgg tggttacgcg 60
   cagcgtgacc gctacacttg ccagcgccct agcgcccgct cctttcgctt tcttcccttc 120
   ctttctcgcc acgttcgccg gctttccccg tcaagctcta aatcgggggc tccctttagg 180
   gttccgattt agtgctttac ggcacctcga ccccaaaaaa cttgattagg gtgatggttc 240
   acgtagtggg ccatcgccct gatagacggt ttttcgccct ttgacgttgg agtccacgtt 300
   ctttaatagt ggactcttgt tccaaactgg aacaacactc aaccctatct cggtctattc 360
   ttttgattta taagggattt tgccgatttc ggcctattgg ttaaaaaatg agctgattta 420
   acaaaaattt aacgcgaatt ttaacaaaat attaacgttt acaatttcag gtggcacttt 480
   tcggggaaat gtgcgcggaa cccctatttg tttatttttc taaatacatt caaatatgta 540
   tccgctcatg agacaataac cctgataaat gcttcaataa tattgaaaaa ggaagagtat 600
   gagtattcaa catttccgtg tcgcccttat tccctttttt gcggcatttt gccttcctgt 660
   ttttgctcac ccagaaacgc tggtgaaagt aaaagatgct gaagatcagt tgggtgcacg 720
   agtgggttac atcgaactgg atctcaacag cggtaagatc cttgagagtt ttcgccccga 780
   agaacgtttt ccaatgatga gcacttttaa agttctgcta tgtggcgcgg tattatcccg 840
   tattgacgcc gggcaagagc aactcggtcg ccgcatacac tattctcaga atgacttggt 900
   tgagtactca ccagtcacag aaaagcatct tacggatggc atgacagtaa gagaattatg 960
   cagtgctgcc ataaccatga gtgataacac tgcggccaac ttacttctga caacgatcgg 1020
   aggaccgaag gagctaaccg cttttttgca caacatgggg gatcatgtaa ctcgccttga 1080
   tcgttgggaa ccggagctga atgaagccat accaaacgac gagcgtgaca ccacgatgcc 1140
   tgcagcaatg gcaacaacgt tgcgcaaact attaactggc gaactactta ctctagcttc 1200
   ccggcaacaa ttaatagact ggatggaggc ggataaagtt gcaggaccac ttctgcgctc 1260
   ggcccttccg gctggctggt ttattgctga taaatctgga gccggtgagc gtgggtctcg 1320
   cggtatcatt gcagcactgg ggccagatgg taagccctcc cgtatcgtag ttatctacac 1380
   gacggggagt caggcaacta tggatgaacg aaatagacag atcgctgaga taggtgcctc 1440
   actgattaag cattggtaac tgtcagacca agtttactca tatatacttt agattgattt 1500
   aaaacttcat ttttaattta aaaggatcta ggtgaagatc ctttttgata atctcatgac 1560
   caaaatccct taacgtgagt tttcgttcca ctgagcgtca gaccccgtag aaaagatcaa 1620
   aggatcttct tgagatcctt tttttctgcg cgtaatctgc tgcttgcaaa caaaaaaacc 1680
   accgctacca gcggtggttt gtttgccgga tcaagagcta ccaactcttt ttccgaaggt 1740
   aactggcttc agcagagcgc agataccaaa tactgtcctt ctagtgtagc cgtagttagg 1800
   ccaccacttc aagaactctg tagcaccgcc tacatacctc gctctgctaa tcctgttacc 1860
   agtggctgct gccagtggcg ataagtcgtg tcttaccggg ttggactcaa gacgatagtt 1920
   accggataag gcgcagcggt cgggctgaac ggggggttcg tgcacacagc ccagcttgga 1980
   gcgaacgacc tacaccgaac tgagatacct acagcgtgag ctatgagaaa gcgccacgct 2040
   tcccgaaggg agaaaggcgg acaggtatcc ggtaagcggc agggtcggaa caggagagcg 2100
   cacgagggag cttccagggg gaaacgcctg gtatctttat agtcctgtcg ggtttcgcca 2160
   cctctgactt gagcgtcgat ttttgtgatg ctcgtcaggg gggcggagcc tatggaaaaa 2220
   cgccagcaac gcggcctttt tacggttcct ggccttttgc tggccttttg ctcacatgtt 2280
   ctttcctgcg ttatcccctg attctgtgga taaccgtatt accgcctttg agtgagctga 2340
   taccgctcgc cgcagccgaa cgaccgagcg cagcgagtca gtgagcgagg aagcggaaga 2400
   gcgcctgatg cggtattttc tccttacgca tctgtgcggt atttcacacc gcatatatgg 2460
   tgcactctca gtacaatctg ctctgatgcc gcatagttaa gccagtatac actccgctat 2520
   cgctacgtga ctgggtcatg gctgcgcccc gacacccgcc aacacccgct gacgcgccct 2580
   gacgggcttg tctgctcccg gcatccgctt acagacaagc tgtgaccgtc tccgggagct 2640
   gcatgtgtca gaggttttca ccgtcatcac cgaaacgcgc gaggcagctg cggtaaagct 2700
   catcagcgtg gtcgtgaagc gattcacaga tgtctgcctg ttcatccgcg tccagctcgt 2760
   tgagtttctc cagaagcgtt aatgtctggc ttctgataaa gcgggccatg ttaagggcgg 2820
   ttttttcctg tttggtcact gatgcctccg tgtaaggggg atttctgttc atgggggtaa 2880
   tgataccgat gaaacgagag aggatgctca cgatacgggt tactgatgat gaacatgccc 2940
   ggttactgga acgttgtgag ggtaaacaac tggcggtatg gatgcggcgg gaccagagaa 3000
   aaatcactca gggtcaatgc cagcgcttcg ttaatacaga tgtaggtgtt ccacagggta 3060
   gccagcagca tcctgcgatg cagatccgga acataatggt gcagggcgct gacttccgcg 3120
   tttccagact ttacgaaaca cggaaaccga agaccattca tgttgttgct caggtcgcag 3180
   acgttttgca gcagcagtcg cttcacgttc gctcgcgtat cggtgattca ttctgctaac 3240
   cagtaaggca accccgccag cctagccggg tcctcaacga caggagcacg atcatgcgca 3300
   cccgtggcca ggacccaacg ctgcccgaga tctcgatccc gcgaaattaa tacgactcac 3360
   tatagggaga ccacaacggt ttccctctag aaataatttt gtttaacttt aagaaggaga 3420
   tatacat atg agt aat cat ggc cca gat gcc aca gaa gct gaa gag gat    3469
           Met Ser Asn His Gly Pro Asp Ala Thr Glu Ala Glu Glu Asp
            1               5                   10
   ttt gtg gac cca tgg aca gta cag aca agc agt gca aaa ggc ata gac    3517
   Phe Val Asp Pro Trp Thr Val Gln Thr Ser Ser Ala Lys Gly Ile Asp
    15                  20                  25                  30
   tac gat aag ctc att gtt cgg ttt gga agt agt aaa att gac aaa gag    3565
   Tyr Asp Lys Leu Ile Val Arg Phe Gly Ser Ser Lys Ile Asp Lys Glu
                    35                  40                  45
   cta ata aac cga ata gag aga gcc acc ggc caa aga cca cac cac ttc    3613
   Leu Ile Asn Arg Ile Glu Arg Ala Thr Gly Gln Arg Pro His His Phe
                   50                      55                      60
   ctg cgc aga ggc atc ttc ttc tca cac aga gat atg aat cag gtt ctt     3661
   Leu Arg Arg Gly Ile Phe Phe Ser His Arg Asp Met Asn Gln Val Leu
           65                  70                      75
   gat gcc tat gaa aat aag aag cca ttt tat ctg tac acg ggc cgg ggc     3709
   Asp Ala Tyr Glu Asn Lys Lys Pro Phe Tyr Leu Tyr Thr Gly Arg Gly
       80                  85                      90
   ccc tct tct gaa gca atg cat gta ggt cac ctc att cca ttt att ttc     3757
   Pro Ser Ser Glu Ala Met His Val Gly His Leu Ile Pro Phe Ile Phe
   95                  100                     105                 110
   aca aag tgg ctc cag gat gta ttt aac gtg ccc ttg gtc atc cag atg     3805
   Thr Lys Trp Leu Gln Asp Val Phe Asn Val Pro Leu Val Ile Gln Met
                   115                     120                 125
   acg gat gac gag aag tat ctg tgg aag gac ctg acc ctg gac cag gcc     3853
   Thr Asp Asp Glu Lys Tyr Leu Trp Lys Asp Leu Thr Leu Asp Gln Ala
               130                     135                 140
   tat ggc gat gct gtt gag aat gcc aag gac atc atc gcc tgt ggc ttt     3901
   Tyr Gly Asp Ala Val Glu Asn Ala Lys Asp Ile Ile Ala Cys Gly Phe
           145                 150                     155
   gac atc aac aag act ttc ata ttc tct gac ctg gac tac atg ggg atg     3949
   Asp Ile Asn Lys Thr Phe Ile Phe Ser Asp Leu Asp Tyr Met Gly Met
       160                 165                     170
   agc tca ggt ttc tac aaa aat gtg gtg aag att caa aag cat gtt acc     3997
   Ser Ser Gly Phe Tyr Lys Asn Val Val Lys Ile Gln Lys His Val Thr
   175                 180                     185                 190
   ttc aac caa gtg aaa ggc att ttc ggc ttc act gac agc gac tgc att     4045
   Phe Asn Gln Val Lys Gly Ile Phe Gly Phe Thr Asp Ser Asp Cys Ile
                   195                     200                 205
   ggg aag atc agt ttt cct gcc atc cag gct gct ccc tcc ttc agc aac     4093
   Gly Lys Ile Ser Phe Pro Ala Ile Gln Ala Ala Pro Ser Phe Ser Asn
               210                     215                 220
   tca ttc cca cag atc ttc cga gac agg acg gat atc cag tgc ctt atc     4141
   Ser Phe Pro Gln Ile Phe Arg Asp Arg Thr Asp Ile Gln Cys Leu Ile
           225                 230                     235
   cca tgt gcc att gac cag gat cct tac ttt aga atg aca agg gac gtc     4189
   Pro Cys Ala Ile Asp Gln Asp Pro Tyr Phe Arg Met Thr Arg Asp Val
       240                 245                     250
   gcc ccc agg atc ggc tat cct aaa cca gcc ctg ttg cac tcc acc ttc     4237
   Ala Pro Arg Ile Gly Tyr Pro Lys Pro Ala Leu Leu His Ser Thr Phe
   255                 260                     265                 270
   ttc cca gcc ctg cag ggc gcc cag acc aaa atg agt gcc agc gac cca     4285
   Phe Pro Ala Leu Gln Gly Ala Gln Thr Lys Met Ser Ala Ser Asp Pro
                   275                     280                 285
   aac tcc tcc atc ttc ctc acc gac acg gcc aag cag atc aaa acc aag     4333
   Asn Ser Ser Ile Phe Leu Thr Asp Thr Ala Lys Gln Ile Lys Thr Lys
               290                     295                 300
   gtc aat aag cat gcg ttt tct gga ggg aga gac acc atc gag gag cac     4381
   Val Asn Lys His Ala Phe Ser Gly Gly Arg Asp Thr Ile Glu Glu His
           305                 310                     315
   agg cag ttt ggg ggc aac tgt gat gtg gac gtg tct ttc atg tac ctg     4429
   Arg Gln Phe Gly Gly Asn Cys Asp Val Asp Val Ser Phe Met Tyr Leu
       320                 325                 330
   acc ttc ttc ctc gag gac gac gac aag ctc gag cag atc agg aag gat    4477
   Thr Phe Phe Leu Glu Asp Asp Asp Lys Leu Glu Gln Ile Arg Lys Asp
   335                 340                 345                 350
   tac acc agc gga gcc atg ctc acc ggt gag ctc aag aag gca ctc ata    4525
   Tyr Thr Ser Gly Ala Met Leu Thr Gly Glu Leu Lys Lys Ala Leu Ile
                   355                 360                 365
   gag gtt ctg cag ccc ttg atc gca gag cac cag gcc cgg cgc aag gag    4573
   Glu Val Leu Gln Pro Leu Ile Ala Glu His Gln Ala Arg Arg Lys Glu
               370                 375                 380
   gtc acg gat gag ata gtg aaa gag ttc atg act ccc cgg aag ctg tcc    4621
   Val Thr Asp Glu Ile Val Lys Glu Phe Met Thr Pro Arg Lys Leu Ser
           385                 390                 395
   ttc gac ttt cag aag ctt gcg gcc gca ctc gag cac cac cac cac cac    4669
   Phe Asp Phe Gln Lys Leu Ala Ala Ala Leu Glu His His His His His
       400                 405                 410
   cac tgagatccgg ctgctaacaa agcccgaaag gaagctgagt tggctgctgc         4722
   His
   415
   caccgctgag caataactag cataacccct tggggcctct aaacgggtct tgaggggttt 4782
   tttgctgaaa ggaggaacta tatccggat                                    4811
<210> 5
   <211> 415
   <212> PRT
   <213> Artificial Sequence
<220>
   <223> Recombinant human Met-T1-His tag protein construct
<400> 5
   Met Ser Asn His Gly Pro Asp Ala Thr Glu Ala Glu Glu Asp Phe Val
   1               5                   10                  15
   Asp Pro Trp Thr Val Gln Thr Ser Ser Ala Lys Gly Ile Asp Tyr Asp
               20                  25                  30
   Lys Leu Ile Val Arg Phe Gly Ser Ser Lys Ile Asp Lys Glu Leu Ile
           35                  40                  45
   Asn Arg Ile Glu Arg Ala Thr Gly Gln Arg Pro His His Phe Leu Arg
       50                  55                  60
   Arg Gly Ile Phe Phe Ser His Arg Asp Met Asn Gln Val Leu Asp Ala
   65                  70                  75                  80
   Tyr Glu Asn Lys Lys Pro Phe Tyr Leu Tyr Thr Gly Arg Gly Pro Ser
                   85                  90                  95
   Ser Glu Ala Met His Val Gly His Leu Ile Pro Phe Ile Phe Thr Lys
               100                 105                 110
   Trp Leu Gln Asp Val Phe Asn Val Pro Leu Val Ile Gln Met Thr Asp
           115                 120                 125
   Asp Glu Lys Tyr Leu Trp Lys Asp Leu Thr Leu Asp Gln Ala Tyr Gly
       130                 135                 140
   Asp Ala Val Glu Asn Ala Lys Asp Ile Ile Ala Cys Gly Phe Asp Ile
   145                 150                 155                 160
   Asn Lys Thr Phe Ile Phe Ser Asp Leu Asp Tyr Met Gly Met Ser Ser
                   165                 170                 175
   Gly Phe Tyr Lys Asn Val Val Lys Ile Gln Lys His Val Thr Phe Asn
               180                 185                 190
   Gln Val Lys Gly Ile Phe Gly Phe Thr Asp Ser Asp Cys Ile Gly Lys
           195                 200                 205
   Ile Ser Phe Pro Ala Ile Gln Ala Ala Pro Ser Phe Ser Asn Ser Phe
       210                 215                 220
   Pro Gln Ile Phe Arg Asp Arg Thr Asp Ile Gln Cys Leu Ile Pro Cys
   225                 230                 235                 240
   Ala Ile Asp Gln Asp Pro Tyr Phe Arg Met Thr Arg Asp Val Ala Pro
                   245                 250                 255
   Arg Ile Gly Tyr Pro Lys Pro Ala Leu Leu His Ser Thr Phe Phe Pro
               260                 265                 270
   Ala Leu Gln Gly Ala Gln Thr Lys Met Ser Ala Ser Asp Pro Asn Ser
           275                 280                 285
   Ser Ile Phe Leu Thr Asp Thr Ala Lys Gln Ile Lys Thr Lys Val Asn
       290                 295                 300
   Lys His Ala Phe Ser Gly Gly Arg Asp Thr Ile Glu Glu His Arg Gln
   305                 310                 315                 320
   Phe Gly Gly Asn Cys Asp Val Asp Val Ser Phe Met Tyr Leu Thr Phe
                   325                 330                 335
   Phe Leu Glu Asp Asp Asp Lys Leu Glu Gln Ile Arg Lys Asp Tyr Thr
               340                 345                 350
   Ser Gly Ala Met Leu Thr Gly Glu Leu Lys Lys Ala Leu Ile Glu Val
           355                 360                 365
   Leu Gln Pro Leu Ile Ala Glu His Gln Ala Arg Arg Lys Glu Val Thr
       370                 375                 380
   Asp Glu Ile Val Lys Glu Phe Met Thr Pro Arg Lys Leu Ser Phe Asp
   385                 390                 395                 400
   Phe Gln Lys Leu Ala Ala Ala Leu Glu His His His His His His
                   405                 410                 415
<210> 6
   <211> 4742
   <212> DNA
   <213> Artificial sequence
<220>
   <223> Recombinant human Met-T2-His tag nucleotide construct
<220>
   <221> CDS
   <222> (3428)..(4603)
<400> 6
   tggcgaatgg gacgcgccct gtagcggcgc attaagcgcg gcgggtgtgg tggttacgcg 60
   cagcgtgacc gctacacttg ccagcgccct agcgcccgct cctttcgctt tcttcccttc 120
   ctttctcgcc acgttcgccg gctttccccg tcaagctcta aatcgggggc tccctttagg 180
   gttccgattt agtgctttac ggcacctcga ccccaaaaaa cttgattagg gtgatggttc 240
   acgtagtggg ccatcgccct gatagacggt ttttcgccct ttgacgttgg agtccacgtt 300
   ctttaatagt ggactcttgt tccaaactgg aacaacactc aaccctatct cggtctattc 360
   ttttgattta taagggattt tgccgatttc ggcctattgg ttaaaaaatg agctgattta 420
   acaaaaattt aacgcgaatt ttaacaaaat attaacgttt acaatttcag gtggcacttt 480
   tcggggaaat gtgcgcggaa cccctatttg tttatttttc taaatacatt caaatatgta 540
   tccgctcatg agacaataac cctgataaat gcttcaataa tattgaaaaa ggaagagtat 600
   gagtattcaa catttccgtg tcgcccttat tccctttttt gcggcatttt gccttcctgt 660
   ttttgctcac ccagaaacgc tggtgaaagt aaaagatgct gaagatcagt tgggtgcacg 720
   agtgggttac atcgaactgg atctcaacag cggtaagatc cttgagagtt ttcgccccga 780
   agaacgtttt ccaatgatga gcacttttaa agttctgcta tgtggcgcgg tattatcccg 840
   tattgacgcc gggcaagagc aactcggtcg ccgcatacac tattctcaga atgacttggt 900
   tgagtactca ccagtcacag aaaagcatct tacggatggc atgacagtaa gagaattatg 960
   cagtgctgcc ataaccatga gtgataacac tgcggccaac ttacttctga caacgatcgg 1020
   aggaccgaag gagctaaccg cttttttgca caacatgggg gatcatgtaa ctcgccttga 1080
   tcgttgggaa ccggagctga atgaagccat accaaacgac gagcgtgaca ccacgatgcc 1140
   tgcagcaatg gcaacaacgt tgcgcaaact attaactggc gaactactta ctctagcttc 1200
   ccggcaacaa ttaatagact ggatggaggc ggataaagtt gcaggaccac ttctgcgctc 1260
   ggcccttccg gctggctggt ttattgctga taaatctgga gccggtgagc gtgggtctcg 1320
   cggtatcatt gcagcactgg ggccagatgg taagccctcc cgtatcgtag ttatctacac 1380
   gacggggagt caggcaacta tggatgaacg aaatagacag atcgctgaga taggtgcctc 1440
   actgattaag cattggtaac tgtcagacca agtttactca tatatacttt agattgattt 1500
   aaaacttcat ttttaattta aaaggatcta ggtgaagatc ctttttgata atctcatgac 1560
   caaaatccct taacgtgagt tttcgttcca ctgagcgtca gaccccgtag aaaagatcaa 1620
   aggatcttct tgagatcctt tttttctgcg cgtaatctgc tgcttgcaaa caaaaaaacc 1680
   accgctacca gcggtggttt gtttgccgga tcaagagcta ccaactcttt ttccgaaggt 1740
   aactggcttc agcagagcgc agataccaaa tactgtcctt ctagtgtagc cgtagttagg 1800
   ccaccacttc aagaactctg tagcaccgcc tacatacctc gctctgctaa tcctgttacc 1860
   agtggctgct gccagtggcg ataagtcgtg tcttaccggg ttggactcaa gacgatagtt 1920
   accggataag gcgcagcggt cgggctgaac ggggggttcg tgcacacagc ccagcttgga 1980
   gcgaacgacc tacaccgaac tgagatacct acagcgtgag ctatgagaaa gcgccacgct 2040
   tcccgaaggg agaaaggcgg acaggtatcc ggtaagcggc agggtcggaa caggagagcg 2100
   cacgagggag cttccagggg gaaacgcctg gtatctttat agtcctgtcg ggtttcgcca 2160
   cctctgactt gagcgtcgat ttttgtgatg ctcgtcaggg gggcggagcc tatggaaaaa 2220
   cgccagcaac gcggcctttt tacggttcct ggccttttgc tggccttttg ctcacatgtt 2280
   ctttcctgcg ttatcccctg attctgtgga taaccgtatt accgcctttg agtgagctga 2340
   taccgctcgc cgcagccgaa cgaccgagcg cagcgagtca gtgagcgagg aagcggaaga 2400
   gcgcctgatg cggtattttc tccttacgca tctgtgcggt atttcacacc gcatatatgg 2460
   tgcactctca gtacaatctg ctctgatgcc gcatagttaa gccagtatac actccgctat 2520
   cgctacgtga ctgggtcatg gctgcgcccc gacacccgcc aacacccgct gacgcgccct 2580
   gacgggcttg tctgctcccg gcatccgctt acagacaagc tgtgaccgtc tccgggagct 2640
   gcatgtgtca gaggttttca ccgtcatcac cgaaacgcgc gaggcagctg cggtaaagct 2700
   catcagcgtg gtcgtgaagc gattcacaga tgtctgcctg ttcatccgcg tccagctcgt 2760
   tgagtttctc cagaagcgtt aatgtctggc ttctgataaa gcgggccatg ttaagggcgg 2820
   ttttttcctg tttggtcact gatgcctccg tgtaaggggg atttctgttc atgggggtaa 2880
   tgataccgat gaaacgagag aggatgctca cgatacgggt tactgatgat gaacatgccc 2940
   ggttactgga acgttgtgag ggtaaacaac tggcggtatg gatgcggcgg gaccagagaa 3000
   aaatcactca gggtcaatgc cagcgcttcg ttaatacaga tgtaggtgtt ccacagggta 3060
   gccagcagca tcctgcgatg cagatccgga acataatggt gcagggcgct gacttccgcg 3120
   tttccagact ttacgaaaca cggaaaccga agaccattca tgttgttgct caggtcgcag 3180
   acgttttgca gcagcagtcg cttcacgttc gctcgcgtat cggtgattca ttctgctaac 3240
   cagtaaggca accccgccag cctagccggg tcctcaacga caggagcacg atcatgcgca 3300
   cccgtggcca ggacccaacg ctgcccgaga tctcgatccc gcgaaattaa tacgactcac 3360
   tatagggaga ccacaacggt ttccctctag aaataatttt gtttaacttt aagaaggaga 3420
   tatacat atg agt gca aaa ggc ata gac tac gat aag ctc att gtt cgg    3469
           Met Ser Ala Lys Gly Ile Asp Tyr Asp Lys Leu Ile Val Arg
            1               5                  10
   ttt gga agt agt aaa att gac aaa gag cta ata aac cga ata gag aga    3517
   Phe Gly Ser Ser Lys Ile Asp Lys Glu Leu Ile Asn Arg Ile Glu Arg
    15                  20                  25                  30
   gcc acc ggc caa aga cca cac cac ttc ctg cgc aga ggc atc ttc ttc    3565
   Ala Thr Gly Gln Arg Pro His His Phe Leu Arg Arg Gly Ile Phe Phe
                    35                  40                  45
   tca cac aga gat atg aat cag gtt ctt gat gcc tat gaa aat aag aag    3613
   Ser His Arg Asp Met Asn Gln Val Leu Asp Ala Tyr Glu Asn Lys Lys
                50                  55                  60
   cca ttt tat ctg tac acg ggc cgg ggc ccc tct tct gaa gca atg cat    3661
   Pro Phe Tyr Leu Tyr Thr Gly Arg Gly Pro Ser Ser Glu Ala Met His
           65                  70                  75
   gta ggt cac ctc att cca ttt att ttc aca aag tgg ctc cag gat gta     3709
   Val Gly His Leu Ile Pro Phe Ile Phe Thr Lys Trp Leu Gln Asp Val
       80                  85                  90
   ttt aac gtg ccc ttg gtc atc cag atg acg gat gac gag aag tat ctg     3757
   Phe Asn Val Pro Leu Val Ile Gln Met Thr Asp Asp Glu Lys Tyr Leu
   95                  100                 105                 110
   tgg aag gac ctg acc ctg gac cag gcc tat ggc gat gct gtt gag aat     3805
   Trp Lys Asp Leu Thr Leu Asp Gln Ala Tyr Gly Asp Ala Val Glu Asn
                   115                 120                 125
   gcc aag gac atc atc gcc tgt ggc ttt gac atc aac aag act ttc ata     3853
   Ala Lys Asp Ile Ile Ala Cys Gly Phe Asp Ile Asn Lys Thr Phe Ile
               130                 135                 140
   ttc tct gac ctg gac tac atg ggg atg agc tca ggt ttc tac aaa aat     3901
   Phe Ser Asp Leu Asp Tyr Met Gly Met Ser Ser Gly Phe Tyr Lys Asn
           145                 150                 155
   gtg gtg aag att caa aag cat gtt acc ttc aac caa gtg aaa ggc att     3949
   Val Val Lys Ile Gln Lys His Val Thr Phe Asn Gln Val Lys Gly Ile
       160                 165                 170
   ttc ggc ttc act gac agc gac tgc att ggg aag atc agt ttt cct gcc     3997
   Phe Gly Phe Thr Asp Ser Asp Cys Ile Gly Lys Ile Ser Phe Pro Ala
   175                 180                 185                 190
   atc cag gct gct ccc tcc ttc agc aac tca ttc cca cag atc ttc cga     4045
   Ile Gln Ala Ala Pro Ser Phe Ser Asn Ser Phe Pro Gln Ile Phe Arg
                   195                 200                 205
   gac agg acg gat atc cag tgc ctt atc cca tgt gcc att gac cag gat     4093
   Asp Arg Thr Asp Ile Gln Cys Leu Ile Pro Cys Ala Ile Asp Gln Asp
               210                 215                 220
   cct tac ttt aga atg aca agg gac gtc gcc ccc agg atc ggc tat cct     4141
   Pro Tyr Phe Arg Met Thr Arg Asp Val Ala Pro Arg Ile Gly Tyr Pro
           225                 230                 235
   aaa cca gcc ctg ttg cac tcc acc ttc ttc cca gcc ctg cag ggc gcc     4189
   Lys Pro Ala Leu Leu His Ser Thr Phe Phe Pro Ala Leu Gln Gly Ala
       240                 245                 250
   cag acc aaa atg agt gcc agc gac cca aac tcc tcc atc ttc ctc acc     4237
   Gln Thr Lys Met Ser Ala Ser Asp Pro Asn Ser Ser Ile Phe Leu Thr
   255                 260                 265                 270
   gac acg gcc aag cag atc aaa acc aag gtc aat aag cat gcg ttt tct     4285
   Asp Thr Ala Lys Gln Ile Lys Thr Lys Val Asn Lys His Ala Phe Ser
                   275                 280                 285
   gga ggg aga gac acc atc gag gag cac agg cag ttt ggg ggc aac tgt     4333
   Gly Gly Arg Asp Thr Ile Glu Glu His Arg Gln Phe Gly Gly Asn Cys
               290                 295                 300
   gat gtg gac gtg tct ttc atg tac ctg acc ttc ttc ctc gag gac gac     4381
   Asp Val Asp Val Ser Phe Met Tyr Leu Thr Phe Phe Leu Glu Asp Asp
           305                 310                 315
   gac aag ctc gag cag atc agg aag gat tac acc agc gga gcc atg ctc     4429
   Asp Lys Leu Glu Gln Ile Arg Lys Asp Tyr Thr Ser Gly Ala Met Leu
       320                 325                 330
   acc ggt gag ctc aag aag gca ctc ata gag gtt ctg cag ccc ttg atc     4477
   Thr Gly Glu Leu Lys Lys Ala Leu Ile Glu Val Leu Gln Pro Leu Ile
   335                 340                 345                 350
   gca gag cac cag gcc cgg cgc aag gag gtc acg gat gag ata gtg aaa     4525
   Ala Glu His Gln Ala Arg Arg Lys Glu Val Thr Asp Glu Ile Val Lys
                   355                 360                 365
   gag ttc atg act ccc cgg aag ctg tcc ttc gac ttt cag aag ctt gcg     4573
   Glu Phe Met Thr Pro Arg Lys Leu Ser Phe Asp Phe Gln Lys Leu Ala
               370                 375                 380
   gcc gca ctc gag cac cac cac cac cac cac tgagatccgg ctgctaacaa      4623
   Ala Ala Leu Glu His His His His His His
           385                 390
   agcccgaaag gaagctgagt tggctgctgc caccgctgag caataactag cataacccct 4683
   tggggcctct aaacgggtct tgaggggttt tttgctgaaa ggaggaacta tatccggat  4742
<210> 7
   <211> 392
   <212> PRT
   <213> Artificial sequence
<220>
   <223> Recombinant human Met-T2-His tag protein construct
<400> 7
   Met Ser Ala Lys Gly Ile Asp Tyr Asp Lys Leu Ile Val Arg Phe Gly
   1               5                   10                  15
   Ser Ser Lys Ile Asp Lys Glu Leu Ile Asn Arg Ile Glu Arg Ala Thr
               20                  25                  30
   Gly Gln Arg Pro His His Phe Leu Arg Arg Gly Ile Phe Phe Ser His
           35                  40                  45
   Arg Asp Met Asn Gln Val Leu Asp Ala Tyr Glu Asn Lys Lys Pro Phe
       50                  55                  60
   Tyr Leu Tyr Thr Gly Arg Gly Pro Ser Ser Glu Ala Met His Val Gly
   65                  70                  75                  80
   His Leu Ile Pro Phe Ile Phe Thr Lys Trp Leu Gln Asp Val Phe Asn
                   85                  90                  95
   Val Pro Leu Val Ile Gln Met Thr Asp Asp Glu Lys Tyr Leu Trp Lys
               100                 105                 110
   Asp Leu Thr Leu Asp Gln Ala Tyr Gly Asp Ala Val Glu Asn Ala Lys
           115                 120                 125
   Asp Ile Ile Ala Cys Gly Phe Asp Ile Asn Lys Thr Phe Ile Phe Ser
       130                 135                 140
   Asp Leu Asp Tyr Met Gly Met Ser Ser Gly Phe Tyr Lys Asn Val Val
   145                 150                 155                 160
   Lys Ile Gln Lys His Val Thr Phe Asn Gln Val Lys Gly Ile Phe Gly
                   165                 170                 175
   Phe Thr Asp Ser Asp Cys Ile Gly Lys Ile Ser Phe Pro Ala Ile Gln
               180                 185                 190
   Ala Ala Pro Ser Phe Ser Asn Ser Phe Pro Gln Ile Phe Arg Asp Arg
           195                 200                 205
   Thr Asp Ile Gln Cys Leu Ile Pro Cys Ala Ile Asp Gln Asp Pro Tyr
       210                 215                 220
   Phe Arg Met Thr Arg Asp Val Ala Pro Arg Ile Gly Tyr Pro Lys Pro
   225                 230                 235                 240
   Ala Leu Leu His Ser Thr Phe Phe Pro Ala Leu Gln Gly Ala Gln Thr
                   245                 250                 255
   Lys Met Ser Ala Ser Asp Pro Asn Ser Ser Ile Phe Leu Thr Asp Thr
               260                 265                 270
   Ala Lys Gln Ile Lys Thr Lys Val Asn Lys His Ala Phe Ser Gly Gly
           275                 280                 285
   Arg Asp Thr Ile Glu Glu His Arg Gln Phe Gly Gly Asn Cys Asp Val
       290                 295                 300
   Asp Val Ser Phe Met Tyr Leu Thr Phe Phe Leu Glu Asp Asp Asp Lys
   305                 310                 315                 320
   Leu Glu Gln Ile Arg Lys Asp Tyr Thr Ser Gly Ala Met Leu Thr Gly
                   325                 330                 335
   Glu Leu Lys Lys Ala Leu Ile Glu Val Leu Gln Pro Leu Ile Ala Glu
               340                 345                 350
   His Gln Ala Arg Arg Lys Glu Val Thr Asp Glu Ile Val Lys Glu Phe
           355                 360                 365
   Met Thr Pro Arg Lys Leu Ser Phe Asp Phe Gln Lys Leu Ala Ala Ala
       370                 375                 380
   Leu Glu His His His His His His
   385                 390
<210> 8
   <211> 5
   <212> PRT
   <213> Homo sapiens
<400> 8
   Ser Asn His Gly Pro
   1               5
<210> 9
   <211> 5
   <212> PRT
   <213> Homo sapiens
<400> 9
   Ser Ala Lys Gly Ile
   1               5
<210> 10
   <211> 4
   <212> PRT
   <213> Homo sapiens
<400> 10
   His Val Gly His
   1
<210> 11
   <211> 5
   <212> PRT
   <213> Homo sapiens
<400> 11
   Lys Met Ser Ala Ser
   1 5
<210> 12
   <211> 378
   <212> PRT
   <213> Homo sapiens
<400> 12
   Ser Ala Lys Gly Ile Asp Tyr Asp Lys Leu Ile Val Arg Phe Gly Ser
   1 5 10 15
   Ser Lys Ile Asp Lys Glu Leu Ile Asn Arg Ile Glu Arg Ala Thr Gly
   20 25 30
   Gln Arg Pro His His Phe Leu Arg Arg Gly Ile Phe Phe Ser His Arg
   35 40 45
   Asp Met Asn Gln Val Leu Asp Ala Tyr Glu Asn Lys Lys Pro Phe Tyr
   50 55 60
   Leu Tyr Thr Gly Arg Gly Pro Ser Ser Glu Ala Met His Val Gly His
   65 70 75 80
   Leu Ile Pro Phe Ile Phe Thr Lys Trp Leu Gln Asp Val Phe Asn Val
   85 90 95
   Pro Leu Val Ile Gln Met Thr Asp Asp Glu Lys Tyr Leu Trp Lys Asp
   100 105 110
   Leu Thr Leu Asp Gln Ala Tyr Gly Asp Ala Val Glu Asn Ala Lys Asp
   115 120 125
   Ile Ile Ala Cys Gly Phe Asp Ile Asn Lys Thr Phe Ile Phe Ser Asp
   130 135 140
   Leu Asp Tyr Met Gly Met Ser Ser Gly Phe Tyr Lys Asn Val Val Lys
   145 150 155 160
   Ile Gln Lys His Val Thr Phe Asn Gln Val Lys Gly Ile Phe Gly Phe
   165 170 175
   Thr Asp Ser Asp Cys Ile Gly Lys Ile Ser Phe Pro Ala Ile Gln Ala
   180 185 190
   Ala Pro Ser Phe Ser Asn Ser Phe Pro Gln Ile Phe Arg Asp Arg Thr
   195 200 205
   Asp Ile Gln Cys Leu Ile Pro Cys Ala Ile Asp Gln Asp Pro Tyr Phe
   210 215 220
   Arg Met Thr Arg Asp Val Ala Pro Arg Ile Gly Tyr Pro Lys Pro Ala
   225 230 235 240
   Leu Leu His Ser Thr Phe Phe Pro Ala Leu Gln Gly Ala Gln Thr Lys
   245 250 255
   Met Ser Ala Ser Asp Pro Asn Ser Ser Ile Phe Leu Thr Asp Thr Ala
   260 265 270
   Lys Gln Ile Lys Thr Lys Val Asn Lys His Ala Phe Ser Gly Gly Arg
   275 280 285
   Asp Thr Ile Glu Glu His Arg Gln Phe Gly Gly Asn Cys Asp Val Asp
   290 295 300
   Val Ser Phe Met Tyr Leu Thr Phe Phe Leu Glu Asp Asp Asp Lys Leu
   305 310 315 320
   Glu Gln Ile Arg Lys Asp Tyr Thr Ser Gly Ala Met Leu Thr Gly Glu
   325 330 335
   Leu Lys Lys Ala Leu Ile Glu Val Leu Gln Pro Leu Ile Ala Glu His
   340 345 350
   Gln Ala Arg Arg Lys Glu Val Thr Asp Glu Ile Val Lys Glu Phe Met
   355 360 365
   Thr Pro Arg Lys Leu Ser Phe Asp Phe Gln
   370 375
<210> 13
   <211> 401
   <212> PRT
   <213> Homo sapiens
<400> 13
   Ser Asn His Gly Pro Asp Ala Thr Glu Ala Glu Glu Asp Phe Val Asp
   1 5 10 15
   Pro Trp Thr Val Gln Thr Ser Ser Ala Lys Gly Ile Asp Tyr Asp Lys
   20 25 30
   Leu Ile Val Arg Phe Gly Ser Ser Lys Ile Asp Lys Glu Leu Ile Asn
   35 40 45
   Arg Ile Glu Arg Ala Thr Gly Gln Arg Pro His His Phe Leu Arg Arg
   50 55 60
   Gly Ile Phe Phe Ser His Arg Asp Met Asn Gln Val Leu Asp Ala Tyr
   65 70 75 80
   Glu Asn Lys Lys Pro Phe Tyr Leu Tyr Thr Gly Arg Gly Pro Ser Ser
   85 90 95
   Glu Ala Met His Val Gly His Leu Ile Pro Phe Ile Phe Thr Lys Trp
   100 105 110
   Leu Gln Asp Val Phe Asn Val Pro Leu Val Ile Gln Met Thr Asp Asp
   115 120 125
   Glu Lys Tyr Leu Trp Lys Asp Leu Thr Leu Asp Gln Ala Tyr Gly Asp
   130 135 140
   Ala Val Glu Asn Ala Lys Asp Ile Ile Ala Cys Gly Phe Asp Ile Asn
   145 150 155 160
   Lys Thr Phe Ile Phe Ser Asp Leu Asp Tyr Met Gly Met Ser Ser Gly
   165 170 175
   Phe Tyr Lys Asn Val Val Lys Ile Gln Lys His Val Thr Phe Asn Gln
   180 185 190
   Val Lys Gly Ile Phe Gly Phe Thr Asp Ser Asp Cys Ile Gly Lys Ile
   195 200 205
   Ser Phe Pro Ala Ile Gln Ala Ala Pro Ser Phe Ser Asn Ser Phe Pro
   210 215 220
   Gln Ile Phe Arg Asp Arg Thr Asp Ile Gln Cys Leu Ile Pro Cys Ala
   225 230 235 240
   Ile Asp Gln Asp Pro Tyr Phe Arg Met Thr Arg Asp Val Ala Pro Arg
   245 250 255
   Ile Gly Tyr Pro Lys Pro Ala Leu Leu His Ser Thr Phe Phe Pro Ala
   260 265 270
   Leu Gln Gly Ala Gln Thr Lys Met Ser Ala Ser Asp Pro Asn Ser Ser
   275 280 285
   Ile Phe Leu Thr Asp Thr Ala Lys Gln Ile Lys Thr Lys Val Asn Lys
   290 295 300
   His Ala Phe Ser Gly Gly Arg Asp Thr Ile Glu Glu His Arg Gln Phe
   305 310 315 320
   Gly Gly Asn Cys Asp Val Asp Val Ser Phe Met Tyr Leu Thr Phe Phe
   325 330 335
   Leu Glu Asp Asp Asp Lys Leu Glu Gln Ile Arg Lys Asp Tyr Thr Ser
   340 345 350
   Gly Ala Met Leu Thr Gly Glu Leu Lys Lys Ala Leu Ile Glu Val Leu
   355 360 365
   Gln Pro Leu Ile Ala Glu His Gln Ala Arg Arg Lys Glu Val Thr Asp
   370 375 380
   Glu Ile Val Lys Glu Phe Met Thr Pro Arg Lys Leu Ser Phe Asp Phe
   385 390 395 400
   Gln
<210> 14
   <211> 423
   <212> PRT
   <213> Artificial sequence
<220>
   <223> Recombinant human mini-TrpRS (GD variant) construct
<400> 14
   Ser Tyr Lys Ala Ala Ala Gly Glu Asp Tyr Lys Ala Asp Cys Pro Pro
   1 5 10 15
   Gly Asn Pro Ala Pro Thr Ser Asn His Gly Pro Asp Ala Thr Glu Ala
   20 25 30
   Glu Glu Asp Phe Val Asp Pro Trp Thr Val Gln Thr Ser Ser Ala Lys
   35 40 45
   Gly Ile Asp Tyr Asp Lys Leu Ile Val Arg Phe Gly Ser Ser Lys Ile
   50 55 60
   Asp Lys Glu Leu Ile Asn Arg Ile Glu Arg Ala Thr Gly Gln Arg Pro
   65 70 75 80
   His His Phe Leu Arg Arg Gly Ile Phe Phe Ser His Arg Asp Met Asn
   85 90 95
   Gln Val Leu Asp Ala Tyr Glu Asn Lys Lys Pro Phe Tyr Leu Tyr Thr
   100 105 110
   Gly Arg Gly Pro Ser Ser Glu Ala Met His Val Gly His Leu Ile Pro
   115 120 125
   Phe Ile Phe Thr Lys Trp Leu Gln Asp Val Phe Asn Val Pro Leu Val
   130 135 140
   Ile Gln Met Thr Asp Asp Glu Lys Tyr Leu Trp Lys Asp Leu Thr Leu
   145 150 155 160
   Asp Gln Ala Tyr Gly Asp Ala Val Glu Asn Ala Lys Asp Ile Ile Ala
   165 170 175
   Cys Gly Phe Asp Ile Asn Lys Thr Phe Ile Phe Ser Asp Leu Asp Tyr
   180 185 190
   Met Gly Met Ser Ser Gly Phe Tyr Lys Asn Val Val Lys Ile Gln Lys
   195 200 205
   His Val Thr Phe Asn Gln Val Lys Gly Ile Phe Gly Phe Thr Asp Ser
   210 215 220
   Asp Cys Ile Gly Lys Ile Ser Phe Pro Ala Ile Gln Ala Ala Pro Ser
   225 230 235 240
   Phe Ser Asn Ser Phe Pro Gln Ile Phe Arg Asp Arg Thr Asp Ile Gln
   245 250 255
   Cys Leu Ile Pro Cys Ala Ile Asp Gln Asp Pro Tyr Phe Arg Met Thr
   260 265 270
   Arg Asp Val Ala Pro Arg Ile Gly Tyr Pro Lys Pro Ala Leu Leu His
   275 280 285
   Ser Thr Phe Phe Pro Ala Leu Gln Gly Ala Gln Thr Lys Met Ser Ala
   290 295 300
   Ser Asp Pro Asn Ser Ser Ile Phe Leu Thr Asp Thr Ala Lys Gln Ile
   305 310 315 320
   Lys Thr Lys Val Asn Lys His Ala Phe Ser Gly Gly Arg Asp Thr Ile
   325 330 335
   Glu Glu His Arg Gln Phe Gly Gly Asn Cys Asp Val Asp Val Ser Phe
   340 345 350
   Met Tyr Leu Thr Phe Phe Leu Glu Asp Asp Asp Lys Leu Glu Gln Ile
   355 360 365
   Arg Lys Asp Tyr Thr Ser Gly Ala Met Leu Thr Gly Glu Leu Lys Lys
   370 375 380
   Ala Leu Ile Glu Val Leu Gln Pro Leu Ile Ala Glu His Gln Ala Arg
   385 390 395 400
   Arg Lys Glu Val Thr Asp Glu Ile Val Lys Glu Phe Met Thr Pro Arg
   405 410 415
   Lys Leu Ser Phe Asp Phe Gln
   420
<210> 15
   <211> 379
   <212> PRT
   <213> Artificial sequence
<220>
   <223> Recombinant human Met-T2 (GD variant) construct
<400> 15
   Met Ser Ala Lys Gly Ile Asp Tyr Asp Lys Leu Ile Val Arg Phe Gly
    1               5                  10                  15
   Ser Ser Lys Ile Asp Lys Glu Leu Ile Asn Arg Ile Glu Arg Ala Thr
                   20                  25                  30
   Gly Gln Arg Pro His His Phe Leu Arg Arg Gly Ile Phe Phe Ser His
           35                  40                  45
   Arg Asp Met Asn Gln Val Leu Asp Ala Tyr Glu Asn Lys Lys Pro Phe
       50                  55                  60
   Tyr Leu Tyr Thr Gly Arg Gly Pro Ser Ser Glu Ala Met His Val Gly
   65                  70                  75                  80
   His Leu Ile Pro Phe Ile Phe Thr Lys Trp Leu Gln Asp Val Phe Asn
                   85                  90                  95
   Val Pro Leu Val Ile Gln Met Thr Asp Asp Glu Lys Tyr Leu Trp Lys
               100                 105                 110
   Asp Leu Thr Leu Asp Gln Ala Tyr Gly Asp Ala Val Glu Asn Ala Lys
           115                 120                 125
   Asp Ile Ile Ala Cys Gly Phe Asp Ile Asn Lys Thr Phe Ile Phe Ser
       130                 135                 140
   Asp Leu Asp Tyr Met Gly Met Ser Ser Gly Phe Tyr Lys Asn Val Val
   145                 150                 155                 160
   Lys Ile Gln Lys His Val Thr Phe Asn Gln Val Lys Gly Ile Phe Gly
                   165                 170                 175
   Phe Thr Asp Ser Asp Cys Ile Gly Lys Ile Ser Phe Pro Ala Ile Gln
               180                 185                 190
   Ala Ala Pro Ser Phe Ser Asn Ser Phe Pro Gln Ile Phe Arg Asp Arg
           195                 200                 205
   Thr Asp Ile Gln Cys Leu Ile Pro Cys Ala Ile Asp Gln Asp Pro Tyr
       210                 215                 220
   Phe Arg Met Thr Arg Asp Val Ala Pro Arg Ile Gly Tyr Pro Lys Pro
   225                 230                 235                 240
   Ala Leu Leu His Ser Thr Phe Phe Pro Ala Leu Gln Gly Ala Gln Thr
                   245                 250                 255
   Lys Met Ser Ala Ser Asp Pro Asn Ser Ser Ile Phe Leu Thr Asp Thr
               260                 265                 270
   Ala Lys Gln Ile Lys Thr Lys Val Asn Lys His Ala Phe Ser Gly Gly
           275                 280                 285
   Arg Asp Thr Ile Glu Glu His Arg Gln Phe Gly Gly Asn Cys Asp Val
   290 295 300
   Asp Val Ser Phe Met Tyr Leu Thr Phe Phe Leu Glu Asp Asp Asp Lys
   305 310 315 320
   Leu Glu Gln Ile Arg Lys Asp Tyr Thr Ser Gly Ala Met Leu Thr Gly
   325 330 335
   Glu Leu Lys Lys Ala Leu Ile Glu Val Leu Gln Pro Leu Ile Ala Glu
   340 345 350
   His Gln Ala Arg Arg Lys Glu Val Thr Asp Glu Ile Val Lys Glu Phe
   355 360 365
   Met Thr Pro Arg Lys Leu Ser Phe Asp Phe Gln
   370 375
<210> 16
   <211> 402
   <212> PRT
   <213> Artificial sequence
<220>
   <223> Recombinant human Met-T1 (GD variant) construct
<400> 16
   Met Ser Asn His Gly Pro Asp Ala Thr Glu Ala Glu Glu Asp Phe Val
   1 5 10 15
   Asp Pro Trp Thr Val Gln Thr Ser Ser Ala Lys Gly Ile Asp Tyr Asp
   20 25 30
   Lys Leu Ile Val Arg Phe Gly Ser Ser Lys Ile Asp Lys Glu Leu Ile
   35 40 45
   Asn Arg Ile Glu Arg Ala Thr Gly Gln Arg Pro His His Phe Leu Arg
   50 55 60
   Arg Gly Ile Phe Phe Ser His Arg Asp Met Asn Gln Val Leu Asp Ala
   65 70 75 80
   Tyr Glu Asn Lys Lys Pro Phe Tyr Leu Tyr Thr Gly Arg Gly Pro Ser
   85 90 95
   Ser Glu Ala Met His Val Gly His Leu Ile Pro Phe Ile Phe Thr Lys
   100 105 110
   Trp Leu Gln Asp Val Phe Asn Val Pro Leu Val Ile Gln Met Thr Asp
   115 120 125
   Asp Glu Lys Tyr Leu Trp Lys Asp Leu Thr Leu Asp Gln Ala Tyr Gly
   130 135 140
   Asp Ala Val Glu Asn Ala Lys Asp Ile Ile Ala Cys Gly Phe Asp Ile
   145 150 155 160
   Asn Lys Thr Phe Ile Phe Ser Asp Leu Asp Tyr Met Gly Met Ser Ser
   165 170 175
   Gly Phe Tyr Lys Asn Val Val Lys Ile Gln Lys His Val Thr Phe Asn
   180 185 190
   Gln Val Lys Gly Ile Phe Gly Phe Thr Asp Ser Asp Cys Ile Gly Lys
   195 200 205
   Ile Ser Phe Pro Ala Ile Gln Ala Ala Pro Ser Phe Ser Asn Ser Phe
   210 215 220
   Pro Gln Ile Phe Arg Asp Arg Thr Asp Ile Gln Cys Leu Ile Pro Cys
   225 230 235 240
   Ala Ile Asp Gln Asp Pro Tyr Phe Arg Met Thr Arg Asp Val Ala Pro
   245 250 255
   Arg Ile Gly Tyr Pro Lys Pro Ala Leu Leu His Ser Thr Phe Phe Pro
   260 265 270
   Ala Leu Gln Gly Ala Gln Thr Lys Met Ser Ala Ser Asp Pro Asn Ser
   275 280 285
   Ser Ile Phe Leu Thr Asp Thr Ala Lys Gln Ile Lys Thr Lys Val Asn
   290 295 300
   Lys His Ala Phe Ser Gly Gly Arg Asp Thr Ile Glu Glu His Arg Gln
   305 310 315 320
   Phe Gly Gly Asn Cys Asp Val Asp Val Ser Phe Met Tyr Leu Thr Phe
   325 330 335
   Phe Leu Glu Asp Asp Asp Lys Leu Glu Gln Ile Arg Lys Asp Tyr Thr
   340 345 350
   Ser Gly Ala Met Leu Thr Gly Glu Leu Lys Lys Ala Leu Ile Glu Val
   355 360 365
   Leu Gln Pro Leu Ile Ala Glu His Gln Ala Arg Arg Lys Glu Val Thr
   370 375 380
   Asp Glu Ile Val Lys Glu Phe Met Thr Pro Arg Lys Leu Ser Phe Asp
   385 390 395 400
   Phe Gln
<210> 17
   <211> 424
   <212> PRT
   <213> Artificial sequence
<220>
   <223> Recombinant human Met-mini-TrpRS (GD variant) construct
<400> 17
   Met Ser Tyr Lys Ala Ala Ala Gly Glu Asp Tyr Lys Ala Asp Cys Pro
   1 5 10 15
   Pro Gly Asn Pro Ala Pro Thr Ser Asn His Gly Pro Asp Ala Thr Glu
   20 25 30
   Ala Glu Glu Asp Phe Val Asp Pro Trp Thr Val Gln Thr Ser Ser Ala
   35 40 45
   Lys Gly Ile Asp Tyr Asp Lys Leu Ile Val Arg Phe Gly Ser Ser Lys
   50 55 60
   Ile Asp Lys Glu Leu Ile Asn Arg Ile Glu Arg Ala Thr Gly Gln Arg
   65 70 75 80
   Pro His His Phe Leu Arg Arg Gly Ile Phe Phe Ser His Arg Asp Met
   85 90 95
   Asn Gln Val Leu Asp Ala Tyr Glu Asn Lys Lys Pro Phe Tyr Leu Tyr
   100 105 110
   Thr Gly Arg Gly Pro Ser Ser Glu Ala Met His Val Gly His Leu Ile
   115 120 125
   Pro Phe Ile Phe Thr Lys Trp Leu Gln Asp Val Phe Asn Val Pro Leu
   130 135 140
   Val Ile Gln Met Thr Asp Asp Glu Lys Tyr Leu Trp Lys Asp Leu Thr
   145 150 155 160
   Leu Asp Gln Ala Tyr Gly Asp Ala Val Glu Asn Ala Lys Asp Ile Ile
   165 170 175
   Ala Cys Gly Phe Asp Ile Asn Lys Thr Phe Ile Phe Ser Asp Leu Asp
   180 185 190
   Tyr Met Gly Met Ser Ser Gly Phe Tyr Lys Asn Val Val Lys Ile Gln
   195 200 205
   Lys His Val Thr Phe Asn Gln Val Lys Gly Ile Phe Gly Phe Thr Asp
   210 215 220
   Ser Asp Cys Ile Gly Lys Ile Ser Phe Pro Ala Ile Gln Ala Ala Pro
   225 230 235 240
   Ser Phe Ser Asn Ser Phe Pro Gln Ile Phe Arg Asp Arg Thr Asp Ile
   245 250 255
   Gln Cys Leu Ile Pro Cys Ala Ile Asp Gln Asp Pro Tyr Phe Arg Met
   260 265 270
   Thr Arg Asp Val Ala Pro Arg Ile Gly Tyr Pro Lys Pro Ala Leu Leu
   275 280 285
   His Ser Thr Phe Phe Pro Ala Leu Gln Gly Ala Gln Thr Lys Met Ser
   290 295 300
   Ala Ser Asp Pro Asn Ser Ser Ile Phe Leu Thr Asp Thr Ala Lys Gln
   305 310 315 320
   Ile Lys Thr Lys Val Asn Lys His Ala Phe Ser Gly Gly Arg Asp Thr
   325 330 335
   Ile Glu Glu His Arg Gln Phe Gly Gly Asn Cys Asp Val Asp Val Ser
   340 345 350
   Phe Met Tyr Leu Thr Phe Phe Leu Glu Asp Asp Asp Lys Leu Glu Gln
   355 360 365
   Ile Arg Lys Asp Tyr Thr Ser Gly Ala Met Leu Thr Gly Glu Leu Lys
   370 375 380
   Lys Ala Leu Ile Glu Val Leu Gln Pro Leu Ile Ala Glu His Gln Ala
   385 390 395 400
   Arg Arg Lys Glu Val Thr Asp Glu Ile Val Lys Glu Phe Met Thr Pro
   405 410 415
   Arg Lys Leu Ser Phe Asp Phe Gln
   420
<210> 18
   <211> 1134
   <212> DNA
   <213> Artificial Sequence
<220>
   <223> Recombinant human T2 (GD variant) construct
<220>
   <221> CDS
   <222> (1)..(1134)
<400> 18
   agt gca aaa ggc ata gac tac gat aag ctc att gtt cgg ttt gga agt      48
   Ser Ala Lys Gly Ile Asp Tyr Asp Lys Leu Ile Val Arg Phe Gly Ser
   1               5                   10                  15
   agt aaa att gac aaa gag cta ata aac cga ata gag aga gcc acc ggc      96
   Ser Lys Ile Asp Lys Glu Leu Ile Asn Arg Ile Glu Arg Ala Thr Gly
               20                  25                  30
   caa aga cca cac cac ttc ctg cgc aga ggc atc ttc ttc tca cac aga      144
   Gln Arg Pro His His Phe Leu Arg Arg Gly Ile Phe Phe Ser His Arg
           35                  40                  45
   gat atg aat cag gtt ctt gat gcc tat gaa aat aag aag cca ttt tat      192
   Asp Met Asn Gln Val Leu Asp Ala Tyr Glu Asn Lys Lys Pro Phe Tyr
       50                  55                  60
   ctg tac acg ggc cgg ggc ccc tct tct gaa gca atg cat gta ggt cac      240
   Leu Tyr Thr Gly Arg Gly Pro Ser Ser Glu Ala Met His Val Gly His
   65                  70                  75                  80
   ctc att cca ttt att ttc aca aag tgg ctc cag gat gta ttt aac gtg      288
   Leu Ile Pro Phe Ile Phe Thr Lys Trp Leu Gln Asp Val Phe Asn Val
                   85                  90                  95
   ccc ttg gtc atc cag atg acg gat gac gag aag tat ctg tgg aag gac      336
   Pro Leu Val Ile Gln Met Thr Asp Asp Glu Lys Tyr Leu Trp Lys Asp
               100                 105                 110
   ctg acc ctg gac cag gcc tat ggc gat gct gtt gag aat gcc aag gac      384
   Leu Thr Leu Asp Gln Ala Tyr Gly Asp Ala Val Glu Asn Ala Lys Asp
           115                 120                 125
   atc atc gcc tgt ggc ttt gac atc aac aag act ttc ata ttc tct gac      432
   Ile Ile Ala Cys Gly Phe Asp Ile Asn Lys Thr Phe Ile Phe Ser Asp
       130                 135                 140
   ctg gac tac atg ggg atg agc tca ggt ttc tac aaa aat gtg gtg aag      480
   Leu Asp Tyr Met Gly Met Ser Ser Gly Phe Tyr Lys Asn Val Val Lys
   145                 150                 155                 160
   att caa aag cat gtt acc ttc aac caa gtg aaa ggc att ttc ggc ttc      528
   Ile Gln Lys His Val Thr Phe Asn Gln Val Lys Gly Ile Phe Gly Phe
                   165                 170                 175
   act gac agc gac tgc att ggg aag atc agt ttt cct gcc atc cag gct      576
   Thr Asp Ser Asp Cys Ile Gly Lys Ile Ser Phe Pro Ala Ile Gln Ala
               180                 185                 190
   gct ccc tcc ttc agc aac tca ttc cca cag atc ttc cga gac agg acg      624
   Ala Pro Ser Phe Ser Asn Ser Phe Pro Gln Ile Phe Arg Asp Arg Thr
           195                 200                 205
   gat atc cag tgc ctt atc cca tgt gcc att gac cag gat cct tac ttt      672
   Asp Ile Gln Cys Leu Ile Pro Cys Ala Ile Asp Gln Asp Pro Tyr Phe
       210                 215                 220
   aga atg aca agg gac gtc gcc ccc agg atc ggc tat cct aaa cca gcc     720
   Arg Met Thr Arg Asp Val Ala Pro Arg Ile Gly Tyr Pro Lys Pro Ala
   225                 230                 235                 240
   ctg ttg cac tcc acc ttc ttc cca gcc ctg cag ggc gcc cag acc aaa     768
   Leu Leu His Ser Thr Phe Phe Pro Ala Leu Gln Gly Ala Gln Thr Lys
                   245                 250                 255
   atg agt gcc agc gac cca aac tcc tcc atc ttc ctc acc gac acg gcc     816
   Met Ser Ala Ser Asp Pro Asn Ser Ser Ile Phe Leu Thr Asp Thr Ala
               260                 265                 270
   aag cag atc aaa acc aag gtc aat aag cat gcg ttt tct gga ggg aga     864
   Lys Gln Ile Lys Thr Lys Val Asn Lys His Ala Phe Ser Gly Gly Arg
           275                 280                 285
   gac acc atc gag gag cac agg cag ttt ggg ggc aac tgt gat gtg gac     912
   Asp Thr Ile Glu Glu His Arg Gln Phe Gly Gly Asn Cys Asp Val Asp
       290                 295                 300
   gtg tct ttc atg tac ctg acc ttc ttc ctc gag gac gac gac aag ctc     960
   Val Ser Phe Met Tyr Leu Thr Phe Phe Leu Glu Asp Asp Asp Lys Leu
   305                 310                 315                 320
   gag cag atc agg aag gat tac acc agc gga gcc atg ctc acc ggt gag     1008
   Glu Gln Ile Arg Lys Asp Tyr Thr Ser Gly Ala Met Leu Thr Gly Glu
                   325                 330                 335
   ctc aag aag gca ctc ata gag gtt ctg cag ccc ttg atc gca gag cac     1056
   Leu Lys Lys Ala Leu Ile Glu Val Leu Gln Pro Leu Ile Ala Glu His
               340                 345                 350
   cag gcc cgg cgc aag gag gtc acg gat gag ata gtg aaa gag ttc atg     1104
   Gln Ala Arg Arg Lys Glu Val Thr Asp Glu Ile Val Lys Glu Phe Met
           355                 360                 365
   act ccc cgg aag ctg tcc ttc gac ttt cag                             1134
   Thr Pro Arg Lys Leu Ser Phe Asp Phe Gln
       370                 375
<210> 19
   <211> 1137
   <212> DNA
   <213> Artificial sequence
<220>
   <223> Recombinant human Met-T2 (GD variant) construct
<220>
   <221> CDS
   <222> (1)..(1137)
<400> 19
   atg agt gca aaa ggc ata gac tac gat aag ctc att gtt cgg ttt gga     48
   Met Ser Ala Lys Gly Ile Asp Tyr Asp Lys Leu Ile Val Arg Phe Gly
   1               5                   10                  15
   agt agt aaa att gac aaa gag cta ata aac cga ata gag aga gcc acc     96
   Ser Ser Lys Ile Asp Lys Glu Leu Ile Asn Arg Ile Glu Arg Ala Thr
               20                  25                  30
   ggc caa aga cca cac cac ttc ctg cgc aga ggc atc ttc ttc tca cac     144
   Gly Gln Arg Pro His His Phe Leu Arg Arg Gly Ile Phe Phe Ser His
               35                  40                  45
   aga gat atg aat cag gtt ctt gat gcc tat gaa aat aag aag cca ttt     192
   Arg Asp Met Asn Gln Val Leu Asp Ala Tyr Glu Asn Lys Lys Pro Phe
       50                  55                  60
   tat ctg tac acg ggc cgg ggc ccc tct tct gaa gca atg cat gta ggt     240
   Tyr Leu Tyr Thr Gly Arg Gly Pro Ser Ser Glu Ala Met His Val Gly
   65                  70                  75                  80
   cac ctc att cca ttt att ttc aca aag tgg ctc cag gat gta ttt aac     288
   His Leu Ile Pro Phe Ile Phe Thr Lys Trp Leu Gln Asp Val Phe Asn
                   85                  90                  95
   gtg ccc ttg gtc atc cag atg acg gat gac gag aag tat ctg tgg aag     336
   Val Pro Leu Val Ile Gln Met Thr Asp Asp Glu Lys Tyr Leu Trp Lys
               100                 105                 110
   gac ctg acc ctg gac cag gcc tat ggc gat gct gtt gag aat gcc aag     384
   Asp Leu Thr Leu Asp Gln Ala Tyr Gly Asp Ala Val Glu Asn Ala Lys
           115                 120                 125
   gac atc atc gcc tgt ggc ttt gac atc aac aag act ttc ata ttc tct     432
   Asp Ile Ile Ala Cys Gly Phe Asp Ile Asn Lys Thr Phe Ile Phe Ser
       130                 135                 140
   gac ctg gac tac atg ggg atg agc tca ggt ttc tac aaa aat gtg gtg     480
   Asp Leu Asp Tyr Met Gly Met Ser Ser Gly Phe Tyr Lys Asn Val Val
   145                 150                 155                 160
   aag att caa aag cat gtt acc ttc aac caa gtg aaa ggc att ttc ggc     528
   Lys Ile Gln Lys His Val Thr Phe Asn Gln Val Lys Gly Ile Phe Gly
                   165                 170                 175
   ttc act gac agc gac tgc att ggg aag atc agt ttt cct gcc atc cag     576
   Phe Thr Asp Ser Asp Cys Ile Gly Lys Ile Ser Phe Pro Ala Ile Gln
               180                 185                 190
   gct gct ccc tcc ttc agc aac tca ttc cca cag atc ttc cga gac agg     624
   Ala Ala Pro Ser Phe Ser Asn Ser Phe Pro Gln Ile Phe Arg Asp Arg
           195                 200                 205
   acg gat atc cag tgc ctt atc cca tgt gcc att gac cag gat cct tac     672
   Thr Asp Ile Gln Cys Leu Ile Pro Cys Ala Ile Asp Gln Asp Pro Tyr
       210                 215                 220
   ttt aga atg aca agg gac gtc gcc ccc agg atc ggc tat cct aaa cca     720
   Phe Arg Met Thr Arg Asp Val Ala Pro Arg Ile Gly Tyr Pro Lys Pro
   225                 230                 235                 240
   gcc ctg ttg cac tcc acc ttc ttc cca gcc ctg cag ggc gcc cag acc     768
   Ala Leu Leu His Ser Thr Phe Phe Pro Ala Leu Gln Gly Ala Gln Thr
                   245                 250                 255
   aaa atg agt gcc agc gac cca aac tcc tcc atc ttc ctc acc gac acg     816
   Lys Met Ser Ala Ser Asp Pro Asn Ser Ser Ile Phe Leu Thr Asp Thr
               260                 265                 270
   gcc aag cag atc aaa acc aag gtc aat aag cat gcg ttt tct gga ggg     864
   Ala Lys Gln Ile Lys Thr Lys Val Asn Lys His Ala Phe Ser Gly Gly
           275                 280                 285
   aga gac acc atc gag gag cac agg cag ttt ggg ggc aac tgt gat gtg     912
   Arg Asp Thr Ile Glu Glu His Arg Gln Phe Gly Gly Asn Cys Asp Val
       290                 295                 300
   gac gtg tct ttc atg tac ctg acc ttc ttc ctc gag gac gac gac aag     960
   Asp Val Ser Phe Met Tyr Leu Thr Phe Phe Leu Glu Asp Asp Asp Lys
   305                 310                 315                 320
   ctc gag cag atc agg aag gat tac acc agc gga gcc atg ctc acc ggt    1008
   Leu Glu Gln Ile Arg Lys Asp Tyr Thr Ser Gly Ala Met Leu Thr Gly
                   325                 330                 335
   gag ctc aag aag gca ctc ata gag gtt ctg cag ccc ttg atc gca gag    1056
   Glu Leu Lys Lys Ala Leu Ile Glu Val Leu Gln Pro Leu Ile Ala Glu
               340                 345                 350
   cac cag gcc cgg cgc aag gag gtc acg gat gag ata gtg aaa gag ttc    1104
   His Gln Ala Arg Arg Lys Glu Val Thr Asp Glu Ile Val Lys Glu Phe
           355                 360                 365
   atg act ccc cgg aag ctg tcc ttc gac ttt cag                        1137
   Met Thr Pro Arg Lys Leu Ser Phe Asp Phe Gln
       370                 375
<210> 20
   <211> 1203
   <212> DNA
   <213> Artificial Sequence
<220>
   <223> Recombinant human T1 (GD variant) construct
<220>
   <221> CDS
   <222> (1)..(1203)
<400> 20
   agt aat cat ggc cca gat gcc aca gaa gct gaa gag gat ttt gtg gac    48
   Ser Asn His Gly Pro Asp Ala Thr Glu Ala Glu Glu Asp Phe Val Asp
   1               5                   10                  15
   cca tgg aca gta cag aca agc agt gca aaa ggc ata gac tac gat aag    96
   Pro Trp Thr Val Gln Thr Ser Ser Ala Lys Gly Ile Asp Tyr Asp Lys
               20                  25                  30
   ctc att gtt cgg ttt gga agt agt aaa att gac aaa gag cta ata aac    144
   Leu Ile Val Arg Phe Gly Ser Ser Lys Ile Asp Lys Glu Leu Ile Asn
           35                  40                  45
   cga ata gag aga gcc acc ggc caa aga cca cac cac ttc ctg cgc aga    192
   Arg Ile Glu Arg Ala Thr Gly Gln Arg Pro His His Phe Leu Arg Arg
       50                  55                  60
   ggc atc ttc ttc tca cac aga gat atg aat cag gtt ctt gat gcc tat    240
   Gly Ile Phe Phe Ser His Arg Asp Met Asn Gln Val Leu Asp Ala Tyr
   65                  70                  75                  80
   gaa aat aag aag cca ttt tat ctg tac acg ggc cgg ggc ccc tct tct    288
   Glu Asn Lys Lys Pro Phe Tyr Leu Tyr Thr Gly Arg Gly Pro Ser Ser
                   85                  90                  95
   gaa gca atg cat gta ggt cac ctc att cca ttt att ttc aca aag tgg    336
   Glu Ala Met His Val Gly His Leu Ile Pro Phe Ile Phe Thr Lys Trp
               100                 105                 110
   ctc cag gat gta ttt aac gtg ccc ttg gtc atc cag atg acg gat gac    384
   Leu Gln Asp Val Phe Asn Val Pro Leu Val Ile Gln Met Thr Asp Asp
           115                 120                 125
   gag aag tat ctg tgg aag gac ctg acc ctg gac cag gcc tat ggc gat    432
   Glu Lys Tyr Leu Trp Lys Asp Leu Thr Leu Asp Gln Ala Tyr Gly Asp
       130                 135                 140
   gct gtt gag aat gcc aag gac atc atc gcc tgt ggc ttt gac atc aac      480
   Ala Val Glu Asn Ala Lys Asp Ile Ile Ala Cys Gly Phe Asp Ile Asn
   145                 150                 155                 160
   aag act ttc ata ttc tct gac ctg gac tac atg ggg atg agc tca ggt      528
   Lys Thr Phe Ile Phe Ser Asp Leu Asp Tyr Met Gly Met Ser Ser Gly
                   165                 170                 175
   ttc tac aaa aat gtg gtg aag att caa aag cat gtt acc ttc aac caa      576
   Phe Tyr Lys Asn Val Val Lys Ile Gln Lys His Val Thr Phe Asn Gln
               180                 185                 190
   gtg aaa ggc att ttc ggc ttc act gac agc gac tgc att ggg aag atc      624
   Val Lys Gly Ile Phe Gly Phe Thr Asp Ser Asp Cys Ile Gly Lys Ile
           195                 200                 205
   agt ttt cct gcc atc cag gct gct ccc tcc ttc agc aac tca ttc cca      672
   Ser Phe Pro Ala Ile Gln Ala Ala Pro Ser Phe Ser Asn Ser Phe Pro
       210                 215                 220
   cag atc ttc cga gac agg acg gat atc cag tgc ctt atc cca tgt gcc      720
   Gln Ile Phe Arg Asp Arg Thr Asp Ile Gln Cys Leu Ile Pro Cys Ala
   225                 230                 235                 240
   att gac cag gat cct tac ttt aga atg aca agg gac gtc gcc ccc agg      768
   Ile Asp Gln Asp Pro Tyr Phe Arg Met Thr Arg Asp Val Ala Pro Arg
                   245                 250                 255
   atc ggc tat cct aaa cca gcc ctg ttg cac tcc acc ttc ttc cca gcc      816
   Ile Gly Tyr Pro Lys Pro Ala Leu Leu His Ser Thr Phe Phe Pro Ala
               260                 265                 270
   ctg cag ggc gcc cag acc aaa atg agt gcc agc gac cca aac tcc tcc      864
   Leu Gln Gly Ala Gln Thr Lys Met Ser Ala Ser Asp Pro Asn Ser Ser
           275                 280                 285
   atc ttc ctc acc gac acg gcc aag cag atc aaa acc aag gtc aat aag      912
   Ile Phe Leu Thr Asp Thr Ala Lys Gln Ile Lys Thr Lys Val Asn Lys
       290                 295                 300
   cat gcg ttt tct gga ggg aga gac acc atc gag gag cac agg cag ttt      960
   His Ala Phe Ser Gly Gly Arg Asp Thr Ile Glu Glu His Arg Gln Phe
   305                 310                 315                 320
   ggg ggc aac tgt gat gtg gac gtg tct ttc atg tac ctg acc ttc ttc      1008
   Gly Gly Asn Cys Asp Val Asp Val Ser Phe Met Tyr Leu Thr Phe Phe
                   325                 330                 335
   ctc gag gac gac gac aag ctc gag cag atc agg aag gat tac acc agc      1056
   Leu Glu Asp Asp Asp Lys Leu Glu Gln Ile Arg Lys Asp Tyr Thr Ser
               340                 345                 350
   gga gcc atg ctc acc ggt gag ctc aag aag gca ctc ata gag gtt ctg      1104
   Gly Ala Met Leu Thr Gly Glu Leu Lys Lys Ala Leu Ile Glu Val Leu
           355                 360                 365
   cag ccc ttg atc gca gag cac cag gcc cgg cgc aag gag gtc acg gat      1152
   Gln Pro Leu Ile Ala Glu His Gln Ala Arg Arg Lys Glu Val Thr Asp
       370                 375                 380
   gag ata gtg aaa gag ttc atg act ccc cgg aag ctg tcc ttc gac ttt      1200
   Glu Ile Val Lys Glu Phe Met Thr Pro Arg Lys Leu Ser Phe Asp Phe
   385                 390                 395                 400
   cag 1203
   Gln
<210> 21
   <211> 1206
   <212> DNA
   <213> Artificial sequence
<220>
   <223> Recombinant human Met-T1 (GD variant) construct
<220>
   <221> CDS
   <222> (1)..(1206)
<400> 21
   atg agt aat cat ggc cca gat gcc aca gaa gct gaa gag gat ttt gtg      48
   Met Ser Asn His Gly Pro Asp Ala Thr Glu Ala Glu Glu Asp Phe Val
   1               5                   10                  15
   gac cca tgg aca gta cag aca agc agt gca aaa ggc ata gac tac gat      96
   Asp Pro Trp Thr Val Gln Thr Ser Ser Ala Lys Gly Ile Asp Tyr Asp
               20                  25                  30
   aag ctc att gtt cgg ttt gga agt agt aaa att gac aaa gag cta ata      144
   Lys Leu Ile Val Arg Phe Gly Ser Ser Lys Ile Asp Lys Glu Leu Ile
           35                  40                  45
   aac cga ata gag aga gcc acc ggc caa aga cca cac cac ttc ctg cgc      192
   Asn Arg Ile Glu Arg Ala Thr Gly Gln Arg Pro His His Phe Leu Arg
       50                  55                  60
   aga ggc atc ttc ttc tca cac aga gat atg aat cag gtt ctt gat gcc      240
   Arg Gly Ile Phe Phe Ser His Arg Asp Met Asn Gln Val Leu Asp Ala
   65                  70                  75                  80
   tat gaa aat aag aag cca ttt tat ctg tac acg ggc cgg ggc ccc tct      288
   Tyr Glu Asn Lys Lys Pro Phe Tyr Leu Tyr Thr Gly Arg Gly Pro Ser
                   85                  90                  95
   tct gaa gca atg cat gta ggt cac ctc att cca ttt att ttc aca aag      336
   Ser Glu Ala Met His Val Gly His Leu Ile Pro Phe Ile Phe Thr Lys
               100                 105                 110
   tgg ctc cag gat gta ttt aac gtg ccc ttg gtc atc cag atg acg gat      384
   Trp Leu Gln Asp Val Phe Asn Val Pro Leu Val Ile Gln Met Thr Asp
           115                 120                 125
   gac gag aag tat ctg tgg aag gac ctg acc ctg gac cag gcc tat ggc      432
   Asp Glu Lys Tyr Leu Trp Lys Asp Leu Thr Leu Asp Gln Ala Tyr Gly
       130                 135                 140
   gat gct gtt gag aat gcc aag gac atc atc gcc tgt ggc ttt gac atc      480
   Asp Ala Val Glu Asn Ala Lys Asp Ile Ile Ala Cys Gly Phe Asp Ile
   145                 150                 155                 160
   aac aag act ttc ata ttc tct gac ctg gac tac atg ggg atg agc tca      528
   Asn Lys Thr Phe Ile Phe Ser Asp Leu Asp Tyr Met Gly Met Ser Ser
                   165                 170                 175
   ggt ttc tac aaa aat gtg gtg aag att caa aag cat gtt acc ttc aac      576
   Gly Phe Tyr Lys Asn Val Val Lys Ile Gln Lys His Val Thr Phe Asn
               180                 185                 190
   caa gtg aaa ggc att ttc ggc ttc act gac agc gac tgc att ggg aag     624
   Gln Val Lys Gly Ile Phe Gly Phe Thr Asp Ser Asp Cys Ile Gly Lys
           195                 200                 205
   atc agt ttt cct gcc atc cag gct gct ccc tcc ttc agc aac tca ttc     672
   Ile Ser Phe Pro Ala Ile Gln Ala Ala Pro Ser Phe Ser Asn Ser Phe
       210                 215                 220
   cca cag atc ttc cga gac agg acg gat atc cag tgc ctt atc cca tgt     720
   Pro Gln Ile Phe Arg Asp Arg Thr Asp Ile Gln Cys Leu Ile Pro Cys
   225                 230                 235                 240
   gcc att gac cag gat cct tac ttt aga atg aca agg gac gtc gcc ccc     768
   Ala Ile Asp Gln Asp Pro Tyr Phe Arg Met Thr Arg Asp Val Ala Pro
                   245                 250                 255
   agg atc ggc tat cct aaa cca gcc ctg ttg cac tcc acc ttc ttc cca     816
   Arg Ile Gly Tyr Pro Lys Pro Ala Leu Leu His Ser Thr Phe Phe Pro
               260                 265                 270
   gcc ctg cag ggc gcc cag acc aaa atg agt gcc agc gac cca aac tcc     864
   Ala Leu Gln Gly Ala Gln Thr Lys Met Ser Ala Ser Asp Pro Asn Ser
           275                 280                 285
   tcc atc ttc ctc acc gac acg gcc aag cag atc aaa acc aag gtc aat     912
   Ser Ile Phe Leu Thr Asp Thr Ala Lys Gln Ile Lys Thr Lys Val Asn
       290                 295                 300
   aag cat gcg ttt tct gga ggg aga gac acc atc gag gag cac agg cag     960
   Lys His Ala Phe Ser Gly Gly Arg Asp Thr Ile Glu Glu His Arg Gln
   305                 310                 315                 320
   ttt ggg ggc aac tgt gat gtg gac gtg tct ttc atg tac ctg acc ttc     1008
   Phe Gly Gly Asn Cys Asp Val Asp Val Ser Phe Met Tyr Leu Thr Phe
                   325                 330                 335
   ttc ctc gag gac gac gac aag ctc gag cag atc agg aag gat tac acc     1056
   Phe Leu Glu Asp Asp Asp Lys Leu Glu Gln Ile Arg Lys Asp Tyr Thr
               340                 345                 350
   agc gga gcc atg ctc acc ggt gag ctc aag aag gca ctc ata gag gtt     1104
   Ser Gly Ala Met Leu Thr Gly Glu Leu Lys Lys Ala Leu Ile Glu Val
           355                 360                 365
   ctg cag ccc ttg atc gca gag cac cag gcc cgg cgc aag gag gtc acg     1152
   Leu Gln Pro Leu Ile Ala Glu His Gln Ala Arg Arg Lys Glu Val Thr
       370                 375                 380
   gat gag ata gtg aaa gag ttc atg act ccc cgg aag ctg tcc ttc gac     1200
   Asp Glu Ile Val Lys Glu Phe Met Thr Pro Arg Lys Leu Ser Phe Asp
   385                 390                 395                 400
   ttt cag                                                             1206
   Phe Gln
<210> 22
   <211> 1269
   <212> DNA
   <213> Artificial sequence
<220>
   <223> Recombinant human mini-TrpRS (GD variant) construct
<220>
   <221> CDS
   <222> (1)..(1269)
<400> 22
   agc tac aaa gct gcc gcg ggg gag gat tac aag gct gac tgt cct cca      48
   Ser Tyr Lys Ala Ala Ala Gly Glu Asp Tyr Lys Ala Asp Cys Pro Pro
   1               5                   10                  15
   ggg aac cca gca cct acc agt aat cat ggc cca gat gcc aca gaa gct      96
   Gly Asn Pro Ala Pro Thr Ser Asn His Gly Pro Asp Ala Thr Glu Ala
               20                  25                  30
   gaa gag gat ttt gtg gac cca tgg aca gta cag aca agc agt gca aaa      144
   Glu Glu Asp Phe Val Asp Pro Trp Thr Val Gln Thr Ser Ser Ala Lys
           35                  40                  45
   ggc ata gac tac gat aag ctc att gtt cgg ttt gga agt agt aaa att      192
   Gly Ile Asp Tyr Asp Lys Leu Ile Val Arg Phe Gly Ser Ser Lys Ile
       50                  55                  60
   gac aaa gag cta ata aac cga ata gag aga gcc acc ggc caa aga cca      240
   Asp Lys Glu Leu Ile Asn Arg Ile Glu Arg Ala Thr Gly Gln Arg Pro
   65                  70                  75                  80
   cac cac ttc ctg cgc aga ggc atc ttc ttc tca cac aga gat atg aat      288
   His His Phe Leu Arg Arg Gly Ile Phe Phe Ser His Arg Asp Met Asn
                   85                  90                  95
   cag gtt ctt gat gcc tat gaa aat aag aag cca ttt tat ctg tac acg      336
   Gln Val Leu Asp Ala Tyr Glu Asn Lys Lys Pro Phe Tyr Leu Tyr Thr
               100                 105                 110
   ggc cgg ggc ccc tct tct gaa gca atg cat gta ggt cac ctc att cca      384
   Gly Arg Gly Pro Ser Ser Glu Ala Met His Val Gly His Leu Ile Pro
           115                 120                 125
   ttt att ttc aca aag tgg ctc cag gat gta ttt aac gtg ccc ttg gtc      432
   Phe Ile Phe Thr Lys Trp Leu Gln Asp Val Phe Asn Val Pro Leu Val
       130                 135                 140
   atc cag atg acg gat gac gag aag tat ctg tgg aag gac ctg acc ctg      480
   Ile Gln Met Thr Asp Asp Glu Lys Tyr Leu Trp Lys Asp Leu Thr Leu
   145                 150                 155                 160
   gac cag gcc tat ggc gat gct gtt gag aat gcc aag gac atc atc gcc      528
   Asp Gln Ala Tyr Gly Asp Ala Val Glu Asn Ala Lys Asp Ile Ile Ala
                   165                 170                 175
   tgt ggc ttt gac atc aac aag act ttc ata ttc tct gac ctg gac tac      576
   Cys Gly Phe Asp Ile Asn Lys Thr Phe Ile Phe Ser Asp Leu Asp Tyr
               180                 185                 190
   atg ggg atg agc tca ggt ttc tac aaa aat gtg gtg aag att caa aag      624
   Met Gly Met Ser Ser Gly Phe Tyr Lys Asn Val Val Lys Ile Gln Lys
           195                 200                 205
   cat gtt acc ttc aac caa gtg aaa ggc att ttc ggc ttc act gac agc      672
   His Val Thr Phe Asn Gln Val Lys Gly Ile Phe Gly Phe Thr Asp Ser
       210                 215                 220
   gac tgc att ggg aag atc agt ttt cct gcc atc cag gct gct ccc tcc      720
   Asp Cys Ile Gly Lys Ile Ser Phe Pro Ala Ile Gln Ala Ala Pro Ser
   225                 230                 235                 240
   ttc agc aac tca ttc cca cag atc ttc cga gac agg acg gat atc cag      768
   Phe Ser Asn Ser Phe Pro Gln Ile Phe Arg Asp Arg Thr Asp Ile Gln
                         245                       250                       255
   tgc ctt atc cca tgt gcc att gac cag gat cct tac ttt aga atg aca      816
   Cys Leu Ile Pro Cys Ala Ile Asp Gln Asp Pro Tyr Phe Arg Met Thr
               260                     265                     270
   agg gac gtc gcc ccc agg atc ggc tat cct aaa cca gcc ctg ttg cac      864
   Arg Asp Val Ala Pro Arg Ile Gly Tyr Pro Lys Pro Ala Leu Leu His
           275                     280                     285
   tcc acc ttc ttc cca gcc ctg cag ggc gcc cag acc aaa atg agt gcc      912
   Ser Thr Phe Phe Pro Ala Leu Gln Gly Ala Gln Thr Lys Met Ser Ala
       290                     295                     300
   agc gac cca aac tcc tcc atc ttc ctc acc gac acg gcc aag cag atc      960
   Ser Asp Pro Asn Ser Ser Ile Phe Leu Thr Asp Thr Ala Lys Gln Ile
   305                     310                     315                     320
   aaa acc aag gtc aat aag cat gcg ttt tct gga ggg aga gac acc atc      1008
   Lys Thr Lys Val Asn Lys His Ala Phe Ser Gly Gly Arg Asp Thr Ile
                   325                     330                     335
   gag gag cac agg cag ttt ggg ggc aac tgt gat gtg gac gtg tct ttc      1056
   Glu Glu His Arg Gln Phe Gly Gly Asn Cys Asp Val Asp Val Ser Phe
               340                     345                     350
   atg tac ctg acc ttc ttc ctc gag gac gac gac aag ctc gag cag atc      1104
   Met Tyr Leu Thr Phe Phe Leu Glu Asp Asp Asp Lys Leu Glu Gln Ile
           355                     360                     365
   agg aag gat tac acc agc gga gcc atg ctc acc ggt gag ctc aag aag      1152
   Arg Lys Asp Tyr Thr Ser Gly Ala Met Leu Thr Gly Glu Leu Lys Lys
       370                     375                     380
   gca ctc ata gag gtt ctg cag ccc ttg atc gca gag cac cag gcc cgg      1200
   Ala Leu Ile Glu Val Leu Gln Pro Leu Ile Ala Glu His Gln Ala Arg
   385                     390                     395                     400
   cgc aag gag gtc acg gat gag ata gtg aaa gag ttc atg act ccc cgg      1248
   Arg Lys Glu Val Thr Asp Glu Ile Val Lys Glu Phe Met Thr Pro Arg
                   405                     410                     415
   aag ctg tcc ttc gac ttt cag                                          1269
   Lys Leu Ser Phe Asp Phe Gln
               420
<210> 23
   <211> 1272
   <212> DNA
   <213> Artificial Sequence
<220>
   <223> Recombinant human Met-mini-TrpRS (GD variant) construct
<220>
   <221> CDS
   <222> (1)..(1272)
<400> 23
   atg agc tac aaa gct gcc gcg ggg gag gat tac aag gct gac tgt cct      48
   Met Ser Tyr Lys Ala Ala Ala Gly Glu Asp Tyr Lys Ala Asp Cys Pro
   1               5                   10                  15
   cca ggg aac cca gca cct acc agt aat cat ggc cca gat gcc aca gaa      96
   Pro Gly Asn Pro Ala Pro Thr Ser Asn His Gly Pro Asp Ala Thr Glu
               20                  25                  30
   gct gaa gag gat ttt gtg gac cca tgg aca gta cag aca agc agt gca      144
   Ala Glu Glu Asp Phe Val Asp Pro Trp Thr Val Gln Thr Ser Ser Ala
           35                  40                  45
   aaa ggc ata gac tac gat aag ctc att gtt cgg ttt gga agt agt aaa      192
   Lys Gly Ile Asp Tyr Asp Lys Leu Ile Val Arg Phe Gly Ser Ser Lys
       50                  55                  60
   att gac aaa gag cta ata aac cga ata gag aga gcc acc ggc caa aga      240
   Ile Asp Lys Glu Leu Ile Asn Arg Ile Glu Arg Ala Thr Gly Gln Arg
   65                  70                  75                  80
   cca cac cac ttc ctg cgc aga ggc atc ttc ttc tca cac aga gat atg      288
   Pro His His Phe Leu Arg Arg Gly Ile Phe Phe Ser His Arg Asp Met
                   85                  90                  95
   aat cag gtt ctt gat gcc tat gaa aat aag aag cca ttt tat ctg tac      336
   Asn Gln Val Leu Asp Ala Tyr Glu Asn Lys Lys Pro Phe Tyr Leu Tyr
               100                 105                 110
   acg ggc cgg ggc ccc tct tct gaa gca atg cat gta ggt cac ctc att      384
   Thr Gly Arg Gly Pro Ser Ser Glu Ala Met His Val Gly His Leu Ile
           115                 120                 125
   cca ttt att ttc aca aag tgg ctc cag gat gta ttt aac gtg ccc ttg      432
   Pro Phe Ile Phe Thr Lys Trp Leu Gln Asp Val Phe Asn Val Pro Leu
       130                 135                 140
   gtc atc cag atg acg gat gac gag aag tat ctg tgg aag gac ctg acc      480
   Val Ile Gln Met Thr Asp Asp Glu Lys Tyr Leu Trp Lys Asp Leu Thr
   145                 150                 155                 160
   ctg gac cag gcc tat ggc gat gct gtt gag aat gcc aag gac atc atc      528
   Leu Asp Gln Ala Tyr Gly Asp Ala Val Glu Asn Ala Lys Asp Ile Ile
                   165                 170                 175
   gcc tgt ggc ttt gac atc aac aag act ttc ata ttc tct gac ctg gac      576
   Ala Cys Gly Phe Asp Ile Asn Lys Thr Phe Ile Phe Ser Asp Leu Asp
               180                 185                 190
   tac atg ggg atg agc tca ggt ttc tac aaa aat gtg gtg aag att caa      624
   Tyr Met Gly Met Ser Ser Gly Phe Tyr Lys Asn Val Val Lys Ile Gln
           195                 200                 205
   aag cat gtt acc ttc aac caa gtg aaa ggc att ttc ggc ttc act gac      672
   Lys His Val Thr Phe Asn Gln Val Lys Gly Ile Phe Gly Phe Thr Asp
       210                 215                 220
   agc gac tgc att ggg aag atc agt ttt cct gcc atc cag gct gct ccc      720
   Ser Asp Cys Ile Gly Lys Ile Ser Phe Pro Ala Ile Gln Ala Ala Pro
   225                 230                 235                 240
   tcc ttc agc aac tca ttc cca cag atc ttc cga gac agg acg gat atc      768
   Ser Phe Ser Asn Ser Phe Pro Gln Ile Phe Arg Asp Arg Thr Asp Ile
                   245                 250                 255
   cag tgc ctt atc cca tgt gcc att gac cag gat cct tac ttt aga atg      816
   Gln Cys Leu Ile Pro Cys Ala Ile Asp Gln Asp Pro Tyr Phe Arg Met
               260                 265                 270
   aca agg gac gtc gcc ccc agg atc ggc tat cct aaa cca gcc ctg ttg      864
   Thr Arg Asp Val Ala Pro Arg Ile Gly Tyr Pro Lys Pro Ala Leu Leu
           275                 280                 285
   cac tcc acc ttc ttc cca gcc ctg cag ggc gcc cag acc aaa atg agt      912
   His Ser Thr Phe Phe Pro Ala Leu Gln Gly Ala Gln Thr Lys Met Ser
       290                 295                 300
   gcc agc gac cca aac tcc tcc atc ttc ctc acc gac acg gcc aag cag      960
   Ala Ser Asp Pro Asn Ser Ser Ile Phe Leu Thr Asp Thr Ala Lys Gln
   305                 310                 315                 320
   atc aaa acc aag gtc aat aag cat gcg ttt tct gga ggg aga gac acc     1008
   Ile Lys Thr Lys Val Asn Lys His Ala Phe Ser Gly Gly Arg Asp Thr
                   325                 330                 335
   atc gag gag cac agg cag ttt ggg ggc aac tgt gat gtg gac gtg tct     1056
   Ile Glu Glu His Arg Gln Phe Gly Gly Asn Cys Asp Val Asp Val Ser
               340                 345                 350
   ttc atg tac ctg acc ttc ttc ctc gag gac gac gac aag ctc gag cag     1104
   Phe Met Tyr Leu Thr Phe Phe Leu Glu Asp Asp Asp Lys Leu Glu Gln
           355                 360                 365
   atc agg aag gat tac acc agc gga gcc atg ctc acc ggt gag ctc aag     1152
   Ile Arg Lys Asp Tyr Thr Ser Gly Ala Met Leu Thr Gly Glu Leu Lys
       370                 375                 380
   aag gca ctc ata gag gtt ctg cag ccc ttg atc gca gag cac cag gcc     1200
   Lys Ala Leu Ile Glu Val Leu Gln Pro Leu Ile Ala Glu His Gln Ala
   385                 390                 395                 400
   cgg cgc aag gag gtc acg gat gag ata gtg aaa gag ttc atg act ccc     1248
   Arg Arg Lys Glu Val Thr Asp Glu Ile Val Lys Glu Phe Met Thr Pro
                   405                 410                 415
   cgg aag ctg tcc ttc gac ttt cag                                     1272
   Arg Lys Leu Ser Phe Asp Phe Gln
               420
<210> 24
   <211> 378
   <212> PRT
   <213> Artificial sequence
<220>
   <223> Recombinant human T2 (SY variant) construct
<400> 24 <210> 25
   <211> 401
   <212> PRT
   <213> Artificial Sequence
<220>
   <223> Recombinant human T1 (SY variant) construct
<400> 25
<210> 26
   <211> 423
   <212> PRT
   <213> Artificial Sequence
<220>
   <223> Recombinant human mini-TrpRS (SY variant) construct
<400> 26
   Ser Tyr Lys Ala Ala Ala Gly Glu Asp Tyr Lys Ala Asp Cys Pro Pro
   1 5 10 15
   Gly Asn Pro Ala Pro Thr Ser Asn His Gly Pro Asp Ala Thr Glu Ala
   20 25 30
   Glu Glu Asp Phe Val Asp Pro Trp Thr Val Gln Thr Ser Ser Ala Lys
   35 40 45
   Gly Ile Asp Tyr Asp Lys Leu Ile Val Arg Phe Gly Ser Ser Lys Ile
   50 55 60
   Asp Lys Glu Leu Ile Asn Arg Ile Glu Arg Ala Thr Gly Gln Arg Pro
   65 70 75 80
   His His Phe Leu Arg Arg Gly Ile Phe Phe Ser His Arg Asp Met Asn
   85 90 95
   Gln Val Leu Asp Ala Tyr Glu Asn Lys Lys Pro Phe Tyr Leu Tyr Thr
   100 105 110
   Gly Arg Gly Pro Ser Ser Glu Ala Met His Val Gly His Leu Ile Pro
   115 120 125
   Phe Ile Phe Thr Lys Trp Leu Gln Asp Val Phe Asn Val Pro Leu Val
   130 135 140
   Ile Gln Met Thr Asp Asp Glu Lys Tyr Leu Trp Lys Asp Leu Thr Leu
   145 150 155 160
   Asp Gln Ala TYr Ser Tyr Ala Val Glu Asn Ala Lys Asp Ile Ile Ala
   165 170 175
   Cys Gly Phe Asp Ile Asn Lys Thr Phe Ile Phe Ser Asp Leu Asp Tyr
   180 185 190
   Met Gly Met Ser Ser Gly Phe Tyr Lys Asn Val Val Lys Ile Gln Lys
   195 200 205
   His Val Thr Phe Asn Gln Val Lys Gly Ile Phe Gly Phe Thr Asp Ser
   210 215 220
   Asp Cys Ile Gly Lys Ile Ser Phe Pro Ala Ile Gln Ala Ala Pro Ser
   225 230 235 240
   Phe Ser Asn Ser Phe Pro Gln Ile Phe Arg Asp Arg Thr Asp Ile Gln
   245 250 255
   Cys Leu Ile Pro Cys Ala Ile Asp Gln Asp Pro Tyr Phe Arg Met Thr
   260 265 270
   Arg Asp Val Ala Pro Arg Ile Gly Tyr Pro Lys Pro Ala Leu Leu His
   275 280 285
   Ser Thr Phe Phe Pro Ala Leu Gln Gly Ala Gln Thr Lys Met Ser Ala
   290 295 300
   Ser Asp Pro Asn Ser Ser Ile Phe Leu Thr Asp Thr Ala Lys Gln Ile
   305 310 315 320
   Lys Thr Lys Val Asn Lys His Ala Phe Ser Gly Gly Arg Asp Thr Ile
   325 330 335
   Glu Glu His Arg Gln Phe Gly Gly Asn Cys Asp Val Asp Val Ser Phe
   340 345 350
   Met Tyr Leu Thr Phe Phe Leu Glu Asp Asp Asp Lys Leu Glu Gln Ile
   355 360 365
   Arg Lys Asp Tyr Thr Ser Gly Ala Met Leu Thr Gly Glu Leu Lys Lys
   370 375 380
   Ala Leu Ile Glu Val Leu Gln Pro Leu Ile Ala Glu His Gln Ala Arg
   385 390 395 400
   Arg Lys Glu Val Thr Asp Glu Ile Val Lys Glu Phe Met Thr Pro Arg
   405 410 415
   Lys Leu Ser Phe Asp Phe Gln
   420
<210> 27
   <211> 379
   <212> PRT
   <213> Artificial Sequence
<220>
   <223> Recombinant human Met-T2 (SY variant) construct
<400> 27
   Met Ser Ala Lys Gly Ile Asp Tyr Asp Lys Leu Ile Val Arg Phe Gly
   1 5 10 15
   Ser Ser Lys Ile Asp Lys Glu Leu Ile Asn Arg Ile Glu Arg Ala Thr
   20 25 30
   Gly Gln Arg Pro His His Phe Leu Arg Arg Gly Ile Phe Phe Ser His
   35 40 45
   Arg Asp Met Asn Gln Val Leu Asp Ala Tyr Glu Asn Lys Lys Pro Phe
   50 55 60
   Tyr Leu Tyr Thr Gly Arg Gly Pro Ser Ser Glu Ala Met His Val Gly
   65 70 75 80
   His Leu Ile Pro Phe Ile Phe Thr Lys Trp Leu Gln Asp Val Phe Asn
   85 90 95
   Val Pro Leu Val Ile Gln Met Thr Asp Asp Glu Lys Tyr Leu Trp Lys
   100 105 110
   Asp Leu Thr Leu Asp Gln Ala Tyr Ser Tyr Ala Val Glu Asn Ala Lys
   115 120 125
   Asp Ile Ile Ala Cys Gly Phe Asp Ile Asn Lys Thr Phe Ile Phe Ser
   130 135 140
   Asp Leu Asp Tyr Met Gly Met Ser Ser Gly Phe Tyr Lys Asn Val Val
   145 150 155 160
   Lys Ile Gln Lys His Val Thr Phe Asn Gln Val Lys Gly Ile Phe Gly
   165 170 175
   Phe Thr Asp Ser Asp Cys Ile Gly Lys Ile Ser Phe Pro Ala Ile Gln
   180 185 190
   Ala Ala Pro Ser Phe Ser Asn Ser Phe Pro Gln Ile Phe Arg Asp Arg
   195 200 205
   Thr Asp Ile Gln Cys Leu Ile Pro Cys Ala Ile Asp Gln Asp Pro Tyr
   210 215 220
   Phe Arg Met Thr Arg Asp Val Ala Pro Arg Ile Gly Tyr Pro Lys Pro
   225 230 235 240
   Ala Leu Leu His Ser Thr Phe Phe Pro Ala Leu Gln Gly Ala Gln Thr
   245 250 255
   Lys Met Ser Ala Ser Asp Pro Asn Ser Ser Ile Phe Leu Thr Asp Thr
   260 265 270
   Ala Lys Gln Ile Lys Thr Lys Val Asn Lys His Ala Phe Ser Gly Gly
   275 280 285
   Arg Asp Thr Ile Glu Glu His Arg Gln Phe Gly Gly Asn Cys Asp Val
   290 295 300
   Asp Val Ser Phe Met Tyr Leu Thr Phe Phe Leu Glu Asp Asp Asp Lys
   305 310 315 320
   Leu Glu Gln Ile Arg Lys Asp Tyr Thr Ser Gly Ala Met Leu Thr Gly
   325 330 335
   Glu Leu Lys Lys Ala Leu Ile Glu Val Leu Gln Pro Leu Ile Ala Glu
   340 345 350
   His Gln Ala Arg Arg Lys Glu Val Thr Asp Glu Ile Val Lys Glu Phe
   355 360 365
   Met Thr Pro Arg Lys Leu Ser Phe Asp Phe Gln
   370 375
<210> 28
   <211> 402
   <212> PRT
   <213> Artificial Sequence
<220>
   <223> Recombinant human Met-T1 (SY variant) construct
<400> 28
   Met Ser Asn His Gly Pro Asp Ala Thr Glu Ala Glu Glu Asp Phe Val
   1 5 10 15
   Asp Pro Trp Thr Val Gln Thr Ser Ser Ala Lys Gly Ile Asp Tyr Asp
   20 25 30
   Lys Leu Ile Val Arg Phe Gly Ser Ser Lys Ile Asp Lys Glu Leu Ile
   35 40 45
   Asn Arg Ile Glu Arg Ala Thr Gly Gln Arg Pro His His Phe Leu Arg
   50 55 60
   Arg Gly Ile Phe Phe Ser His Arg Asp Met Asn Gln Val Leu Asp Ala
   65 70 75 80
   Tyr Glu Asn Lys Lys Pro Phe Tyr Leu Tyr Thr Gly Arg Gly Pro Ser
   85 90 95
   Ser Glu Ala Met His Val Gly His Leu Ile Pro Phe Ile Phe Thr Lys
   100 105 110
   Trp Leu Gln Asp Val Phe Asn Val Pro Leu Val Ile Gln Met Thr Asp
   115 120 125
   Asp Glu Lys Tyr Leu Trp Lys Asp Leu Thr Leu Asp Gln Ala Tyr Ser
   130 135 140
   Tyr Ala Val Glu Asn Ala Lys Asp Ile Ile Ala Cys Gly Phe Asp Ile
   145 150 155 160
   Asn Lys Thr Phe Ile Phe Ser Asp Leu Asp Tyr Met Gly Met Ser Ser
   165 170 175
   Gly Phe Tyr Lys Asn Val Val Lys Ile Gln Lys His Val Thr Phe Asn
   180 185 190
   Gln Val Lys Gly Ile Phe Gly Phe Thr Asp Ser Asp Cys Ile Gly Lys
   195 200 205
   Ile Ser Phe Pro Ala Ile Gln Ala Ala Pro Ser Phe Ser Asn Ser Phe
   210 215 220
   Pro Gln Ile Phe Arg Asp Arg Thr Asp Ile Gln Cys Leu Ile Pro Cys
   225 230 235 240
   Ala Ile Asp Gln Asp Pro Tyr Phe Arg Met Thr Arg Asp Val Ala Pro
   245 250 255
   Arg Ile Gly Tyr Pro Lys Pro Ala Leu Leu His Ser Thr Phe Phe Pro
   260 265 270
   Ala Leu Gln Gly Ala Gln Thr Lys Met Ser Ala Ser Asp Pro Asn Ser
   275 280 285
   Ser Ile Phe Leu Thr Asp Thr Ala Lys Gln Ile Lys Thr Lys Val Asn
   290 295 300
   Lys His Ala Phe Ser Gly Gly Arg Asp Thr Ile Glu Glu His Arg Gln
   305 310 315 320
   Phe Gly Gly Asn Cys Asp Val Asp Val Ser Phe Met Tyr Leu Thr Phe
   325 330 335
   Phe Leu Glu Asp Asp Asp Lys Leu Glu Gln Ile Arg Lys Asp Tyr Thr
   340 345 350
   Ser Gly Ala Met Leu Thr Gly Glu Leu Lys Lys Ala Leu Ile Glu Val
   355 360 365
   Leu Gln Pro Leu Ile Ala Glu His Gln Ala Arg Arg Lys Glu Val Thr
   370 375 380
   Asp Glu Ile Val Lys Glu Phe Met Thr Pro Arg Lys Leu Ser Phe Asp
   385 390 395 400
   Phe Gln
<210> 29
   <211> 424
   <212> PRT
   <213> Artificial Sequence
<220>
   <223> Recombinant human Met-mini-TrpRS (SY variant) construct
<400> 29
<210> 30
   <211> 1134
   <212> DNA
   <213> Artificial sequence
<220>
   <223> Recombinant human T2 (SY variant) construct
<220>
   <221> CDS
   <222> (1)..(1134)
<220>
   <221> misc_feature
   <222> (358)..(360)
   <223> nnn is agy, tcy or tcr
<400> 30
   agt gca aaa ggc ata gac tac gat aag ctc att gtt cgg ttt gga agt      48
   Ser Ala Lys Gly Ile Asp Tyr Asp Lys Leu Ile Val Arg Phe Gly Ser
   1               5                   10                  15
   agt aaa att gac aaa gag cta ata aac cga ata gag aga gcc acc ggc      96
   Ser Lys Ile Asp Lys Glu Leu Ile Asn Arg Ile Glu Arg Ala Thr Gly
               20                  25                  30
   caa aga cca cac cac ttc ctg cgc aga ggc atc ttc ttc tca cac aga      144
   Gln Arg Pro His His Phe Leu Arg Arg Gly Ile Phe Phe Ser His Arg
           35                  40                  45
   gat atg aat cag gtt ctt gat gcc tat gaa aat aag aag cca ttt tat      192
   Asp Met Asn Gln Val Leu Asp Ala Tyr Glu Asn Lys Lys Pro Phe Tyr
       50                  55                  60
   ctg tac acg ggc cgg ggc ccc tct tct gaa gca atg cat gta ggt cac      240
   Leu Tyr Thr Gly Arg Gly Pro Ser Ser Glu Ala Met His Val Gly His
   65                  70                  75                  80
   ctc att cca ttt att ttc aca aag tgg ctc cag gat gta ttt aac gtg      288
   Leu Ile Pro Phe Ile Phe Thr Lys Trp Leu Gln Asp Val Phe Asn Val
                   85                  90                  95
   ccc ttg gtc atc cag atg acg gat gac gag aag tat ctg tgg aag gac      336
   Pro Leu Val Ile Gln Met Thr Asp Asp Glu Lys Tyr Leu Trp Lys Asp
               100                 105                 110
   ctg acc ctg gac cag gcc tat nnn tay gct gtt gag aat gcc aag gac     384
   Leu Thr Leu Asp Gln Ala Tyr Ser Tyr Ala Val Glu Asn Ala Lys Asp
           115                 120                 125
   atc atc gcc tgt ggc ttt gac atc aac aag act ttc ata ttc tct gac     432
   Ile Ile Ala Cys Gly Phe Asp Ile Asn Lys Thr Phe Ile Phe Ser Asp
       130                 135                 140
   ctg gac tac atg ggg atg agc tca ggt ttc tac aaa aat gtg gtg aag     480
   Leu Asp Tyr Met Gly Met Ser Ser Gly Phe Tyr Lys Asn Val Val Lys
   145                 150                 155                 160
   att caa aag cat gtt acc ttc aac caa gtg aaa ggc att ttc ggc ttc     528
   Ile Gln Lys His Val Thr Phe Asn Gln Val Lys Gly Ile Phe Gly Phe
                   165                 170                 175
   act gac agc gac tgc att ggg aag atc agt ttt cct gcc atc cag gct     576
   Thr Asp Ser Asp Cys Ile Gly Lys Ile Ser Phe Pro Ala Ile Gln Ala
               180                 185                 190
   gct ccc tcc ttc agc aac tca ttc cca cag atc ttc cga gac agg acg     624
   Ala Pro Ser Phe Ser Asn Ser Phe Pro Gln Ile Phe Arg Asp Arg Thr
           195                 200                 205
   gat atc cag tgc ctt atc cca tgt gcc att gac cag gat cct tac ttt     672
   Asp Ile Gln Cys Leu Ile Pro Cys Ala Ile Asp Gln Asp Pro Tyr Phe
       210                 215                 220
   aga atg aca agg gac gtc gcc ccc agg atc ggc tat cct aaa cca gcc     720
   Arg Met Thr Arg Asp Val Ala Pro Arg Ile Gly Tyr Pro Lys Pro Ala
   225                 230                 235                 240
   ctg ttg cac tcc acc ttc ttc cca gcc ctg cag ggc gcc cag acc aaa     768
   Leu Leu His Ser Thr Phe Phe Pro Ala Leu Gln Gly Ala Gln Thr Lys
                   245                 250                 255
   atg agt gcc agc gac cca aac tcc tcc atc ttc ctc acc gac acg gcc     816
   Met Ser Ala Ser Asp Pro Asn Ser Ser Ile Phe Leu Thr Asp Thr Ala
               260                 265                 270
   aag cag atc aaa acc aag gtc aat aag cat gcg ttt tct gga ggg aga     864
   Lys Gln Ile Lys Thr Lys Val Asn Lys His Ala Phe Ser Gly Gly Arg
           275                 280                 285
   gac acc atc gag gag cac agg cag ttt ggg ggc aac tgt gat gtg gac     912
   Asp Thr Ile Glu Glu His Arg Gln Phe Gly Gly Asn Cys Asp Val Asp
       290                 295                 300
   gtg tct ttc atg tac ctg acc ttc ttc ctc gag gac gac gac aag ctc     960
   Val Ser Phe Met Tyr Leu Thr Phe Phe Leu Glu Asp Asp Asp Lys Leu
   305                 310                 315                 320
   gag cag atc agg aag gat tac acc agc gga gcc atg ctc acc ggt gag     1008
   Glu Gln Ile Arg Lys Asp Tyr Thr Ser Gly Ala Met Leu Thr Gly Glu
                   325                 330                 335
   ctc aag aag gca ctc ata gag gtt ctg cag ccc ttg atc gca gag cac     1056
   Leu Lys Lys Ala Leu Ile Glu Val Leu Gln Pro Leu Ile Ala Glu His
               340                 345                 350
   cag gcc cgg cgc aag gag gtc acg gat gag ata gtg aaa gag ttc atg     1104
   Gln Ala Arg Arg Lys Glu Val Thr Asp Glu Ile Val Lys Glu Phe Met
           355                 360                 365
   act ccc cgg aag ctg tcc ttc gac ttt cag                       1134
   Thr Pro Arg Lys Leu Ser Phe Asp Phe Gln
       370                 375
<210> 31
   <211> 1137
   <212> DNA
   <213> Artificial sequence
<220>
   <223> Recombinant human Met-T2 (SY variant) construct
<220>
   <221> CDS
   <222> (1)..(1137)
<220>
   <221> misc_feature
   <222> (361)..(363)
   <223> nnn is agy, tcy or tcr
<400> 31
   atg agt gca aaa ggc ata gac tac gat aag ctc att gtt cgg ttt gga    48
   Met Ser Ala Lys Gly Ile Asp Tyr Asp Lys Leu Ile Val Arg Phe Gly
     1               5                  10                  15
   agt agt aaa att gac aaa gag cta ata aac cga ata gag aga gcc acc    96
   Ser Ser Lys Ile Asp Lys Glu Leu Ile Asn Arg Ile Glu Arg Ala Thr
                   20                  25                  30
   ggc caa aga cca cac cac ttc ctg cgc aga ggc atc ttc ttc tca cac   144
   Gly Gln Arg Pro His His Phe Leu Arg Arg Gly Ile Phe Phe Ser His
               35                  40                  45
   aga gat atg aat cag gtt ctt gat gcc tat gaa aat aag aag cca ttt   192
   Arg Asp Met Asn Gln Val Leu Asp Ala Tyr Glu Asn Lys Lys Pro Phe
       50                  55                  60
   tat ctg tac acg ggc cgg ggc ccc tct tct gaa gca atg cat gta ggt   240
   Tyr Leu Tyr Thr Gly Arg Gly Pro Ser Ser Glu Ala Met His Val Gly
    65                  70                  75                  80
   cac ctc att cca ttt att ttc aca aag tgg ctc cag gat gta ttt aac   288
   His Leu Ile Pro Phe Ile Phe Thr Lys Trp Leu Gln Asp Val Phe Asn
                   85                  90                  95
   gtg ccc ttg gtc atc cag atg acg gat gac gag aag tat ctg tgg aag   336
   Val Pro Leu Val Ile Gln Met Thr Asp Asp Glu Lys Tyr Leu Trp Lys
               100                 105                 110
   gac ctg acc ctg gac cag gcc tat nnn tay gct gtt gag aat gcc aag   384
   Asp Leu Thr Leu Asp Gln Ala Tyr Ser Tyr Ala Val Glu Asn Ala Lys
           115                 120                 125
   gac atc atc gcc tgt ggc ttt gac atc aac aag act ttc ata ttc tct   432
   Asp Ile Ile Ala Cys Gly Phe Asp Ile Asn Lys Thr Phe Ile Phe Ser
       130                 135                 140
   gac ctg gac tac atg ggg atg agc tca ggt ttc tac aaa aat gtg gtg   480
   Asp Leu Asp Tyr Met Gly Met Ser Ser Gly Phe Tyr Lys Asn Val Val
   145                 150                 155                 160
   aag att caa aag cat gtt acc ttc aac caa gtg aaa ggc att ttc ggc   528
   Lys Ile Gln Lys His Val Thr Phe Asn Gln Val Lys Gly Ile Phe Gly
                   165                 170                 175
   ttc act gac agc gac tgc att ggg aag atc agt ttt cct gcc atc cag      576
   Phe Thr Asp Ser Asp Cys Ile Gly Lys Ile Ser Phe Pro Ala Ile Gln
               180                 185                 190
   gct gct ccc tcc ttc agc aac tca ttc cca cag atc ttc cga gac agg      624
   Ala Ala Pro Ser Phe Ser Asn Ser Phe Pro Gln Ile Phe Arg Asp Arg
           195                 200                 205
   acg gat atc cag tgc ctt atc cca tgt gcc att gac cag gat cct tac      672
   Thr Asp Ile Gln Cys Leu Ile Pro Cys Ala Ile Asp Gln Asp Pro Tyr
       210                 215                 220
   ttt aga atg aca agg gac gtc gcc ccc agg atc ggc tat cct aaa cca      720
   Phe Arg Met Thr Arg Asp Val Ala Pro Arg Ile Gly Tyr Pro Lys Pro
   225                 230                 235                 240
   gcc ctg ttg cac tcc acc ttc ttc cca gcc ctg cag ggc gcc cag acc      768
   Ala Leu Leu His Ser Thr Phe Phe Pro Ala Leu Gln Gly Ala Gln Thr
                   245                 250                 255
   aaa atg agt gcc agc gac cca aac tcc tcc atc ttc ctc acc gac acg      816
   Lys Met Ser Ala Ser Asp Pro Asn Ser Ser Ile Phe Leu Thr Asp Thr
               260                 265                 270
   gcc aag cag atc aaa acc aag gtc aat aag cat gcg ttt tct gga ggg      864
   Ala Lys Gln Ile Lys Thr Lys Val Asn Lys His Ala Phe Ser Gly Gly
           275                 280                 285
   aga gac acc atc gag gag cac agg cag ttt ggg ggc aac tgt gat gtg      912
   Arg Asp Thr Ile Glu Glu His Arg Gln Phe Gly Gly Asn Cys Asp Val
       290                 295                 300
   gac gtg tct ttc atg tac ctg acc ttc ttc ctc gag gac gac gac aag      960
   Asp Val Ser Phe Met Tyr Leu Thr Phe Phe Leu Glu Asp Asp Asp Lys
   305                 310                 315                 320
   ctc gag cag atc agg aag gat tac acc agc gga gcc atg ctc acc ggt      1008
   Leu Glu Gln Ile Arg Lys Asp Tyr Thr Ser Gly Ala Met Leu Thr Gly
                   325                 330                 335
   gag ctc aag aag gca ctc ata gag gtt ctg cag ccc ttg atc gca gag      1056
   Glu Leu Lys Lys Ala Leu Ile Glu Val Leu Gln Pro Leu Ile Ala Glu
               340                 345                 350
   cac cag gcc cgg cgc aag gag gtc acg gat gag ata gtg aaa gag ttc      1104
   His Gln Ala Arg Arg Lys Glu Val Thr Asp Glu Ile Val Lys Glu Phe
           355                 360                 365
   atg act ccc cgg aag ctg tcc ttc gac ttt cag                          1137
   Met Thr Pro Arg Lys Leu Ser Phe Asp Phe Gln
       370                 375
<210> 32
   <211> 1203
   <212> DNA
   <213> Artificial Sequence
<220>
   <223> Recombinant human T1 (SY variant) construct
<220>
   <221> CDS
   <222> (1)..(1203)
<220>
   <221> misc_feature
   <222> (427)..(429)
   <223> nnn is agy, tcy or tcr
<400> 32
   agt aat cat ggc cca gat gcc aca gaa gct gaa gag gat ttt gtg gac 48
   Ser Asn His Gly Pro Asp Ala Thr Glu Ala Glu Glu Asp Phe Val Asp
   1 5 10 15
   cca tgg aca gta cag aca agc agt gca aaa ggc ata gac tac gat aag 96
   Pro Trp Thr Val Gln Thr Ser Ser Ala Lys Gly Ile Asp Tyr Asp Lys
   20 25 30
   ctc att gtt cgg ttt gga agt agt aaa att gac aaa gag cta ata aac 144
   Leu Ile Val Arg Phe Gly Ser Ser Lys Ile Asp Lys Glu Leu Ile Asn
   35 40 45
   cga ata gag aga gcc acc ggc caa aga cca cac cac ttc ctg cgc aga 192
   Arg Ile Glu Arg Ala Thr Gly Gln Arg Pro His His Phe Leu Arg Arg
   50 55 60
   ggc atc ttc ttc tca cac aga gat atg aat cag gtt ctt gat gcc tat 240
   Gly Ile Phe Phe Ser His Arg Asp Met Asn Gln Val Leu Asp Ala Tyr
   65 70 75 80
   gaa aat aag aag cca ttt tat ctg tac acg ggc cgg ggc ccc tct tct 288
   Glu Asn Lys Lys Pro Phe Tyr Leu Tyr Thr Gly Arg Gly Pro Ser Ser
   85 90 95
   gaa gca atg cat gta ggt cac ctc att cca ttt att ttc aca aag tgg 336
   Glu Ala Met His Val Gly His Leu Ile Pro Phe Ile Phe Thr Lys Trp
   100 105 110
   ctc cag gat gta ttt aac gtg ccc ttg gtc atc cag atg acg gat gac 384
   Leu Gln Asp Val Phe Asn Val Pro Leu Val Ile Gln Met Thr Asp Asp
   115 120 125
   gag aag tat ctg tgg aag gac ctg acc ctg gac cag gcc tat nnn tay 432
   Glu Lys Tyr Leu Trp Lys Asp Leu Thr Leu Asp Gln Ala Tyr Ser Tyr
   130 135 140
   gct gtt gag aat gcc aag gac atc atc gcc tgt ggc ttt gac atc aac 480
   Ala Val Glu Asn Ala Lys Asp Ile Ile Ala Cys Gly Phe Asp Ile Asn
   145 150 155 160
   aag act ttc ata ttc tct gac ctg gac tac atg ggg atg agc tca ggt 528
   Lys Thr Phe Ile Phe Ser Asp Leu Asp Tyr Met Gly Met Ser Ser Gly
   165 170 175
   ttc tac aaa aat gtg gtg aag att caa aag cat gtt acc ttc aac caa 576
   Phe Tyr Lys Asn Val Val Lys Ile Gln Lys His Val Thr Phe Asn Gln
   180 185 190
   gtg aaa ggc att ttc ggc ttc act gac agc gac tgc att ggg aag atc 624
   Val Lys Gly Ile Phe Gly Phe Thr Asp Ser Asp Cys Ile Gly Lys Ile
   195 200 205
   agt ttt cct gcc atc cag gct gct ccc tcc ttc agc aac tca ttc cca 672
   Ser Phe Pro Ala Ile Gln Ala Ala Pro Ser Phe Ser Asn Ser Phe Pro
   210 215 220
   cag atc ttc cga gac agg acg gat atc cag tgc ctt atc cca tgt gcc 720
   Gln Ile Phe Arg Asp Arg Thr Asp Ile Gln Cys Leu Ile Pro Cys Ala
   225 230 235 240
   att gac cag gat cct tac ttt aga atg aca agg gac gtc gcc ccc agg      768
   Ile Asp Gln Asp Pro Tyr Phe Arg Met Thr Arg Asp Val Ala Pro Arg
                   245                     250                 255
   atc ggc tat cct aaa cca gcc ctg ttg cac tcc acc ttc ttc cca gcc      816
   Ile Gly Tyr Pro Lys Pro Ala Leu Leu His Ser Thr Phe Phe Pro Ala
               260                 265                 270
   ctg cag ggc gcc cag acc aaa atg agt gcc agc gac cca aac tcc tcc      864
   Leu Gln Gly Ala Gln Thr Lys Met Ser Ala Ser Asp Pro Asn Ser Ser
           275                 280                 285
   atc ttc ctc acc gac acg gcc aag cag atc aaa acc aag gtc aat aag      912
   Ile Phe Leu Thr Asp Thr Ala Lys Gln Ile Lys Thr Lys Val Asn Lys
       290                 295                 300
   cat gcg ttt tct gga ggg aga gac acc atc gag gag cac agg cag ttt      960
   His Ala Phe Ser Gly Gly Arg Asp Thr Ile Glu Glu His Arg Gln Phe
   305                 310                 315                 320
   ggg ggc aac tgt gat gtg gac gtg tct ttc atg tac ctg acc ttc ttc     1008
   Gly Gly Asn Cys Asp Val Asp Val Ser Phe Met Tyr Leu Thr Phe Phe
                   325                 330                 335
   ctc gag gac gac gac aag ctc gag cag atc agg aag gat tac acc agc     1056
   Leu Glu Asp Asp Asp Lys Leu Glu Gln Ile Arg Lys Asp Tyr Thr Ser
               340                 345                 350
   gga gcc atg ctc acc ggt gag ctc aag aag gca ctc ata gag gtt ctg     1104
   Gly Ala Met Leu Thr Gly Glu Leu Lys Lys Ala Leu Ile Glu Val Leu
           355                 360                 365
   cag ccc ttg atc gca gag cac cag gcc cgg cgc aag gag gtc acg gat     1152
   Gln Pro Leu Ile Ala Glu His Gln Ala Arg Arg Lys Glu Val Thr Asp
       370                 375                 380
   gag ata gtg aaa gag ttc atg act ccc cgg aag ctg tcc ttc gac ttt     1200
   Glu Ile Val Lys Glu Phe Met Thr Pro Arg Lys Leu Ser Phe Asp Phe
   385                 390                 395                 400
   cag                                                                 1203
   Gln
<210> 33
   <211> 1206
   <212> DNA
   <213> Artificial Sequence
<220>
   <223> Recombinant human Met-T1 (SY variant) construct
<220>
   <221> CDS
   <222> (1)..(1206)
<220>
   <221> misc_feature
   <222> (430)..(432)
   <223> nnn is agy, tcy or tcr
<400> 33
   atg agt aat cat ggc cca gat gcc aca gaa gct gaa gag gat ttt gtg      48
   Met Ser Asn His Gly Pro Asp Ala Thr Glu Ala Glu Glu Asp Phe Val
   1               5                   10                  15
   gac cca tgg aca gta cag aca agc agt gca aaa ggc ata gac tac gat      96
   Asp Pro Trp Thr Val Gln Thr Ser Ser Ala Lys Gly Ile Asp Tyr Asp
               20                  25                  30
   aag ctc att gtt cgg ttt gga agt agt aaa att gac aaa gag cta ata     144
   Lys Leu Ile Val Arg Phe Gly Ser Ser Lys Ile Asp Lys Glu Leu Ile
           35                  40                  45
   aac cga ata gag aga gcc acc ggc caa aga cca cac cac ttc ctg cgc     192
   Asn Arg Ile Glu Arg Ala Thr Gly Gln Arg Pro His His Phe Leu Arg
       50                  55                  60
   aga ggc atc ttc ttc tca cac aga gat atg aat cag gtt ctt gat gcc     240
   Arg Gly Ile Phe Phe Ser His Arg Asp Met Asn Gln Val Leu Asp Ala
   65                  70                  75                  80
   tat gaa aat aag aag cca ttt tat ctg tac acg ggc cgg ggc ccc tct     288
   Tyr Glu Asn Lys Lys Pro Phe Tyr Leu Tyr Thr Gly Arg Gly Pro Ser
                   85                  90                  95
   tct gaa gca atg cat gta ggt cac ctc att cca ttt att ttc aca aag     336
   Ser Glu Ala Met His Val Gly His Leu Ile Pro Phe Ile Phe Thr Lys
               100                 105                 110
   tgg ctc cag gat gta ttt aac gtg ccc ttg gtc atc cag atg acg gat     384
   Trp Leu Gln Asp Val Phe Asn Val Pro Leu Val Ile Gln Met Thr Asp
           115                 120                 125
   gac gag aag tat ctg tgg aag gac ctg acc ctg gac cag gcc tat nnn     432
   Asp Glu Lys Tyr Leu Trp Lys Asp Leu Thr Leu Asp Gln Ala Tyr Ser
       130                 135                 140
   tay gct gtt gag aat gcc aag gac atc atc gcc tgt ggc ttt gac atc     480
   Tyr Ala Val Glu Asn Ala Lys Asp Ile Ile Ala Cys Gly Phe Asp Ile
   145                 150                 155                 160
   aac aag act ttc ata ttc tct gac ctg gac tac atg ggg atg agc tca     528
   Asn Lys Thr Phe Ile Phe Ser Asp Leu Asp Tyr Met Gly Met Ser Ser
                   165                 170                 175
   ggt ttc tac aaa aat gtg gtg aag att caa aag cat gtt acc ttc aac     576
   Gly Phe Tyr Lys Asn Val Val Lys Ile Gln Lys His Val Thr Phe Asn
               180                 185                 190
   caa gtg aaa ggc att ttc ggc ttc act gac agc gac tgc att ggg aag     624
   Gln Val Lys Gly Ile Phe Gly Phe Thr Asp Ser Asp Cys Ile Gly Lys
           195                 200                 205
   atc agt ttt cct gcc atc cag gct gct ccc tcc ttc agc aac tca ttc     672
   Ile Ser Phe Pro Ala Ile Gln Ala Ala Pro Ser Phe Ser Asn Ser Phe
       210                 215                 220
   cca cag atc ttc cga gac agg acg gat atc cag tgc ctt atc cca tgt     720
   Pro Gln Ile Phe Arg Asp Arg Thr Asp Ile Gln Cys Leu Ile Pro Cys
   225                 230                 235                 240
   gcc att gac cag gat cct tac ttt aga atg aca agg gac gtc gcc ccc     768
   Ala Ile Asp Gln Asp Pro Tyr Phe Arg Met Thr Arg Asp Val Ala Pro
                   245                 250                 255
   agg atc ggc tat cct aaa cca gcc ctg ttg cac tcc acc ttc ttc cca     816
   Arg Ile Gly Tyr Pro Lys Pro Ala Leu Leu His Ser Thr Phe Phe Pro
               260                 265                 270
   gcc ctg cag ggc gcc cag acc aaa atg agt gcc agc gac cca aac tcc     864
   Ala Leu Gln Gly Ala Gln Thr Lys Met Ser Ala Ser Asp Pro Asn Ser
           275                     280                     285
   tcc atc ttc ctc acc gac acg gcc aag cag atc aaa acc aag gtc aat      912
   Ser Ile Phe Leu Thr Asp Thr Ala Lys Gln Ile Lys Thr Lys Val Asn
       290                     295                     300
   aag cat gcg ttt tct gga ggg aga gac acc atc gag gag cac agg cag      960
   Lys His Ala Phe Ser Gly Gly Arg Asp Thr Ile Glu Glu His Arg Gln
   305                     310                     315                 320
   ttt ggg ggc aac tgt gat gtg gac gtg tct ttc atg tac ctg acc ttc     1008
   Phe Gly Gly Asn Cys Asp Val Asp Val Ser Phe Met Tyr Leu Thr Phe
                   325                     330                     335
   ttc ctc gag gac gac gac aag ctc gag cag atc agg aag gat tac acc     1056
   Phe Leu Glu Asp Asp Asp Lys Leu Glu Gln Ile Arg Lys Asp Tyr Thr
               340                     345                     350
   agc gga gcc atg ctc acc ggt gag ctc aag aag gca ctc ata gag gtt     1104
   Ser Gly Ala Met Leu Thr Gly Glu Leu Lys Lys Ala Leu Ile Glu Val
           355                     360                     365
   ctg cag ccc ttg atc gca gag cac cag gcc cgg cgc aag gag gtc acg     1152
   Leu Gln Pro Leu Ile Ala Glu His Gln Ala Arg Arg Lys Glu Val Thr
       370                     375                     380
   gat gag ata gtg aaa gag ttc atg act ccc cgg aag ctg tcc ttc gac     1200
   Asp Glu Ile Val Lys Glu Phe Met Thr Pro Arg Lys Leu Ser Phe Asp
   385                     390                     395                 400
   ttt cag                                                             1206
   Phe Gln
<210> 34
   <211> 1269
   <212> DNA
   <213> Artificial Sequence
<220>
   <223> Recombinant human mini-TrpRS (SY variant) construct
<220>
   <221> CDS
   <222> (1)..(1269)
<220>
   <221> misc_feature
   <222> (493)..(495)
   <223> nnn is agy, tcy or tcr
<400> 34
   agc tac aaa gct gcc gcg ggg gag gat tac aag gct gac tgt cct cca      48
   Ser Tyr Lys Ala Ala Ala Gly Glu Asp Tyr Lys Ala Asp Cys Pro Pro
     1               5                  10                  15
   ggg aac cca gca cct acc agt aat cat ggc cca gat gcc aca gaa gct      96
   Gly Asn Pro Ala Pro Thr Ser Asn His Gly Pro Asp Ala Thr Glu Ala
               20                  25                  30
   gaa gag gat ttt gtg gac cca tgg aca gta cag aca agc agt gca aaa     144
   Glu Glu Asp Phe Val Asp Pro Trp Thr Val Gln Thr Ser Ser Ala Lys
           35                  40                  45
   ggc ata gac tac gat aag ctc att gtt cgg ttt gga agt agt aaa att     192
   Gly Ile Asp Tyr Asp Lys Leu Ile Val Arg Phe Gly Ser Ser Lys Ile
   50 55 60
   gac aaa gag cta ata aac cga ata gag aga gcc acc ggc caa aga cca 240
   Asp Lys Glu Leu Ile Asn Arg Ile Glu Arg Ala Thr Gly Gln Arg Pro
   65 70 75 80
   cac cac ttc ctg cgc aga ggc atc ttc ttc tca cac aga gat atg aat 288
   His His Phe Leu Arg Arg Gly Ile Phe Phe Ser His Arg Asp Met Asn
   85 90 95
   cag gtt ctt gat gcc tat gaa aat aag aag cca ttt tat ctg tac acg 336
   Gln Val Leu Asp Ala Tyr Glu Asn Lys Lys Pro Phe Tyr Leu Tyr Thr
   100 105 110
   ggc cgg ggc ccc tct tct gaa gca atg cat gta ggt cac ctc att cca 384
   Gly Arg Gly Pro Ser Ser Glu Ala Met His Val Gly His Leu Ile Pro
   115 120 125
   ttt att ttc aca aag tgg ctc cag gat gta ttt aac gtg ccc ttg gtc 432
   Phe Ile Phe Thr Lys Trp Leu Gln Asp Val Phe Asn Val Pro Leu Val
   130 135 140
   atc cag atg acg gat gac gag aag tat ctg tgg aag gac ctg acc ctg 480
   Ile Gln Met Thr Asp Asp Glu Lys Tyr Leu Trp Lys Asp Leu Thr Leu
   145 150 155 160
   gac cag gcc tat nnn tay gct gtt gag aat gcc aag gac atc atc gcc 528
   Asp Gln Ala Tyr Ser Tyr Ala Val Glu Asn Ala Lys Asp Ile Ile Ala
   165 170 175
   tgt ggc ttt gac atc aac aag act ttc ata ttc tct gac ctg gac tac 576
   Cys Gly Phe Asp Ile Asn Lys Thr Phe Ile Phe Ser Asp Leu Asp Tyr
   180 185 190
   atg ggg atg agc tca ggt ttc tac aaa aat gtg gtg aag att caa aag 624
   Met Gly Met Ser Ser Gly Phe Tyr Lys Asn Val Val Lys Ile Gln Lys
   195 200 205
   cat gtt acc ttc aac caa gtg aaa ggc att ttc ggc ttc act gac agc 672
   His Val Thr Phe Asn Gln Val Lys Gly Ile Phe Gly Phe Thr Asp Ser
   210 215 220
   gac tgc att ggg aag atc agt ttt cct gcc atc cag gct gct ccc tcc 720
   Asp Cys Ile Gly Lys Ile Ser Phe Pro Ala Ile Gln Ala Ala Pro Ser
   225 230 235 240
   ttc agc aac tca ttc cca cag atc ttc cga gac agg acg gat atc cag 768
   Phe Ser Asn Ser Phe Pro Gln Ile Phe Arg Asp Arg Thr Asp Ile Gln
   245 250 255
   tgc ctt atc cca tgt gcc att gac cag gat cct tac ttt aga atg aca 816
   Cys Leu Ile Pro Cys Ala Ile Asp Gln Asp Pro Tyr Phe Arg Met Thr
   260 265 270
   agg gac gtc gcc ccc agg atc ggc tat cct aaa cca gcc ctg ttg cac 864
   Arg Asp Val Ala Pro Arg Ile Gly Tyr Pro Lys Pro Ala Leu Leu His
   275 280 285
   tcc acc ttc ttc cca gcc ctg cag ggc gcc cag acc aaa atg agt gcc 912
   Ser Thr Phe Phe Pro Ala Leu Gln Gly Ala Gln Thr Lys Met Ser Ala
   290 295 300
   agc gac cca aac tcc tcc atc ttc ctc acc gac acg gcc aag cag atc 960
   Ser Asp Pro Asn Ser Ser Ile Phe Leu Thr Asp Thr Ala Lys Gln Ile
   305 310 315 320
   aaa acc aag gtc aat aag cat gcg ttt tct gga ggg aga gac acc atc 1008
   Lys Thr Lys Val Asn Lys His Ala Phe Ser Gly Gly Arg Asp Thr Ile
                   325                 330                 335
   gag gag cac agg cag ttt ggg ggc aac tgt gat gtg gac gtg tct ttc      1056
   Glu Glu His Arg Gln Phe Gly Gly Asn Cys Asp Val Asp Val Ser Phe
               340                 345                 350
   atg tac ctg acc ttc ttc ctc gag gac gac gac aag ctc gag cag atc      1104
   Met Tyr Leu Thr Phe Phe Leu Glu Asp Asp Asp Lys Leu Glu Gln Ile
           355                 360                 365
   agg aag gat tac acc agc gga gcc atg ctc acc ggt gag ctc aag aag      1152
   Arg Lys Asp Tyr Thr Ser Gly Ala Met Leu Thr Gly Glu Leu Lys Lys
       370                 375                 380
   gca ctc ata gag gtt ctg cag ccc ttg atc gca gag cac cag gcc cgg      1200
   Ala Leu Ile Glu Val Leu Gln Pro Leu Ile Ala Glu His Gln Ala Arg
   385                 390                 395                 400
   cgc aag gag gtc acg gat gag ata gtg aaa gag ttc atg act ccc cgg      1248
   Arg Lys Glu Val Thr Asp Glu Ile Val Lys Glu Phe Met Thr Pro Arg
                   405                 410                 415
   aag ctg tcc ttc gac ttt cag                                          1269
   Lys Leu Ser Phe Asp Phe Gln
               420
<210> 35
   <211> 1272
   <212> DNA
   <213> Artificial Sequence
<220>
   <223> Recombinant human Met-mini-TrpRS (SY variant) construct
<220>
   <221> CDS
   <222> (1)..(1272)
<220>
   <221> misc_feature
   <222> (496)..(498)
   <223> nnn is agy, tcy or tcr
<400> 35
   atg agc tac aaa gct gcc gcg ggg gag gat tac aag gct gac tgt cct      48
   cca ggg aac cca gca cct acc agt aat cat ggc cca gat gcc aca gaa      96
   gct gaa gag gat ttt gtg gac cca tgg aca gta cag aca agc agt gca      144
   aaa ggc ata gac tac gat aag ctc att gtt cgg ttt gga agt agt aaa      192
   att gac aaa gag cta ata aac cga ata gag aga gcc acc ggc caa aga      240
   cca cac cac ttc ctg cgc aga ggc atc ttc ttc tca cac aga gat atg 288
   aat cag gtt ctt gat gcc tat gaa aat aag aag cca ttt tat ctg tac 336
   acg ggc cgg ggc ccc tct tct gaa gca atg cat gta ggt cac ctc att 384
   cca ttt att ttc aca aag tgg ctc cag gat gta ttt aac gtg ccc ttg 432
   gtc atc cag atg acg gat gac gag aag tat ctg tgg aag gac ctg acc 480
   ctg gac cag gcc tat nnn tay gct gtt gag aat gcc aag gac atc atc 528
   gcc tgt ggc ttt gac atc aac aag act ttc ata ttc tct gac ctg gac 576
   tac atg ggg atg agc tca ggt ttc tac aaa aat gtg gtg aag att caa 624
   aag cat gtt acc ttc aac caa gtg aaa ggc att ttc ggc ttc act gac 672
   agc gac tgc att ggg aag atc agt ttt cct gcc atc cag gct gct ccc 720
   tcc ttc agc aac tca ttc cca cag atc ttc cga gac agg acg gat atc 768
   cag tgc ctt atc cca tgt gcc att gac cag gat cct tac ttt aga atg 816
   aca agg gac gtc gcc ccc agg atc ggc tat cct aaa cca gcc ctg ttg 864
   cac tcc acc ttc ttc cca gcc ctg cag ggc gcc cag acc aaa atg agt 912
   gcc agc gac cca aac tcc tcc atc ttc ctc acc gac acg gcc aag cag 960
   atc aaa acc aag gtc aat aag cat gcg ttt tct gga ggg aga gac acc 1008
   atc gag gag cac agg cag ttt ggg ggc aac tgt gat gtg gac gtg tct 1056
   ttc atg tac ctg acc ttc ttc ctc gag gac gac gac aag ctc gag cag      1104
   atc agg aag gat tac acc agc gga gcc atg ctc acc ggt gag ctc aag      1152
   aag gca ctc ata gag gtt ctg cag ccc ttg atc gca gag cac cag gcc      1200
   cgg cgc aag gag gtc acg gat gag ata gtg aaa gag ttc atg act ccc      1248
   cgg aag ctg tcc ttc gac ttt cag                                      1272
<210> 36
   <211> 378
   <212> PRT
   <213> Artificial Sequence
<220>
   <223> Recombinant human T2 (GY variant) construct
<400> 36
   Ser Ala Lys Gly Ile Asp Tyr Asp Lys Leu Ile Val Arg Phe Gly Ser
   1               5                   10                  15
   Ser Lys Ile Asp Lys Glu Leu Ile Asn Arg Ile Glu Arg Ala Thr Gly
               20                  25                  30
   Gln Arg Pro His His Phe Leu Arg Arg Gly Ile Phe Phe Ser His Arg
           35                  40                  45
   Asp Met Asn Gln Val Leu Asp Ala Tyr Glu Asn Lys Lys Pro Phe Tyr
       50                  55                  60
   Leu Tyr Thr Gly Arg Gly Pro Ser Ser Glu Ala Met His Val Gly His
   65                  70                  75                  80
   Leu Ile Pro Phe Ile Phe Thr Lys Trp Leu Gln Asp Val Phe Asn Val
                   85                  90                  95
   Pro Leu Val Ile Gln Met Thr Asp Asp Glu Lys Tyr Leu Trp Lys Asp
               100                 105                 110
   Leu Thr Leu Asp Gln Ala Tyr Gly Tyr Ala Val Glu Asn Ala Lys Asp
           115                 120                 125
   Ile Ile Ala Cys Gly Phe Asp Ile Asn Lys Thr Phe Ile Phe Ser Asp
       130                 135                 140
   Leu Asp Tyr Met Gly Met Ser Ser Gly Phe Tyr Lys Asn Val Val Lys
   145                 150                 155                 160
   Ile Gln Lys His Val Thr Phe Asn Gln Val Lys Gly Ile Phe Gly Phe
                   165                 170                 175
   Thr Asp Ser Asp Cys Ile Gly Lys Ile Ser Phe Pro Ala Ile Gln Ala
   180 185 190
   Ala Pro Ser Phe Ser Asn Ser Phe Pro Gln Ile Phe Arg Asp Arg Thr
   195 200 205
   Asp Ile Gln Cys Leu Ile Pro Cys Ala Ile Asp Gln Asp Pro Tyr Phe
   210 215 220
   Arg Met Thr Arg Asp Val Ala Pro Arg Ile Gly Tyr Pro Lys Pro Ala
   225 230 235 240
   Leu Leu His Ser Thr Phe Phe Pro Ala Leu Gln Gly Ala Gln Thr Lys
   245 250 255
   Met Ser Ala Ser Asp Pro Asn Ser Ser Ile Phe Leu Thr Asp Thr Ala
   260 265 270
   Lys Gln Ile Lys Thr Lys Val Asn Lys His Ala Phe Ser Gly Gly Arg
   275 280 285
   Asp Thr Ile Glu Glu His Arg Gln Phe Gly Gly Asn Cys Asp Val Asp
   290 295 300
   Val Ser Phe Met Tyr Leu Thr Phe Phe Leu Glu Asp Asp Asp Lys Leu
   305 310 315 320
   Glu Gln Ile Arg Lys Asp Tyr Thr Ser Gly Ala Met Leu Thr Gly Glu
   325 330 335
   Leu Lys Lys Ala Leu Ile Glu Val Leu Gln Pro Leu Ile Ala Glu His
   340 345 350
   Gln Ala Arg Arg Lys Glu Val Thr Asp Glu Ile Val Lys Glu Phe Met
   355 360 365
   Thr Pro Arg Lys Leu Ser Phe Asp Phe Gln
   370 375
<210> 37
   <211> 401
   <212> PRT
   <213> Artificial Sequence
<220>
   <223> Recombinant human T1 (GY variant) construct
<400> 37
   Ser Asn His Gly Pro Asp Ala Thr Glu Ala Glu Glu Asp Phe Val Asp
   1 5 10 15
   Pro Trp Thr Val Gln Thr Ser Ser Ala Lys Gly Ile Asp Tyr Asp Lys
   20 25 30
   Leu Ile Val Arg Phe Gly Ser Ser Lys Ile Asp Lys Glu Leu Ile Asn
   35 40 45
   Arg Ile Glu Arg Ala Thr Gly Gln Arg Pro His His Phe Leu Arg Arg
   50 55 60
   Gly Ile Phe Phe Ser His Arg Asp Met Asn Gln Val Leu Asp Ala Tyr
   65 70 75 80
   Glu Asn Lys Lys Pro Phe Tyr Leu Tyr Thr Gly Arg Gly Pro Ser Ser
   85 90 95
   Glu Ala Met His Val Gly His Leu Ile Pro Phe Ile Phe Thr Lys Trp
   100 105 110
   Leu Gln Asp Val Phe Asn Val Pro Leu Val Ile Gln Met Thr Asp Asp
   115 120 125
   Glu Lys Tyr Leu Trp Lys Asp Leu Thr Leu Asp Gln Ala Tyr Gly Tyr
   130 135 140
   Ala Val Glu Asn Ala Lys Asp Ile Ile Ala Cys Gly Phe Asp Ile Asn
   145 150 155 160
   Lys Thr Phe Ile Phe Ser Asp Leu Asp Tyr Met Gly Met Ser Ser Gly
   165 170 175
   Phe Tyr Lys Asn Val Val Lys Ile Gln Lys His Val Thr Phe Asn Gln
   180 185 190
   Val Lys Gly Ile Phe Gly Phe Thr Asp Ser Asp Cys Ile Gly Lys Ile
   195 200 205
   Ser Phe Pro Ala Ile Gln Ala Ala Pro Ser Phe Ser Asn Ser Phe Pro
   210 215 220
   Gln Ile Phe Arg Asp Arg Thr Asp Ile Gln Cys Leu Ile Pro Cys Ala
   225 230 235 240
   Ile Asp Gln Asp Pro Tyr Phe Arg Met Thr Arg Asp Val Ala Pro Arg
   245 250 255
   Ile Gly Tyr Pro Lys Pro Ala Leu Leu His Ser Thr Phe Phe Pro Ala
   260 265 270
   Leu Gln Gly Ala Gln Thr Lys Met Ser Ala Ser Asp Pro Asn Ser Ser
   275 280 285
   Ile Phe Leu Thr Asp Thr Ala Lys Gln Ile Lys Thr Lys Val Asn Lys
   290 295 300
   His Ala Phe Ser Gly Gly Arg Asp Thr Ile Glu Glu His Arg Gln Phe
   305 310 315 320
   Gly Gly Asn Cys Asp Val Asp Val Ser Phe Met Tyr Leu Thr Phe Phe
   325 330 335
   Leu Glu Asp Asp Asp Lys Leu Glu Gln Ile Arg Lys Asp Tyr Thr Ser
   340 345 350
   Gly Ala Met Leu Thr Gly Glu Leu Lys Lys Ala Leu Ile Glu Val Leu
   355 360 365
   Gln Pro Leu Ile Ala Glu His Gln Ala Arg Arg Lys Glu Val Thr Asp
   370 375 380
   Glu Ile Val Lys Glu Phe Met Thr Pro Arg Lys Leu Ser Phe Asp Phe
   385 390 395 400
   Gln
<210> 38
   <211> 423
   <212> PRT
   <213> Artificial Sequence
<220>
   <223> Recombinant human mini-TrpRS (GY variant) construct
<400> 38
   Ser Tyr Lys Ala Ala Ala Gly Glu Asp Tyr Lys Ala Asp Cys Pro Pro
   1               5                   10                  15
   Gly Asn Pro Ala Pro Thr Ser Asn His Gly Pro Asp Ala Thr Glu Ala
               20                  25                  30
   Glu Glu Asp Phe Val Asp Pro Trp Thr Val Gln Thr Ser Ser Ala Lys
           35                  40                  45
   Gly Ile Asp Tyr Asp Lys Leu Ile Val Arg Phe Gly Ser Ser Lys Ile
       50                  55                  60
   Asp Lys Glu Leu Ile Asn Arg Ile Glu Arg Ala Thr Gly Gln Arg Pro
   65                  70                  75                  80
   His His Phe Leu Arg Arg Gly Ile Phe Phe Ser His Arg Asp Met Asn
                   85                  90                  95
   Gln Val Leu Asp Ala Tyr Glu Asn Lys Lys Pro Phe Tyr Leu Tyr Thr
               100                 105                 110
   Gly Arg Gly Pro Ser Ser Glu Ala Met His Val Gly His Leu Ile Pro
           115                 120                 125
   Phe Ile Phe Thr Lys Trp Leu Gln Asp Val Phe Asn Val Pro Leu Val
       130                 135                 140
   Ile Gln Met Thr Asp Asp Glu Lys Tyr Leu Trp Lys Asp Leu Thr Leu
   145                 150                 155                 160
   Asp Gln Ala Tyr Gly Tyr Ala Val Glu Asn Ala Lys Asp Ile Ile Ala
                   165                 170                 175
   Cys Gly Phe Asp Ile Asn Lys Thr Phe Ile Phe Ser Asp Leu Asp Tyr
               180                 185                 190
   Met Gly Met Ser Ser Gly Phe Tyr Lys Asn Val Val Lys Ile Gln Lys
           195                 200                 205
   His Val Thr Phe Asn Gln Val Lys Gly Ile Phe Gly Phe Thr Asp Ser
       210                 215                 220
   Asp Cys Ile Gly Lys Ile Ser Phe Pro Ala Ile Gln Ala Ala Pro Ser
   225                 230                 235                 240
   Phe Ser Asn Ser Phe Pro Gln Ile Phe Arg Asp Arg Thr Asp Ile Gln
                   245                 250                 255
   Cys Leu Ile Pro Cys Ala Ile Asp Gln Asp Pro Tyr Phe Arg Met Thr
               260                 265                 270
   Arg Asp Val Ala Pro Arg Ile Gly Tyr Pro Lys Pro Ala Leu Leu His
           275                 280                 285
   Ser Thr Phe Phe Pro Ala Leu Gln Gly Ala Gln Thr Lys Met Ser Ala
       290                 295                 300
   Ser Asp Pro Asn Ser Ser Ile Phe Leu Thr Asp Thr Ala Lys Gln Ile
   305                 310                 315                 320
   Lys Thr Lys Val Asn Lys His Ala Phe Ser Gly Gly Arg Asp Thr Ile
                   325                 330                 335
   Glu Glu His Arg Gln Phe Gly Gly Asn Cys Asp Val Asp Val Ser Phe
               340                 345                 350
   Met Tyr Leu Thr Phe Phe Leu Glu Asp Asp Asp Lys Leu Glu Gln Ile
           355                 360                 365
   Arg Lys Asp Tyr Thr Ser Gly Ala Met Leu Thr Gly Glu Leu Lys Lys
       370                 375                 380
   Ala Leu Ile Glu Val Leu Gln Pro Leu Ile Ala Glu His Gln Ala Arg
   385                 390                 395                 400
   Arg Lys Glu Val Thr Asp Glu Ile Val Lys Glu Phe Met Thr Pro Arg
                   405                 410                 415
   Lys Leu Ser Phe Asp Phe Gln
               420
<210> 39
   <211> 379
   <212> PRT
   <213> Artificial sequence
<220>
   <223> Recombinant human Met-T2 (GY variant) construct
<400> 39
   Met Ser Ala Lys Gly Ile Asp Tyr Asp Lys Leu Ile Val Arg Phe Gly
   1               5                   10                  15
   Ser Ser Lys Ile Asp Lys Glu Leu Ile Asn Arg Ile Glu Arg Ala Thr
               20                  25                  30
   Gly Gln Arg Pro His His Phe Leu Arg Arg Gly Ile Phe Phe Ser His
           35                  40                  45
   Arg Asp Met Asn Gln Val Leu Asp Ala Tyr Glu Asn Lys Lys Pro Phe
       50                  55                  60
   Tyr Leu Tyr Thr Gly Arg Gly Pro Ser Ser Glu Ala Met His Val Gly
   65                  70                  75                  80
   His Leu Ile Pro Phe Ile Phe Thr Lys Trp Leu Gln Asp Val Phe Asn
                   85                  90                  95
   Val Pro Leu Val Ile Gln Met Thr Asp Asp Glu Lys Tyr Leu Trp Lys
               100                 105                 110
   Asp Leu Thr Leu Asp Gln Ala Tyr Gly Tyr Ala Val Glu Asn Ala Lys
           115                 120                 125
   Asp Ile Ile Ala Cys Gly Phe Asp Ile Asn Lys Thr Phe Ile Phe Ser
       130                 135                 140
   Asp Leu Asp Tyr Met Gly Met Ser Ser Gly Phe Tyr Lys Asn Val Val
   145                 150                 155                 160
   Lys Ile Gln Lys His Val Thr Phe Asn Gln Val Lys Gly Ile Phe Gly
                   165                 170                 175
   Phe Thr Asp Ser Asp Cys Ile Gly Lys Ile Ser Phe Pro Ala Ile Gln
               180                 185                 190
   Ala Ala Pro Ser Phe Ser Asn Ser Phe Pro Gln Ile Phe Arg Asp Arg
           195                 200                 205
   Thr Asp Ile Gln Cys Leu Ile Pro Cys Ala Ile Asp Gln Asp Pro Tyr
       210                 215                 220
   Phe Arg Met Thr Arg Asp Val Ala Pro Arg Ile Gly Tyr Pro Lys Pro
   225 230 235 240
   Ala Leu Leu His Ser Thr Phe Phe Pro Ala Leu Gln Gly Ala Gln Thr
   245 250 255
   Lys Met Ser Ala Ser Asp Pro Asn Ser Ser Ile Phe Leu Thr Asp Thr
   260 265 270
   Ala Lys Gln Ile Lys Thr Lys Val Asn Lys His Ala Phe Ser Gly Gly
   275 280 285
   Arg Asp Thr Ile Glu Glu His Arg Gln Phe Gly Gly Asn Cys Asp Val
   290 295 300
   Asp Val Ser Phe Met Tyr Leu Thr Phe Phe Leu Glu Asp Asp Asp Lys
   305 310 315 320
   Leu Glu Gln Ile Arg Lys Asp Tyr Thr Ser Gly Ala Met Leu Thr Gly
   325 330 335
   Glu Leu Lys Lys Ala Leu Ile Glu Val Leu Gln Pro Leu Ile Ala Glu
   340 345 350
   His Gln Ala Arg Arg Lys Glu Val Thr Asp Glu Ile Val Lys Glu Phe
   355 360 365
   Met Thr Pro Arg Lys Leu Ser Phe Asp Phe Gln
   370 375
<210> 40
   <211> 402
   <212> PRT
   <213> Artificial sequence
<220>
   <223> Recombinant human Met-T1 (GY variant) construct
<400> 40
   Met Ser Asn His Gly Pro Asp Ala Thr Glu Ala Glu Glu Asp Phe Val
   1               5                   10                  15
   Asp Pro Trp Thr Val Gln Thr Ser Ser Ala Lys Gly Ile Asp Tyr Asp
               20                  25                  30
   Lys Leu Ile Val Arg Phe Gly Ser Ser Lys Ile Asp Lys Glu Leu Ile
           35                  40                  45
   Asn Arg Ile Glu Arg Ala Thr Gly Gln Arg Pro His His Phe Leu Arg
       50                  55                  60
   Arg Gly Ile Phe Phe Ser His Arg Asp Met Asn Gln Val Leu Asp Ala
   65                  70                  75                  80
   Tyr Glu Asn Lys Lys Pro Phe Tyr Leu Tyr Thr Gly Arg Gly Pro Ser
                   85                  90                  95
   Ser Glu Ala Met His Val Gly His Leu Ile Pro Phe Ile Phe Thr Lys
               100                 105                 110
   Trp Leu Gln Asp Val Phe Asn Val Pro Leu Val Ile Gln Met Thr Asp
           115                 120                 125
   Asp Glu Lys Tyr Leu Trp Lys Asp Leu Thr Leu Asp Gln Ala Tyr Gly
       130                 135                 140
   Tyr Ala Val Glu Asn Ala Lys Asp Ile Ile Ala Cys Gly Phe Asp Ile
   145 150 155 160
   Asn Lys Thr Phe Ile Phe Ser Asp Leu Asp Tyr Met Gly Met Ser Ser
   165 170 175
   Gly Phe Tyr Lys Asn Val Val Lys Ile Gln Lys His Val Thr Phe Asn
   180 185 190
   Gln Val Lys Gly Ile Phe Gly Phe Thr Asp Ser Asp Cys Ile Gly Lys
   195 200 205
   Ile Ser Phe Pro Ala Ile Gln Ala Ala Pro Ser Phe Ser Asn Ser Phe
   210 215 220
   Pro Gln Ile Phe Arg Asp Arg Thr Asp Ile Gln Cys Leu Ile Pro Cys
   225 230 235 240
   Ala Ile Asp Gln Asp Pro Tyr Phe Arg Met Thr Arg Asp Val Ala Pro
   245 250 255
   Arg Ile Gly Tyr Pro Lys Pro Ala Leu Leu His Ser Thr Phe Phe Pro
   260 265 270
   Ala Leu Gln Gly Ala Gln Thr Lys Met Ser Ala Ser Asp Pro Asn Ser
   275 280 285
   Ser Ile Phe Leu Thr Asp Thr Ala Lys Gln Ile Lys Thr Lys Val Asn
   290 295 300
   Lys His Ala Phe Ser Gly Gly Arg Asp Thr Ile Glu Glu His Arg Gln
   305 310 315 320
   Phe Gly Gly Asn Cys Asp Val Asp Val Ser Phe Met Tyr Leu Thr Phe
   325 330 335
   Phe Leu Glu Asp Asp Asp Lys Leu Glu Gln Ile Arg Lys Asp Tyr Thr
   340 345 350
   Ser Gly Ala Met Leu Thr Gly Glu Leu Lys Lys Ala Leu Ile Glu Val
   355 360 365
   Leu Gln Pro Leu Ile Ala Glu His Gln Ala Arg Arg Lys Glu Val Thr
   370 375 380
   Asp Glu Ile Val Lys Glu Phe Met Thr Pro Arg Lys Leu Ser Phe Asp
   385 390 395 400
   Phe Gln
<210> 41
   <211> 424
   <212> PRT
   <213> Artificial Sequence
<220>
   <223> Recombinant human Met-mini-TrpRS (GY variant) construct
<400> 41
   Met Ser Tyr Lys Ala Ala Ala Gly Glu Asp Tyr Lys Ala Asp Cys Pro
   1 5 10 15
   Pro Gly Asn Pro Ala Pro Thr Ser Asn His Gly Pro Asp Ala Thr Glu
   20 25 30
   Ala Glu Glu Asp Phe Val Asp Pro Trp Thr Val Gln Thr Ser Ser Ala
   35 40 45
   Lys Gly Ile Asp Tyr Asp Lys Leu Ile Val Arg Phe Gly Ser Ser Lys
   50 55 60
   Ile Asp Lys Glu Leu Ile Asn Arg Ile Glu Arg Ala Thr Gly Gln Arg
   65 70 75 80
   Pro His His Phe Leu Arg Arg Gly Ile Phe Phe Ser His Arg Asp Met
   85 90 95
   Asn Gln Val Leu Asp Ala Tyr Glu Asn Lys Lys Pro Phe Tyr Leu Tyr
   100 105 110
   Thr Gly Arg Gly Pro Ser Ser Glu Ala Met His Val Gly His Leu Ile
   115 120 125
   Pro Phe Ile Phe Thr Lys Trp Leu Gln Asp Val Phe Asn Val Pro Leu
   130 135 140
   Val Ile Gln Met Thr Asp Asp Glu Lys Tyr Leu Trp Lys Asp Leu Thr
   145 150 155 160
   Leu Asp Gln Ala Tyr Gly Tyr Ala Val Glu Asn Ala Lys Asp Ile Ile
   165 170 175
   Ala Cys Gly Phe Asp Ile Asn Lys Thr Phe Ile Phe Ser Asp Leu Asp
   180 185 190
   Tyr Met Gly Met Ser Ser Gly Phe Tyr Lys Asn Val Val Lys Ile Gln
   195 200 205
   Lys His Val Thr Phe Asn Gln Val Lys Gly Ile Phe Gly Phe Thr Asp
   210 215 220
   Ser Asp Cys Ile Gly Lys Ile Ser Phe Pro Ala Ile Gln Ala Ala Pro
   225 230 235 240
   Ser Phe Ser Asn Ser Phe Pro Gln Ile Phe Arg Asp Arg Thr Asp Ile
   245 250 255
   Gln Cys Leu Ile Pro Cys Ala Ile Asp Gln Asp Pro Tyr Phe Arg Met
   260 265 270
   Thr Arg Asp Val Ala Pro Arg Ile Gly Tyr Pro Lys Pro Ala Leu Leu
   275 280 285
   His Ser Thr Phe Phe Pro Ala Leu Gln Gly Ala Gln Thr Lys Met Ser
   290 295 300
   Ala Ser Asp Pro Asn Ser Ser Ile Phe Leu Thr Asp Thr Ala Lys Gln
   305 310 315 320
   Ile Lys Thr Lys Val Asn Lys His Ala Phe Ser Gly Gly Arg Asp Thr
   325 330 335
   Ile Glu Glu His Arg Gln Phe Gly Gly Asn Cys Asp Val Asp Val Ser
   340 345 350
   Phe Met Tyr Leu Thr Phe Phe Leu Glu Asp Asp Asp Lys Leu Glu Gln
   355 360 365
   Ile Arg Lys Asp Tyr Thr Ser Gly Ala Met Leu Thr Gly Glu Leu Lys
   370 375 380
   Lys Ala Leu Ile Glu Val Leu Gln Pro Leu Ile Ala Glu His Gln Ala
   385 390 395 400
   Arg Arg Lys Glu Val Thr Asp Glu Ile Val Lys Glu Phe Met Thr Pro
   405 410 415
   Arg Lys Leu Ser Phe Asp Phe Gln
   420
<210> 42
   <211> 1134
   <212> DNA
   <213> Artificial sequence
<220>
   <223> Recombinant human T2 (GY variant) construct
<220>
   <221> CDS
   <222> (1)..(1134)
<220>
   <221> modified_base
   <222> (360)
   <223> a, t, c or g
<400> 42
   agt gca aaa ggc ata gac tac gat aag ctc att gtt cgg ttt gga agt 48
   Ser Ala Lys Gly Ile Asp Tyr Asp Lys Leu Ile Val Arg Phe Gly Ser
   1 5 10 15
   agt aaa att gac aaa gag cta ata aac cga ata gag aga gcc acc ggc 96
   Ser Lys Ile Asp Lys Glu Leu Ile Asn Arg Ile Glu Arg Ala Thr Gly
   20 25 30
   caa aga cca cac cac ttc ctg cgc aga ggc atc ttc ttc tca cac aga 144
   Gln Arg Pro His His Phe Leu Arg Arg Gly Ile Phe Phe Ser His Arg
   35 40 45
   gat atg aat cag gtt ctt gat gcc tat gaa aat aag aag cca ttt tat 192
   Asp Met Asn Gln Val Leu Asp Ala Tyr Glu Asn Lys Lys Pro Phe Tyr
   50 55 60
   ctg tac acg ggc cgg ggc ccc tct tct gaa gca atg cat gta ggt cac 240
   Leu Tyr Thr Gly Arg Gly Pro Ser Ser Glu Ala Met His Val Gly His
   65 70 75 80
   ctc att cca ttt att ttc aca aag tgg ctc cag gat gta ttt aac gtg 288
   Leu Ile Pro Phe Ile Phe Thr Lys Trp Leu Gln Asp Val Phe Asn Val
   85 90 95
   ccc ttg gtc atc cag atg acg gat gac gag aag tat ctg tgg aag gac 336
   Pro Leu Val Ile Gln Met Thr Asp Asp Glu Lys Tyr Leu Trp Lys Asp
   100 105 110
   ctg acc ctg gac cag gcc tat ggn tay gct gtt gag aat gcc aag gac 384
   Leu Thr Leu Asp Gln Ala Tyr Gly Tyr Ala Val Glu Asn Ala Lys Asp
   115 120 125
   atc atc gcc tgt ggc ttt gac atc aac aag act ttc ata ttc tct gac 432
   Ile Ile Ala Cys Gly Phe Asp Ile Asn Lys Thr Phe Ile Phe Ser Asp
   130 135 140
   ctg gac tac atg ggg atg agc tca ggt ttc tac aaa aat gtg gtg aag 480
   Leu Asp Tyr Met Gly Met Ser Ser Gly Phe Tyr Lys Asn Val Val Lys
   145                 150                 155                 160
   att caa aag cat gtt acc ttc aac caa gtg aaa ggc att ttc ggc ttc      528
   Ile Gln Lys His Val Thr Phe Asn Gln Val Lys Gly Ile Phe Gly Phe
                   165                 170                 175
   act gac agc gac tgc att ggg aag atc agt ttt cct gcc atc cag gct      576
   Thr Asp Ser Asp Cys Ile Gly Lys Ile Ser Phe Pro Ala Ile Gln Ala
               180                 185                 190
   gct ccc tcc ttc agc aac tca ttc cca cag atc ttc cga gac agg acg      624
   Ala Pro Ser Phe Ser Asn Ser Phe Pro Gln Ile Phe Arg Asp Arg Thr
           195                 200                 205
   gat atc cag tgc ctt atc cca tgt gcc att gac cag gat cct tac ttt      672
   Asp Ile Gln Cys Leu Ile Pro Cys Ala Ile Asp Gln Asp Pro Tyr Phe
       210                 215                 220
   aga atg aca agg gac gtc gcc ccc agg atc ggc tat cct aaa cca gcc      720
   Arg Met Thr Arg Asp Val Ala Pro Arg Ile Gly Tyr Pro Lys Pro Ala
   225                 230                 235                 240
   ctg ttg cac tcc acc ttc ttc cca gcc ctg cag ggc gcc cag acc aaa      768
   Leu Leu His Ser Thr Phe Phe Pro Ala Leu Gln Gly Ala Gln Thr Lys
                   245                 250                 255
   atg agt gcc agc gac cca aac tcc tcc atc ttc ctc acc gac acg gcc      816
   Met Ser Ala Ser Asp Pro Asn Ser Ser Ile Phe Leu Thr Asp Thr Ala
               260                 265                 270
   aag cag atc aaa acc aag gtc aat aag cat gcg ttt tct gga ggg aga      864
   Lys Gln Ile Lys Thr Lys Val Asn Lys His Ala Phe Ser Gly Gly Arg
           275                 280                 285
   gac acc atc gag gag cac agg cag ttt ggg ggc aac tgt gat gtg gac      912
   Asp Thr Ile Glu Glu His Arg Gln Phe Gly Gly Asn Cys Asp Val Asp
       290                 295                 300
   gtg tct ttc atg tac ctg acc ttc ttc ctc gag gac gac gac aag ctc      960
   Val Ser Phe Met Tyr Leu Thr Phe Phe Leu Glu Asp Asp Asp Lys Leu
   305                 310                 315                 320
   gag cag atc agg aag gat tac acc agc gga gcc atg ctc acc ggt gag      1008
   Glu Gln Ile Arg Lys Asp Tyr Thr Ser Gly Ala Met Leu Thr Gly Glu
                   325                 330                 335
   ctc aag aag gca ctc ata gag gtt ctg cag ccc ttg atc gca gag cac      1056
   Leu Lys Lys Ala Leu Ile Glu Val Leu Gln Pro Leu Ile Ala Glu His
               340                 345                 350
   cag gcc cgg cgc aag gag gtc acg gat gag ata gtg aaa gag ttc atg      1104
   Gln Ala Arg Arg Lys Glu Val Thr Asp Glu Ile Val Lys Glu Phe Met
           355                 360                 365
   act ccc cgg aag ctg tcc ttc gac ttt cag                              1134
   Thr Pro Arg Lys Leu Ser Phe Asp Phe Gln
       370                 375
<210> 43
   <211> 1137
   <212> DNA
   <213> Artificial Sequence
<220>
   <223> Recombinant human Met-T2 (GY variant) construct
<220>
   <221> CDS
   <222> (1)..(1137)
<220>
   <221> modified_base
<222> (363)
   <223> a, t, c or g
<400> 43
   atg agt gca aaa ggc ata gac tac gat aag ctc att gtt cgg ttt gga      48
   Met Ser Ala Lys Gly Ile Asp Tyr Asp Lys Leu Ile Val Arg Phe Gly
   1               5                   10                  15
   agt agt aaa att gac aaa gag cta ata aac cga ata gag aga gcc acc      96
   Ser Ser Lys Ile Asp Lys Glu Leu Ile Asn Arg Ile Glu Arg Ala Thr
               20                  25                  30
   ggc caa aga cca cac cac ttc ctg cgc aga ggc atc ttc ttc tca cac      144
   Gly Gln Arg Pro His His Phe Leu Arg Arg Gly Ile Phe Phe Ser His
           35                  40                  45
   aga gat atg aat cag gtt ctt gat gcc tat gaa aat aag aag cca ttt      192
   Arg Asp Met Asn Gln Val Leu Asp Ala Tyr Glu Asn Lys Lys Pro Phe
       50                  55                  60
   tat ctg tac acg ggc cgg ggc ccc tct tct gaa gca atg cat gta ggt      240
   Tyr Leu Tyr Thr Gly Arg Gly Pro Ser Ser Glu Ala Met His Val Gly
   65                  70                  75                  80
   cac ctc att cca ttt att ttc aca aag tgg ctc cag gat gta ttt aac      288
   His Leu Ile Pro Phe Ile Phe Thr Lys Trp Leu Gln Asp Val Phe Asn
                   85                  90                  95
   gtg ccc ttg gtc atc cag atg acg gat gac gag aag tat ctg tgg aag      336
   Val Pro Leu Val Ile Gln Met Thr Asp Asp Glu Lys Tyr Leu Trp Lys
               100                 105                 110
   gac ctg acc ctg gac cag gcc tat ggn tay gct gtt gag aat gcc aag      384
   Asp Leu Thr Leu Asp Gln Ala Tyr Gly Tyr Ala Val Glu Asn Ala Lys
           115                 120                 125
   gac atc atc gcc tgt ggc ttt gac atc aac aag act ttc ata ttc tct      432
   Asp Ile Ile Ala Cys Gly Phe Asp Ile Asn Lys Thr Phe Ile Phe Ser
       130                 135                 140
   gac ctg gac tac atg ggg atg agc tca ggt ttc tac aaa aat gtg gtg      480
   Asp Leu Asp Tyr Met Gly Met Ser Ser Gly Phe Tyr Lys Asn Val Val
   145                 150                 155                 160
   aag att caa aag cat gtt acc ttc aac caa gtg aaa ggc att ttc ggc      528
   Lys Ile Gln Lys His Val Thr Phe Asn Gln Val Lys Gly Ile Phe Gly
                   165                 170                 175
   ttc act gac agc gac tgc att ggg aag atc agt ttt cct gcc atc cag      576
   Phe Thr Asp Ser Asp Cys Ile Gly Lys Ile Ser Phe Pro Ala Ile Gln
               180                 185                 190
   gct gct ccc tcc ttc agc aac tca ttc cca cag atc ttc cga gac agg      624
   Ala Ala Pro Ser Phe Ser Asn Ser Phe Pro Gln Ile Phe Arg Asp Arg
           195                 200                 205
   acg gat atc cag tgc ctt atc cca tgt gcc att gac cag gat cct tac      672
   Thr Asp Ile Gln Cys Leu Ile Pro Cys Ala Ile Asp Gln Asp Pro Tyr
       210                 215                 220
   ttt aga atg aca agg gac gtc gcc ccc agg atc ggc tat cct aaa cca      720
   Phe Arg Met Thr Arg Asp Val Ala Pro Arg Ile Gly Tyr Pro Lys Pro
   225                 230                 235                 240
   gcc ctg ttg cac tcc acc ttc ttc cca gcc ctg cag ggc gcc cag acc      768
   Ala Leu Leu His Ser Thr Phe Phe Pro Ala Leu Gln Gly Ala Gln Thr
                   245                 250                 255
   aaa atg agt gcc agc gac cca aac tcc tcc atc ttc ctc acc gac acg      816
   Lys Met Ser Ala Ser Asp Pro Asn Ser Ser Ile Phe Leu Thr Asp Thr
               260                 265                 270
   gcc aag cag atc aaa acc aag gtc aat aag cat gcg ttt tct gga ggg      864
   Ala Lys Gln Ile Lys Thr Lys Val Asn Lys His Ala Phe Ser Gly Gly
           275                 280                 285
   aga gac acc atc gag gag cac agg cag ttt ggg ggc aac tgt gat gtg      912
   Arg Asp Thr Ile Glu Glu His Arg Gln Phe Gly Gly Asn Cys Asp Val
       290                 295                 300
   gac gtg tct ttc atg tac ctg acc ttc ttc ctc gag gac gac gac aag      960
   Asp Val Ser Phe Met Tyr Leu Thr Phe Phe Leu Glu Asp Asp Asp Lys
   305                 310                 315                 320
   ctc gag cag atc agg aag gat tac acc agc gga gcc atg ctc acc ggt     1008
   Leu Glu Gln Ile Arg Lys Asp Tyr Thr Ser Gly Ala Met Leu Thr Gly
                   325                 330                 335
   gag ctc aag aag gca ctc ata gag gtt ctg cag ccc ttg atc gca gag     1056
   Glu Leu Lys Lys Ala Leu Ile Glu Val Leu Gln Pro Leu Ile Ala Glu
               340                 345                 350
   cac cag gcc cgg cgc aag gag gtc acg gat gag ata gtg aaa gag ttc     1104
   His Gln Ala Arg Arg Lys Glu Val Thr Asp Glu Ile Val Lys Glu Phe
           355                 360                 365
   atg act ccc cgg aag ctg tcc ttc gac ttt cag                         1137
   Met Thr Pro Arg Lys Leu Ser Phe Asp Phe Gln
       370                 375
<210> 44
   <211> 1203
   <212> DNA
   <213> Artificial sequence
<220>
   <223> Recombinant human T1 (GY variant) construct
<220>
   <221> CDS
   <222> (1)..(1203)
<220>
   <221> modified_base
   <222> (429)
   <223> a, t, c or g
<400> 44
   agt aat cat ggc cca gat gcc aca gaa gct gaa gag gat ttt gtg gac      48
   Ser Asn His Gly Pro Asp Ala Thr Glu Ala Glu Glu Asp Phe Val Asp
    1               5                  10                  15
   cca tgg aca gta cag aca agc agt gca aaa ggc ata gac tac gat aag       96
   Pro Trp Thr Val Gln Thr Ser Ser Ala Lys Gly Ile Asp Tyr Asp Lys
               20                  25                  30
   ctc att gtt cgg ttt gga agt agt aaa att gac aaa gag cta ata aac      144
   Leu Ile Val Arg Phe Gly Ser Ser Lys Ile Asp Lys Glu Leu Ile Asn
           35                  40                  45
   cga ata gag aga gcc acc ggc caa aga cca cac cac ttc ctg cgc aga      192
   Arg Ile Glu Arg Ala Thr Gly Gln Arg Pro His His Phe Leu Arg Arg
       50                  55                  60
   ggc atc ttc ttc tca cac aga gat atg aat cag gtt ctt gat gcc tat      240
   Gly Ile Phe Phe Ser His Arg Asp Met Asn Gln Val Leu Asp Ala Tyr
   65                  70                  75                  80
   gaa aat aag aag cca ttt tat ctg tac acg ggc cgg ggc ccc tct tct      288
   Glu Asn Lys Lys Pro Phe Tyr Leu Tyr Thr Gly Arg Gly Pro Ser Ser
                   85                  90                  95
   gaa gca atg cat gta ggt cac ctc att cca ttt att ttc aca aag tgg      336
   Glu Ala Met His Val Gly His Leu Ile Pro Phe Ile Phe Thr Lys Trp
               100                 105                 110
   ctc cag gat gta ttt aac gtg ccc ttg gtc atc cag atg acg gat gac      384
   Leu Gln Asp Val Phe Asn Val Pro Leu Val Ile Gln Met Thr Asp Asp
           115                 120                 125
   gag aag tat ctg tgg aag gac ctg acc ctg gac cag gcc tat ggn tay      432
   Glu Lys Tyr Leu Trp Lys Asp Leu Thr Leu Asp Gln Ala Tyr Gly Tyr
       130                 135                 140
   gct gtt gag aat gcc aag gac atc atc gcc tgt ggc ttt gac atc aac      480
   Ala Val Glu Asn Ala Lys Asp Ile Ile Ala Cys Gly Phe Asp Ile Asn
   145                 150                 155                 160
   aag act ttc ata ttc tct gac ctg gac tac atg ggg atg agc tca ggt      528
   Lys Thr Phe Ile Phe Ser Asp Leu Asp Tyr Met Gly Met Ser Ser Gly
                   165                 170                 175
   ttc tac aaa aat gtg gtg aag att caa aag cat gtt acc ttc aac caa      576
   Phe Tyr Lys Asn Val Val Lys Ile Gln Lys His Val Thr Phe Asn Gln
               180                 185                 190
   gtg aaa ggc att ttc ggc ttc act gac agc gac tgc att ggg aag atc      624
   Val Lys Gly Ile Phe Gly Phe Thr Asp Ser Asp Cys Ile Gly Lys Ile
           195                 200                 205
   agt ttt cct gcc atc cag gct gct ccc tcc ttc agc aac tca ttc cca      672
   Ser Phe Pro Ala Ile Gln Ala Ala Pro Ser Phe Ser Asn Ser Phe Pro
       210                 215                 220
   cag atc ttc cga gac agg acg gat atc cag tgc ctt atc cca tgt gcc      720
   Gln Ile Phe Arg Asp Arg Thr Asp Ile Gln Cys Leu Ile Pro Cys Ala
   225                 230                 235                 240
   att gac cag gat cct tac ttt aga atg aca agg gac gtc gcc ccc agg      768
   Ile Asp Gln Asp Pro Tyr Phe Arg Met Thr Arg Asp Val Ala Pro Arg
                   245                 250                 255
   atc ggc tat cct aaa cca gcc ctg ttg cac tcc acc ttc ttc cca gcc      816
   Ile Gly Tyr Pro Lys Pro Ala Leu Leu His Ser Thr Phe Phe Pro Ala
               260                 265                 270
   ctg cag ggc gcc cag acc aaa atg agt gcc agc gac cca aac tcc tcc      864
   Leu Gln Gly Ala Gln Thr Lys Met Ser Ala Ser Asp Pro Asn Ser Ser
                   275                           280                           285
   atc ttc ctc acc gac acg gcc aag cag atc aaa acc aag gtc aat aag     912
   Ile Phe Leu Thr Asp Thr Ala Lys Gln Ile Lys Thr Lys Val Asn Lys
       290                     295                     300
   cat gcg ttt tct gga ggg aga gac acc atc gag gag cac agg cag ttt     960
   His Ala Phe Ser Gly Gly Arg Asp Thr Ile Glu Glu His Arg Gln Phe
   305                     310                     315                 320
   ggg ggc aac tgt gat gtg gac gtg tct ttc atg tac ctg acc ttc ttc     1008
   Gly Gly Asn Cys Asp Val Asp Val Ser Phe Met Tyr Leu Thr Phe Phe
                   325                     330                     335
   ctc gag gac gac gac aag ctc gag cag atc agg aag gat tac acc agc     1056
   Leu Glu Asp Asp Asp Lys Leu Glu Gln Ile Arg Lys Asp Tyr Thr Ser
               340                     345                     350
   gga gcc atg ctc acc ggt gag ctc aag aag gca ctc ata gag gtt ctg     1104
   Gly Ala Met Leu Thr Gly Glu Leu Lys Lys Ala Leu Ile Glu Val Leu
           355                     360                     365
   cag ccc ttg atc gca gag cac cag gcc cgg cgc aag gag gtc acg gat     1152
   Gln Pro Leu Ile Ala Glu His Gln Ala Arg Arg Lys Glu Val Thr Asp
       370                     375                     380
   gag ata gtg aaa gag ttc atg act ccc cgg aag ctg tcc ttc gac ttt     1200
   Glu Ile Val Lys Glu Phe Met Thr Pro Arg Lys Leu Ser Phe Asp Phe
   385                     390                     395                 400
   cag                                                                 1203
   Gln
<210> 45
   <211> 1206
   <212> DNA
   <213> Artificial Sequence
<220>
   <223> Recombinant human Met-T1 (GY variant) construct
<220>
   <221> CDS
   <222> (1)..(1206)
<220>
   <221> modified_base
   <222> (432)
   <223> a, t, c or g
<400> 45
   atg agt aat cat ggc cca gat gcc aca gaa gct gaa gag gat ttt gtg     48
   Met Ser Asn His Gly Pro Asp Ala Thr Glu Ala Glu Glu Asp Phe Val
     1               5                  10                  15
   gac cca tgg aca gta cag aca agc agt gca aaa ggc ata gac tac gat     96
   Asp Pro Trp Thr Val Gln Thr Ser Ser Ala Lys Gly Ile Asp Tyr Asp
               20                  25                  30
   aag ctc att gtt cgg ttt gga agt agt aaa att gac aaa gag cta ata     144
   Lys Leu Ile Val Arg Phe Gly Ser Ser Lys Ile Asp Lys Glu Leu Ile
           35                  40                  45
   aac cga ata gag aga gcc acc ggc caa aga cca cac cac ttc ctg cgc     192
   Asn Arg Ile Glu Arg Ala Thr Gly Gln Arg Pro His His Phe Leu Arg
        50                  55                  60
   aga ggc atc ttc ttc tca cac aga gat atg aat cag gtt ctt gat gcc      240
   Arg Gly Ile Phe Phe Ser His Arg Asp Met Asn Gln Val Leu Asp Ala
    65                  70                  75                  80
   tat gaa aat aag aag cca ttt tat ctg tac acg ggc cgg ggc ccc tct      288
   Tyr Glu Asn Lys Lys Pro Phe Tyr Leu Tyr Thr Gly Arg Gly Pro Ser
                    85                  90                  95
   tct gaa gca atg cat gta ggt cac ctc att cca ttt att ttc aca aag      336
   Ser Glu Ala Met His Val Gly His Leu Ile Pro Phe Ile Phe Thr Lys
               100                 105                 110
   tgg ctc cag gat gta ttt aac gtg ccc ttg gtc atc cag atg acg gat      384
   Trp Leu Gln Asp Val Phe Asn Val Pro Leu Val Ile Gln Met Thr Asp
           115                 120                 125
   gac gag aag tat ctg tgg aag gac ctg acc ctg gac cag gcc tat ggn      432
   Asp Glu Lys Tyr Leu Trp Lys Asp Leu Thr Leu Asp Gln Ala Tyr Gly
       130                 135                 140
   tay gct gtt gag aat gcc aag gac atc atc gcc tgt ggc ttt gac atc      480
   Tyr Ala Val Glu Asn Ala Lys Asp Ile Ile Ala Cys Gly Phe Asp Ile
   145                 150                 155                 160
   aac aag act ttc ata ttc tct gac ctg gac tac atg ggg atg agc tca      528
   Asn Lys Thr Phe Ile Phe Ser Asp Leu Asp Tyr Met Gly Met Ser Ser
                   165                 170                 175
   ggt ttc tac aaa aat gtg gtg aag att caa aag cat gtt acc ttc aac      576
   Gly Phe Tyr Lys Asn Val Val Lys Ile Gln Lys His Val Thr Phe Asn
               180                 185                 190
   caa gtg aaa ggc att ttc ggc ttc act gac agc gac tgc att ggg aag      624
   Gln Val Lys Gly Ile Phe Gly Phe Thr Asp Ser Asp Cys Ile Gly Lys
           195                 200                 205
   atc agt ttt cct gcc atc cag gct gct ccc tcc ttc agc aac tca ttc      672
   Ile Ser Phe Pro Ala Ile Gln Ala Ala Pro Ser Phe Ser Asn Ser Phe
       210                 215                 220
   cca cag atc ttc cga gac agg acg gat atc cag tgc ctt atc cca tgt      720
   Pro Gln Ile Phe Arg Asp Arg Thr Asp Ile Gln Cys Leu Ile Pro Cys
   225                 230                 235                 240
   gcc att gac cag gat cct tac ttt aga atg aca agg gac gtc gcc ccc      768
   Ala Ile Asp Gln Asp Pro Tyr Phe Arg Met Thr Arg Asp Val Ala Pro
                   245                 250                 255
   agg atc ggc tat cct aaa cca gcc ctg ttg cac tcc acc ttc ttc cca      816
   Arg Ile Gly Tyr Pro Lys Pro Ala Leu Leu His Ser Thr Phe Phe Pro
               260                 265                 270
   gcc ctg cag ggc gcc cag acc aaa atg agt gcc agc gac cca aac tcc      864
   Ala Leu Gln Gly Ala Gln Thr Lys Met Ser Ala Ser Asp Pro Asn Ser
           275                 280                 285
   tcc atc ttc ctc acc gac acg gcc aag cag atc aaa acc aag gtc aat      912
   Ser Ile Phe Leu Thr Asp Thr Ala Lys Gln Ile Lys Thr Lys Val Asn
       290                 295                 300
   aag cat gcg ttt tct gga ggg aga gac acc atc gag gag cac agg cag      960
   Lys His Ala Phe Ser Gly Gly Arg Asp Thr Ile Glu Glu His Arg Gln
   305                 310                 315                 320
   ttt ggg ggc aac tgt gat gtg gac gtg tct ttc atg tac ctg acc ttc      1008
   Phe Gly Gly Asn Cys Asp Val Asp Val Ser Phe Met Tyr Leu Thr Phe
                   325                 330                 335
   ttc ctc gag gac gac gac aag ctc gag cag atc agg aag gat tac acc      1056
   Phe Leu Glu Asp Asp Asp Lys Leu Glu Gln Ile Arg Lys Asp Tyr Thr
               340                 345                 350
   agc gga gcc atg ctc acc ggt gag ctc aag aag gca ctc ata gag gtt      1104
   Ser Gly Ala Met Leu Thr Gly Glu Leu Lys Lys Ala Leu Ile Glu Val
           355                 360                 365
   ctg cag ccc ttg atc gca gag cac cag gcc cgg cgc aag gag gtc acg      1152
   Leu Gln Pro Leu Ile Ala Glu His Gln Ala Arg Arg Lys Glu Val Thr
       370                 375                 380
   gat gag ata gtg aaa gag ttc atg act ccc cgg aag ctg tcc ttc gac      1200
   Asp Glu Ile Val Lys Glu Phe Met Thr Pro Arg Lys Leu Ser Phe Asp
   385                 390                 395                 400
   ttt cag                                                              1206
   Phe Gln
<210> 46
   <211> 1269
   <212> DNA
   <213> Artificial sequence
<220>
<223> Recombinant human mini-TrpRS (GY variant) construct
<220>
   <221> CDS
   <222> (1)..(1269)
<220>
   <221> modified_base
   <222> (495)
   <223> a, t, c or g
<400> 46
   agc tac aaa gct gcc gcg ggg gag gat tac aag gct gac tgt cct cca      48
   Ser Tyr Lys Ala Ala Ala Gly Glu Asp Tyr Lys Ala Asp Cys Pro Pro
   1               5                   10                  15
   ggg aac cca gca cct acc agt aat cat ggc cca gat gcc aca gaa gct      96
   Gly Asn Pro Ala Pro Thr Ser Asn His Gly Pro Asp Ala Thr Glu Ala
               20                  25                  30
   gaa gag gat ttt gtg gac cca tgg aca gta cag aca agc agt gca aaa      144
   Glu Glu Asp Phe Val Asp Pro Trp Thr Val Gln Thr Ser Ser Ala Lys
           35                  40                  45
   ggc ata gac tac gat aag ctc att gtt cgg ttt gga agt agt aaa att      192
   Gly Ile Asp Tyr Asp Lys Leu Ile Val Arg Phe Gly Ser Ser Lys Ile
       50                  55                  60
   gac aaa gag cta ata aac cga ata gag aga gcc acc ggc caa aga cca      240
   Asp Lys Glu Leu Ile Asn Arg Ile Glu Arg Ala Thr Gly Gln Arg Pro
   65                  70                  75                  80
   cac cac ttc ctg cgc aga ggc atc ttc ttc tca cac aga gat atg aat      288
   His His Phe Leu Arg Arg Gly Ile Phe Phe Ser His Arg Asp Met Asn
                   85                  90                  95
   cag gtt ctt gat gcc tat gaa aat aag aag cca ttt tat ctg tac acg    336
   Gln Val Leu Asp Ala Tyr Glu Asn Lys Lys Pro Phe Tyr Leu Tyr Thr
               100                 105                 110
   ggc cgg ggc ccc tct tct gaa gca atg cat gta ggt cac ctc att cca    384
   Gly Arg Gly Pro Ser Ser Glu Ala Met His Val Gly His Leu Ile Pro
           115                 120                 125
   ttt att ttc aca aag tgg ctc cag gat gta ttt aac gtg ccc ttg gtc    432
   Phe Ile Phe Thr Lys Trp Leu Gln Asp Val Phe Asn Val Pro Leu Val
       130                 135                 140
   atc cag atg acg gat gac gag aag tat ctg tgg aag gac ctg acc ctg    480
   Ile Gln Met Thr Asp Asp Glu Lys Tyr Leu Trp Lys Asp Leu Thr Leu
   145                 150                 155                 160
   gac cag gcc tat ggn tay gct gtt gag aat gcc aag gac atc atc gcc    528
   Asp Gln Ala Tyr Gly Tyr Ala Val Glu Asn Ala Lys Asp Ile Ile Ala
                   165                 170                 175
   tgt ggc ttt gac atc aac aag act ttc ata ttc tct gac ctg gac tac    576
   Cys Gly Phe Asp Ile Asn Lys Thr Phe Ile Phe Ser Asp Leu Asp Tyr
               180                 185                 190
   atg ggg atg agc tca ggt ttc tac aaa aat gtg gtg aag att caa aag    624
   Met Gly Met Ser Ser Gly Phe Tyr Lys Asn Val Val Lys Ile Gln Lys
           195                 200                 205
   cat gtt acc ttc aac caa gtg aaa ggc att ttc ggc ttc act gac agc    672
   His Val Thr Phe Asn Gln Val Lys Gly Ile Phe Gly Phe Thr Asp Ser
       210                 215                 220
   gac tgc att ggg aag atc agt ttt cct gcc atc cag gct gct ccc tcc    720
   Asp Cys Ile Gly Lys Ile Ser Phe Pro Ala Ile Gln Ala Ala Pro Ser
   225                 230                 235                 240
   ttc agc aac tca ttc cca cag atc ttc cga gac agg acg gat atc cag    768
   Phe Ser Asn Ser Phe Pro Gln Ile Phe Arg Asp Arg Thr Asp Ile Gln
                   245                 250                 255
   tgc ctt atc cca tgt gcc att gac cag gat cct tac ttt aga atg aca    816
   Cys Leu Ile Pro Cys Ala Ile Asp Gln Asp Pro Tyr Phe Arg Met Thr
               260                 265                 270
   agg gac gtc gcc ccc agg atc ggc tat cct aaa cca gcc ctg ttg cac    864
   Arg Asp Val Ala Pro Arg Ile Gly Tyr Pro Lys Pro Ala Leu Leu His
           275                 280                 285
   tcc acc ttc ttc cca gcc ctg cag ggc gcc cag acc aaa atg agt gcc    912
   Ser Thr Phe Phe Pro Ala Leu Gln Gly Ala Gln Thr Lys Met Ser Ala
       290                 295                 300
   agc gac cca aac tcc tcc atc ttc ctc acc gac acg gcc aag cag atc    960
   Ser Asp Pro Asn Ser Ser Ile Phe Leu Thr Asp Thr Ala Lys Gln Ile
   305                 310                 315                 320
   aaa acc aag gtc aat aag cat gcg ttt tct gga ggg aga gac acc atc    1008
   Lys Thr Lys Val Asn Lys His Ala Phe Ser Gly Gly Arg Asp Thr Ile
                   325                 330                 335
   gag gag cac agg cag ttt ggg ggc aac tgt gat gtg gac gtg tct ttc    1056
   Glu Glu His Arg Gln Phe Gly Gly Asn Cys Asp Val Asp Val Ser Phe
               340                 345                 350
   atg tac ctg acc ttc ttc ctc gag gac gac gac aag ctc gag cag atc    1104
   Met Tyr Leu Thr Phe Phe Leu Glu Asp Asp Asp Lys Leu Glu Gln Ile
   355 360 365
   agg aag gat tac acc agc gga gcc atg ctc acc ggt gag ctc aag aag 1152
   Arg Lys Asp Tyr Thr Ser Gly Ala Met Leu Thr Gly Glu Leu Lys Lys
   370 375 380
   gca ctc ata gag gtt ctg cag ccc ttg atc gca gag cac cag gcc cgg 1200
   Ala Leu Ile Glu Val Leu Gln Pro Leu Ile Ala Glu His Gln Ala Arg
   385 390 395 400
   cgc aag gag gtc acg gat gag ata gtg aaa gag ttc atg act ccc cgg 1248
   Arg Lys Glu Val Thr Asp Glu Ile Val Lys Glu Phe Met Thr Pro Arg
   405 410 415
   aag ctg tcc ttc gac ttt cag 1269
   Lys Leu Ser Phe Asp Phe Gln
   420
<210> 47
   <211> 1272
   <212> DNA
   <213> Artificial sequence
<220>
   <223> Recombinant human Met-mini-TrpRS (GY variant) construct
<220>
   <221> CDS
   <222> (1)..(1272)
<220>
   <221> modified_base
   <222> (498)
   <223> a, t, c or g
<400> 47
   atg agc tac aaa gct gcc gcg ggg gag gat tac aag gct gac tgt cct 48
   Met Ser Tyr Lys Ala Ala Ala Gly Glu Asp Tyr Lys Ala Asp Cys Pro
   1 5 10 15
   cca ggg aac cca gca cct acc agt aat cat ggc cca gat gcc aca gaa 96
   Pro Gly Asn Pro Ala Pro Thr Ser Asn His Gly Pro Asp Ala Thr Glu
   20 25 30
   gct gaa gag gat ttt gtg gac cca tgg aca gta cag aca agc agt gca 144
   Ala Glu Glu Asp Phe Val Asp Pro Trp Thr Val Gln Thr Ser Ser Ala
   35 40 45
   aaa ggc ata gac tac gat aag ctc att gtt cgg ttt gga agt agt aaa 192
   Lys Gly Ile Asp Tyr Asp Lys Leu Ile Val Arg Phe Gly Ser Ser Lys
   50 55 60
   att gac aaa gag cta ata aac cga ata gag aga gcc acc ggc caa aga 240
   Ile Asp Lys Glu Leu Ile Asn Arg Ile Glu Arg Ala Thr Gly Gln Arg
   65 70 75 80
   cca cac cac ttc ctg cgc aga ggc atc ttc ttc tca cac aga gat atg 288
   Pro His His Phe Leu Arg Arg Gly Ile Phe Phe Ser His Arg Asp Met
   85 90 95
   aat cag gtt ctt gat gcc tat gaa aat aag aag cca ttt tat ctg tac 336
   Asn Gln Val Leu Asp Ala Tyr Glu Asn Lys Lys Pro Phe Tyr Leu Tyr
   100 105 110
   acg ggc cgg ggc ccc tct tct gaa gca atg cat gta ggt cac ctc att 384
   Thr Gly Arg Gly Pro Ser Ser Glu Ala Met His Val Gly His Leu Ile
   115 120 125
   cca ttt att ttc aca aag tgg ctc cag gat gta ttt aac gtg ccc ttg 432
   Pro Phe Ile Phe Thr Lys Trp Leu Gln Asp Val Phe Asn Val Pro Leu
   130 135 140
   gtc atc cag atg acg gat gac gag aag tat ctg tgg aag gac ctg acc 480
   Val Ile Gln Met Thr Asp Asp Glu Lys Tyr Leu Trp Lys Asp Leu Thr
   145 150 155 160
   ctg gac cag gcc tat ggn tay gct gtt gag aat gcc aag gac atc atc 528
   Leu Asp Gln Ala Tyr Gly Tyr Ala Val Glu Asn Ala Lys Asp Ile Ile
   165 170 175
   gcc tgt ggc ttt gac atc aac aag act ttc ata ttc tct gac ctg gac 576
   Ala Cys Gly Phe Asp Ile Asn Lys Thr Phe Ile Phe Ser Asp Leu Asp
   180 185 190
   tac atg ggg atg agc tca ggt ttc tac aaa aat gtg gtg aag att caa 624
   Tyr Met Gly Met Ser Ser Gly Phe Tyr Lys Asn Val Val Lys Ile Gln
   195 200 205
   aag cat gtt acc ttc aac caa gtg aaa ggc att ttc ggc ttc act gac 672
   Lys His Val Thr Phe Asn Gln Val Lys Gly Ile Phe Gly Phe Thr Asp
   210 215 220
   agc gac tgc att ggg aag atc agt ttt cct gcc atc cag gct gct ccc 720
   Ser Asp Cys Ile Gly Lys Ile Ser Phe Pro Ala Ile Gln Ala Ala Pro
   225 230 235 240
   tcc ttc agc aac tca ttc cca cag atc ttc cga gac agg acg gat atc 768
   Ser Phe Ser Asn Ser Phe Pro Gln Ile Phe Arg Asp Arg Thr Asp Ile
   245 250 255
   cag tgc ctt atc cca tgt gcc att gac cag gat cct tac ttt aga atg 816
   Gln Cys Leu Ile Pro Cys Ala Ile Asp Gln Asp Pro Tyr Phe Arg Met
   260 265 270
   aca agg gac gtc gcc ccc agg atc ggc tat cct aaa cca gcc ctg ttg 864
   Thr Arg Asp Val Ala Pro Arg Ile Gly Tyr Pro Lys Pro Ala Leu Leu
   275 280 285
   cac tcc acc ttc ttc cca gcc ctg cag ggc gcc cag acc aaa atg agt 912
   His Ser Thr Phe Phe Pro Ala Leu Gln Gly Ala Gln Thr Lys Met Ser
   290 295 300
   gcc agc gac cca aac tcc tcc atc ttc ctc acc gac acg gcc aag cag 960
   Ala Ser Asp Pro Asn Ser Ser Ile Phe Leu Thr Asp Thr Ala Lys Gln
   305 310 315 320
   atc aaa acc aag gtc aat aag cat gcg ttt tct gga ggg aga gac acc 1008
   Ile Lys Thr Lys Val Asn Lys His Ala Phe Ser Gly Gly Arg Asp Thr
   325 330 335
   atc gag gag cac agg cag ttt ggg ggc aac tgt gat gtg gac gtg tct 1056
   Ile Glu Glu His Arg Gln Phe Gly Gly Asn Cys Asp Val Asp Val Ser
   340 345 350
   ttc atg tac ctg acc ttc ttc ctc gag gac gac gac aag ctc gag cag 1104
   Phe Met Tyr Leu Thr Phe Phe Leu Glu Asp Asp Asp Lys Leu Glu Gln
   355 360 365
   atc agg aag gat tac acc agc gga gcc atg ctc acc ggt gag ctc aag 1152
   Ile Arg Lys Asp Tyr Thr Ser Gly Ala Met Leu Thr Gly Glu Leu Lys
   370 375 380
   aag gca ctc ata gag gtt ctg cag ccc ttg atc gca gag cac cag gcc     1200
   Lys Ala Leu Ile Glu Val Leu Gln Pro Leu Ile Ala Glu His Gln Ala
   385                 390                 395                 400
   cgg cgc aag gag gtc acg gat gag ata gtg aaa gag ttc atg act ccc     1248
   Arg Arg Lys Glu Val Thr Asp Glu Ile Val Lys Glu Phe Met Thr Pro
                   405                 410                 415
   cgg aag ctg tcc ttc gac ttt cag                                     1272
   Arg Lys Leu Ser Phe Asp Phe Gln
               420
<210> 48
   <211> 378
   <212> PRT
   <213> Artificial Sequence
<220>
   <223> Recombinant human T2 (SD variant) construct
<400> 48
   Ser Ala Lys Gly Ile Asp Tyr Asp Lys Leu Ile Val Arg Phe Gly Ser
   1               5                   10                  15
   Ser Lys Ile Asp Lys Glu Leu Ile Asn Arg Ile Glu Arg Ala Thr Gly
               20                  25                  30
   Gln Arg Pro His His Phe Leu Arg Arg Gly Ile Phe Phe Ser His Arg
           35                  40                  45
   Asp Met Asn Gln Val Leu Asp Ala Tyr Glu Asn Lys Lys Pro Phe Tyr
       50                  55                  60
   Leu Tyr Thr Gly Arg Gly Pro Ser Ser Glu Ala Met His Val Gly His
   65                  70                  75                  80
   Leu Ile Pro Phe Ile Phe Thr Lys Trp Leu Gln Asp Val Phe Asn Val
                   85                  90                  95
   Pro Leu Val Ile Gln Met Thr Asp Asp Glu Lys Tyr Leu Trp Lys Asp
               100                 105                 110
   Leu Thr Leu Asp Gln Ala Tyr Ser Asp Ala Val Glu Asn Ala Lys Asp
           115                 120                 125
   Ile Ile Ala Cys Gly Phe Asp Ile Asn Lys Thr Phe Ile Phe Ser Asp
       130                 135                 140
   Leu Asp Tyr Met Gly Met Ser Ser Gly Phe Tyr Lys Asn Val Val Lys
   145                 150                 155                 160
   Ile Gln Lys His Val Thr Phe Asn Gln Val Lys Gly Ile Phe Gly Phe
                   165                 170                 175
   Thr Asp Ser Asp Cys Ile Gly Lys Ile Ser Phe Pro Ala Ile Gln Ala
               180                 185                 190
   Ala Pro Ser Phe Ser Asn Ser Phe Pro Gln Ile Phe Arg Asp Arg Thr
           195                 200                 205
   Asp Ile Gln Cys Leu Ile Pro Cys Ala Ile Asp Gln Asp Pro Tyr Phe
       210                 215                 220
   Arg Met Thr Arg Asp Val Ala Pro Arg Ile Gly Tyr Pro Lys Pro Ala
   225                 230                 235                 240
   Leu Leu His Ser Thr Phe Phe Pro Ala Leu Gln Gly Ala Gln Thr Lys
   245 250 255
   Met Ser Ala Ser Asp Pro Asn Ser Ser Ile Phe Leu Thr Asp Thr Ala
   260 265 270
   Lys Gln Ile Lys Thr Lys Val Asn Lys His Ala Phe Ser Gly Gly Arg
   275 280 285
   Asp Thr Ile Glu Glu His Arg Gln Phe Gly Gly Asn Cys Asp Val Asp
   290 295 300
   Val Ser Phe Met Tyr Leu Thr Phe Phe Leu Glu Asp Asp Asp Lys Leu
   305 310 315 320
   Glu Gln Ile Arg Lys Asp Tyr Thr Ser Gly Ala Met Leu Thr Gly Glu
   325 330 335
   Leu Lys Lys Ala Leu Ile Glu Val Leu Gln Pro Leu Ile Ala Glu His
   340 345 350
   Gln Ala Arg Arg Lys Glu Val Thr Asp Glu Ile Val Lys Glu Phe Met
   355 360 365
   Thr Pro Arg Lys Leu Ser Phe Asp Phe Gln
   370 375
<210> 49
   <211> 401
   <212> PRT
   <213> Artificial sequence
<220>
   <223> Recombinant human T1 (SD variant) construct
<400> 49
   Ser Asn His Gly Pro Asp Ala Thr Glu Ala Glu Glu Asp Phe Val Asp
   1               5                   10                  15
   Pro Trp Thr Val Gln Thr Ser Ser Ala Lys Gly Ile Asp Tyr Asp Lys
               20                  25                  30
   Leu Ile Val Arg Phe Gly Ser Ser Lys Ile Asp Lys Glu Leu Ile Asn
           35                  40                  45
   Arg Ile Glu Arg Ala Thr Gly Gln Arg Pro His His Phe Leu Arg Arg
       50                  55                  60
   Gly Ile Phe Phe Ser His Arg Asp Met Asn Gln Val Leu Asp Ala Tyr
   65                  70                  75                  80
   Glu Asn Lys Lys Pro Phe Tyr Leu Tyr Thr Gly Arg Gly Pro Ser Ser
                   85                  90                  95
   Glu Ala Met His Val Gly His Leu Ile Pro Phe Ile Phe Thr Lys Trp
               100                 105                 110
   Leu Gln Asp Val Phe Asn Val Pro Leu Val Ile Gln Met Thr Asp Asp
           115                 120                 125
   Glu Lys Tyr Leu Trp Lys Asp Leu Thr Leu Asp Gln Ala Tyr Ser Asp
       130                 135                 140
   Ala Val Glu Asn Ala Lys Asp Ile Ile Ala Cys Gly Phe Asp Ile Asn
   145 150 155 160
   Lys Thr Phe Ile Phe Ser Asp Leu Asp Tyr Met Gly Met Ser Ser Gly
   165 170 175
   Phe Tyr Lys Asn Val Val Lys Ile Gln Lys His Val Thr Phe Asn Gln
   180 185 190
   Val Lys Gly Ile Phe Gly Phe Thr Asp Ser Asp Cys Ile Gly Lys Ile
   195 200 205
   Ser Phe Pro Ala Ile Gln Ala Ala Pro Ser Phe Ser Asn Ser Phe Pro
   210 215 220
   Gln Ile Phe Arg Asp Arg Thr Asp Ile Gln Cys Leu Ile Pro Cys Ala
   225 230 235 240
   Ile Asp Gln Asp Pro Tyr Phe Arg Met Thr Arg Asp Val Ala Pro Arg
   245 250 255
   Ile Gly Tyr Pro Lys Pro Ala Leu Leu His Ser Thr Phe Phe Pro Ala
   260 265 270
   Leu Gln Gly Ala Gln Thr Lys Met Ser Ala Ser Asp Pro Asn Ser Ser
   275 280 285
   Ile Phe Leu Thr Asp Thr Ala Lys Gln Ile Lys Thr Lys Val Asn Lys
   290 295 300
   His Ala Phe Ser Gly Gly Arg Asp Thr Ile Glu Glu His Arg Gln Phe
   305 310 315 320
   Gly Gly Asn Cys Asp Val Asp Val Ser Phe Met Tyr Leu Thr Phe Phe
   325 330 335
   Leu Glu Asp Asp Asp Lys Leu Glu Gln Ile Arg Lys Asp Tyr Thr Ser
   340 345 350
   Gly Ala Met Leu Thr Gly Glu Leu Lys Lys Ala Leu Ile Glu Val Leu
   355 360 365
   Gln Pro Leu Ile Ala Glu His Gln Ala Arg Arg Lys Glu Val Thr Asp
   370 375 380
   Glu Ile Val Lys Glu Phe Met Thr Pro Arg Lys Leu Ser Phe Asp Phe
   385 390 395 400
   Gln
<210> 50
   <211> 423
   <212> PRT
   <213> Artificial sequence
<220>
   <223> Recombinant human mini-TrpRS (SD variant) construct
<400> 50
   Ser Tyr Lys Ala Ala Ala Gly Glu Asp Tyr Lys Ala Asp Cys Pro Pro
   1 5 10 15
   Gly Asn Pro Ala Pro Thr Ser Asn His Gly Pro Asp Ala Thr Glu Ala
   20 25 30
   Glu Glu Asp Phe Val Asp Pro Trp Thr Val Gln Thr Ser Ser Ala Lys
               35                  40                  45
   Gly Ile Asp Tyr Asp Lys Leu Ile Val Arg Phe Gly Ser Ser Lys Ile
       50                  55                  60
   Asp Lys Glu Leu Ile Asn Arg Ile Glu Arg Ala Thr Gly Gln Arg Pro
   65                  70                  75                  80
   His His Phe Leu Arg Arg Gly Ile Phe Phe Ser His Arg Asp Met Asn
                   85                  90                  95
   Gln Val Leu Asp Ala Tyr Glu Asn Lys Lys Pro Phe Tyr Leu Tyr Thr
               100                 105                 110
   Gly Arg Gly Pro Ser Ser Glu Ala Met His Val Gly His Leu Ile Pro
           115                 120                 125
   Phe Ile Phe Thr Lys Trp Leu Gln Asp Val Phe Asn Val Pro Leu Val
       130                 135                 140
   Ile Gln Met Thr Asp Asp Glu Lys Tyr Leu Trp Lys Asp Leu Thr Leu
   145                 150                 155                 160
   Asp Gln Ala Tyr Ser Asp Ala Val Glu Asn Ala Lys Asp Ile Ile Ala
                   165                 170                 175
   Cys Gly Phe Asp Ile Asn Lys Thr Phe Ile Phe Ser Asp Leu Asp Tyr
               180                 185                 190
   Met Gly Met Ser Ser Gly Phe Tyr Lys Asn Val Val Lys Ile Gln Lys
           195                 200                 205
   His Val Thr Phe Asn Gln Val Lys Gly Ile Phe Gly Phe Thr Asp Ser
       210                 215                 220
   Asp Cys Ile Gly Lys Ile Ser Phe Pro Ala Ile Gln Ala Ala Pro Ser
   225                 230                 235                 240
   Phe Ser Asn Ser Phe Pro Gln Ile Phe Arg Asp Arg Thr Asp Ile Gln
                   245                 250                 255
   Cys Leu Ile Pro Cys Ala Ile Asp Gln Asp Pro Tyr Phe Arg Met Thr
               260                 265                 270
   Arg Asp Val Ala Pro Arg Ile Gly Tyr Pro Lys Pro Ala Leu Leu His
           275                 280                 285
   Ser Thr Phe Phe Pro Ala Leu Gln Gly Ala Gln Thr Lys Met Ser Ala
       290                 295                 300
   Ser Asp Pro Asn Ser Ser Ile Phe Leu Thr Asp Thr Ala Lys Gln Ile
   305                 310                 315                 320
   Lys Thr Lys Val Asn Lys His Ala Phe Ser Gly Gly Arg Asp Thr Ile
                   325                 330                 335
   Glu Glu His Arg Gln Phe Gly Gly Asn Cys Asp Val Asp Val Ser Phe
               340                 345                 350
   Met Tyr Leu Thr Phe Phe Leu Glu Asp Asp Asp Lys Leu Glu Gln Ile
           355                 360                 365
   Arg Lys Asp Tyr Thr Ser Gly Ala Met Leu Thr Gly Glu Leu Lys Lys
       370                 375                 380
   Ala Leu Ile Glu Val Leu Gln Pro Leu Ile Ala Glu His Gln Ala Arg
   385                 390                 395                 400
   Arg Lys Glu Val Thr Asp Glu Ile Val Lys Glu Phe Met Thr Pro Arg
   405 410 415
   Lys Leu Ser Phe Asp Phe Gln
   420
<210> 51
   <211> 379
   <212> PRT
   <213> Artificial sequence
<220>
   <223> Recombinant human Met-T2 (SD variant) construct
<400> 51
   Met Ser Ala Lys Gly Ile Asp Tyr Asp Lys Leu Ile Val Arg Phe Gly
   1 5 10 15
   Ser Ser Lys Ile Asp Lys Glu Leu Ile Asn Arg Ile Glu Arg Ala Thr
   20 25 30
   Gly Gln Arg Pro His His Phe Leu Arg Arg Gly Ile Phe Phe Ser His
   35 40 45
   Arg Asp Met Asn Gln Val Leu Asp Ala Tyr Glu Asn Lys Lys Pro Phe
   50 55 60
   Tyr Leu Tyr Thr Gly Arg Gly Pro Ser Ser Glu Ala Met His Val Gly
   65 70 75 80
   His Leu Ile Pro Phe Ile Phe Thr Lys Trp Leu Gln Asp Val Phe Asn
   85 90 95
   Val Pro Leu Val Ile Gln Met Thr Asp Asp Glu Lys Tyr Leu Trp Lys
   100 105 110
   Asp Leu Thr Leu Asp Gln Ala Tyr Ser Asp Ala Val Glu Asn Ala Lys
   115 120 125
   Asp Ile Ile Ala Cys Gly Phe Asp Ile Asn Lys Thr Phe Ile Phe Ser
   130 135 140
   Asp Leu Asp Tyr Met Gly Met Ser Ser Gly Phe Tyr Lys Asn Val Val
   145 150 155 160
   Lys Ile Gln Lys His Val Thr Phe Asn Gln Val Lys Gly Ile Phe Gly
   165 170 175
   Phe Thr Asp Ser Asp Cys Ile Gly Lys Ile Ser Phe Pro Ala Ile Gln
   180 185 190
   Ala Ala Pro Ser Phe Ser Asn Ser Phe Pro Gln Ile Phe Arg Asp Arg
   195 200 205
   Thr Asp Ile Gln Cys Leu Ile Pro Cys Ala Ile Asp Gln Asp Pro Tyr
   210 215 220
   Phe Arg Met Thr Arg Asp Val Ala Pro Arg Ile Gly Tyr Pro Lys Pro
   225 230 235 240
   Ala Leu Leu His Ser Thr Phe Phe Pro Ala Leu Gln Gly Ala Gln Thr
   245 250 255
   Lys Met Ser Ala Ser Asp Pro Asn Ser Ser Ile Phe Leu Thr Asp Thr
   260 265 270
   Ala Lys Gln Ile Lys Thr Lys Val Asn Lys His Ala Phe Ser Gly Gly
           275                 280                 285
   Arg Asp Thr Ile Glu Glu His Arg Gln Phe Gly Gly Asn Cys Asp Val
       290                 295                 300
   Asp Val Ser Phe Met Tyr Leu Thr Phe Phe Leu Glu Asp Asp Asp Lys
   305                 310                 315                     320
   Leu Glu Gln Ile Arg Lys Asp Tyr Thr Ser Gly Ala Met Leu Thr Gly
                   325                 330                 335
   Glu Leu Lys Lys Ala Leu Ile Glu Val Leu Gln Pro Leu Ile Ala Glu
               340                 345                 350
   His Gln Ala Arg Arg Lys Glu Val Thr Asp Glu Ile Val Lys Glu Phe
           355                 360                 365
   Met Thr Pro Arg Lys Leu Ser Phe Asp Phe Gln
       370                 375
<210> 52
   <211> 402
   <212> PRT
   <213> Artificial sequence
<220>
   <223> Recombinant human Met-T1 (SD variant) construct
<400> 52
   Met Ser Asn His Gly Pro Asp Ala Thr Glu Ala Glu Glu Asp Phe Val
   1               5                   10                  15
   Asp Pro Trp Thr Val Gln Thr Ser Ser Ala Lys Gly Ile Asp Tyr Asp
               20                  25                  30
   Lys Leu Ile Val Arg Phe Gly Ser Ser Lys Ile Asp Lys Glu Leu Ile
           35                  40                  45
   Asn Arg Ile Glu Arg Ala Thr Gly Gln Arg Pro His His Phe Leu Arg
       50                  55                  60
   Arg Gly Ile Phe Phe Ser His Arg Asp Met Asn Gln Val Leu Asp Ala
   65                  70                  75                  80
   Tyr Glu Asn Lys Lys Pro Phe Tyr Leu Tyr Thr Gly Arg Gly Pro Ser
                   85                  90                  95
   Ser Glu Ala Met His Val Gly His Leu Ile Pro Phe Ile Phe Thr Lys
               100                 105                 110
   Trp Leu Gln Asp Val Phe Asn Val Pro Leu Val Ile Gln Met Thr Asp
           115                 120                 125
   Asp Glu Lys Tyr Leu Trp Lys Asp Leu Thr Leu Asp Gln Ala Tyr Ser
       130                 135                 140
   Asp Ala Val Glu Asn Ala Lys Asp Ile Ile Ala Cys Gly Phe Asp Ile
   145                 150                 155                 160
   Asn Lys Thr Phe Ile Phe Ser Asp Leu Asp Tyr Met Gly Met Ser Ser
                   165                 170                 175
   Gly Phe Tyr Lys Asn Val Val Lys Ile Gln Lys His Val Thr Phe Asn
               180                 185                 190
   Gln Val Lys Gly Ile Phe Gly Phe Thr Asp Ser Asp Cys Ile Gly Lys
   195 200 205
   Ile Ser Phe Pro Ala Ile Gln Ala Ala Pro Ser Phe Ser Asn Ser Phe
   210 215 220
   Pro Gln Ile Phe Arg Asp Arg Thr Asp Ile Gln Cys Leu Ile Pro Cys
   225 230 235 240
   Ala Ile Asp Gln Asp Pro Tyr Phe Arg Met Thr Arg Asp Val Ala Pro
   245 250 255
   Arg Ile Gly Tyr Pro Lys Pro Ala Leu Leu His Ser Thr Phe Phe Pro
   260 265 270
   Ala Leu Gln Gly Ala Gln Thr Lys Met Ser Ala Ser Asp Pro Asn Ser
   275 280 285
   Ser Ile Phe Leu Thr Asp Thr Ala Lys Gln Ile Lys Thr Lys Val Asn
   290 295 300
   Lys His Ala Phe Ser Gly Gly Arg Asp Thr Ile Glu Glu His Arg Gln
   305 310 315 320
   Phe Gly Gly Asn Cys Asp Val Asp Val Ser Phe Met Tyr Leu Thr Phe
   325 330 335
   Phe Leu Glu Asp Asp Asp Lys Leu Glu Gln Ile Arg Lys Asp Tyr Thr
   340 345 350
   Ser Gly Ala Met Leu Thr Gly Glu Leu Lys Lys Ala Leu Ile Glu Val
   355 360 365
   Leu Gln Pro Leu Ile Ala Glu His Gln Ala Arg Arg Lys Glu Val Thr
   370 375 380
   Asp Glu Ile Val Lys Glu Phe Met Thr Pro Arg Lys Leu Ser Phe Asp
   385 390 395 400
   Phe Gln
<210> 53
   <211> 424
   <212> PRT
   <213> Artificial sequence
<220>
   <223> Recombinant human Met-mini-TrpRS (SD variant) construct
<400> 53
   Met Ser Tyr Lys Ala Ala Ala Gly Glu Asp Tyr Lys Ala Asp Cys Pro
   1 5 10 15
   Pro Gly Asn Pro Ala Pro Thr Ser Asn His Gly Pro Asp Ala Thr Glu
   20 25 30
   Ala Glu Glu Asp Phe Val Asp Pro Trp Thr Val Gln Thr Ser Ser Ala
   35 40 45
   Lys Gly Ile Asp Tyr Asp Lys Leu Ile Val Arg Phe Gly Ser Ser Lys
   50 55 60
   Ile Asp Lys Glu Leu Ile Asn Arg Ile Glu Arg Ala Thr Gly Gln Arg
   65 70 75 80
   Pro His His Phe Leu Arg Arg Gly Ile Phe Phe Ser His Arg Asp Met
   85 90 95
   Asn Gln Val Leu Asp Ala Tyr Glu Asn Lys Lys Pro Phe Tyr Leu Tyr
   100 105 110
   Thr Gly Arg Gly Pro Ser Ser Glu Ala Met His Val Gly His Leu Ile
   115 120 125
   Pro Phe Ile Phe Thr Lys Trp Leu Gln Asp Val Phe Asn Val Pro Leu
   130 135 140
   Val Ile Gln Met Thr Asp Asp Glu Lys Tyr Leu Trp Lys Asp Leu Thr
   145 150 155 160
   Leu Asp Gln Ala Tyr Ser Asp Ala Val Glu Asn Ala Lys Asp Ile Ile
   165 170 175
   Ala Cys Gly Phe Asp Ile Asn Lys Thr Phe Ile Phe Ser Asp Leu Asp
   180 185 190
   Tyr Met Gly Met Ser Ser Gly Phe Tyr Lys Asn Val Val Lys Ile Gln
   195 200 205
   Lys His Val Thr Phe Asn Gln Val Lys Gly Ile Phe Gly Phe Thr Asp
   210 215 220
   Ser Asp Cys Ile Gly Lys Ile Ser Phe Pro Ala Ile Gln Ala Ala Pro
   225 230 235 240
   Ser Phe Ser Asn Ser Phe Pro Gln Ile Phe Arg Asp Arg Thr Asp Ile
   245 250 255
   Gln Cys Leu Ile Pro Cys Ala Ile Asp Gln Asp Pro Tyr Phe Arg Met
   260 265 270
   Thr Arg Asp Val Ala Pro Arg Ile Gly Tyr Pro Lys Pro Ala Leu Leu
   275 280 285
   His Ser Thr Phe Phe Pro Ala Leu Gln Gly Ala Gln Thr Lys Met Ser
   290 295 300
   Ala Ser Asp Pro Asn Ser Ser Ile Phe Leu Thr Asp Thr Ala Lys Gln
   305 310 315 320
   Ile Lys Thr Lys Val Asn Lys His Ala Phe Ser Gly Gly Arg Asp Thr
   325 330 335
   Ile Glu Glu His Arg Gln Phe Gly Gly Asn Cys Asp Val Asp Val Ser
   340 345 350
   Phe Met Tyr Leu Thr Phe Phe Leu Glu Asp Asp Asp Lys Leu Glu Gln
   355 360 365
   Ile Arg Lys Asp Tyr Thr Ser Gly Ala Met Leu Thr Gly Glu Leu Lys
   370 375 380
   Lys Ala Leu Ile Glu Val Leu Gln Pro Leu Ile Ala Glu His Gln Ala
   385 390 395 400
   Arg Arg Lys Glu Val Thr Asp Glu Ile Val Lys Glu Phe Met Thr Pro
   405 410 415
   Arg Lys Leu Ser Phe Asp Phe Gln
   420
<210> 54
   <211> 1134
   <212> DNA
   <213> Artificial sequence
<220>
   <223> Recombinant human T2 (SD variant) construct
<220>
   <221> CDS
   <222> (1)..(1134)
<220>
   <221> misc_feature
   <222> (358)..(360)
   <223> nnn is agy, tcy or tcr
<400> 54
   agt gca aaa ggc ata gac tac gat aag ctc att gtt cgg ttt gga agt    48
   Ser Ala Lys Gly Ile Asp Tyr Asp Lys Leu Ile Val Arg Phe Gly Ser
   1               5                   10                  15
   agt aaa att gac aaa gag cta ata aac cga ata gag aga gcc acc ggc    96
   Ser Lys Ile Asp Lys Glu Leu Ile Asn Arg Ile Glu Arg Ala Thr Gly
               20                  25                  30
   caa aga cca cac cac ttc ctg cgc aga ggc atc ttc ttc tca cac aga    144
   Gln Arg Pro His His Phe Leu Arg Arg Gly Ile Phe Phe Ser His Arg
           35                  40                  45
   gat atg aat cag gtt ctt gat gcc tat gaa aat aag aag cca ttt tat    192
   Asp Met Asn Gln Val Leu Asp Ala Tyr Glu Asn Lys Lys Pro Phe Tyr
       50                  55                  60
   ctg tac acg ggc cgg ggc ccc tct tct gaa gca atg cat gta ggt cac    240
   Leu Tyr Thr Gly Arg Gly Pro Ser Ser Glu Ala Met His Val Gly His
   65                  70                  75                  80
   ctc att cca ttt att ttc aca aag tgg ctc cag gat gta ttt aac gtg    288
   Leu Ile Pro Phe Ile Phe Thr Lys Trp Leu Gln Asp Val Phe Asn Val
                   85                  90                  95
   ccc ttg gtc atc cag atg acg gat gac gag aag tat ctg tgg aag gac    336
   Pro Leu Val Ile Gln Met Thr Asp Asp Glu Lys Tyr Leu Trp Lys Asp
               100                 105                 110
   ctg acc ctg gac cag gcc tat nnn gay gct gtt gag aat gcc aag gac    384
   Leu Thr Leu Asp Gln Ala Tyr Ser Asp Ala Val Glu Asn Ala Lys Asp
           115                 120                 125
   atc atc gcc tgt ggc ttt gac atc aac aag act ttc ata ttc tct gac    432
   Ile Ile Ala Cys Gly Phe Asp Ile Asn Lys Thr Phe Ile Phe Ser Asp
       130                 135                 140
   ctg gac tac atg ggg atg agc tca ggt ttc tac aaa aat gtg gtg aag    480
   Leu Asp Tyr Met Gly Met Ser Ser Gly Phe Tyr Lys Asn Val Val Lys
   145                 150                 155                 160
   att caa aag cat gtt acc ttc aac caa gtg aaa ggc att ttc ggc ttc    528
   Ile Gln Lys His Val Thr Phe Asn Gln Val Lys Gly Ile Phe Gly Phe
                   165                 170                 175
   act gac agc gac tgc att ggg aag atc agt ttt cct gcc atc cag gct    576
   Thr Asp Ser Asp Cys Ile Gly Lys Ile Ser Phe Pro Ala Ile Gln Ala
               180                 185                 190
   gct ccc tcc ttc agc aac tca ttc cca cag atc ttc cga gac agg acg      624
   Ala Pro Ser Phe Ser Asn Ser Phe Pro Gln Ile Phe Arg Asp Arg Thr
           195                 200                 205
   gat atc cag tgc ctt atc cca tgt gcc att gac cag gat cct tac ttt      672
   Asp Ile Gln Cys Leu Ile Pro Cys Ala Ile Asp Gln Asp Pro Tyr Phe
       210                 215                 220
   aga atg aca agg gac gtc gcc ccc agg atc ggc tat cct aaa cca gcc      720
   Arg Met Thr Arg Asp Val Ala Pro Arg Ile Gly Tyr Pro Lys Pro Ala
   225                 230                 235                 240
   ctg ttg cac tcc acc ttc ttc cca gcc ctg cag ggc gcc cag acc aaa      768
   Leu Leu His Ser Thr Phe Phe Pro Ala Leu Gln Gly Ala Gln Thr Lys
                   245                 250                 255
   atg agt gcc agc gac cca aac tcc tcc atc ttc ctc acc gac acg gcc      816
   Met Ser Ala Ser Asp Pro Asn Ser Ser Ile Phe Leu Thr Asp Thr Ala
               260                 265                 270
   aag cag atc aaa acc aag gtc aat aag cat gcg ttt tct gga ggg aga      864
   Lys Gln Ile Lys Thr Lys Val Asn Lys His Ala Phe Ser Gly Gly Arg
           275                 280                 285
   gac acc atc gag gag cac agg cag ttt ggg ggc aac tgt gat gtg gac      912
   Asp Thr Ile Glu Glu His Arg Gln Phe Gly Gly Asn Cys Asp Val Asp
       290                 295                 300
   gtg tct ttc atg tac ctg acc ttc ttc ctc gag gac gac gac aag ctc      960
   Val Ser Phe Met Tyr Leu Thr Phe Phe Leu Glu Asp Asp Asp Lys Leu
   305                 310                 315                 320
   gag cag atc agg aag gat tac acc agc gga gcc atg ctc acc ggt gag      1008
   Glu Gln Ile Arg Lys Asp Tyr Thr Ser Gly Ala Met Leu Thr Gly Glu
                   325                 330                 335
   ctc aag aag gca ctc ata gag gtt ctg cag ccc ttg atc gca gag cac      1056
   Leu Lys Lys Ala Leu Ile Glu Val Leu Gln Pro Leu Ile Ala Glu His
               340                 345                 350
   cag gcc cgg cgc aag gag gtc acg gat gag ata gtg aaa gag ttc atg      1104
   Gln Ala Arg Arg Lys Glu Val Thr Asp Glu Ile Val Lys Glu Phe Met
           355                 360                 365
   act ccc cgg aag ctg tcc ttc gac ttt cag                              1134
   Thr Pro Arg Lys Leu Ser Phe Asp Phe Gln
       370                 375
<210> 55
   <211> 1137
   <212> DNA
   <213> Artificial Sequence
<220>
   <223> Recombinant human Met-T2 (SD variant) construct
<220>
   <221> CDS
   <222> (1)..(1137)
<220>
   <221> misc_feature
   <222> (361)..(363)
   <223> nnn is agy, tcy or tcr
<400> 55
   atg agt gca aaa ggc ata gac tac gat aag ctc att gtt cgg ttt gga     48
   Met Ser Ala Lys Gly Ile Asp Tyr Asp Lys Leu Ile Val Arg Phe Gly
    1               5                  10                  15
   agt agt aaa att gac aaa gag cta ata aac cga ata gag aga gcc acc     96
   Ser Ser Lys Ile Asp Lys Glu Leu Ile Asn Arg Ile Glu Arg Ala Thr
               20                  25                  30
   ggc caa aga cca cac cac ttc ctg cgc aga ggc atc ttc ttc tca cac     144
   Gly Gln Arg Pro His His Phe Leu Arg Arg Gly Ile Phe Phe Ser His
           35                  40                  45
   aga gat atg aat cag gtt ctt gat gcc tat gaa aat aag aag cca ttt     192
   Arg Asp Met Asn Gln Val Leu Asp Ala Tyr Glu Asn Lys Lys Pro Phe
       50                  55                  60
   tat ctg tac acg ggc cgg ggc ccc tct tct gaa gca atg cat gta ggt     240
   Tyr Leu Tyr Thr Gly Arg Gly Pro Ser Ser Glu Ala Met His Val Gly
   65                  70                  75                  80
   cac ctc att cca ttt att ttc aca aag tgg ctc cag gat gta ttt aac     288
   His Leu Ile Pro Phe Ile Phe Thr Lys Trp Leu Gln Asp Val Phe Asn
                   85                  90                  95
   gtg ccc ttg gtc atc cag atg acg gat gac gag aag tat ctg tgg aag     336
   Val Pro Leu Val Ile Gln Met Thr Asp Asp Glu Lys Tyr Leu Trp Lys
               100                 105                 110
   gac ctg acc ctg gac cag gcc tat nnn gay gct gtt gag aat gcc aag     384
   Asp Leu Thr Leu Asp Gln Ala Tyr Ser Asp Ala Val Glu Asn Ala Lys
           115                 120                 125
   gac atc atc gcc tgt ggc ttt gac atc aac aag act ttc ata ttc tct     432
   Asp Ile Ile Ala Cys Gly Phe Asp Ile Asn Lys Thr Phe Ile Phe Ser
       130                 135                 140
   gac ctg gac tac atg ggg atg agc tca ggt ttc tac aaa aat gtg gtg     480
   Asp Leu Asp Tyr Met Gly Met Ser Ser Gly Phe Tyr Lys Asn Val Val
   145                 150                 155                 160
   aag att caa aag cat gtt acc ttc aac caa gtg aaa ggc att ttc ggc     528
   Lys Ile Gln Lys His Val Thr Phe Asn Gln Val Lys Gly Ile Phe Gly
                   165                 170                 175
   ttc act gac agc gac tgc att ggg aag atc agt ttt cct gcc atc cag     576
   Phe Thr Asp Ser Asp Cys Ile Gly Lys Ile Ser Phe Pro Ala Ile Gln
               180                 185                 190
   gct gct ccc tcc ttc agc aac tca ttc cca cag atc ttc cga gac agg     624
   Ala Ala Pro Ser Phe Ser Asn Ser Phe Pro Gln Ile Phe Arg Asp Arg
           195                 200                 205
   acg gat atc cag tgc ctt atc cca tgt gcc att gac cag gat cct tac     672
   Thr Asp Ile Gln Cys Leu Ile Pro Cys Ala Ile Asp Gln Asp Pro Tyr
       210                 215                 220
   ttt aga atg aca agg gac gtc gcc ccc agg atc ggc tat cct aaa cca     720
   Phe Arg Met Thr Arg Asp Val Ala Pro Arg Ile Gly Tyr Pro Lys Pro
   225                 230                 235                 240
   gcc ctg ttg cac tcc acc ttc ttc cca gcc ctg cag ggc gcc cag acc     768
   Ala Leu Leu His Ser Thr Phe Phe Pro Ala Leu Gln Gly Ala Gln Thr
                   245                 250                 255
   aaa atg agt gcc agc gac cca aac tcc tcc atc ttc ctc acc gac acg    816
   Lys Met Ser Ala Ser Asp Pro Asn Ser Ser Ile Phe Leu Thr Asp Thr
               260                 265                 270
   gcc aag cag atc aaa acc aag gtc aat aag cat gcg ttt tct gga ggg    864
   Ala Lys Gln Ile Lys Thr Lys Val Asn Lys His Ala Phe Ser Gly Gly
           275                 280                 285
   aga gac acc atc gag gag cac agg cag ttt ggg ggc aac tgt gat gtg    912
   Arg Asp Thr Ile Glu Glu His Arg Gln Phe Gly Gly Asn Cys Asp Val
       290                 295                 300
   gac gtg tct ttc atg tac ctg acc ttc ttc ctc gag gac gac gac aag    960
   Asp Val Ser Phe Met Tyr Leu Thr Phe Phe Leu Glu Asp Asp Asp Lys
   305                 310                 315                 320
   ctc gag cag atc agg aag gat tac acc agc gga gcc atg ctc acc ggt    1008
   Leu Glu Gln Ile Arg Lys Asp Tyr Thr Ser Gly Ala Met Leu Thr Gly
                   325                 330                 335
   gag ctc aag aag gca ctc ata gag gtt ctg cag ccc ttg atc gca gag    1056
   Glu Leu Lys Lys Ala Leu Ile Glu Val Leu Gln Pro Leu Ile Ala Glu
               340                 345                 350
   cac cag gcc cgg cgc aag gag gtc acg gat gag ata gtg aaa gag ttc    1104
   His Gln Ala Arg Arg Lys Glu Val Thr Asp Glu Ile Val Lys Glu Phe
           355                 360                 365
   atg act ccc cgg aag ctg tcc ttc gac ttt cag                        1137
   Met Thr Pro Arg Lys Leu Ser Phe Asp Phe Gln
       370                 375
<210> 56
   <211> 1203
   <212> DNA
   <213> Artificial Sequence
<220>
   <223> Recombinant human T1 (SD variant) construct
<220>
   <221> CDS
   <222> (1)..(1203)
<220>
   <221> misc_feature
   <222> (427)..(429)
   <223> nnn is agy, tcy or tcr
<400> 56
   agt aat cat ggc cca gat gcc aca gaa gct gaa gag gat ttt gtg gac    48
   Ser Asn His Gly Pro Asp Ala Thr Glu Ala Glu Glu Asp Phe Val Asp
   1               5                   10                  15
   cca tgg aca gta cag aca agc agt gca aaa ggc ata gac tac gat aag    96
   Pro Trp Thr Val Gln Thr Ser Ser Ala Lys Gly Ile Asp Tyr Asp Lys
               20                  25                  30
   ctc att gtt cgg ttt gga agt agt aaa att gac aaa gag cta ata aac    144
   Leu Ile Val Arg Phe Gly Ser Ser Lys Ile Asp Lys Glu Leu Ile Asn
           35                  40                  45
   cga ata gag aga gcc acc ggc caa aga cca cac cac ttc ctg cgc aga    192
   Arg Ile Glu Arg Ala Thr Gly Gln Arg Pro His His Phe Leu Arg Arg
   50 55 60
   ggc atc ttc ttc tca cac aga gat atg aat cag gtt ctt gat gcc tat 240
   Gly Ile Phe Phe Ser His Arg Asp Met Asn Gln Val Leu Asp Ala Tyr
   65 70 75 80
   gaa aat aag aag cca ttt tat ctg tac acg ggc cgg ggc ccc tct tct 288
   Glu Asn Lys Lys Pro Phe Tyr Leu Tyr Thr Gly Arg Gly Pro Ser Ser
   85 90 95
   gaa gca atg cat gta ggt cac ctc att cca ttt att ttc aca aag tgg 336
   Glu Ala Met His Val Gly His Leu Ile Pro Phe Ile Phe Thr Lys Trp
   100 105 110
   ctc cag gat gta ttt aac gtg ccc ttg gtc atc cag atg acg gat gac 384
   Leu Gln Asp Val Phe Asn Val Pro Leu Val Ile Gln Met Thr Asp Asp
   115 120 125
   gag aag tat ctg tgg aag gac ctg acc ctg gac cag gcc tat nnn gay 432
   Glu Lys Tyr Leu Trp Lys Asp Leu Thr Leu Asp Gln Ala Tyr Ser Asp
   130 135 140
   gct gtt gag aat gcc aag gac atc atc gcc tgt ggc ttt gac atc aac 480
   Ala Val Glu Asn Ala Lys Asp Ile Ile Ala Cys Gly Phe Asp Ile Asn
   145 150 155 160
   aag act ttc ata ttc tct gac ctg gac tac atg ggg atg agc tca ggt 528
   Lys Thr Phe Ile Phe Ser Asp Leu Asp Tyr Met Gly Met Ser Ser Gly
   165 170 175
   ttc tac aaa aat gtg gtg aag att caa aag cat gtt acc ttc aac caa 576
   Phe Tyr Lys Asn Val Val Lys Ile Gln Lys His Val Thr Phe Asn Gln
   180 185 190
   gtg aaa ggc att ttc ggc ttc act gac agc gac tgc att ggg aag atc 624
   Val Lys Gly Ile Phe Gly Phe Thr Asp Ser Asp Cys Ile Gly Lys Ile
   195 200 205
   agt ttt cct gcc atc cag gct gct ccc tcc ttc agc aac tca ttc cca 672
   Ser Phe Pro Ala Ile Gln Ala Ala Pro Ser Phe Ser Asn Ser Phe Pro
   210 215 220
   cag atc ttc cga gac agg acg gat atc cag tgc ctt atc cca tgt gcc 720
   Gln Ile Phe Arg Asp Arg Thr Asp Ile Gln Cys Leu Ile Pro Cys Ala
   225 230 235 240
   att gac cag gat cct tac ttt aga atg aca agg gac gtc gcc ccc agg 768
   Ile Asp Gln Asp Pro Tyr Phe Arg Met Thr Arg Asp Val Ala Pro Arg
   245 250 255
   atc ggc tat cct aaa cca gcc ctg ttg cac tcc acc ttc ttc cca gcc 816
   Ile Gly Tyr Pro Lys Pro Ala Leu Leu His Ser Thr Phe Phe Pro Ala
   260 265 270
   ctg cag ggc gcc cag acc aaa atg agt gcc agc gac cca aac tcc tcc 864
   Leu Gln Gly Ala Gln Thr Lys Met Ser Ala Ser Asp Pro Asn Ser Ser
   275 280 285
   atc ttc ctc acc gac acg gcc aag cag atc aaa acc aag gtc aat aag 912
   Ile Phe Leu Thr Asp Thr Ala Lys Gln Ile Lys Thr Lys Val Asn Lys
   290 295 300
   cat gcg ttt tct gga ggg aga gac acc atc gag gag cac agg cag ttt 960
   His Ala Phe Ser Gly Gly Arg Asp Thr Ile Glu Glu His Arg Gln Phe
   305 310 315 320
   ggg ggc aac tgt gat gtg gac gtg tct ttc atg tac ctg acc ttc ttc      1008
   Gly Gly Asn Cys Asp Val Asp Val Ser Phe Met Tyr Leu Thr Phe Phe
                   325                 330                 335
   ctc gag gac gac gac aag ctc gag cag atc agg aag gat tac acc agc      1056
   Leu Glu Asp Asp Asp Lys Leu Glu Gln Ile Arg Lys Asp Tyr Thr Ser
               340                 345                 350
   gga gcc atg ctc acc ggt gag ctc aag aag gca ctc ata gag gtt ctg      1104
   Gly Ala Met Leu Thr Gly Glu Leu Lys Lys Ala Leu Ile Glu Val Leu
           355                 360                 365
   cag ccc ttg atc gca gag cac cag gcc cgg cgc aag gag gtc acg gat      1152
   Gln Pro Leu Ile Ala Glu His Gln Ala Arg Arg Lys Glu Val Thr Asp
       370                 375                 380
   gag ata gtg aaa gag ttc atg act ccc cgg aag ctg tcc ttc gac ttt      1200
   Glu Ile Val Lys Glu Phe Met Thr Pro Arg Lys Leu Ser Phe Asp Phe
   385                 390                 395                 400
   cag                                                                  1203
   Gln
<210> 57
   <211> 1206
   <212> DNA
   <213> Artificial Sequence
<220>
   <223> Recombinant human Met-T1 (SD variant) construct
<220>
   <221> CDS
   <222> (1)..(1206)
<220>
   <221> misc_feature
   <222> (430)..(432)
   <223> nnn is agy, tcy or tcr
<400> 57
   atg agt aat cat ggc cca gat gcc aca gaa gct gaa gag gat ttt gtg      48
   Met Ser Asn His Gly Pro Asp Ala Thr Glu Ala Glu Glu Asp Phe Val
   1               5                   10                  15
   gac cca tgg aca gta cag aca agc agt gca aaa ggc ata gac tac gat      96
   Asp Pro Trp Thr Val Gln Thr Ser Ser Ala Lys Gly Ile Asp Tyr Asp
               20                  25                  30
   aag ctc att gtt cgg ttt gga agt agt aaa att gac aaa gag cta ata      144
   Lys Leu Ile Val Arg Phe Gly Ser Ser Lys Ile Asp Lys Glu Leu Ile
           35                  40                  45
   aac cga ata gag aga gcc acc ggc caa aga cca cac cac ttc ctg cgc      192
   Asn Arg Ile Glu Arg Ala Thr Gly Gln Arg Pro His His Phe Leu Arg
       50                  55                  60
   aga ggc atc ttc ttc tca cac aga gat atg aat cag gtt ctt gat gcc      240
   Arg Gly Ile Phe Phe Ser His Arg Asp Met Asn Gln Val Leu Asp Ala
   65                  70                  75                  80
   tat gaa aat aag aag cca ttt tat ctg tac acg ggc cgg ggc ccc tct      288
   Tyr Glu Asn Lys Lys Pro Phe Tyr Leu Tyr Thr Gly Arg Gly Pro Ser
                   85                  90                  95
   tct gaa gca atg cat gta ggt cac ctc att cca ttt att ttc aca aag    336
   Ser Glu Ala Met His Val Gly His Leu Ile Pro Phe Ile Phe Thr Lys
               100                 105                 110
   tgg ctc cag gat gta ttt aac gtg ccc ttg gtc atc cag atg acg gat    384
   Trp Leu Gln Asp Val Phe Asn Val Pro Leu Val Ile Gln Met Thr Asp
           115                 120                 125
   gac gag aag tat ctg tgg aag gac ctg acc ctg gac cag gcc tat nnn    432
   Asp Glu Lys Tyr Leu Trp Lys Asp Leu Thr Leu Asp Gln Ala Tyr Ser
       130                 135                 140
   gay gct gtt gag aat gcc aag gac atc atc gcc tgt ggc ttt gac atc    480
   Asp Ala Val Glu Asn Ala Lys Asp Ile Ile Ala Cys Gly Phe Asp Ile
   145                 150                 155                 160
   aac aag act ttc ata ttc tct gac ctg gac tac atg ggg atg agc tca    528
   Asn Lys Thr Phe Ile Phe Ser Asp Leu Asp Tyr Met Gly Met Ser Ser
                   165                 170                 175
   ggt ttc tac aaa aat gtg gtg aag att caa aag cat gtt acc ttc aac    576
   Gly Phe Tyr Lys Asn Val Val Lys Ile Gln Lys His Val Thr Phe Asn
               180                 185                 190
   caa gtg aaa ggc att ttc ggc ttc act gac agc gac tgc att ggg aag    624
   Gln Val Lys Gly Ile Phe Gly Phe Thr Asp Ser Asp Cys Ile Gly Lys
           195                 200                 205
   atc agt ttt cct gcc atc cag gct gct ccc tcc ttc agc aac tca ttc    672
   Ile Ser Phe Pro Ala Ile Gln Ala Ala Pro Ser Phe Ser Asn Ser Phe
       210                 215                 220
   cca cag atc ttc cga gac agg acg gat atc cag tgc ctt atc cca tgt    720
   Pro Gln Ile Phe Arg Asp Arg Thr Asp Ile Gln Cys Leu Ile Pro Cys
   225                 230                 235                 240
   gcc att gac cag gat cct tac ttt aga atg aca agg gac gtc gcc ccc    768
   Ala Ile Asp Gln Asp Pro Tyr Phe Arg Met Thr Arg Asp Val Ala Pro
                   245                 250                 255
   agg atc ggc tat cct aaa cca gcc ctg ttg cac tcc acc ttc ttc cca    816
   Arg Ile Gly Tyr Pro Lys Pro Ala Leu Leu His Ser Thr Phe Phe Pro
               260                 265                 270
   gcc ctg cag ggc gcc cag acc aaa atg agt gcc agc gac cca aac tcc    864
   Ala Leu Gln Gly Ala Gln Thr Lys Met Ser Ala Ser Asp Pro Asn Ser
           275                 280                 285
   tcc atc ttc ctc acc gac acg gcc aag cag atc aaa acc aag gtc aat    912
   Ser Ile Phe Leu Thr Asp Thr Ala Lys Gln Ile Lys Thr Lys Val Asn
       290                 295                 300
   aag cat gcg ttt tct gga ggg aga gac acc atc gag gag cac agg cag    960
   Lys His Ala Phe Ser Gly Gly Arg Asp Thr Ile Glu Glu His Arg Gln
   305                 310                 315                 320
   ttt ggg ggc aac tgt gat gtg gac gtg tct ttc atg tac ctg acc ttc    1008
   Phe Gly Gly Asn Cys Asp Val Asp Val Ser Phe Met Tyr Leu Thr Phe
                   325                 330                 335
   ttc ctc gag gac gac gac aag ctc gag cag atc agg aag gat tac acc    1056
   Phe Leu Glu Asp Asp Asp Lys Leu Glu Gln Ile Arg Lys Asp Tyr Thr
               340                 345                 350
   agc gga gcc atg ctc acc ggt gag ctc aag aag gca ctc ata gag gtt    1104
   Ser Gly Ala Met Leu Thr Gly Glu Leu Lys Lys Ala Leu Ile Glu Val
   355 360 365
   ctg cag ccc ttg atc gca gag cac cag gcc cgg cgc aag gag gtc acg 1152
   Leu Gln Pro Leu Ile Ala Glu His Gln Ala Arg Arg Lys Glu Val Thr
   370 375 380
   gat gag ata gtg aaa gag ttc atg act ccc cgg aag ctg tcc ttc gac 1200
   Asp Glu Ile Val Lys Glu Phe Met Thr Pro Arg Lys Leu Ser Phe Asp
   385 390 395 400
   ttt cag 1206
   Phe Gln
<210> 58
   <211> 1269
   <212> DNA
   <213> Artificial Sequence
<220>
   <223> Recombinant human mini-TrpRS (SD variant) construct
<220>
   <221> CDS
   <222> (1)..(1269)
<220>
   <221> misc_feature
   <222> (493)..(495)
   <223> nnn is agy, tcy or tcr
<400> 58
   agc tac aaa gct gcc gcg ggg gag gat tac aag gct gac tgt cct cca 48
   Ser Tyr Lys Ala Ala Ala Gly Glu Asp Tyr Lys Ala Asp Cys Pro Pro
   1 5 10 15
   ggg aac cca gca cct acc agt aat cat ggc cca gat gcc aca gaa gct 96
   Gly Asn Pro Ala Pro Thr Ser Asn His Gly Pro Asp Ala Thr Glu Ala
   20 25 30
   gaa gag gat ttt gtg gac cca tgg aca gta cag aca agc agt gca aaa 144
   Glu Glu Asp Phe Val Asp Pro Trp Thr Val Gln Thr Ser Ser Ala Lys
   35 40 45
   ggc ata gac tac gat aag ctc att gtt cgg ttt gga agt agt aaa att 192
   Gly Ile Asp Tyr Asp Lys Leu Ile Val Arg Phe Gly Ser Ser Lys Ile
   50 55 60
   gac aaa gag cta ata aac cga ata gag aga gcc acc ggc caa aga cca 240
   Asp Lys Glu Leu Ile Asn Arg Ile Glu Arg Ala Thr Gly Gln Arg Pro
   65 70 75 80
   cac cac ttc ctg cgc aga ggc atc ttc ttc tca cac aga gat atg aat 288
   His His Phe Leu Arg Arg Gly Ile Phe Phe Ser His Arg Asp Met Asn
   85 90 95
   cag gtt ctt gat gcc tat gaa aat aag aag cca ttt tat ctg tac acg 336
   Gln Val Leu Asp Ala Tyr Glu Asn Lys Lys Pro Phe Tyr Leu Tyr Thr
   100 105 110
   ggc cgg ggc ccc tct tct gaa gca atg cat gta ggt cac ctc att cca 384
   Gly Arg Gly Pro Ser Ser Glu Ala Met His Val Gly His Leu Ile Pro
   115 120 125
   ttt att ttc aca aag tgg ctc cag gat gta ttt aac gtg ccc ttg gtc 432
   Phe Ile Phe Thr Lys Trp Leu Gln Asp Val Phe Asn Val Pro Leu Val
       130                     135                     140
   atc cag atg acg gat gac gag aag tat ctg tgg aag gac ctg acc ctg     480
   Ile Gln Met Thr Asp Asp Glu Lys Tyr Leu Trp Lys Asp Leu Thr Leu
   145                 150                 155                 160
   gac cag gcc tat nnn gay gct gtt gag aat gcc aag gac atc atc gcc     528
   Asp Gln Ala Tyr Ser Asp Ala Val Glu Asn Ala Lys Asp Ile Ile Ala
                   165                 170                 175
   tgt ggc ttt gac atc aac aag act ttc ata ttc tct gac ctg gac tac     576
   Cys Gly Phe Asp Ile Asn Lys Thr Phe Ile Phe Ser Asp Leu Asp Tyr
               180                 185                 190
   atg ggg atg agc tca ggt ttc tac aaa aat gtg gtg aag att caa aag     624
   Met Gly Met Ser Ser Gly Phe Tyr Lys Asn Val Val Lys Ile Gln Lys
           195                 200                 205
   cat gtt acc ttc aac caa gtg aaa ggc att ttc ggc ttc act gac agc     672
   His Val Thr Phe Asn Gln Val Lys Gly Ile Phe Gly Phe Thr Asp Ser
       210                 215                 220
   gac tgc att ggg aag atc agt ttt cct gcc atc cag gct gct ccc tcc     720
   Asp Cys Ile Gly Lys Ile Ser Phe Pro Ala Ile Gln Ala Ala Pro Ser
   225                 230                 235                 240
   ttc agc aac tca ttc cca cag atc ttc cga gac agg acg gat atc cag     768
   Phe Ser Asn Ser Phe Pro Gln Ile Phe Arg Asp Arg Thr Asp Ile Gln
                   245                 250                 255
   tgc ctt atc cca tgt gcc att gac cag gat cct tac ttt aga atg aca     816
   Cys Leu Ile Pro Cys Ala Ile Asp Gln Asp Pro Tyr Phe Arg Met Thr
               260                 265                 270
   agg gac gtc gcc ccc agg atc ggc tat cct aaa cca gcc ctg ttg cac     864
   Arg Asp Val Ala Pro Arg Ile Gly Tyr Pro Lys Pro Ala Leu Leu His
           275                 280                 285
   tcc acc ttc ttc cca gcc ctg cag ggc gcc cag acc aaa atg agt gcc     912
   Ser Thr Phe Phe Pro Ala Leu Gln Gly Ala Gln Thr Lys Met Ser Ala
       290                 295                 300
   agc gac cca aac tcc tcc atc ttc ctc acc gac acg gcc aag cag atc     960
   Ser Asp Pro Asn Ser Ser Ile Phe Leu Thr Asp Thr Ala Lys Gln Ile
   305                 310                 315                 320
   aaa acc aag gtc aat aag cat gcg ttt tct gga ggg aga gac acc atc    1008
   Lys Thr Lys Val Asn Lys His Ala Phe Ser Gly Gly Arg Asp Thr Ile
                   325                 330                 335
   gag gag cac agg cag ttt ggg ggc aac tgt gat gtg gac gtg tct ttc    1056
   Glu Glu His Arg Gln Phe Gly Gly Asn Cys Asp Val Asp Val Ser Phe
               340                 345                 350
   atg tac ctg acc ttc ttc ctc gag gac gac gac aag ctc gag cag atc    1104
   Met Tyr Leu Thr Phe Phe Leu Glu Asp Asp Asp Lys Leu Glu Gln Ile
           355                 360                 365
   agg aag gat tac acc agc gga gcc atg ctc acc ggt gag ctc aag aag    1152
   Arg Lys Asp Tyr Thr Ser Gly Ala Met Leu Thr Gly Glu Leu Lys Lys
       370                 375                 380
   gca ctc ata gag gtt ctg cag ccc ttg atc gca gag cac cag gcc cgg    1200
   Ala Leu Ile Glu Val Leu Gln Pro Leu Ile Ala Glu His Gln Ala Arg
   385                 390                 395                 400
   cgc aag gag gtc acg gat gag ata gtg aaa gag ttc atg act ccc cgg    1248
   Arg Lys Glu Val Thr Asp Glu Ile Val Lys Glu Phe Met Thr Pro Arg
                   405                 410                 415
   aag ctg tcc ttc gac ttt cag                                        1269
   Lys Leu Ser Phe Asp Phe Gln
               420
<210> 59
   <211> 1272
   <212> DNA
   <213> Artificial Sequence
<220>
   <223> Recombinant human Met-mini-TrpRS (SD variant) construct
<220>
   <221> CDS
   <222> (1)..(1272)
<220>
   <221> misc_feature
   <222> (496)..(498)
   <223> nnn is agy, tcy or tcr
<400> 59
   atg agc tac aaa gct gcc gcg ggg gag gat tac aag gct gac tgt cct    48
   Met Ser Tyr Lys Ala Ala Ala Gly Glu Asp Tyr Lys Ala Asp Cys Pro
   1               5                   10                  15
   cca ggg aac cca gca cct acc agt aat cat ggc cca gat gcc aca gaa    96
   Pro Gly Asn Pro Ala Pro Thr Ser Asn His Gly Pro Asp Ala Thr Glu
               20                  25                  30
   gct gaa gag gat ttt gtg gac cca tgg aca gta cag aca agc agt gca    144
   Ala Glu Glu Asp Phe Val Asp Pro Trp Thr Val Gln Thr Ser Ser Ala
           35                  40                  45
   aaa ggc ata gac tac gat aag ctc att gtt cgg ttt gga agt agt aaa    192
   Lys Gly Ile Asp Tyr Asp Lys Leu Ile Val Arg Phe Gly Ser Ser Lys
       50                  55                  60
   att gac aaa gag cta ata aac cga ata gag aga gcc acc ggc caa aga    240
   Ile Asp Lys Glu Leu Ile Asn Arg Ile Glu Arg Ala Thr Gly Gln Arg
   65                  70                  75                  80
   cca cac cac ttc ctg cgc aga ggc atc ttc ttc tca cac aga gat atg    288
   Pro His His Phe Leu Arg Arg Gly Ile Phe Phe Ser His Arg Asp Met
                   85                  90                  95
   aat cag gtt ctt gat gcc tat gaa aat aag aag cca ttt tat ctg tac    336
   Asn Gln Val Leu Asp Ala Tyr Glu Asn Lys Lys Pro Phe Tyr Leu Tyr
               100                 105                 110
   acg ggc cgg ggc ccc tct tct gaa gca atg cat gta ggt cac ctc att    384
   Thr Gly Arg Gly Pro Ser Ser Glu Ala Met His Val Gly His Leu Ile
           115                 120                 125
   cca ttt att ttc aca aag tgg ctc cag gat gta ttt aac gtg ccc ttg    432
   Pro Phe Ile Phe Thr Lys Trp Leu Gln Asp Val Phe Asn Val Pro Leu
       130                 135                 140
   gtc atc cag atg acg gat gac gag aag tat ctg tgg aag gac ctg acc    480
   Val Ile Gln Met Thr Asp Asp Glu Lys Tyr Leu Trp Lys Asp Leu Thr
   145                 150                 155                 160
   ctg gac cag gcc tat nnn gay gct gtt gag aat gcc aag gac atc atc     528
   Leu Asp Gln Ala Tyr Ser Asp Ala Val Glu Asn Ala Lys Asp Ile Ile
                   165                 170                 175
   gcc tgt ggc ttt gac atc aac aag act ttc ata ttc tct gac ctg gac     576
   Ala Cys Gly Phe Asp Ile Asn Lys Thr Phe Ile Phe Ser Asp Leu Asp
               180                 185                 190
   tac atg ggg atg agc tca ggt ttc tac aaa aat gtg gtg aag att caa     624
   Tyr Met Gly Met Ser Ser Gly Phe Tyr Lys Asn Val Val Lys Ile Gln
           195                 200                 205
   aag cat gtt acc ttc aac caa gtg aaa ggc att ttc ggc ttc act gac     672
   Lys His Val Thr Phe Asn Gln Val Lys Gly Ile Phe Gly Phe Thr Asp
       210                 215                 220
   agc gac tgc att ggg aag atc agt ttt cct gcc atc cag gct gct ccc     720
   Ser Asp Cys Ile Gly Lys Ile Ser Phe Pro Ala Ile Gln Ala Ala Pro
   225                 230                 235                 240
   tcc ttc agc aac tca ttc cca cag atc ttc cga gac agg acg gat atc     768
   Ser Phe Ser Asn Ser Phe Pro Gln Ile Phe Arg Asp Arg Thr Asp Ile
                   245                 250                 255
   cag tgc ctt atc cca tgt gcc att gac cag gat cct tac ttt aga atg     816
   Gln Cys Leu Ile Pro Cys Ala Ile Asp Gln Asp Pro Tyr Phe Arg Met
               260                 265                 270
   aca agg gac gtc gcc ccc agg atc ggc tat cct aaa cca gcc ctg ttg     864
   Thr Arg Asp Val Ala Pro Arg Ile Gly Tyr Pro Lys Pro Ala Leu Leu
           275                 280                 285
   cac tcc acc ttc ttc cca gcc ctg cag ggc gcc cag acc aaa atg agt     912
   His Ser Thr Phe Phe Pro Ala Leu Gln Gly Ala Gln Thr Lys Met Ser
       290                 295                 300
   gcc agc gac cca aac tcc tcc atc ttc ctc acc gac acg gcc aag cag     960
   Ala Ser Asp Pro Asn Ser Ser Ile Phe Leu Thr Asp Thr Ala Lys Gln
   305                 310                 315                 320
   atc aaa acc aag gtc aat aag cat gcg ttt tct gga ggg aga gac acc     1008
   Ile Lys Thr Lys Val Asn Lys His Ala Phe Ser Gly Gly Arg Asp Thr
                   325                 330                 335
   atc gag gag cac agg cag ttt ggg ggc aac tgt gat gtg gac gtg tct     1056
   Ile Glu Glu His Arg Gln Phe Gly Gly Asn Cys Asp Val Asp Val Ser
               340                 345                 350
   ttc atg tac ctg acc ttc ttc ctc gag gac gac gac aag ctc gag cag     1104
   Phe Met Tyr Leu Thr Phe Phe Leu Glu Asp Asp Asp Lys Leu Glu Gln
           355                 360                 365
   atc agg aag gat tac acc agc gga gcc atg ctc acc ggt gag ctc aag     1152
   Ile Arg Lys Asp Tyr Thr Ser Gly Ala Met Leu Thr Gly Glu Leu Lys
       370                 375                 380
   aag gca ctc ata gag gtt ctg cag ccc ttg atc gca gag cac cag gcc     1200
   Lys Ala Leu Ile Glu Val Leu Gln Pro Leu Ile Ala Glu His Gln Ala
   385                 390                 395                 400
   cgg cgc aag gag gtc acg gat gag ata gtg aaa gag ttc atg act ccc     1248
   Arg Arg Lys Glu Val Thr Asp Glu Ile Val Lys Glu Phe Met Thr Pro
                   405                 410                 415
   cgg aag ctg tcc ttc gac ttt cag                                     1272
   Arg Lys Leu Ser Phe Asp Phe Gln
               420
<210> 60
   <211> 56
   <212> PRT
   <213> Homo sapiens
<400> 60
   Asp Leu Asp Tyr Met Gly Met Ser Ser Gly Phe Tyr Lys Asn Val Val
   1               5                   10                  15
   Lys Ile Gln Lys His Val Thr Phe Asn Gln Val Lys Gly Ile Phe Gly
               20                  25                  30
   Phe Thr Asp Ser Asp Cys Ile Gly Lys Ile Ser Phe Pro Ala Ile Gln
           35                  40                  45
   Ala Ala Pro Ser Phe Ser Asn Ser
       50                  55
<210> 61
   <211> 471
   <212> PRT
   <213> Artificial sequence
<220>
   <223> Description of Artificial sequence: Recombinant human GD variant protein
<400> 61
   Met Pro Asn Ser Glu Pro Ala Ser Leu Leu Glu Leu Phe Asn Ser Ile
   1               5                   10                  15
   Ala Thr Gln Gly Glu Leu Val Arg Ser Leu Lys Ala Gly Asn Ala Ser
               20                  25                  30
   Lys Asp Glu Ile Asp Ser Ala Val Lys Met Leu Val Ser Leu Lys Met
           35                  40                  45
   Ser Tyr Lys Ala Ala Ala Gly Glu Asp Tyr Lys Ala Asp Cys Pro Pro
       50                  55                  60
   Gly Asn Pro Ala Pro Thr Ser Asn His Gly Pro Asp Ala Thr Glu Ala
   65                  70                  75                  80
   Glu Glu Asp Phe Val Asp Pro Trp Thr Val Gln Thr Ser Ser Ala Lys
                   85                  90                  95
   Gly Ile Asp Tyr Asp Lys Leu Ile Val Arg Phe Gly Ser Ser Lys Ile
               100                 105                 110
   Asp Lys Glu Leu Ile Asn Arg Ile Glu Arg Ala Thr Gly Gln Arg Pro
           115                 120                 125
   His His Phe Leu Arg Arg Gly Ile Phe Phe Ser His Arg Asp Met Asn
       130                 135                 140
   Gln Val Leu Asp Ala Tyr Glu Asn Lys Lys Pro Phe Tyr Leu Tyr Thr
   145                 150                 155                 160
   Gly Arg Gly Pro Ser Ser Glu Ala Met His Val Gly His Leu Ile Pro
                   165                 170                 175
   Phe Ile Phe Thr Lys Trp Leu Gln Asp Val Phe Asn Val Pro Leu Val
               180                 185                 190
   Ile Gln Met Thr Asp Asp Glu Lys Tyr Leu Trp Lys Asp Leu Thr Leu
   195 200 205
   Asp Gln Ala Tyr Gly Asp Ala Val Glu Asn Ala Lys Asp Ile Ile Ala
   210 215 220
   Cys Gly Phe Asp Ile Asn Lys Thr Phe Ile Phe Ser Asp Leu Asp Tyr
   225 230 235 240
   Met Gly Met Ser Ser Gly Phe Tyr Lys Asn Val Val Lys Ile Gln Lys
   245 250 255
   His Val Thr Phe Asn Gln Val Lys Gly Ile Phe Gly Phe Thr Asp Ser
   260 265 270
   Asp Cys Ile Gly Lys Ile Ser Phe Pro Ala Ile Gln Ala Ala Pro Ser
   275 280 285
   Phe Ser Asn Ser Phe Pro Gln Ile Phe Arg Asp Arg Thr Asp Ile Gln
   290 295 300
   Cys Leu Ile Pro Cys Ala Ile Asp Gln Asp Pro Tyr Phe Arg Met Thr
   305 310 315 320
   Arg Asp Val Ala Pro Arg Ile Gly Tyr Pro Lys Pro Ala Leu Leu His
   325 330 335
   Ser Thr Phe Phe Pro Ala Leu Gln Gly Ala Gln Thr Lys Met Ser Ala
   340 345 350
   Ser Asp Pro Asn Ser Ser Ile Phe Leu Thr Asp Thr Ala Lys Gln Ile
   355 360 365
   Lys Thr Lys Val Asn Lys His Ala Phe Ser Gly Gly Arg Asp Thr Ile
   370 375 380
   Glu Glu His Arg Gln Phe Gly Gly Asn Cys Asp Val Asp Val Ser Phe
   385 390 395 400
   Met Tyr Leu Thr Phe Phe Leu Glu Asp Asp Asp Lys Leu Glu Gln Ile
   405 410 415
   Arg Lys Asp Tyr Thr Ser Gly Ala Met Leu Thr Gly Glu Leu Lys Lys
   420 425 430
   Ala Leu Ile Glu Val Leu Gln Pro Leu Ile Ala Glu His Gln Ala Arg
   435 440 445
   Arg Lys Glu Val Thr Asp Glu Ile Val Lys Glu Phe Met Thr Pro Arg
   450 455 460
   Lys Leu Ser Phe Asp Phe Gln
   465 470
<210> 62
   <211> 470
   <212> PRT
   <213> Artificial sequence
<220>
   <223> Description of Artificial sequence: Recombinant human GD variant protein
<400> 62
   Pro Asn Ser Glu Pro Ala Ser Leu Leu Glu Leu Phe Asn Ser Ile Ala
   1               5                   10                  15
   Thr Gln Gly Glu Leu Val Arg Ser Leu Lys Ala Gly Asn Ala Ser Lys
               20                  25                  30
   Asp Glu Ile Asp Ser Ala Val Lys Met Leu Val Ser Leu Lys Met Ser
           35                  40                  45
   Tyr Lys Ala Ala Ala Gly Glu Asp Tyr Lys Ala Asp Cys Pro Pro Gly
       50                  55                  60
   Asn Pro Ala Pro Thr Ser Asn His Gly Pro Asp Ala Thr Glu Ala Glu
   65                  70                  75                  80
   Glu Asp Phe Val Asp Pro Trp Thr Val Gln Thr Ser Ser Ala Lys Gly
                   85                  90                  95
   Ile Asp Tyr Asp Lys Leu Ile Val Arg Phe Gly Ser Ser Lys Ile Asp
               100                 105                 110
   Lys Glu Leu Ile Asn Arg Ile Glu Arg Ala Thr Gly Gln Arg Pro His
           115                 120                 125
   His Phe Leu Arg Arg Gly Ile Phe Phe Ser His Arg Asp Met Asn Gln
       130                 135                 140
   Val Leu Asp Ala Tyr Glu Asn Lys Lys Pro Phe Tyr Leu Tyr Thr Gly
   145                 150                 155                 160
   Arg Gly Pro Ser Ser Glu Ala Met His Val Gly His Leu Ile Pro Phe
                   165                 170                 175
   Ile Phe Thr Lys Trp Leu Gln Asp Val Phe Asn Val Pro Leu Val Ile
               180                 185                 190
   Gln Met Thr Asp Asp Glu Lys Tyr Leu Trp Lys Asp Leu Thr Leu Asp
           195                 200                 205
   Gln Ala Tyr Gly Asp Ala Val Glu Asn Ala Lys Asp Ile Ile Ala Cys
       210                 215                 220
   Gly Phe Asp Ile Asn Lys Thr Phe Ile Phe Ser Asp Leu Asp Tyr Met
   225                 230                 235                 240
   Gly Met Ser Ser Gly Phe Tyr Lys Asn Val Val Lys Ile Gln Lys His
                   245                 250                 255
   Val Thr Phe Asn Gln Val Lys Gly Ile Phe Gly Phe Thr Asp Ser Asp
               260                 265                 270
   Cys Ile Gly Lys Ile Ser Phe Pro Ala Ile Gln Ala Ala Pro Ser Phe
           275                 280                 285
   Ser Asn Ser Phe Pro Gln Ile Phe Arg Asp Arg Thr Asp Ile Gln Cys
       290                 295                 300
   Leu Ile Pro Cys Ala Ile Asp Gln Asp Pro Tyr Phe Arg Met Thr Arg
   305                 310                 315                 320
   Asp Val Ala Pro Arg Ile Gly Tyr Pro Lys Pro Ala Leu Leu His Ser
                   325                 330                 335
   Thr Phe Phe Pro Ala Leu Gln Gly Ala Gln Thr Lys Met Ser Ala Ser
               340                 345                 350
   Asp Pro Asn Ser Ser Ile Phe Leu Thr Asp Thr Ala Lys Gln Ile Lys
           355                 360                 365
   Thr Lys Val Asn Lys His Ala Phe Ser Gly Gly Arg Asp Thr Ile Glu
   370 375 380
   Glu His Arg Gln Phe Gly Gly Asn Cys Asp Val Asp Val Ser Phe Met
   385 390 395 400
   Tyr Leu Thr Phe Phe Leu Glu Asp Asp Asp Lys Leu Glu Gln Ile Arg
   405 410 415
   Lys Asp Tyr Thr Ser Gly Ala Met Leu Thr Gly Glu Leu Lys Lys Ala
   420 425 430
   Leu Ile Glu Val Leu Gln Pro Leu Ile Ala Glu His Gln Ala Arg Arg
   435 440 445
   Lys Glu Val Thr Asp Glu Ile Val Lys Glu Phe Met Thr Pro Arg Lys
   450 455 460
   Leu Ser Phe Asp Phe Gln
   465 470
<210> 63
   <211> 471
   <212> PRT
   <213> Artificial Sequence
<220>
   <223> Description of Artificial Sequence: Recombinant human SY variant protein
<400> 63
<210> 64
   <211> 470
   <212> PRT
   <213> Artificial sequence
<220>
<223> Description of Artificial Sequence: Recombinant human SY variant protein
<400> 64
   Pro Asn Ser Glu Pro Ala Ser Leu Leu Glu Leu Phe Asn Ser Ile Ala
   1               5                   10                  15
   Thr Gln Gly Glu Leu Val Arg Ser Leu Lys Ala Gly Asn Ala Ser Lys
               20                  25                  30
   Asp Glu Ile Asp Ser Ala Val Lys Met Leu Val Ser Leu Lys Met Ser
           35                  40                  45
   Tyr Lys Ala Ala Ala Gly Glu Asp Tyr Lys Ala Asp Cys Pro Pro Gly
       50                  55                  60
   Asn Pro Ala Pro Thr Ser Asn His Gly Pro Asp Ala Thr Glu Ala Glu
   65                  70                  75                  80
   Glu Asp Phe Val Asp Pro Trp Thr Val Gln Thr Ser Ser Ala Lys Gly
                   85                  90                  95
   Ile Asp Tyr Asp Lys Leu Ile Val Arg Phe Gly Ser Ser Lys Ile Asp
               100                 105                 110
   Lys Glu Leu Ile Asn Arg Ile Glu Arg Ala Thr Gly Gln Arg Pro His
           115                 120                 125
   His Phe Leu Arg Arg Gly Ile Phe Phe Ser His Arg Asp Met Asn Gln
       130                 135                 140
   Val Leu Asp Ala Tyr Glu Asn Lys Lys Pro Phe Tyr Leu Tyr Thr Gly
   145                 150                 155                 160
   Arg Gly Pro Ser Ser Glu Ala Met His Val Gly His Leu Ile Pro Phe
                   165                 170                 175
   Ile Phe Thr Lys Trp Leu Gln Asp Val Phe Asn Val Pro Leu Val Ile
               180                 185                 190
   Gln Met Thr Asp Asp Glu Lys Tyr Leu Trp Lys Asp Leu Thr Leu Asp
           195                 200                 205
   Gln Ala Tyr Ser Tyr Ala Val Glu Asn Ala Lys Asp Ile Ile Ala Cys
       210                 215                 220
   Gly Phe Asp Ile Asn Lys Thr Phe Ile Phe Ser Asp Leu Asp Tyr Met
   225                 230                 235                 240
   Gly Met Ser Ser Gly Phe Tyr Lys Asn Val Val Lys Ile Gln Lys His
                   245                 250                 255
   Val Thr Phe Asn Gln Val Lys Gly Ile Phe Gly Phe Thr Asp Ser Asp
               260                 265                 270
   Cys Ile Gly Lys Ile Ser Phe Pro Ala Ile Gln Ala Ala Pro Ser Phe
           275                 280                 285
   Ser Asn Ser Phe Pro Gln Ile Phe Arg Asp Arg Thr Asp Ile Gln Cys
       290                 295                 300
   Leu Ile Pro Cys Ala Ile Asp Gln Asp Pro Tyr Phe Arg Met Thr Arg
   305                 310                 315                 320
   Asp Val Ala Pro Arg Ile Gly Tyr Pro Lys Pro Ala Leu Leu His Ser
                   325                 330                 335
   Thr Phe Phe Pro Ala Leu Gln Gly Ala Gln Thr Lys Met Ser Ala Ser
               340                 345                 350
   Asp Pro Asn Ser Ser Ile Phe Leu Thr Asp Thr Ala Lys Gln Ile Lys
   355 360 365
   Thr Lys Val Asn Lys His Ala Phe Ser Gly Gly Arg Asp Thr Ile Glu
   370 375 380
   Glu His Arg Gln Phe Gly Gly Asn Cys Asp Val Asp Val Ser Phe Met
   385 390 395 400
   Tyr Leu Thr Phe Phe Leu Glu Asp Asp Asp Lys Leu Glu Gln Ile Arg
   405 410 415
   Lys Asp Tyr Thr Ser Gly Ala Met Leu Thr Gly Glu Leu Lys Lys Ala
   420 425 430
   Leu Ile Glu Val Leu Gln Pro Leu Ile Ala Glu His Gln Ala Arg Arg
   435 440 445
   Lys Glu Val Thr Asp Glu Ile Val Lys Glu Phe Met Thr Pro Arg Lys
   450 455 460
   Leu Ser Phe Asp Phe Gln
   465 470
<210> 65
   <211> 471
   <212> PRT
   <213> Artificial Sequence
<220>
   <223> Description of Artificial sequence: Recombinant human GY variant protein
<400> 65
   Met Pro Asn Ser Glu Pro Ala Ser Leu Leu Glu Leu Phe Asn Ser Ile
   1 5 10 15
   Ala Thr Gln Gly Glu Leu Val Arg Ser Leu Lys Ala Gly Asn Ala Ser
   20 25 30
   Lys Asp Glu Ile Asp Ser Ala Val Lys Met Leu Val Ser Leu Lys Met
   35 40 45
   Ser Tyr Lys Ala Ala Ala Gly Glu Asp Tyr Lys Ala Asp Cys Pro Pro
   50 55 60
   Gly Asn Pro Ala Pro Thr Ser Asn His Gly Pro Asp Ala Thr Glu Ala
   65 70 75 80
   Glu Glu Asp Phe Val Asp Pro Trp Thr Val Gln Thr Ser Ser Ala Lys
   85 90 95
   Gly Ile Asp Tyr Asp Lys Leu Ile Val Arg Phe Gly Ser Ser Lys Ile
   100 105 110
   Asp Lys Glu Leu Ile Asn Arg Ile Glu Arg Ala Thr Gly Gln Arg Pro
   115 120 125
   His His Phe Leu Arg Arg Gly Ile Phe Phe Ser His Arg Asp Met Asn
   130 135 140
   Gln Val Leu Asp Ala Tyr Glu Asn Lys Lys Pro Phe Tyr Leu Tyr Thr
   145 150 155 160
   Gly Arg Gly Pro Ser Ser Glu Ala Met His Val Gly His Leu Ile Pro
   165 170 175
   Phe Ile Phe Thr Lys Trp Leu Gln Asp Val Phe Asn Val Pro Leu Val
   180 185 190
   Ile Gln Met Thr Asp Asp Glu Lys Tyr Leu Trp Lys Asp Leu Thr Leu
   195 200 205
   Asp Gln Ala Tyr Gly Tyr Ala Val Glu Asn Ala Lys Asp Ile Ile Ala
   210 215 220
   Cys Gly Phe Asp Ile Asn Lys Thr Phe Ile Phe Ser Asp Leu Asp Tyr
   225 230 235 240
   Met Gly Met Ser Ser Gly Phe Tyr Lys Asn Val Val Lys Ile Gln Lys
   245 250 255
   His Val Thr Phe Asn Gln Val Lys Gly Ile Phe Gly Phe Thr Asp Ser
   260 265 270
   Asp Cys Ile Gly Lys Ile Ser Phe Pro Ala Ile Gln Ala Ala Pro Ser
   275 280 285
   Phe Ser Asn Ser Phe Pro Gln Ile Phe Arg Asp Arg Thr Asp Ile Gln
   290 295 300
   Cys Leu Ile Pro Cys Ala Ile Asp Gln Asp Pro Tyr Phe Arg Met Thr
   305 310 315 320
   Arg Asp Val Ala Pro Arg Ile Gly Tyr Pro Lys Pro Ala Leu Leu His
   325 330 335
   Ser Thr Phe Phe Pro Ala Leu Gln Gly Ala Gln Thr Lys Met Ser Ala
   340 345 350
   Ser Asp Pro Asn Ser Ser Ile Phe Leu Thr Asp Thr Ala Lys Gln Ile
   355 360 365
   Lys Thr Lys Val Asn Lys His Ala Phe Ser Gly Gly Arg Asp Thr Ile
   370 375 380
   Glu Glu His Arg Gln Phe Gly Gly Asn Cys Asp Val Asp Val Ser Phe
   385 390 395 400
   Met Tyr Leu Thr Phe Phe Leu Glu Asp Asp Asp Lys Leu Glu Gln Ile
   405 410 415
   Arg Lys Asp Tyr Thr Ser Gly Ala Met Leu Thr Gly Glu Leu Lys Lys
   420 425 430
   Ala Leu Ile Glu Val Leu Gln Pro Leu Ile Ala Glu His Gln Ala Arg
   435 440 445
   Arg Lys Glu Val Thr Asp Glu Ile Val Lys Glu Phe Met Thr Pro Arg
   450 455 460
   Lys Leu Ser Phe Asp Phe Gln
   465 470
<210> 66
   <211> 470
   <212> PRT
   <213> Artificial sequence
<220>
   <223> Description of Artificial sequence: Recombinant human GY variant protein
<400> 66
   Pro Asn Ser Glu Pro Ala Ser Leu Leu Glu Leu Phe Asn Ser Ile Ala
   1               5                   10                  15
   Thr Gln Gly Glu Leu Val Arg Ser Leu Lys Ala Gly Asn Ala Ser Lys
               20                  25                  30
   Asp Glu Ile Asp Ser Ala Val Lys Met Leu Val Ser Leu Lys Met Ser
           35                  40                  45
   Tyr Lys Ala Ala Ala Gly Glu Asp Tyr Lys Ala Asp Cys Pro Pro Gly
       50                  55                  60
   Asn Pro Ala Pro Thr Ser Asn His Gly Pro Asp Ala Thr Glu Ala Glu
   65                  70                  75                  80
   Glu Asp Phe Val Asp Pro Trp Thr Val Gln Thr Ser Ser Ala Lys Gly
                   85                  90                  95
   Ile Asp Tyr Asp Lys Leu Ile Val Arg Phe Gly Ser Ser Lys Ile Asp
               100                 105                 110
   Lys Glu Leu Ile Asn Arg Ile Glu Arg Ala Thr Gly Gln Arg Pro His
           115                 120                 125
   His Phe Leu Arg Arg Gly Ile Phe Phe Ser His Arg Asp Met Asn Gln
       130                 135                 140
   Val Leu Asp Ala Tyr Glu Asn Lys Lys Pro Phe Tyr Leu Tyr Thr Gly
   145                 150                 155                 160
   Arg Gly Pro Ser Ser Glu Ala Met His Val Gly His Leu Ile Pro Phe
                   165                 170                 175
   Ile Phe Thr Lys Trp Leu Gln Asp Val Phe Asn Val Pro Leu Val Ile
               180                 185                 190
   Gln Met Thr Asp Asp Glu Lys Tyr Leu Trp Lys Asp Leu Thr Leu Asp
           195                 200                 205
   Gln Ala Tyr Gly Tyr Ala Val Glu Asn Ala Lys Asp Ile Ile Ala Cys
       210                 215                 220
   Gly Phe Asp Ile Asn Lys Thr Phe Ile Phe Ser Asp Leu Asp Tyr Met
   225                 230                 235                 240
   Gly Met Ser Ser Gly Phe Tyr Lys Asn Val Val Lys Ile Gln Lys His
                   245                 250                 255
   Val Thr Phe Asn Gln Val Lys Gly Ile Phe Gly Phe Thr Asp Ser Asp
               260                 265                 270
   Cys Ile Gly Lys Ile Ser Phe Pro Ala Ile Gln Ala Ala Pro Ser Phe
           275                 280                 285
   Ser Asn Ser Phe Pro Gln Ile Phe Arg Asp Arg Thr Asp Ile Gln Cys
       290                 295                 300
   Leu Ile Pro Cys Ala Ile Asp Gln Asp Pro Tyr Phe Arg Met Thr Arg
   305                 310                 315                 320
   Asp Val Ala Pro Arg Ile Gly Tyr Pro Lys Pro Ala Leu Leu His Ser
                   325                 330                 335
   Thr Phe Phe Pro Ala Leu Gln Gly Ala Gln Thr Lys Met Ser Ala Ser
   340 345 350
   Asp Pro Asn Ser Ser Ile Phe Leu Thr Asp Thr Ala Lys Gln Ile Lys
   355 360 365
   Thr Lys Val Asn Lys His Ala Phe Ser Gly Gly Arg Asp Thr Ile Glu
   370 375 380
   Glu His Arg Gln Phe Gly Gly Asn Cys Asp Val Asp Val Ser Phe Met
   385 390 395 400
   Tyr Leu Thr Phe Phe Leu Glu Asp Asp Asp Lys Leu Glu Gln Ile Arg
   405 410 415
   Lys Asp Tyr Thr Ser Gly Ala Met Leu Thr Gly Glu Leu Lys Lys Ala
   420 425 430
   Leu Ile Glu Val Leu Gln Pro Leu Ile Ala Glu His Gln Ala Arg Arg
   435 440 445
   Lys Glu Val Thr Asp Glu Ile Val Lys Glu Phe Met Thr Pro Arg Lys
   450 455 460
   Leu Ser Phe Asp Phe Gln
   465 470
<210> 67
   <211> 471
   <212> PRT
   <213> Artificial sequence
<220>
   <223> Description of Artificial sequence: Recombinant human SD variant protein
<400> 67
   Met Pro Asn Ser Glu Pro Ala Ser Leu Leu Glu Leu Phe Asn Ser Ile
   1 5 10 15
   Ala Thr Gln Gly Glu Leu Val Arg Ser Leu Lys Ala Gly Asn Ala Ser
   20 25 30
   Lys Asp Glu Ile Asp Ser Ala Val Lys Met Leu Val Ser Leu Lys Met
   35 40 45
   Ser Tyr Lys Ala Ala Ala Gly Glu Asp Tyr Lys Ala Asp Cys Pro Pro
   50 55 60
   Gly Asn Pro Ala Pro Thr Ser Asn His Gly Pro Asp Ala Thr Glu Ala
   65 70 75 80
   Glu Glu Asp Phe Val Asp Pro Trp Thr Val Gln Thr Ser Ser Ala Lys
   85 90 95
   Gly Ile Asp Tyr Asp Lys Leu Ile Val Arg Phe Gly Ser Ser Lys Ile
   100 105 110
   Asp Lys Glu Leu Ile Asn Arg Ile Glu Arg Ala Thr Gly Gln Arg Pro
   115 120 125
   His His Phe Leu Arg Arg Gly Ile Phe Phe Ser His Arg Asp Met Asn
   130 135 140
   Gln Val Leu Asp Ala Tyr Glu Asn Lys Lys Pro Phe Tyr Leu Tyr Thr
   145 150 155 160
   Gly Arg Gly Pro Ser Ser Glu Ala Met His Val Gly His Leu Ile Pro
                   165                 170                 175
   Phe Ile Phe Thr Lys Trp Leu Gln Asp Val Phe Asn Val Pro Leu Val
               180                 185                 190
   Ile Gln Met Thr Asp Asp Glu Lys Tyr Leu Trp Lys Asp Leu Thr Leu
           195                 200                 205
   Asp Gln Ala Tyr Ser Asp Ala Val Glu Asn Ala Lys Asp Ile Ile Ala
       210                 215                 220
   Cys Gly Phe Asp Ile Asn Lys Thr Phe Ile Phe Ser Asp Leu Asp Tyr
   225                 230                 235                 240
   Met Gly Met Ser Ser Gly Phe Tyr Lys Asn Val Val Lys Ile Gln Lys
                   245                 250                 255
   His Val Thr Phe Asn Gln Val Lys Gly Ile Phe Gly Phe Thr Asp Ser
               260                 265                 270
   Asp Cys Ile Gly Lys Ile Ser Phe Pro Ala Ile Gln Ala Ala Pro Ser
           275                 280                 285
   Phe Ser Asn Ser Phe Pro Gln Ile Phe Arg Asp Arg Thr Asp Ile Gln
       290                 295                 300
   Cys Leu Ile Pro Cys Ala Ile Asp Gln Asp Pro Tyr Phe Arg Met Thr
   305                 310                 315                 320
   Arg Asp Val Ala Pro Arg Ile Gly Tyr Pro Lys Pro Ala Leu Leu His
                   325                 330                 335
   Ser Thr Phe Phe Pro Ala Leu Gln Gly Ala Gln Thr Lys Met Ser Ala
               340                 345                 350
   Ser Asp Pro Asn Ser Ser Ile Phe Leu Thr Asp Thr Ala Lys Gln Ile
           355                 360                 365
   Lys Thr Lys Val Asn Lys His Ala Phe Ser Gly Gly Arg Asp Thr Ile
       370                 375                 380
   Glu Glu His Arg Gln Phe Gly Gly Asn Cys Asp Val Asp Val Ser Phe
   385                 390                 395                 400
   Met Tyr Leu Thr Phe Phe Leu Glu Asp Asp Asp Lys Leu Glu Gln Ile
                   405                 410                 415
   Arg Lys Asp Tyr Thr Ser Gly Ala Met Leu Thr Gly Glu Leu Lys Lys
               420                 425                 430
   Ala Leu Ile Glu Val Leu Gln Pro Leu Ile Ala Glu His Gln Ala Arg
           435                 440                 445
   Arg Lys Glu Val Thr Asp Glu Ile Val Lys Glu Phe Met Thr Pro Arg
       450                 455                 460
   Lys Leu Ser Phe Asp Phe Gln
   465                 470
<210> 68
   <211> 470
   <212> PRT
   <213> Artificial sequence
<220>
   <223> Description of Artificial Sequence: Recombinant human SD variant protein
<400>68
   Pro Asn Ser Glu Pro Ala Ser Leu Leu Glu Leu Phe Asn Ser Ile Ala
   1               5                   10                  15
   Thr Gln Gly Glu Leu Val Arg Ser Leu Lys Ala Gly Asn Ala Ser Lys
               20                  25                  30
   Asp Glu Ile Asp Ser Ala Val Lys Met Leu Val Ser Leu Lys Met Ser
           35                  40                  45
   Tyr Lys Ala Ala Ala Gly Glu Asp Tyr Lys Ala Asp Cys Pro Pro Gly
       50                  55                  60
   Asn Pro Ala Pro Thr Ser Asn His Gly Pro Asp Ala Thr Glu Ala Glu
   65                  70                  75                  80
   Glu Asp Phe Val Asp Pro Trp Thr Val Gln Thr Ser Ser Ala Lys Gly
                   85                  90                  95
   Ile Asp Tyr Asp Lys Leu Ile Val Arg Phe Gly Ser Ser Lys Ile Asp
               100                 105                 110
   Lys Glu Leu Ile Asn Arg Ile Glu Arg Ala Thr Gly Gln Arg Pro His
           115                 120                 125
   His Phe Leu Arg Arg Gly Ile Phe Phe Ser His Arg Asp Met Asn Gln
       130                 135                 140
   Val Leu Asp Ala Tyr Glu Asn Lys Lys Pro Phe Tyr Leu Tyr Thr Gly
   145                 150                 155                 160
   Arg Gly Pro Ser Ser Glu Ala Met His Val Gly His Leu Ile Pro Phe
                   165                 170                 175
   Ile Phe Thr Lys Trp Leu Gln Asp Val Phe Asn Val Pro Leu Val Ile
               180                 185                 190
   Gln Met Thr Asp Asp Glu Lys Tyr Leu Trp Lys Asp Leu Thr Leu Asp
           195                 200                 205
   Gln Ala Tyr Ser Asp Ala Val Glu Asn Ala Lys Asp Ile Ile Ala Cys
       210                 215                 220
   Gly Phe Asp Ile Asn Lys Thr Phe Ile Phe Ser Asp Leu Asp Tyr Met
   225                 230                 235                 240
   Gly Met Ser Ser Gly Phe Tyr Lys Asn Val Val Lys Ile Gln Lys His
                   245                 250                 255
   Val Thr Phe Asn Gln Val Lys Gly Ile Phe Gly Phe Thr Asp Ser Asp
               260                 265                 270
   Cys Ile Gly Lys Ile Ser Phe Pro Ala Ile Gln Ala Ala Pro Ser Phe
           275                 280                 285
   Ser Asn Ser Phe Pro Gln Ile Phe Arg Asp Arg Thr Asp Ile Gln Cys
       290                 295                 300
   Leu Ile Pro Cys Ala Ile Asp Gln Asp Pro Tyr Phe Arg Met Thr Arg
   305                 310                 315                 320
   Asp Val Ala Pro Arg Ile Gly Tyr Pro Lys Pro Ala Leu Leu His Ser
                   325                 330                 335
   Thr Phe Phe Pro Ala Leu Gln Gly Ala Gln Thr Lys Met Ser Ala Ser
               340                 345                 350
   Asp Pro Asn Ser Ser Ile Phe Leu Thr Asp Thr Ala Lys Gln Ile Lys
           355                 360                 365
   Thr Lys Val Asn Lys His Ala Phe Ser Gly Gly Arg Asp Thr Ile Glu
       370                 375                 380
   Glu His Arg Gln Phe Gly Gly Asn Cys Asp Val Asp Val Ser Phe Met
   385                 390                 395                 400
   Tyr Leu Thr Phe Phe Leu Glu Asp Asp Asp Lys Leu Glu Gln Ile Arg
                   405                 410                 415
   Lys Asp Tyr Thr Ser Gly Ala Met Leu Thr Gly Glu Leu Lys Lys Ala
               420                 425                 430
   Leu Ile Glu Val Leu Gln Pro Leu Ile Ala Glu His Gln Ala Arg Arg
           435                 440                 445
   Lys Glu Val Thr Asp Glu Ile Val Lys Glu Phe Met Thr Pro Arg Lys
       450                 455                 460
   Leu Ser Phe Asp Phe Gln
   465                 470
<210> 69
   <211> 12
   <212> PRT
   <213> Artificial sequence
<220>
   <223> Description of Artificial sequence: C-terminal peptide
<400> 69
   Phe Met Thr Pro Arg Lys Leu Ser Phe Asp Phe Gln
   1               5                   10
<210> 70
   <211> 6389
   <212> DNA
   <213> Artificial sequence
<220>
   <223> Description of Artificial sequence: Plasmid 01 - pET24b+ with a NdeI/HindIII inst of Hu T2-WRS (SY variant) without 6-H Tag
<400> 70
   tggcgaatgg gacgcgccct gtagcggcgc attaagcgcg gcgggtgtgg tggttacgcg 60
   cagcgtgacc gctacacttg ccagcgccct agcgcccgct cctttcgctt tcttcccttc 120
   ctttctcgcc acgttcgccg gctttccccg tcaagctcta aatcgggggc tccctttagg 180
   gttccgattt agtgctttac ggcacctcga ccccaaaaaa cttgattagg gtgatggttc 240
   acgtagtggg ccatcgccct gatagacggt ttttcgccct ttgacgttgg agtccacgtt 300
   ctttaatagt ggactcttgt tccaaactgg aacaacactc aaccctatct cggtctattc 360
   ttttgattta taagggattt tgccgatttc ggcctattgg ttaaaaaatg agctgattta 420
   acaaaaattt aacgcgaatt ttaacaaaat attaacgttt acaatttcag gtggcacttt 480
   tcggggaaat gtgcgcggaa cccctatttg tttatttttc taaatacatt caaatatgta 540
   tccgctcatg aattaattct tagaaaaact catcgagcat caaatgaaac tgcaatttat 600
   tcatatcagg attatcaata ccatattttt gaaaaagccg tttctgtaat gaaggagaaa 660
   actcaccgag gcagttccat aggatggcaa gatcctggta tcggtctgcg attccgactc 720
   gtccaacatc aatacaacct attaatttcc cctcgtcaaa aataaggtta tcaagtgaga 780
   aatcaccatg agtgacgact gaatccggtg agaatggcaa aagtttatgc atttctttcc 840
   agacttgttc aacaggccag ccattacgct cgtcatcaaa atcactcgca tcaaccaaac 900
   cgttattcat tcgtgattgc gcctgagcga gacgaaatac gcgatcgctg ttaaaaggac 960
   aattacaaac aggaatcgaa tgcaaccggc gcaggaacac tgccagcgca tcaacaatat 1020
   tttcacctga atcaggatat tcttctaata cctggaatgc tgttttcccg gggatcgcag 1080
   tggtgagtaa ccatgcatca tcaggagtac ggataaaatg cttgatggtc ggaagaggca 1140
   taaattccgt cagccagttt agtctgacca tctcatctgt aacatcattg gcaacgctac 1200
   ctttgccatg tttcagaaac aactctggcg catcgggctt cccatacaat cgatagattg 1260
   tcgcacctga ttgcccgaca ttatcgcgag cccatttata cccatataaa tcagcatcca 1320
   tgttggaatt taatcgcggc ctagagcaag acgtttcccg ttgaatatgg ctcataacac 1380
   cccttgtatt actgtttatg taagcagaca gttttattgt tcatgaccaa aatcccttaa 1440
   cgtgagtttt cgttccactg agcgtcagac cccgtagaaa agatcaaagg atcttcttga 1500
   gatccttttt ttctgcgcgt aatctgctgc ttgcaaacaa aaaaaccacc gctaccagcg 1560
   gtggtttgtt tgccggatca agagctacca actctttttc cgaaggtaac tggcttcagc 1620
   agagcgcaga taccaaatac tgtccttcta gtgtagccgt agttaggcca ccacttcaag 1680
   aactctgtag caccgcctac atacctcgct ctgctaatcc tgttaccagt ggctgctgcc 1740
   agtggcgata agtcgtgtct taccgggttg gactcaagac gatagttacc ggataaggcg 1800
   cagcggtcgg gctgaacggg gggttcgtgc acacagccca gcttggagcg aacgacctac 1860
   accgaactga gatacctaca gcgtgagcta tgagaaagcg ccacgcttcc cgaagggaga 1920
   aaggcggaca ggtatccggt aagcggcagg gtcggaacag gagagcgcac gagggagctt 1980
   ccagggggaa acgcctggta tctttatagt cctgtcgggt ttcgccacct ctgacttgag 2040
   cgtcgatttt tgtgatgctc gtcagggggg cggagcctat ggaaaaacgc cagcaacgcg 2100
   gcctttttac ggttcctggc cttttgctgg ccttttgctc acatgttctt tcctgcgtta 2160
   tcccctgatt ctgtggataa ccgtattacc gcctttgagt gagctgatac cgctcgccgc 2220
   agccgaacga ccgagcgcag cgagtcagtg agcgaggaag cggaagagcg cctgatgcgg 2280
   tattttctcc ttacgcatct gtgcggtatt tcacaccgca tatatggtgc actctcagta 2340
   caatctgctc tgatgccgca tagttaagcc agtatacact ccgctatcgc tacgtgactg 2400
   ggtcatggct gcgccccgac acccgccaac acccgctgac gcgccctgac gggcttgtct 2460
   gctcccggca tccgcttaca gacaagctgt gaccgtctcc gggagctgca tgtgtcagag 2520
   gttttcaccg tcatcaccga aacgcgcgag gcagctgcgg taaagctcat cagcgtggtc 2580
   gtgaagcgat tcacagatgt ctgcctgttc atccgcgtcc agctcgttga gtttctccag 2640
   aagcgttaat gtctggcttc tgataaagcg ggccatgtta agggcggttt tttcctgttt 2700
   ggtcactgat gcctccgtgt aagggggatt tctgttcatg ggggtaatga taccgatgaa 2760
   acgagagagg atgctcacga tacgggttac tgatgatgaa catgcccggt tactggaacg 2820
   ttgtgagggt aaacaactgg cggtatggat gcggcgggac cagagaaaaa tcactcaggg 2880
   tcaatgccag cgcttcgtta atacagatgt aggtgttcca cagggtagcc agcagcatcc 2940
   tgcgatgcag atccggaaca taatggtgca gggcgctgac ttccgcgttt ccagacttta 3000
   cgaaacacgg aaaccgaaga ccattcatgt tgttgctcag gtcgcagacg ttttgcagca 3060
   gcagtcgctt cacgttcgct cgcgtatcgg tgattcattc tgctaaccag taaggcaacc 3120
   ccgccagcct agccgggtcc tcaacgacag gagcacgatc atgcgcaccc gtggggccgc 3180
   catgccggcg ataatggcct gcttctcgcc gaaacgtttg gtggcgggac cagtgacgaa 3240
   ggcttgagcg agggcgtgca agattccgaa taccgcaagc gacaggccga tcatcgtcgc 3300
   gctccagcga aagcggtcct cgccgaaaat gacccagagc gctgccggca cctgtcctac 3360
   gagttgcatg ataaagaaga cagtcataag tgcggcgacg atagtcatgc cccgcgccca 3420
   ccggaaggag ctgactgggt tgaaggctct caagggcatc ggtcgagatc ccggtgccta 3480
   atgagtgagc taacttacat taattgcgtt gcgctcactg cccgctttcc agtcgggaaa 3540
   cctgtcgtgc cagctgcatt aatgaatcgg ccaacgcgcg gggagaggcg gtttgcgtat 3600
   tgggcgccag ggtggttttt cttttcacca gtgagacggg caacagctga ttgcccttca 3660
   ccgcctggcc ctgagagagt tgcagcaagc ggtccacgct ggtttgcccc agcaggcgaa 3720
   aatcctgttt gatggtggtt aacggcggga tataacatga gctgtcttcg gtatcgtcgt 3780
   atcccactac cgagatatcc gcaccaacgc gcagcccgga ctcggtaatg gcgcgcattg 3840
   cgcccagcgc catctgatcg ttggcaacca gcatcgcagt gggaacgatg ccctcattca 3900
   gcatttgcat ggtttgttga aaaccggaca tggcactcca gtcgccttcc cgttccgcta 3960
   tcggctgaat ttgattgcga gtgagatatt tatgccagcc agccagacgc agacgcgccg 4020
   agacagaact taatgggccc gctaacagcg cgatttgctg gtgacccaat gcgaccagat 4080
   gctccacgcc cagtcgcgta ccgtcttcat gggagaaaat aatactgttg atgggtgtct 4140
   ggtcagagac atcaagaaat aacgccggaa cattagtgca ggcagcttcc acagcaatgg 4200
   catcctggtc atccagcgga tagttaatga tcagcccact gacgcgttgc gcgagaagat 4260
   tgtgcaccgc cgctttacag gcttcgacgc cgcttcgttc taccatcgac accaccacgc 4320
   tggcacccag ttgatcggcg cgagatttaa tcgccgcgac aatttgcgac ggcgcgtgca 4380
   gggccagact ggaggtggca acgccaatca gcaacgactg tttgcccgcc agttgttgtg 4440
   ccacgcggtt gggaatgtaa ttcagctccg ccatcgccgc ttccactttt tcccgcgttt 4500
   tcgcagaaac gtggctggcc tggttcacca cgcgggaaac ggtctgataa gagacaccgg 4560
   catactctgc gacatcgtat aacgttactg gtttcacatt caccaccctg aattgactct 4620
   cttccgggcg ctatcatgcc ataccgcgaa aggttttgcg ccattcgatg gtgtccggga 4680
   tctcgacgct ctcccttatg cgactcctgc attaggaagc agcccagtag taggttgagg 4740
   ccgttgagca ccgccgccgc aaggaatggt gcatgcaagg agatggcgcc caacagtccc 4800
   ccggccacgg ggcctgccac catacccacg ccgaaacaag cgctcatgag cccgaagtgg 4860
   cgagcccgat cttccccatc ggtgatgtcg gcgatatagg cgccagcaac cgcacctgtg 4920
   gcgccggtga tgccggccac gatgcgtccg gcgtagagga tcgagatctc gatcccgcga 4980
   aattaatacg actcactata ggggaattgt gagcggataa caattcccct ctagaaataa 5040
   ttttgtttaa ctttaagaag gagatataca tatgagtgca aaaggcatag actacgataa 5100
   gctcattgtt cggtttggaa gtagtaaaat tgacaaagag ctaataaacc gaatagagag 5160
   agccaccggc caaagaccac accacttcct gcgcagaggc atcttcttct cacacagaga 5220
   tatgaatcag gttcttgatg cctatgaaaa taagaagcca ttttatctgt acacgggccg 5280
   gggcccctct tctgaagcaa tgcatgtagg tcacctcatt ccatttattt tcacaaagtg 5340
   gctccaggat gtatttaacg tgcccttggt catccagatg acggatgacg agaagtatct 5400
   gtggaaggac ctgaccctgg accaggccta tagctatgct gtggagaatg ccaaggacat 5460
   catcgcctgt ggctttgaca tcaacaagac tttcatattc tctgacctgg actacatggg 5520
   gatgagctca ggtttctaca aaaatgtggt gaagattcaa aagcatgtta ccttcaacca 5580
   agtgaaaggc attttcggct tcactgacag cgactgcatt gggaagatca gttttcctgc 5640
   catccaggct gctccctcct tcagcaactc attcccacag atcttccgag acaggacgga 5700
   tatccagtgc cttatcccat gtgccattga ccaggatcct tactttagaa tgacaaggga 5760
   cgtcgccccc aggatcggct atcctaaacc agccctgttg cactccacct tcttcccagc 5820
   cctgcagggc gcccagacca aaatgagtgc cagcgacccc aactcctcca tcttcctcac 5880
   cgacacggcc aagcagatca aaaccaaggt caataagcat gcgttttctg gagggagaga 5940
   caccatcgag gagcacaggc agtttggggg caactgtgat gtggacgtgt ctttcatgta 6000
   cctgaccttc ttcctcgagg acgacgacaa gctcgagcag atcaggaagg attacaccag 6060
   cggagccatg ctcaccggtg agctcaagaa ggcactcata gaggttctgc agcccttgat 6120
   cgcagagcac caggcccggc gcaaggaggt cacggatgag atagtgaaag agttcatgac 6180
   tccccggaag ctgtccttcg actttcagtg aaagcttgcg gccgcactcg agcaccacca 6240
   ccaccaccac tgagatccgg ctgctaacaa agcccgaaag gaagctgagt tggctgctgc 6300
   caccgctgag caataactag cataacccct tggggcctct aaacgggtct tgaggggttt 6360
   tttgctgaaa ggaggaacta tatccggat                                   6389
<210> 71
   <211> 4742
   <212> DNA
   <213> Artificial Sequence
<220>
   <223> Description of Artificial sequence: Plasmid 02: pET20b+ with a NdeI/HindIII inst of Hu T2-WRS (SY variant), with 6-H Tag
<400> 71
   tggcgaatgg gacgcgccct gtagcggcgc attaagcgcg gcgggtgtgg tggttacgcg 60
   cagcgtgacc gctacacttg ccagcgccct agcgcccgct cctttcgctt tcttcccttc 120
   ctttctcgcc acgttcgccg gctttccccg tcaagctcta aatcgggggc tccctttagg 180
   gttccgattt agtgctttac ggcacctcga ccccaaaaaa cttgattagg gtgatggttc 240
   acgtagtggg ccatcgccct gatagacggt ttttcgccct ttgacgttgg agtccacgtt 300
   ctttaatagt ggactcttgt tccaaactgg aacaacactc aaccctatct cggtctattc 360
   ttttgattta taagggattt tgccgatttc ggcctattgg ttaaaaaatg agctgattta 420
   acaaaaattt aacgcgaatt ttaacaaaat attaacgttt acaatttcag gtggcacttt 480
   tcggggaaat gtgcgcggaa cccctatttg tttatttttc taaatacatt caaatatgta 540
   tccgctcatg agacaataac cctgataaat gcttcaataa tattgaaaaa ggaagagtat 600
   gagtattcaa catttccgtg tcgcccttat tccctttttt gcggcatttt gccttcctgt 660
   ttttgctcac ccagaaacgc tggtgaaagt aaaagatgct gaagatcagt tgggtgcacg 720
   agtgggttac atcgaactgg atctcaacag cggtaagatc cttgagagtt ttcgccccga 780
   agaacgtttt ccaatgatga gcacttttaa agttctgcta tgtggcgcgg tattatcccg 840
   tattgacgcc gggcaagagc aactcggtcg ccgcatacac tattctcaga atgacttggt 900
   tgagtactca ccagtcacag aaaagcatct tacggatggc atgacagtaa gagaattatg 960
   cagtgctgcc ataaccatga gtgataacac tgcggccaac ttacttctga caacgatcgg 1020
   aggaccgaag gagctaaccg cttttttgca caacatgggg gatcatgtaa ctcgccttga 1080
   tcgttgggaa ccggagctga atgaagccat accaaacgac gagcgtgaca ccacgatgcc 1140
   tgcagcaatg gcaacaacgt tgcgcaaact attaactggc gaactactta ctctagcttc 1200
   ccggcaacaa ttaatagact ggatggaggc ggataaagtt gcaggaccac ttctgcgctc 1260
   ggcccttccg gctggctggt ttattgctga taaatctgga gccggtgagc gtgggtctcg 1320
   cggtatcatt gcagcactgg ggccagatgg taagccctcc cgtatcgtag ttatctacac 1380
   gacggggagt caggcaacta tggatgaacg aaatagacag atcgctgaga taggtgcctc 1440
   actgattaag cattggtaac tgtcagacca agtttactca tatatacttt agattgattt 1500
   aaaacttcat ttttaattta aaaggatcta ggtgaagatc ctttttgata atctcatgac 1560
   caaaatccct taacgtgagt tttcgttcca ctgagcgtca gaccccgtag aaaagatcaa 1620
   aggatcttct tgagatcctt tttttctgcg cgtaatctgc tgcttgcaaa caaaaaaacc 1680
   accgctacca gcggtggttt gtttgccgga tcaagagcta ccaactcttt ttccgaaggt 1740
   aactggcttc agcagagcgc agataccaaa tactgtcctt ctagtgtagc cgtagttagg 1800
   ccaccacttc aagaactctg tagcaccgcc tacatacctc gctctgctaa tcctgttacc 1860
   agtggctgct gccagtggcg ataagtcgtg tcttaccggg ttggactcaa gacgatagtt 1920
   accggataag gcgcagcggt cgggctgaac ggggggttcg tgcacacagc ccagcttgga 1980
   gcgaacgacc tacaccgaac tgagatacct acagcgtgag ctatgagaaa gcgccacgct 2040
   tcccgaaggg agaaaggcgg acaggtatcc ggtaagcggc agggtcggaa caggagagcg 2100
   cacgagggag cttccagggg gaaacgcctg gtatctttat agtcctgtcg ggtttcgcca 2160
   cctctgactt gagcgtcgat ttttgtgatg ctcgtcaggg gggcggagcc tatggaaaaa 2220
   cgccagcaac gcggcctttt tacggttcct ggccttttgc tggccttttg ctcacatgtt 2280
   ctttcctgcg ttatcccctg attctgtgga taaccgtatt accgcctttg agtgagctga 2340
   taccgctcgc cgcagccgaa cgaccgagcg cagcgagtca gtgagcgagg aagcggaaga 2400
   gcgcctgatg cggtattttc tccttacgca tctgtgcggt atttcacacc gcatatatgg 2460
   tgcactctca gtacaatctg ctctgatgcc gcatagttaa gccagtatac actccgctat 2520
   cgctacgtga ctgggtcatg gctgcgcccc gacacccgcc aacacccgct gacgcgccct 2580
   gacgggcttg tctgctcccg gcatccgctt acagacaagc tgtgaccgtc tccgggagct 2640
   gcatgtgtca gaggttttca ccgtcatcac cgaaacgcgc gaggcagctg cggtaaagct 2700
   catcagcgtg gtcgtgaagc gattcacaga tgtctgcctg ttcatccgcg tccagctcgt 2760
   tgagtttctc cagaagcgtt aatgtctggc ttctgataaa gcgggccatg ttaagggcgg 2820
   ttttttcctg tttggtcact gatgcctccg tgtaaggggg atttctgttc atgggggtaa 2880
   tgataccgat gaaacgagag aggatgctca cgatacgggt tactgatgat gaacatgccc 2940
   ggttactgga acgttgtgag ggtaaacaac tggcggtatg gatgcggcgg gaccagagaa 3000
   aaatcactca gggtcaatgc cagcgcttcg ttaatacaga tgtaggtgtt ccacagggta 3060
   gccagcagca tcctgcgatg cagatccgga acataatggt gcagggcgct gacttccgcg 3120
   tttccagact ttacgaaaca cggaaaccga agaccattca tgttgttgct caggtcgcag 3180
   acgttttgca gcagcagtcg cttcacgttc gctcgcgtat cggtgattca ttctgctaac 3240
   cagtaaggca accccgccag cctagccggg tcctcaacga caggagcacg atcatgcgca 3300
   cccgtggcca ggacccaacg ctgcccgaga tctcgatccc gcgaaattaa tacgactcac 3360
   tatagggaga ccacaacggt ttccctctag aaataatttt gtttaacttt aagaaggaga 3420
   tatacatatg agtgcaaaag gcatagacta cgataagctc attgttcggt ttggaagtag 3480
   taaaattgac aaagagctaa taaaccgaat agagagagcc accggccaaa gaccacacca 3540
   cttcctgcgc agaggcatct tcttctcaca cagagatatg aatcaggttc ttgatgccta 3600
   tgaaaataag aagccatttt atctgtacac gggccggggc ccctcttctg aagcaatgca 3660
   tgtaggtcac ctcattccat ttattttcac aaagtggctc caggatgtat ttaacgtgcc 3720
   cttggtcatc cagatgacgg atgacgagaa gtatctgtgg aaggacctga ccctggacca 3780
   ggcctatagc tatgctgtgg agaatgccaa ggacatcatc gcctgtggct ttgacatcaa 3840
   caagactttc atattctctg acctggacta catggggatg agctcaggtt tctacaaaaa 3900
   tgtggtgaag attcaaaagc atgttacctt caaccaagtg aaaggcattt tcggcttcac 3960
   tgacagcgac tgcattggga agatcagttt tcctgccatc caggctgctc cctccttcag 4020
   caactcattc ccacagatct tccgagacag gacggatatc cagtgcctta tcccatgtgc 4080
   cattgaccag gatccttact ttagaatgac aagggacgtc gcccccagga tcggctatcc 4140
   taaaccagcc ctgttgcact ccaccttctt cccagccctg cagggcgccc agaccaaaat 4200
   gagtgccagc gaccccaact cctccatctt cctcaccgac acggccaagc agatcaaaac 4260
   caaggtcaat aagcatgcgt tttctggagg gagagacacc atcgaggagc acaggcagtt 4320
   tgggggcaac tgtgatgtgg acgtgtcttt catgtacctg accttcttcc tcgaggacga 4380
   cgacaagctc gagcagatca ggaaggatta caccagcgga gccatgctca ccggtgagct 4440
   caagaaggca ctcatagagg ttctgcagcc cttgatcgca gagcaccagg cccggcgcaa 4500
   ggaggtcacg gatgagatag tgaaagagtt catgactccc cggaagctgt ccttcgactt 4560
   tcagaagctt gcggccgcac tcgagcacca ccaccaccac cactgagatc cggctgctaa 4620
   caaagcccga aaggaagctg agttggctgc tgccaccgct gagcaataac tagcataacc 4680
   ccttggggcc tctaaacggg tcttgagggg ttttttgctg aaaggaggaa ctatatccgg 4740
   at 4742
<210> 72
   <211> 4769
   <212> DNA
   <213> Artificial sequence
<220>
   <223> Description of Artificial Sequence: Plasmid 04 pET20b+ with a NdeI/HindIII inSt of T2-WRS (SY variant), 6-H Tag w/Tombin cleavage Site
<400> 72
   tggcgaatgg gacgcgccct gtagcggcgc attaagcgcg gcgggtgtgg tggttacgcg 60
   cagcgtgacc gctacacttg ccagcgccct agcgcccgct cctttcgctt tcttcccttc 120
   ctttctcgcc acgttcgccg gctttccccg tcaagctcta aatcgggggc tccctttagg 180
   gttccgattt agtgctttac ggcacctcga ccccaaaaaa cttgattagg gtgatggttc 240
   acgtagtggg ccatcgccct gatagacggt ttttcgccct ttgacgttgg agtccacgtt 300
   ctttaatagt ggactcttgt tccaaactgg aacaacactc aaccctatct cggtctattc 360
   ttttgattta taagggattt tgccgatttc ggcctattgg ttaaaaaatg agctgattta 420
   acaaaaattt aacgcgaatt ttaacaaaat attaacgttt acaatttcag gtggcacttt 480
   tcggggaaat gtgcgcggaa cccctatttg tttatttttc taaatacatt caaatatgta 540
   tccgctcatg agacaataac cctgataaat gcttcaataa tattgaaaaa ggaagagtat 600
   gagtattcaa catttccgtg tcgcccttat tccctttttt gcggcatttt gccttcctgt 660
   ttttgctcac ccagaaacgc tggtgaaagt aaaagatgct gaagatcagt tgggtgcacg 720
   agtgggttac atcgaactgg atctcaacag cggtaagatc cttgagagtt ttcgccccga 780
   agaacgtttt ccaatgatga gcacttttaa agttctgcta tgtggcgcgg tattatcccg 840
   tattgacgcc gggcaagagc aactcggtcg ccgcatacac tattctcaga atgacttggt 900
   tgagtactca ccagtcacag aaaagcatct tacggatggc atgacagtaa gagaattatg 960
   cagtgctgcc ataaccatga gtgataacac tgcggccaac ttacttctga caacgatcgg 1020
   aggaccgaag gagctaaccg cttttttgca caacatgggg gatcatgtaa ctcgccttga 1080
   tcgttgggaa ccggagctga atgaagccat accaaacgac gagcgtgaca ccacgatgcc 1140
   tgcagcaatg gcaacaacgt tgcgcaaact attaactggc gaactactta ctctagcttc 1200
   ccggcaacaa ttaatagact ggatggaggc ggataaagtt gcaggaccac ttctgcgctc 1260
   ggcccttccg gctggctggt ttattgctga taaatctgga gccggtgagc gtgggtctcg 1320
   cggtatcatt gcagcactgg ggccagatgg taagccctcc cgtatcgtag ttatctacac 1380
   gacggggagt caggcaacta tggatgaacg aaatagacag atcgctgaga taggtgcctc 1440
   actgattaag cattggtaac tgtcagacca agtttactca tatatacttt agattgattt 1500
   aaaacttcat ttttaattta aaaggatcta ggtgaagatc ctttttgata atctcatgac 1560
   caaaatccct taacgtgagt tttcgttcca ctgagcgtca gaccccgtag aaaagatcaa 1620
   aggatcttct tgagatcctt tttttctgcg cgtaatctgc tgcttgcaaa caaaaaaacc 1680
   accgctacca gcggtggttt gtttgccgga tcaagagcta ccaactcttt ttccgaaggt 1740
   aactggcttc agcagagcgc agataccaaa tactgtcctt ctagtgtagc cgtagttagg 1800
   ccaccacttc aagaactctg tagcaccgcc tacatacctc gctctgctaa tcctgttacc 1860
   agtggctgct gccagtggcg ataagtcgtg tcttaccggg ttggactcaa gacgatagtt 1920
   accggataag gcgcagcggt cgggctgaac ggggggttcg tgcacacagc ccagcttgga 1980
   gcgaacgacc tacaccgaac tgagatacct acagcgtgag ctatgagaaa gcgccacgct 2040
   tcccgaaggg agaaaggcgg acaggtatcc ggtaagcggc agggtcggaa caggagagcg 2100
   cacgagggag cttccagggg gaaacgcctg gtatctttat agtcctgtcg ggtttcgcca 2160
   cctctgactt gagcgtcgat ttttgtgatg ctcgtcaggg gggcggagcc tatggaaaaa 2220
   cgccagcaac gcggcctttt tacggttcct ggccttttgc tggccttttg ctcacatgtt 2280
   ctttcctgcg ttatcccctg attctgtgga taaccgtatt accgcctttg agtgagctga 2340
   taccgctcgc cgcagccgaa cgaccgagcg cagcgagtca gtgagcgagg aagcggaaga 2400
   gcgcctgatg cggtattttc tccttacgca tctgtgcggt atttcacacc gcatatatgg 2460
   tgcactctca gtacaatctg ctctgatgcc gcatagttaa gccagtatac actccgctat 2520
   cgctacgtga ctgggtcatg gctgcgcccc gacacccgcc aacacccgct gacgcgccct 2580
   gacgggcttg tctgctcccg gcatccgctt acagacaagc tgtgaccgtc tccgggagct 2640
   gcatgtgtca gaggttttca ccgtcatcac cgaaacgcgc gaggcagctg cggtaaagct 2700
   catcagcgtg gtcgtgaagc gattcacaga tgtctgcctg ttcatccgcg tccagctcgt 2760
   tgagtttctc cagaagcgtt aatgtctggc ttctgataaa gcgggccatg ttaagggcgg 2820
   ttttttcctg tttggtcact gatgcctccg tgtaaggggg atttctgttc atgggggtaa 2880
   tgataccgat gaaacgagag aggatgctca cgatacgggt tactgatgat gaacatgccc 2940
   ggttactgga acgttgtgag ggtaaacaac tggcggtatg gatgcggcgg gaccagagaa 3000
   aaatcactca gggtcaatgc cagcgcttcg ttaatacaga tgtaggtgtt ccacagggta 3060
   gccagcagca tcctgcgatg cagatccgga acataatggt gcagggcgct gacttccgcg 3120
   tttccagact ttacgaaaca cggaaaccga agaccattca tgttgttgct caggtcgcag 3180
   acgttttgca gcagcagtcg cttcacgttc gctcgcgtat cggtgattca ttctgctaac 3240
   cagtaaggca accccgccag cctagccggg tcctcaacga caggagcacg atcatgcgca 3300
   cccgtggcca ggacccaacg ctgcccgaga tctcgatccc gcgaaattaa tacgactcac 3360
   tatagggaga ccacaacggt ttccctctag aaataatttt gtttaacttt aagaaggaga 3420
   tatacatatg agtgcaaaag gcatagacta cgataagctc attgttcggt ttggaagtag 3480
   taaaattgac aaagagctaa taaaccgaat agagagagcc accggccaaa gaccacacca 3540
   cttcctgcgc agaggcatct tcttctcaca cagagatatg aatcaggttc ttgatgccta 3600
   tgaaaataag aagccatttt atctgtacac gggccggggc ccctcttctg aagcaatgca 3660
   tgtaggtcac ctcattccat ttattttcac aaagtggctc caggatgtat ttaacgtgcc 3720
   cttggtcatc cagatgacgg atgacgagaa gtatctgtgg aaggacctga ccctggacca 3780
   ggcctatagc tatgctgtgg agaatgccaa ggacatcatc gcctgtggct ttgacatcaa 3840
   caagactttc atattctctg acctggacta catggggatg agctcaggtt tctacaaaaa 3900
   tgtggtgaag attcaaaagc atgttacctt caaccaagtg aaaggcattt tcggcttcac 3960
   tgacagcgac tgcattggga agatcagttt tcctgccatc caggctgctc cctccttcag 4020
   caactcattc ccacagatct tccgagacag gacggatatc cagtgcctta tcccatgtgc 4080
   cattgaccag gatccttact ttagaatgac aagggacgtc gcccccagga tcggctatcc 4140
   taaaccagcc ctgttgcact ccaccttctt cccagccctg cagggcgccc agaccaaaat 4200
   gagtgccagc gaccccaact cctccatctt cctcaccgac acggccaagc agatcaaaac 4260
   caaggtcaat aagcatgcgt tttctggagg gagagacacc atcgaggagc acaggcagtt 4320
   tgggggcaac tgtgatgtgg acgtgtcttt catgtacctg accttcttcc tcgaggacga 4380
   cgacaagctc gagcagatca ggaaggatta caccagcgga gccatgctca ccggtgagct 4440
   caagaaggca ctcatagagg ttctgcagcc cttgatcgca gagcaccagg cccggcgcaa 4500
   ggaggtcacg gatgagatag tgaaagagtt catgactccc cggaagctgt ccttcgactt 4560
   tcagtcttct ggtctggtgc cacgcggttc taagcttgcg gccgcactcg agcaccacca 4620
   ccaccaccac tgagatccgg ctgctaacaa agcccgaaag gaagctgagt tggctgctgc 4680
   caccgctgag caataactag cataacccct tggggcctct aaacgggtct tgaggggttt 4740
   tttgctgaaa ggaggaacta tatccggat 4769
<210> 73
   <211> 6326
   <212> DNA
   <213> Artificial sequence
<220>
   <223> Description of Artificial Sequence: Plasmid 06 pET24b+ with a NdeI/XoI inst of Hu mini-YRS, 6-H Tag
<400> 73
   tggcgaatgg gacgcgccct gtagcggcgc attaagcgcg gcgggtgtgg tggttacgcg 60
   cagcgtgacc gctacacttg ccagcgccct agcgcccgct cctttcgctt tcttcccttc 120
   ctttctcgcc acgttcgccg gctttccccg tcaagctcta aatcgggggc tccctttagg 180
   gttccgattt agtgctttac ggcacctcga ccccaaaaaa cttgattagg gtgatggttc 240
   acgtagtggg ccatcgccct gatagacggt ttttcgccct ttgacgttgg agtccacgtt 300
   ctttaatagt ggactcttgt tccaaactgg aacaacactc aaccctatct cggtctattc 360
   ttttgattta taagggattt tgccgatttc ggcctattgg ttaaaaaatg agctgattta 420
   acaaaaattt aacgcgaatt ttaacaaaat attaacgttt acaatttcag gtggcacttt 480
   tcggggaaat gtgcgcggaa cccctatttg tttatttttc taaatacatt caaatatgta 540
   tccgctcatg aattaattct tagaaaaact catcgagcat caaatgaaac tgcaatttat 600
   tcatatcagg attatcaata ccatattttt gaaaaagccg tttctgtaat gaaggagaaa 660
   actcaccgag gcagttccat aggatggcaa gatcctggta tcggtctgcg attccgactc 720
   gtccaacatc aatacaacct attaatttcc cctcgtcaaa aataaggtta tcaagtgaga 780
   aatcaccatg agtgacgact gaatccggtg agaatggcaa aagtttatgc atttctttcc 840
   agacttgttc aacaggccag ccattacgct cgtcatcaaa atcactcgca tcaaccaaac 900
   cgttattcat tcgtgattgc gcctgagcga gacgaaatac gcgatcgctg ttaaaaggac 960
   aattacaaac aggaatcgaa tgcaaccggc gcaggaacac tgccagcgca tcaacaatat 1020
   tttcacctga atcaggatat tcttctaata cctggaatgc tgttttcccg gggatcgcag 1080
   tggtgagtaa ccatgcatca tcaggagtac ggataaaatg cttgatggtc ggaagaggca 1140
   taaattccgt cagccagttt agtctgacca tctcatctgt aacatcattg gcaacgctac 1200
   ctttgccatg tttcagaaac aactctggcg catcgggctt cccatacaat cgatagattg 1260
   tcgcacctga ttgcccgaca ttatcgcgag cccatttata cccatataaa tcagcatcca 1320
   tgttggaatt taatcgcggc ctagagcaag acgtttcccg ttgaatatgg ctcataacac 1380
   cccttgtatt actgtttatg taagcagaca gttttattgt tcatgaccaa aatcccttaa 1440
   cgtgagtttt cgttccactg agcgtcagac cccgtagaaa agatcaaagg atcttcttga 1500
   gatccttttt ttctgcgcgt aatctgctgc ttgcaaacaa aaaaaccacc gctaccagcg 1560
   gtggtttgtt tgccggatca agagctacca actctttttc cgaaggtaac tggcttcagc 1620
   agagcgcaga taccaaatac tgtccttcta gtgtagccgt agttaggcca ccacttcaag 1680
   aactctgtag caccgcctac atacctcgct ctgctaatcc tgttaccagt ggctgctgcc 1740
   agtggcgata agtcgtgtct taccgggttg gactcaagac gatagttacc ggataaggcg 1800
   cagcggtcgg gctgaacggg gggttcgtgc acacagccca gcttggagcg aacgacctac 1860
   accgaactga gatacctaca gcgtgagcta tgagaaagcg ccacgcttcc cgaagggaga 1920
   aaggcggaca ggtatccggt aagcggcagg gtcggaacag gagagcgcac gagggagctt 1980
   ccagggggaa acgcctggta tctttatagt cctgtcgggt ttcgccacct ctgacttgag 2040
   cgtcgatttt tgtgatgctc gtcagggggg cggagcctat ggaaaaacgc cagcaacgcg 2100
   gcctttttac ggttcctggc cttttgctgg ccttttgctc acatgttctt tcctgcgtta 2160
   tcccctgatt ctgtggataa ccgtattacc gcctttgagt gagctgatac cgctcgccgc 2220
   agccgaacga ccgagcgcag cgagtcagtg agcgaggaag cggaagagcg cctgatgcgg 2280
   tattttctcc ttacgcatct gtgcggtatt tcacaccgca tatatggtgc actctcagta 2340
   caatctgctc tgatgccgca tagttaagcc agtatacact ccgctatcgc tacgtgactg 2400
   ggtcatggct gcgccccgac acccgccaac acccgctgac gcgccctgac gggcttgtct 2460
   gctcccggca tccgcttaca gacaagctgt gaccgtctcc gggagctgca tgtgtcagag 2520
   gttttcaccg tcatcaccga aacgcgcgag gcagctgcgg taaagctcat cagcgtggtc 2580
   gtgaagcgat tcacagatgt ctgcctgttc atccgcgtcc agctcgttga gtttctccag 2640
   aagcgttaat gtctggcttc tgataaagcg ggccatgtta agggcggttt tttcctgttt 2700
   ggtcactgat gcctccgtgt aagggggatt tctgttcatg ggggtaatga taccgatgaa 2760
   acgagagagg atgctcacga tacgggttac tgatgatgaa catgcccggt tactggaacg 2820
   ttgtgagggt aaacaactgg cggtatggat gcggcgggac cagagaaaaa tcactcaggg 2880
   tcaatgccag cgcttcgtta atacagatgt aggtgttcca cagggtagcc agcagcatcc 2940
   tgcgatgcag atccggaaca taatggtgca gggcgctgac ttccgcgttt ccagacttta 3000
   cgaaacacgg aaaccgaaga ccattcatgt tgttgctcag gtcgcagacg ttttgcagca 3060
   gcagtcgctt cacgttcgct cgcgtatcgg tgattcattc tgctaaccag taaggcaacc 3120
   ccgccagcct agccgggtcc tcaacgacag gagcacgatc atgcgcaccc gtggggccgc 3180
   catgccggcg ataatggcct gcttctcgcc gaaacgtttg gtggcgggac cagtgacgaa 3240
   ggcttgagcg agggcgtgca agattccgaa taccgcaagc gacaggccga tcatcgtcgc 3300
   gctccagcga aagcggtcct cgccgaaaat gacccagagc gctgccggca cctgtcctac 3360
   gagttgcatg ataaagaaga cagtcataag tgcggcgacg atagtcatgc cccgcgccca 3420
   ccggaaggag ctgactgggt tgaaggctct caagggcatc ggtcgagatc ccggtgccta 3480
   atgagtgagc taacttacat taattgcgtt gcgctcactg cccgctttcc agtcgggaaa 3540
   cctgtcgtgc cagctgcatt aatgaatcgg ccaacgcgcg gggagaggcg gtttgcgtat 3600
   tgggcgccag ggtggttttt cttttcacca gtgagacggg caacagctga ttgcccttca 3660
   ccgcctggcc ctgagagagt tgcagcaagc ggtccacgct ggtttgcccc agcaggcgaa 3720
   aatcctgttt gatggtggtt aacggcggga tataacatga gctgtcttcg gtatcgtcgt 3780
   atcccactac cgagatatcc gcaccaacgc gcagcccgga ctcggtaatg gcgcgcattg 3840
   cgcccagcgc catctgatcg ttggcaacca gcatcgcagt gggaacgatg ccctcattca 3900
   gcatttgcat ggtttgttga aaaccggaca tggcactcca gtcgccttcc cgttccgcta 3960
   tcggctgaat ttgattgcga gtgagatatt tatgccagcc agccagacgc agacgcgccg 4020
   agacagaact taatgggccc gctaacagcg cgatttgctg gtgacccaat gcgaccagat 4080
   gctccacgcc cagtcgcgta ccgtcttcat gggagaaaat aatactgttg atgggtgtct 4140
   ggtcagagac atcaagaaat aacgccggaa cattagtgca ggcagcttcc acagcaatgg 4200
   catcctggtc atccagcgga tagttaatga tcagcccact gacgcgttgc gcgagaagat 4260
   tgtgcaccgc cgctttacag gcttcgacgc cgcttcgttc taccatcgac accaccacgc 4320
   tggcacccag ttgatcggcg cgagatttaa tcgccgcgac aatttgcgac ggcgcgtgca 4380
   gggccagact ggaggtggca acgccaatca gcaacgactg tttgcccgcc agttgttgtg 4440
   ccacgcggtt gggaatgtaa ttcagctccg ccatcgccgc ttccactttt tcccgcgttt 4500
   tcgcagaaac gtggctggcc tggttcacca cgcgggaaac ggtctgataa gagacaccgg 4560
   catactctgc gacatcgtat aacgttactg gtttcacatt caccaccctg aattgactct 4620
   cttccgggcg ctatcatgcc ataccgcgaa aggttttgcg ccattcgatg gtgtccggga 4680
   tctcgacgct ctcccttatg cgactcctgc attaggaagc agcccagtag taggttgagg 4740
   ccgttgagca ccgccgccgc aaggaatggt gcatgcaagg agatggcgcc caacagtccc 4800
   ccggccacgg ggcctgccac catacccacg ccgaaacaag cgctcatgag cccgaagtgg 4860
   cgagcccgat cttccccatc ggtgatgtcg gcgatatagg cgccagcaac cgcacctgtg 4920
   gcgccggtga tgccggccac gatgcgtccg gcgtagagga tcgagatctc gatcccgcga 4980
   aattaatacg actcactata ggggaattgt gagcggataa caattcccct ctagaaataa 5040
   ttttgtttaa ctttaagaag gagatataca tatgggggac gctcccagcc ctgaagagaa 5100
   actgcacctt atcacccgga acctgcagga ggttctgggg gaagagaagc tgaaggagat 5160
   actgaaggag cgggaactta aaatttactg gggaacggca accacgggca aaccacatgt 5220
   ggcttacttt gtgcccatgt caaagattgc agacttctta aaggcagggt gtgaggtaac 5280
   aattctgttt gcggacctcc acgcatacct ggataacatg aaagccccat gggaacttct 5340
   agaactccga gtcagttact atgagaatgt gatcaaagca atgctggaga gcattggtgt 5400
   gcccttggag aagctcaagt tcatcaaagg cactgattac cagctcagca aagagtacac 5460
   actagatgtg tacagactct cctccgtggt cacacagcac gattccaaga aggctggagc 5520
   tgaggtggta aagcaggtgg agcacccttt gctgagtggc ctcttatacc ccggactgca 5580
   ggctttggat gaagagtatt taaaagtaga tgcccaattt ggaggcattg atcagagaaa 5640
   gattttcacc tttgcagaga agtacctccc tgcacttggc tattcaaaac gggtccatct 5700
   gatgaatcct atggttccag gattaacagg cagcaaaatg agctcttcag aagaggagtc 5760
   caagattgat ctccttgatc ggaaggagga tgtgaagaaa aaactgaaga aggccttctg 5820
   tgagccagga aatgtggaga acaatggggt tctgtccttc atcaagcatg tcctttttcc 5880
   ccttaagtcc gagtttgtga tcctacgaga tgagaaatgg ggtggaaaca aaacctacac 5940
   agcttacgtg gacctggaaa aggactttgc tgctgaggtt gtacatcctg gagacctgaa 6000
   gaattctgtt gaagtcgcac tgaacaagtt gctggatcca atccgggaaa agtttaatac 6060
   ccctgccctg aaaaaactgg ccagcgctgc ctacccagat ccctcaaagc agaagccaat 6120
   ggccaaaggc cctgccaaga attcagaacc agaggaggtc atcctcgagc accaccacca 6180
   ccaccactga gatccggctg ctaacaaagc ccgaaaggaa gctgagttgg ctgctgccac 6240
   cgctgagcaa taactagcat aaccccttgg ggcctctaaa cgggtcttga ggggtttttt 6300
   gctgaaagga ggaactatat ccggat 6326
<210> 74
   <211> 6563
   <212> DNA
   <213> Artificial Sequence
<220>
   <223> Description of Artificial Sequence: Plasmid 07 pET24b+ with a NdeI/HindIII inst of Hu mini-WRS, (SY variant) 6-H Tag
<400> 74
   tggcgaatgg gacgcgccct gtagcggcgc attaagcgcg gcgggtgtgg tggttacgcg 60
   cagcgtgacc gctacacttg ccagcgccct agcgcccgct cctttcgctt tcttcccttc 120
   ctttctcgcc acgttcgccg gctttccccg tcaagctcta aatcgggggc tccctttagg 180
   gttccgattt agtgctttac ggcacctcga ccccaaaaaa cttgattagg gtgatggttc 240
   acgtagtggg ccatcgccct gatagacggt ttttcgccct ttgacgttgg agtccacgtt 300
   ctttaatagt ggactcttgt tccaaactgg aacaacactc aaccctatct cggtctattc 360
   ttttgattta taagggattt tgccgatttc ggcctattgg ttaaaaaatg agctgattta 420
   acaaaaattt aacgcgaatt ttaacaaaat attaacgttt acaatttcag gtggcacttt 480
   tcggggaaat gtgcgcggaa cccctatttg tttatttttc taaatacatt caaatatgta 540
   tccgctcatg aattaattct tagaaaaact catcgagcat caaatgaaac tgcaatttat 600
   tcatatcagg attatcaata ccatattttt gaaaaagccg tttctgtaat gaaggagaaa 660
   actcaccgag gcagttccat aggatggcaa gatcctggta tcggtctgcg attccgactc 720
   gtccaacatc aatacaacct attaatttcc cctcgtcaaa aataaggtta tcaagtgaga 780
   aatcaccatg agtgacgact gaatccggtg agaatggcaa aagtttatgc atttctttcc 840
   agacttgttc aacaggccag ccattacgct cgtcatcaaa atcactcgca tcaaccaaac 900
   cgttattcat tcgtgattgc gcctgagcga gacgaaatac gcgatcgctg ttaaaaggac 960
   aattacaaac aggaatcgaa tgcaaccggc gcaggaacac tgccagcgca tcaacaatat 1020
   tttcacctga atcaggatat tcttctaata cctggaatgc tgttttcccg gggatcgcag 1080
   tggtgagtaa ccatgcatca tcaggagtac ggataaaatg cttgatggtc ggaagaggca 1140
   taaattccgt cagccagttt agtctgacca tctcatctgt aacatcattg gcaacgctac 1200
   ctttgccatg tttcagaaac aactctggcg catcgggctt cccatacaat cgatagattg 1260
   tcgcacctga ttgcccgaca ttatcgcgag cccatttata cccatataaa tcagcatcca 1320
   tgttggaatt taatcgcggc ctagagcaag acgtttcccg ttgaatatgg ctcataacac 1380
   cccttgtatt actgtttatg taagcagaca gttttattgt tcatgaccaa aatcccttaa 1440
   cgtgagtttt cgttccactg agcgtcagac cccgtagaaa agatcaaagg atcttcttga 1500
   gatccttttt ttctgcgcgt aatctgctgc ttgcaaacaa aaaaaccacc gctaccagcg 1560
   gtggtttgtt tgccggatca agagctacca actctttttc cgaaggtaac tggcttcagc 1620
   agagcgcaga taccaaatac tgtccttcta gtgtagccgt agttaggcca ccacttcaag 1680
   aactctgtag caccgcctac atacctcgct ctgctaatcc tgttaccagt ggctgctgcc 1740
   agtggcgata agtcgtgtct taccgggttg gactcaagac gatagttacc ggataaggcg 1800
   cagcggtcgg gctgaacggg gggttcgtgc acacagccca gcttggagcg aacgacctac 1860
   accgaactga gatacctaca gcgtgagcta tgagaaagcg ccacgcttcc cgaagggaga 1920
   aaggcggaca ggtatccggt aagcggcagg gtcggaacag gagagcgcac gagggagctt 1980
   ccagggggaa acgcctggta tctttatagt cctgtcgggt ttcgccacct ctgacttgag 2040
   cgtcgatttt tgtgatgctc gtcagggggg cggagcctat ggaaaaacgc cagcaacgcg 2100
   gcctttttac ggttcctggc cttttgctgg ccttttgctc acatgttctt tcctgcgtta 2160
   tcccctgatt ctgtggataa ccgtattacc gcctttgagt gagctgatac cgctcgccgc 2220
   agccgaacga ccgagcgcag cgagtcagtg agcgaggaag cggaagagcg cctgatgcgg 2280
   tattttctcc ttacgcatct gtgcggtatt tcacaccgca tatatggtgc actctcagta 2340
   caatctgctc tgatgccgca tagttaagcc agtatacact ccgctatcgc tacgtgactg 2400
   ggtcatggct gcgccccgac acccgccaac acccgctgac gcgccctgac gggcttgtct 2460
   gctcccggca tccgcttaca gacaagctgt gaccgtctcc gggagctgca tgtgtcagag 2520
   gttttcaccg tcatcaccga aacgcgcgag gcagctgcgg taaagctcat cagcgtggtc 2580
   gtgaagcgat tcacagatgt ctgcctgttc atccgcgtcc agctcgttga gtttctccag 2640
   aagcgttaat gtctggcttc tgataaagcg ggccatgtta agggcggttt tttcctgttt 2700
   ggtcactgat gcctccgtgt aagggggatt tctgttcatg ggggtaatga taccgatgaa 2760
   acgagagagg atgctcacga tacgggttac tgatgatgaa catgcccggt tactggaacg 2820
   ttgtgagggt aaacaactgg cggtatggat gcggcgggac cagagaaaaa tcactcaggg 2880
   tcaatgccag cgcttcgtta atacagatgt aggtgttcca cagggtagcc agcagcatcc 2940
   tgcgatgcag atccggaaca taatggtgca gggcgctgac ttccgcgttt ccagacttta 3000
   cgaaacacgg aaaccgaaga ccattcatgt tgttgctcag gtcgcagacg ttttgcagca 3060
   gcagtcgctt cacgttcgct cgcgtatcgg tgattcattc tgctaaccag taaggcaacc 3120
   ccgccagcct agccgggtcc tcaacgacag gagcacgatc atgcgcaccc gtggggccgc 3180
   catgccggcg ataatggcct gcttctcgcc gaaacgtttg gtggcgggac cagtgacgaa 3240
   ggcttgagcg agggcgtgca agattccgaa taccgcaagc gacaggccga tcatcgtcgc 3300
   gctccagcga aagcggtcct cgccgaaaat gacccagagc gctgccggca cctgtcctac 3360
   gagttgcatg ataaagaaga cagtcataag tgcggcgacg atagtcatgc cccgcgccca 3420
   ccggaaggag ctgactgggt tgaaggctct caagggcatc ggtcgagatc ccggtgccta 3480
   atgagtgagc taacttacat taattgcgtt gcgctcactg cccgctttcc agtcgggaaa 3540
   cctgtcgtgc cagctgcatt aatgaatcgg ccaacgcgcg gggagaggcg gtttgcgtat 3600
   tgggcgccag ggtggttttt cttttcacca gtgagacggg caacagctga ttgcccttca 3660
   ccgcctggcc ctgagagagt tgcagcaagc ggtccacgct ggtttgcccc agcaggcgaa 3720
   aatcctgttt gatggtggtt aacggcggga tataacatga gctgtcttcg gtatcgtcgt 3780
   atcccactac cgagatatcc gcaccaacgc gcagcccgga ctcggtaatg gcgcgcattg 3840
   cgcccagcgc catctgatcg ttggcaacca gcatcgcagt gggaacgatg ccctcattca 3900
   gcatttgcat ggtttgttga aaaccggaca tggcactcca gtcgccttcc cgttccgcta 3960
   tcggctgaat ttgattgcga gtgagatatt tatgccagcc agccagacgc agacgcgccg 4020
   agacagaact taatgggccc gctaacagcg cgatttgctg gtgacccaat gcgaccagat 4080
   gctccacgcc cagtcgcgta ccgtcttcat gggagaaaat aatactgttg atgggtgtct 4140
   ggtcagagac atcaagaaat aacgccggaa cattagtgca ggcagcttcc acagcaatgg 4200
   catcctggtc atccagcgga tagttaatga tcagcccact gacgcgttgc gcgagaagat 4260
   tgtgcaccgc cgctttacag gcttcgacgc cgcttcgttc taccatcgac accaccacgc 4320
   tggcacccag ttgatcggcg cgagatttaa tcgccgcgac aatttgcgac ggcgcgtgca 4380
   gggccagact ggaggtggca acgccaatca gcaacgactg tttgcccgcc agttgttgtg 4440
   ccacgcggtt gggaatgtaa ttcagctccg ccatcgccgc ttccactttt tcccgcgttt 4500
   tcgcagaaac gtggctggcc tggttcacca cgcgggaaac ggtctgataa gagacaccgg 4560
   catactctgc gacatcgtat aacgttactg gtttcacatt caccaccctg aattgactct 4620
   cttccgggcg ctatcatgcc ataccgcgaa aggttttgcg ccattcgatg gtgtccggga 4680
   tctcgacgct ctcccttatg cgactcctgc attaggaagc agcccagtag taggttgagg 4740
   ccgttgagca ccgccgccgc aaggaatggt gcatgcaagg agatggcgcc caacagtccc 4800
   ccggccacgg ggcctgccac catacccacg ccgaaacaag cgctcatgag cccgaagtgg 4860
   cgagcccgat cttccccatc ggtgatgtcg gcgatatagg cgccagcaac cgcacctgtg 4920
   gcgccggtga tgccggccac gatgcgtccg gcgtagagga tcgagatctc gatcccgcga 4980
   aattaatacg actcactata ggggaattgt gagcggataa caattcccct ctagaaataa 5040
   ttttgtttaa ctttaagaag gagatataca tatgagctac aaagctgccg cgggggagga 5100
   ttacaaggct gactgtcctc cagggaaccc agcacctacc agtaatcatg gcccagatgc 5160
   cacagaagct gaagaggatt ttgtggaccc atggacagta cagacaagca gtgcaaaagg 5220
   catagactac gataagctca ttgttcggtt tggaagtagt aaaattgaca aagagctaat 5280
   aaaccgaata gagagagcca ccggccaaag accacaccac ttcctgcgca gaggcatctt 5340
   cttctcacac agagatatga atcaggttct tgatgcctat gaaaataaga agccatttta 5400
   tctgtacacg ggccggggcc cctcttctga agcaatgcat gtaggtcacc tcattccatt 5460
   tattttcaca aagtggctcc aggatgtatt taacgtgccc ttggtcatcc agatgacgga 5520
   tgacgagaag tatctgtgga aggacctgac cctggaccag gcctatagct atgctgtgga 5580
   gaatgccaag gacatcatcg cctgtggctt tgacatcaac aagactttca tattctctga 5640
   cctggactac atggggatga gctcaggttt ctacaaaaat gtggtgaaga ttcaaaagca 5700
   tgttaccttc aaccaagtga aaggcatttt cggcttcact gacagcgact gcattgggaa 5760
   gatcagtttt cctgccatcc aggctgctcc ctccttcagc aactcattcc cacagatctt 5820
   ccgagacagg acggatatcc agtgccttat cccatgtgcc attgaccagg atccttactt 5880
   tagaatgaca agggacgtcg cccccaggat cggctatcct aaaccagccc tgttgcactc 5940
   caccttcttc ccagccctgc agggcgccca gaccaaaatg agtgccagcg accccaactc 6000
   ctccatcttc ctcaccgaca cggccaagca gatcaaaacc aaggtcaata agcatgcgtt 6060
   ttctggaggg agagacacca tcgaggagca caggcagttt gggggcaact gtgatgtgga 6120
   cgtgtctttc atgtacctga ccttcttcct cgaggacgac gacaagctcg agcagatcag 6180
   gaaggattac accagcggag ccatgctcac cggtgagctc aagaaggcac tcatagaggt 6240
   tctgcagccc ttgatcgcag agcaccaggc ccggcgcaag gaggtcacgg atgagatagt 6300
   gaaagagttc atgactcccc ggaagctgtc cttcgacttt cagaagcttg cggccgcact 6360
   cgagcaccac caccaccacc actgaaagct tgcggccgca ctcgagcacc accaccacca 6420
   ccactgagat ccggctgcta acaaagcccg aaaggaagct gagttggctg ctgccaccgc 6480
   tgagcaataa ctagcataac cccttggggc ctctaaacgg gtcttgaggg gttttttgct 6540
   gaaaggagga actatatccg gat                                         6563
<210> 75
   <211> 6329
   <212> DNA
   <213> Artificial Sequence
<220>
   <223> Description of Artificial sequence: Plasmid 09: pET24b+ with a NdeI/XhoI inst of Hu mini-YRS, No H Tag
<400> 75
   tggcgaatgg gacgcgccct gtagcggcgc attaagcgcg gcgggtgtgg tggttacgcg 60
   cagcgtgacc gctacacttg ccagcgccct agcgcccgct cctttcgctt tcttcccttc 120
   ctttctcgcc acgttcgccg gctttccccg tcaagctcta aatcgggggc tccctttagg 180
   gttccgattt agtgctttac ggcacctcga ccccaaaaaa cttgattagg gtgatggttc 240
   acgtagtggg ccatcgccct gatagacggt ttttcgccct ttgacgttgg agtccacgtt 300
   ctttaatagt ggactcttgt tccaaactgg aacaacactc aaccctatct cggtctattc 360
   ttttgattta taagggattt tgccgatttc ggcctattgg ttaaaaaatg agctgattta 420
   acaaaaattt aacgcgaatt ttaacaaaat attaacgttt acaatttcag gtggcacttt 480
   tcggggaaat gtgcgcggaa cccctatttg tttatttttc taaatacatt caaatatgta 540
   tccgctcatg aattaattct tagaaaaact catcgagcat caaatgaaac tgcaatttat 600
   tcatatcagg attatcaata ccatattttt gaaaaagccg tttctgtaat gaaggagaaa 660
   actcaccgag gcagttccat aggatggcaa gatcctggta tcggtctgcg attccgactc 720
   gtccaacatc aatacaacct attaatttcc cctcgtcaaa aataaggtta tcaagtgaga 780
   aatcaccatg agtgacgact gaatccggtg agaatggcaa aagtttatgc atttctttcc 840
   agacttgttc aacaggccag ccattacgct cgtcatcaaa atcactcgca tcaaccaaac 900
   cgttattcat tcgtgattgc gcctgagcga gacgaaatac gcgatcgctg ttaaaaggac 960
   aattacaaac aggaatcgaa tgcaaccggc gcaggaacac tgccagcgca tcaacaatat 1020
   tttcacctga atcaggatat tcttctaata cctggaatgc tgttttcccg gggatcgcag 1080
   tggtgagtaa ccatgcatca tcaggagtac ggataaaatg cttgatggtc ggaagaggca 1140
   taaattccgt cagccagttt agtctgacca tctcatctgt aacatcattg gcaacgctac 1200
   ctttgccatg tttcagaaac aactctggcg catcgggctt cccatacaat cgatagattg 1260
   tcgcacctga ttgcccgaca ttatcgcgag cccatttata cccatataaa tcagcatcca 1320
   tgttggaatt taatcgcggc ctagagcaag acgtttcccg ttgaatatgg ctcataacac 1380
   cccttgtatt actgtttatg taagcagaca gttttattgt tcatgaccaa aatcccttaa 1440
   cgtgagtttt cgttccactg agcgtcagac cccgtagaaa agatcaaagg atcttcttga 1500
   gatccttttt ttctgcgcgt aatctgctgc ttgcaaacaa aaaaaccacc gctaccagcg 1560
   gtggtttgtt tgccggatca agagctacca actctttttc cgaaggtaac tggcttcagc 1620
   agagcgcaga taccaaatac tgtccttcta gtgtagccgt agttaggcca ccacttcaag 1680
   aactctgtag caccgcctac atacctcgct ctgctaatcc tgttaccagt ggctgctgcc 1740
   agtggcgata agtcgtgtct taccgggttg gactcaagac gatagttacc ggataaggcg 1800
   cagcggtcgg gctgaacggg gggttcgtgc acacagccca gcttggagcg aacgacctac 1860
   accgaactga gatacctaca gcgtgagcta tgagaaagcg ccacgcttcc cgaagggaga 1920
   aaggcggaca ggtatccggt aagcggcagg gtcggaacag gagagcgcac gagggagctt 1980
   ccagggggaa acgcctggta tctttatagt cctgtcgggt ttcgccacct ctgacttgag 2040
   cgtcgatttt tgtgatgctc gtcagggggg cggagcctat ggaaaaacgc cagcaacgcg 2100
   gcctttttac ggttcctggc cttttgctgg ccttttgctc acatgttctt tcctgcgtta 2160
   tcccctgatt ctgtggataa ccgtattacc gcctttgagt gagctgatac cgctcgccgc 2220
   agccgaacga ccgagcgcag cgagtcagtg agcgaggaag cggaagagcg cctgatgcgg 2280
   tattttctcc ttacgcatct gtgcggtatt tcacaccgca tatatggtgc actctcagta 2340
   caatctgctc tgatgccgca tagttaagcc agtatacact ccgctatcgc tacgtgactg 2400
   ggtcatggct gcgccccgac acccgccaac acccgctgac gcgccctgac gggcttgtct 2460
   gctcccggca tccgcttaca gacaagctgt gaccgtctcc gggagctgca tgtgtcagag 2520
   gttttcaccg tcatcaccga aacgcgcgag gcagctgcgg taaagctcat cagcgtggtc 2580
   gtgaagcgat tcacagatgt ctgcctgttc atccgcgtcc agctcgttga gtttctccag 2640
   aagcgttaat gtctggcttc tgataaagcg ggccatgtta agggcggttt tttcctgttt 2700
   ggtcactgat gcctccgtgt aagggggatt tctgttcatg ggggtaatga taccgatgaa 2760
   acgagagagg atgctcacga tacgggttac tgatgatgaa catgcccggt tactggaacg 2820
   ttgtgagggt aaacaactgg cggtatggat gcggcgggac cagagaaaaa tcactcaggg 2880
   tcaatgccag cgcttcgtta atacagatgt aggtgttcca cagggtagcc agcagcatcc 2940
   tgcgatgcag atccggaaca taatggtgca gggcgctgac ttccgcgttt ccagacttta 3000
   cgaaacacgg aaaccgaaga ccattcatgt tgttgctcag gtcgcagacg ttttgcagca 3060
   gcagtcgctt cacgttcgct cgcgtatcgg tgattcattc tgctaaccag taaggcaacc 3120
   ccgccagcct agccgggtcc tcaacgacag gagcacgatc atgcgcaccc gtggggccgc 3180
   catgccggcg ataatggcct gcttctcgcc gaaacgtttg gtggcgggac cagtgacgaa 3240
   ggcttgagcg agggcgtgca agattccgaa taccgcaagc gacaggccga tcatcgtcgc 3300
   gctccagcga aagcggtcct cgccgaaaat gacccagagc gctgccggca cctgtcctac 3360
   gagttgcatg ataaagaaga cagtcataag tgcggcgacg atagtcatgc cccgcgccca 3420
   ccggaaggag ctgactgggt tgaaggctct caagggcatc ggtcgagatc ccggtgccta 3480
   atgagtgagc taacttacat taattgcgtt gcgctcactg cccgctttcc agtcgggaaa 3540
   cctgtcgtgc cagctgcatt aatgaatcgg ccaacgcgcg gggagaggcg gtttgcgtat 3600
   tgggcgccag ggtggttttt cttttcacca gtgagacggg caacagctga ttgcccttca 3660
   ccgcctggcc ctgagagagt tgcagcaagc ggtccacgct ggtttgcccc agcaggcgaa 3720
   aatcctgttt gatggtggtt aacggcggga tataacatga gctgtcttcg gtatcgtcgt 3780
   atcccactac cgagatatcc gcaccaacgc gcagcccgga ctcggtaatg gcgcgcattg 3840
   cgcccagcgc catctgatcg ttggcaacca gcatcgcagt gggaacgatg ccctcattca 3900
   gcatttgcat ggtttgttga aaaccggaca tggcactcca gtcgccttcc cgttccgcta 3960
   tcggctgaat ttgattgcga gtgagatatt tatgccagcc agccagacgc agacgcgccg 4020
   agacagaact taatgggccc gctaacagcg cgatttgctg gtgacccaat gcgaccagat 4080
   gctccacgcc cagtcgcgta ccgtcttcat gggagaaaat aatactgttg atgggtgtct 4140
   ggtcagagac atcaagaaat aacgccggaa cattagtgca ggcagcttcc acagcaatgg 4200
   catcctggtc atccagcgga tagttaatga tcagcccact gacgcgttgc gcgagaagat 4260
   tgtgcaccgc cgctttacag gcttcgacgc cgcttcgttc taccatcgac accaccacgc 4320
   tggcacccag ttgatcggcg cgagatttaa tcgccgcgac aatttgcgac ggcgcgtgca 4380
   gggccagact ggaggtggca acgccaatca gcaacgactg tttgcccgcc agttgttgtg 4440
   ccacgcggtt gggaatgtaa ttcagctccg ccatcgccgc ttccactttt tcccgcgttt 4500
   tcgcagaaac gtggctggcc tggttcacca cgcgggaaac ggtctgataa gagacaccgg 4560
   catactctgc gacatcgtat aacgttactg gtttcacatt caccaccctg aattgactct 4620
   cttccgggcg ctatcatgcc ataccgcgaa aggttttgcg ccattcgatg gtgtccggga 4680
   tctcgacgct ctcccttatg cgactcctgc attaggaagc agcccagtag taggttgagg 4740
   ccgttgagca ccgccgccgc aaggaatggt gcatgcaagg agatggcgcc caacagtccc 4800
   ccggccacgg ggcctgccac catacccacg ccgaaacaag cgctcatgag cccgaagtgg 4860
   cgagcccgat cttccccatc ggtgatgtcg gcgatatagg cgccagcaac cgcacctgtg 4920
   gcgccggtga tgccggccac gatgcgtccg gcgtagagga tcgagatctc gatcccgcga 4980
   aattaatacg actcactata ggggaattgt gagcggataa caattcccct ctagaaataa 5040
   ttttgtttaa ctttaagaag gagatataca tatgggggac gctcccagcc ctgaagagaa 5100
   actgcacctt atcacccgga acctgcagga ggttctgggg gaagagaagc tgaaggagat 5160
   actgaaggag cgggaactta aaatttactg gggaacggca accacgggca aaccacatgt 5220
   ggcttacttt gtgcccatgt caaagattgc agacttctta aaggcagggt gtgaggtaac 5280
   aattctgttt gcggacctcc acgcatacct ggataacatg aaagccccat gggaacttct 5340
   agaactccga gtcagttact atgagaatgt gatcaaagca atgctggaga gcattggtgt 5400
   gcccttggag aagctcaagt tcatcaaagg cactgattac cagctcagca aagagtacac 5460
   actagatgtg tacagactct cctccgtggt cacacagcac gattccaaga aggctggagc 5520
   tgaggtggta aagcaggtgg agcacccttt gctgagtggc ctcttatacc ccggactgca 5580
   ggctttggat gaagagtatt taaaagtaga tgcccaattt ggaggcattg atcagagaaa 5640
   gattttcacc tttgcagaga agtacctccc tgcacttggc tattcaaaac gggtccatct 5700
   gatgaatcct atggttccag gattaacagg cagcaaaatg agctcttcag aagaggagtc 5760
   caagattgat ctccttgatc ggaaggagga tgtgaagaaa aaactgaaga aggccttctg 5820
   tgagccagga aatgtggaga acaatggggt tctgtccttc atcaagcatg tcctttttcc 5880
   ccttaagtcc gagtttgtga tcctacgaga tgagaaatgg ggtggaaaca aaacctacac 5940
   agcttacgtg gacctggaaa aggactttgc tgctgaggtt gtacatcctg gagacctgaa 6000
   gaattctgtt gaagtcgcac tgaacaagtt gctggatcca atccgggaaa agtttaatac 6060
   ccctgccctg aaaaaactgg ccagcgctgc ctacccagat ccctcaaagc agaagccaat 6120
   ggccaaaggc cctgccaaga attcagaacc agaggaggtc atctgactcg agcaccacca 6180
   ccaccaccac tgagatccgg ctgctaacaa agcccgaaag gaagctgagt tggctgctgc 6240
   caccgctgag caataactag cataacccct tggggcctct aaacgggtct tgaggggttt 6300
   tttgctgaaa ggaggaacta tatccggat                                  6329